(19) Europäisches Patentamt
European Patent Office
Office européen des brevets

(11) EP 1 757 602 A1

(12) **EUROPEAN PATENT APPLICATION**
published in accordance with Art. 158(3) EPC

(43) Date of publication:
**28.02.2007 Bulletin 2007/09**

(21) Application number: **05751029.9**

(22) Date of filing: **14.06.2005**

(51) Int Cl.:
*C07D 413/14* (2006.01) *A61K 31/41* (2006.01)
*A61K 31/422* (2006.01) *A61K 31/423* (2006.01)
*A61K 31/427* (2006.01) *A61K 31/4439* (2006.01)
*A61K 31/454* (2006.01) *A61K 31/496* (2006.01)
*A61K 31/5377* (2006.01) *A61P 1/00* (2006.01)
*A61P 3/10* (2006.01) *A61P 11/02* (2006.01)
*A61P 11/06* (2006.01) *A61P 17/06* (2006.01)
*A61P 19/02* (2006.01) *A61P 25/00* (2006.01)
*A61P 29/00* (2006.01) *A61P 35/00* (2006.01)
*A61P 37/02* (2006.01) *A61P 37/06* (2006.01)
*A61P 43/00* (2006.01)

(86) International application number:
**PCT/JP2005/010894**

(87) International publication number:
**WO 2005/121135 (22.12.2005 Gazette 2005/51)**

(84) Designated Contracting States:
**AT BE BG CH CY CZ DE DK EE ES FI FR GB GR HU IE IS IT LI LT LU MC NL PL PT RO SE SI SK TR**

(30) Priority: **14.06.2004 JP 2004175094**

(71) Applicant: **DAIICHI PHARMACEUTICAL CO., LTD.**
**Chuo-ku,**
**Tokyo 103-8234 (JP)**

(72) Inventors:
- **YONEDA, Yoshiyuki,**
  **Daiichi Phamaceutical Co., Ltd.**
  **Edogawa-ku, Tokyo 134-8630 (JP)**
- **NAKAYAMA, Atsushi,**
  **Daiichi Phamaceutical Co., Ltd.**
  **Edogawa-ku, Tokyo 134-8630 (JP)**
- **MACHINAGA, Nobuo,**
  **Daiichi Phamaceutical Co., Ltd.**
  **Edogawa-ku, Tokyo 134-8630 (JP)**
- **CHIBA, Jun,**
  **Daiichi Phamaceutical Co., Ltd.**
  **Edogawa-ku, Tokyo 134-8630 (JP)**
- **MURO, Fumihito,**
  **Daiichi Phamaceutical Co., Ltd.**
  **Edogawa-ku, Tokyo 134-8630 (JP)**

(74) Representative: **Hartz, Nikolai et al**
**Wächtershauser & Hartz**
**Weinstrasse 8**
**80333 München (DE)**

(54) **VLA-4 INHIBITOR**

(57) An object of the present invention is to provide a compound which selectively inhibits binding of a ligand and α4β1 integrin (VLA-4), a process for producing the compound, and a medicament containing the compound.
A compound represented by the formula (I) etc. orasaltthereof, a process for producing the compound or a salt thereof, a medicament containing the compound or a salt thereof, as well as a preventive and/or a therapeutic agent for a disease caused by cell adhesion, for example, inflammatory reaction, autoimmune disease, cancer metastasis, bronchial asthma, nasal obstruction, diabetes, arthritis, psoriasis, multiple sclerosis, inflammatory bowel disease and rejection reaction at transplantation, containing the compound or a salt thereof as a primary component.



Printed by Jouve, 75001 PARIS (FR)

(Cont. next page)

[Chem. 1]

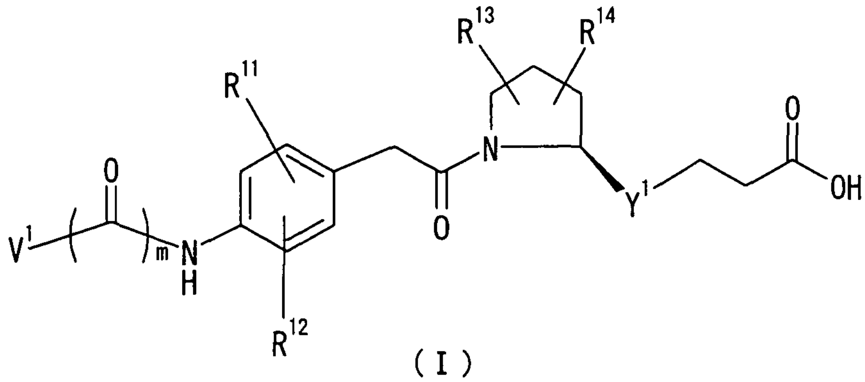

(I)

[wherein $Y^1$ represents a divalent aryl group etc. , $V^1$ represents an aryl group etc., and $R^{11}$ to $R^{14}$ represent H, OH or a halogen atom etc.]

## Description

Technical field

The present invention relates to a compound which selectively inhibits a ligand from combining with an adhesion receptor known as α4β1 integrin or VLA-4 (Very Late Antigen-4). This invention compound is useful in preventing and/or treating an inflammatory disease and an autoimmune disease, and pathologicalconditionscaused bycelladhesion associated with VLA-4 such as cancer metastasis.

Background art

The primary pathological characteristics of an inflammatory disease and an autoimmune disease lie in the accumulation of activated leukocyte into a damaged tissue (tissue affected with inflammation). A process by infiltration of leukocyte into an inflammatory site from the circulating system is divided into the following four phases of a cascade reaction, which synergically react with one another, i.e., tethering and rolling, activation, firm adhesion, and infiltration (Non-Patent Document 1). First, leukocytes subtly tether to the vessel endothelium, and then roll on the surface thereof. Second, activation of cells mediated by soluble chemotactic irritation is triggered after that. Third, the adhesion between individual leukocytes and vessel endothelial cells makes progress more firmly. Fourth, infiltration of leukocytes into vessel endothelial cells are induced owing to such firm adhesion. These phases take place one by one, and any phase is essential to the development of leukocyte infiltration.
While many receptors have been known so far, a receptor associated with leukocyte infiltration especially is characterized by belonging to a cell adhesion molecule family (Non-Patent Document 2). Initial tethering and rolling are mediated by an adhesion receptor called selectin. Firm adhesion is mediated by interaction between integrin on the suface of a leukocyte, and an immunoglobulin superfamily molecule expressed on the surface of a vessel endothelium. Both of integrin and an immunoglobulin-type adhesion molecule are mainly involved in infiltration of leukocytes. After infiltration, whether leukocytes passe through an extracellular matrix and stay in an inflammatory site depends on integrin.
Integrin is a large family consisting of the heterodimers of a glycoprotein in which two non-equal α and β-subunits are associated (Non-Patent Document 3). At least 16 different α-subunits (α1-α9, αL, αM, αD, αX, αE, αIIb, αV) and at least 9 different β(β1-β9) subunits are present. Integrin is divided into subfamilies based on a β-subunit. Leukocyte expresses many different integrins including α4β1, α5β1, α6β1, α4β7, αLβ2, αXβ2 and αVβ3.
α4β1 is also known as very late antigen-4 (VLA-4) or CD49d/CD29, and is expressed on monocyte, lymphocyte, eosinophil and basophil, and these are all modifying factors which are key to various inflammatory deficiencies (non-Patent Document 4). α4β1 integrin works as a receptor for vascular cell adhesion molecule-1 (VCAM-1), and fibronectin which is an extracellular protein functions as a ligand for integrin α4β1 like VCAM-1 (Non-Patent Document 5). Delay in the anti-inflammatory reaction and development of a disease has been demonstrated in an in vivo experiment using a monoclonal antibody which inhibits binding of α4β1 integrin and VCAM-1 (Non-Patent Document 6). In an inflammation model of lung using a guinea pig, an anti-α4 antibody inhibited both of hypersensitivity of an airway induced by an antigen and accumulation of leukocyte into an alveolar secrete in an airway (Non-Patent Document 7). A α4 antibody or VCAM-1 antibody inhibited infiltration of eosinophil into a mouse airway in an antigen-induced model (Non-Patent Document 8). In addition, development of a late cutaneous hypersensitive reaction in a mouse or a monkey was delayed or inhibited by treatment with a α4 antibody or VCAM-1 monoclonal antibody (Non-Patent Document 9, Non-Patent Document 10). Suppression of a graft vs host disease due to a specific immunosuppressing effect after bone marrow transplantation in a cardiac transplantation rejection reaction model in a mouse (Non-Patent Document 11), or experimental autoimmune encephalomyelitis in a rat or a mouse (Non-Patent Document 13, Non-Patent Document 14) by an anti-α4 antibody or an anti-VCAM-1 antibody was reported.
By Rational Drug Design, soluble VCAM-immunoglobulin (Ig) in which human two immunoglobulin (Ig)-like regions (domain 1 and domain 2) on a N-terminal domain are fused with a constant region of human IgG1 was made. When this fused protein was administered in an in vivo model using a nonobese diabetic mouse, spontaneous development was greatly delayed (significantly delays the onset of adoptively transferred autoimmune diabetes in nonobese mice, Non-Patent Document 15). As another approach, using a three-dimensional crystallographic structure of a VCAM-1 fragment, a cyclic peptide antagonist which structurally mimics a binding loop part of α4 integrin present on a domain1 of VCAM-1 was synthesized. CQIDSPC, which is a synthetic VCAM-1 peptide, could inhibit adhesion of a cell expressing VLA-4 to synthetic VCAM-1 (Non-Patent Document 16).
On the other hand, it is thought that, by inhibiting binding to a connecting segment-1 (CS-1) motif which is a connecting region with fibronectin being another ligand of integrin α4β1, the aforementioned disease is improved. A synthetic CS-1 polypeptide (phenylacetic acid-Leu-Asp-Phe-d isomer Pro-amide) inhibits VLA-4-mediatedleukocyte adhesion in vitro, and decreases promotion of a coronary disease in cardiac allografts of a rabbit (Non-Patent Document 17). The results of these study demonstrate that selective inhibition of adhesion mediated with α4β1/VCAM-1 is a solving

means for treating an autoimmune disease and an allergic inflammation disease.
On the other hand, many low-molecular VLA-4 inhibiting medicaments are reported (Non-Patent Document 18), but none of them has been clinically used as yet. The reasons lie in the problems of intracorporeal kinetics such as low oral absorbing property, low blood retention and the like, and a problem in a physical aspect such as low water-solubility.
An isoxazolepropionic acid derivative (isoxazolepropionic acid derivative represented by CP-665411, Non-Patent Document 19) which is a representative compound described in specifications of US Patent (Patent Document 1) and PCT Application (Patent Document 2) exhibits high in vivo VLA-4 inhibitory activity, but is low in oral absorbing property, and does not exhibit efficacy in an in vivo model in oral administration.
In addition, a representative compound described in the specification of PCT Application (Patent Document 3) has the problem that water-solubility is low, and it is necessary to separate and purify cis/trans isomers in a synthesis process of the compound.
Therefore, although these previous techniques have been disclosed, a low-molecular, non-peptidic selective VLA-4-dependent inhibitor which exhibits efficacy in oral administration, can be administered to a chronic inflammatory disease for a long term, and is suitable for treating a disease mediated with chemotaxis and adhesion of other leukocyte is desired.

[Patent Document 1] US 6,355,662
[Patent Document 2] WO 01/051487
[Patent Document 3] WO 02/053534
[Non-Patent Document 1] Springer, T., Ann. Rev. Physiol., 57:827(1995)
[Non-Patent Document 2] Carlos and Harlan, Blood, 82: 2068 (1994)
[Non-Patent Document 3] Heynes, R., Cell., 69: 11 (1992)
[Non-Patent Document 4] Helmer, M. Ann. Rev. Immunol., 8: 365 (1990)
[Non-Patent Document 5] Elices, et al., Cell., 60: 577 (1990)
[Non-Patent Document 6] Lobb et al., J. Clin., Invest., 94: 1722-28 (1994)
[Non-Patent Document 7] Pretolani et al., J. Exp. Med., 180: 795 (1994)
[Non-Patent Document 8] Nakajima et al., J. Exp. Med., 179: 1145 (1994)
[Non-Patent Document 9] Chisholm et al., Eur. J. Immunol., 179: 1145 (1994)
[Non-Patent Document 10] Silber et al., J. Clin., Invest., 93: 1554 (1993)
[Non-Patent Document 11] Isobe et al., J. Immunol., 153: 5810 (1994)
[Non-Patent Document 12] Yang et al., Proc. Natl. Acad. Sci. USA, 90; 10494 (1993)
[Non-Patent Document 13] Yednock et al., Nature., 356: 63 (1992)
[Non-Patent Document 14] Baron, et al., J. Exp. Med., 177: 57 (1993)
[Non-Patent Document 15] Jakubowski et al. , J. Immunol. , 155: 938 (1995)
[Non-Patent Document 16] Wang et al., Proc. Natl. Acad. Sci. USA, 92; 5714 (1995)
[Non-Patent Document 17] Molossi et al., J. Clin. Invest., 95; 2601 (1995)
[Non-Patent Document 18] Jefferson W. Tilley and Achyutharao Sidduri, Drugs of the Future, 26(1), 985-998 (2001)
[Non-Patent Document 19] E. Kudlucz et al. , J. Pharmacol. Exp. Ther., 301, 747-752 (2002)

Disclosure of the invention

Problems to be solved by the invention

An object of the present invention is to provide a medicament, which is a selective inhibitor of VLA-4, exhibits efficacy by oral administration, and can be administered for a long term.

Means to solve the problems

In view of such circumstances, the present inventors studied a low-molecular and non-peptidic compound. The present inventor found out that a compound represented by the following general formula (I) and a compound represented by the following general formula (II) selectively inhibit binding of a cell adhesion molecule to VLA-4, and is useful in preventing and/or treating various diseases mediated with chemotaxis and adhesion of leukocyte, which resulted in completion of the invention. That is, the present invention provides a compound represented by the following general formula (I):
[Chem.1]

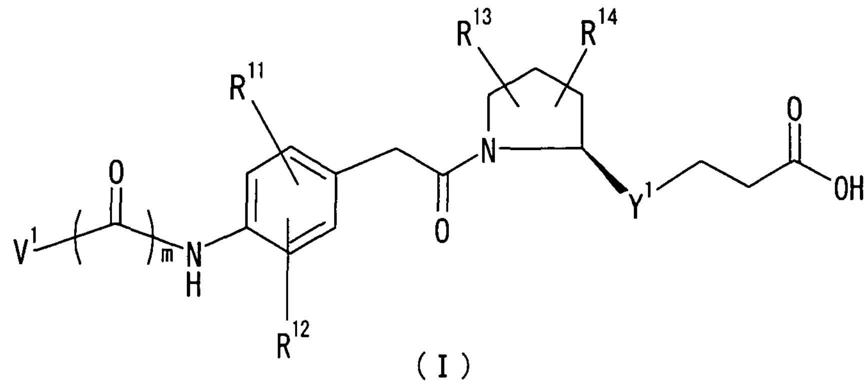

(I)

[wherein $Y^1$ represents a divalent aryl group optionally having a substituent or a divalent heteroaryl group optionally having a substituent, $V^1$ represents an aryl group optionally having a substituent or a heteroaryl group optionally having a substituent, $R^{11}$ and $R^{12}$ each independently represent a hydrogen atom, a hydroxy group, a halogen atom, a lower alkyl group, a lower alkoxy group, or an amino group optionally having a substituent, $R^{13}$ and $R^{14}$ each represent independently a hydrogen atom, a hydroxy group, a halogen atom, an amino group, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkylamino group optionally having a substituent, a dialkylamino group optionally having a substituent, a cyclic amino group optionally having a substituent, an alkylsulfonyl amino group optionally having a substituent, an arylsufonylamino group optionally having a substituent, a heteroaryloxy group optionally having a substituent or an aryloxy group optionally having a substituent, $R^{13}$ and $R^{14}$ may be taken together with a carbon atom constituting a pyrrolidine ring to which $R^{13}$ and $R^{14}$ are bound, to form a 3- to 7- membered cyclic hydrocarbon or a heterocycle (optionally having 1 to 3 substituents selected independently from a hydrxy group, a halogen atom, an amino group, an alkyl group, an alkoxy group, an aryl group, an aryloxy group, an alkyl amino group, a cyclic amino group, a benzyloxy group and a heteroaryl group on the ring), and m represents a number of 0 or 1] or a salt thereof, and a medicament containing this.

Also, the present invention provides a compound represented by the following general formula (II).

[Chem.2]

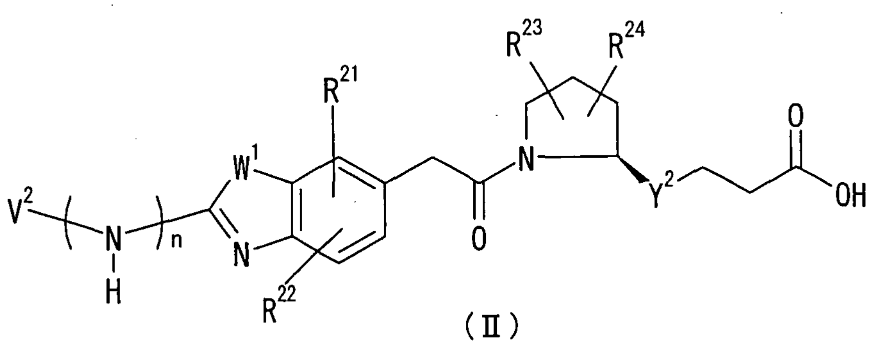

(II)

[wherein $Y^2$ represents a divalent aryl group optionally having a substituent, or a divalent heteroaryl group optionally having a substituent, $V^2$ represents an aryl group optionally having a substituent, or a heteroaryl group optionally having a substituent, $W^1$ represents an oxygen atom or a sulfur atom, $R^{21}$ and $R^{22}$ each independently represent a hydrogen atom, a hydroxy group, a halogen atom, a lower alkyl group, a lower alkoxy group, or an amino group optionally having a substituent, $R^{23}$ and $R^{24}$ each independently represent a hydrogen atom, a hydroxy group, an amino group, a halogen atom, an alkyl group optionally having a substituent, an aryl group optionally having a substituent, a heterocyclic group optionally having a substituent, an alkoxy group optionally having a substituent, an alkoxyalkyl group optionally

having a substituent, a cycloalkoxy group optionally having a substituent, a monoalkylamino group optionally having a substituent, a dialkylamino group optionally having a substituent, a cyclic amino group optionally having a substituent, an alkylsulfonyl amino group optionally having a substituent, an arylsulfonylamino group optionally having a substituent, a heteroaryloxy group optionally having a substituent, or an aryloxy group optionally having a substituent, $R^{23}$ and $R^{24}$ may be taken together with a carbon atom constituting a pyrrolidine ring to which $R^{23}$ and $R^{24}$ are bound, to form a 3- to 7- membered cyclic hydrocarbon or a heterocycle (optionally having 1 to 3 substituents selected independently from a hydroxy group, a halogen atom, an amino group, an alkyl group, an alkoxy group, an aryl group, an aryloxy group, an alkylamino group, a cyclic amino group, a benzyloxy group and a heteroaryl group on the ring), and n represents a number of 0 or 1]
or a salt thereof, and a medicament containing this.

Also, the present invention provides use of a compound represented by the formula (I) or (II) or a salt thereof for preparing a medicament. Also, the present invention provides a method of treating a disease resulting from cell adhesion, comprising administering a compound represented by the formula (I) or (II) or a salt thereof.

Effect of the invention

The VLA-4 inhibitor of the present invention is useful as a preventive and/or a therapeutic agent for various diseases mediated with chemotaxis and adhesion of leukocyte, for example, a disease such as an inflammatory reaction, and an autoimmune disease.

[Best mode for carrying out the invention]

The present compound is represented by the formula (I) or (II).
The aryl group in the formula (I) or (II) represents a monocyclic or fused-cyclic aromatic hydrocarbon group having a carbon number of 6 to 18, preferably 6 to 10, for example, a phenyl group and a naphthyl group.
The heteroaryl group represents a monovalent group formed of a monocyclic or fused-cyclic aromatic heterocycle in which the number of atoms constituting a ring is 5 to 18, and at least one of oxygen, nitrogen and sulfur atoms is an atom constituting a ring, preferably an aromatic heterocycle having an atom number of 5 to 14. Examples of the monocyclic aromatic heterocycle include pyrrole, thiophene, furan, imidazole, pyrazole, isoxazole, isothiazole, thiazole, oxazole, oxadiazole, 1,3,4-thiadiazole, triazole, tetrazole, pyrimidine, pyridine, pyrazine, pyridazine, and the like. Examples of a bicyclic aromatic heterocycle among the fused-cyclic aromatic heterocycle include indole, indolizine, isoindole, indazole, purine, 4H-quinolizine, isoquinoline, quinoline, phthalazine, naphthyridine, quinoxaline, quinazoline, cinnoline, benzopyran, benzothiophene, benzofuran, benzoxazole, benzothiazole, benzoisoxazole, benzoisothiazole, benzoimidazole, benzotriazole, 1H-pyrrolo[2,3-b]pyridine, 1H-pyrrolo[2,3-c]pyridine, 1H-pyrrolo[3,2-c]pyridine, 1H-pyrrolo[3,2-b]pyridine, 3H-pyrrolo[2,3-b]pyridine, 3H-pyrrolo[2,3-c]pyridine, 3H-pyrrolo[3,2-c]pyridine, 3H-pyrrolo[3,2-b]pyridine, dihydropyrrolo [2,3-c]pyridine, dihydropyrrolo[2,3-c]pyridine, dihydropyrrolo[3,2-c]pyridine, dihydropyrrolo[3,2-b]pyridine, oxazolo[4,5-b]pyridine, oxazolo[4,5-c]pyridine, oxazolo[5,4-c]pyridine, oxazolo[5,4-b]pyridine, thiazolo[4,5-b]pyridine, thiazolo[4,5-c]pyridine, thiazolo[5,4-c]pyridine, thiazolo[5,4-b]pyridine, pyrido[1,2-a]pyrimidine and the like. Examples of the tricyclic aromatic heterocycle include carbazole, carboline, dibenzofuran, dibenzothiophene and the like.
Examples of the halogen atom include a fluorine atom, a chlorine atom, a bromine atom and an iodine atom. The alkyl group represents a straight or branched saturated hydrocarbon group having a carbon number of 1 to 12, preferably 1 to 8, and examples include a methyl group, an ethyl group, a propyl group, an isopropyl group, a normal butyl group, an isobutyl group, a tert-butyl group, a pentyl group, a heptyl group and an octyl group.
The cycloalkyl group is a cyclic alkyl group having a carbon number of an integer of 3 to 10, preferably a cyclic alkyl group having a carbon number of an integer of 3 to 6, and examples include a cyclopropyl group, a cyclobutyl group, a cyclopentyl group, and a cyclohexyl group.
The lower alkyl group represents a straight or branched saturated hydrocarbon group having a carbon number of 1 to 8, preferably 1 to 6, and examples include a methyl group, an ethyl group, a propyl group, an isopropyl group, a normal butyl group, an isobutyl group, a tert-butyl group, a pentyl group and the like.
Examples of the alkoxy group include a methoxy group, an ethoxy group, a propoxy group, an isopropoxy group, a normal butoxy group, an isobutyloxy group, a tert-butoxy group, a pentyloxy group, a hexyloxy group, a heptyloxy group, and an octyloxy group.
Examples of the alkoxyalkyl group include methoxymethyl, ethoxymethyl, propoxymethyl, isopropoxymethyl, normalbutoxymethyl, methoxyethyl, ethoxyethyl, propoxyethyl, isopropoxyethyl, normalbutoxyethyl, methoxypropyl, ethoxypropyl, propoxypropyl, isopropoxypropyl, and normal butoxy groups.
The lower alkoxy group represents a straight or branched alkoxy group having a carbon number of 1 to 8, preferably 1 to 6, and examples include a methoxy group, an ethoxy group, a propoxy group, an isopropoxy group, a normal butoxy group, an isobutyloxy group, a tertiary butoxy group, a pentyloxy group, a hexyloxy group and the like.

Examples of the cycloalkoxy group include a cyclopropoxy group, a cyclobutoxy group, a cyclopentyloxy group, a cyclohexyloxy group, a cycloheptyloxy group, and a cyclooctyloxy group.

The heterocyclic group represents a monocyclic or fused-cyclic aliphatic or aromatic heterocyclic in which the number of atoms constituting a ring is 5 to 18, preferably 5 to 14, and at least one of oxygen, nitrogen, and sulfur atoms is an atom constituting a ring, the aromatic heterocyclic group is as defined for the heteroaryl group, and examples of the aliphatic heterocyclic group include groups formed of pyrrolidine, pyrroline, imidazolidine, imidazoline, indoline, tetrahydrofuran, dihydrofuran, tetrahydrothiophene, dihydrothiophene, thiazolidine, thiazoline, oxazoline, oxazolidine, isoxazolidine, isoxazoline , piperidine, morpholine, thiomorpholine, piperazine, tetrahydropyran, tetrahydroquinoline, tetrahydroisoquinoline, tetrahydroquinazoline and the like.

The monoalkyl amino group represents an amino group

in which a hydrogen atom of the amino group is substituted with one alkyl group, and examples include a monomethylamino group, a monoethylamino group, an isopropylamino group and the like.

The dialkylamino group represents an amino group in which hydrogen atoms of an amino group are substituted with two same or different alkyl groups, and examples include a dimethylamino group, a diethylamino group, a methylethylamino group, a methylpropylamino group and the like.

The cyclic amino group represents a cyclic amino group

in which the number of atoms constituting a ring is an integer of 3 to 10, preferably 4 to 7, and examples include an azetidinyl group, a pyrrolidinyl group, a piperidinyl group, a morpholinyl group, a piperazinyl group, a thiomorpholinyl group and a homopiperidinyl group.

Examples of the alkylsulfonyl group include a methylsulfonyl group, an ethylsulfonyl group, a propylsulfonyl group, an isopropylsulfonyl group, a normalbutylsulfonyl group, an isobutylsulfonyl group, a tert-butylsulfonyl group, a pentylsulfonyl group, a hexylsulfonyl group, a heptylsulfonyl group, and an octylsulfonyl group.

Examples of the alkylthio group include a methylthio group, an ethylthio group, a propylthio group, an isopropylthiogroup, anormalbutylthiogroup, anisobutylthio group, a tert-butylthio group, a pentylthio group, a hexylthio group, a heptylthio group, and an octylthio group.

The alkylsulfonyl amino group represents an amino group

in which a hydrogen atom of the amino group is substituted with an alkylsulfonyl group, and examples include a methanesulfonylamino group, and an ethanesulfonylamino group.

Examples of the arylsulfonyl group include a phenylsulfonyl group and a naphthylsulfonyl group.

The arylsulfonylamino group represents an amino group in which a hydrogen atom of the amino group is substituted with an arylsulfonyl group, and examples include a benzenesulfonylamino group and a naphthylsulfonylamino group.

Examples of the cycloalkyloxy group include a cyclopropoxy group, a cyclobutoxy group, a cyclopentyloxy group, a cyclohexyloxy group and the like.

Examples of the heteroaryloxy group include a pyridyloxy group, a pyrazinyloxy group and a pyridazinyloxy group.

Examples of the aryloxy group include a phenoxy group and a naphthyloxy group. The cyclic hydrocarbon group represents a saturated or unsaturated (including aromatic) monocyclic group constructed of 3 to 7 carbon atoms, and examples of the ring include cyclopropane, cyclobutane, cyclopentane, cyclopentene, cyclohexane, cyclohexene, cycloheptane, benzene and the like.

The alkylamino group represents an amino group in which hydrogen atoms of the amino group is substituted with alkyl, and examples include a mono or dialkylamino group, more specifically, amonomethylamino group, amonoethylamino group, an isopropylamino group, a dimethylamino group and a diethylamino group.

In addition, examples of a substituent of the alkyl amino group having a substituent include a N,O-dialkylhydroxylamino group in which a hydrogen atom of an alkylamino group is substituted with a lower alkoxy group. The N,O-dialkylhydroxylamino group represents a hydroxylamino group in which two hydrogen atoms of a hydroxylamino group are substituted with same or different alkyl groups, and examples include a N,O-dimethylhydroxylamino group and the like.

[Chem.3]

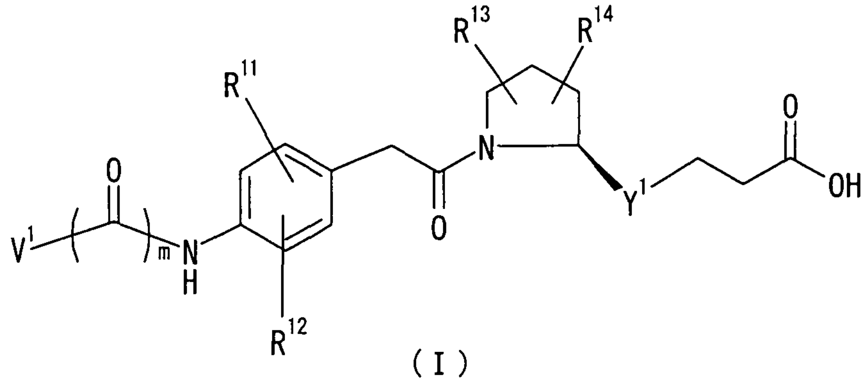

(I)

[Chem.4]

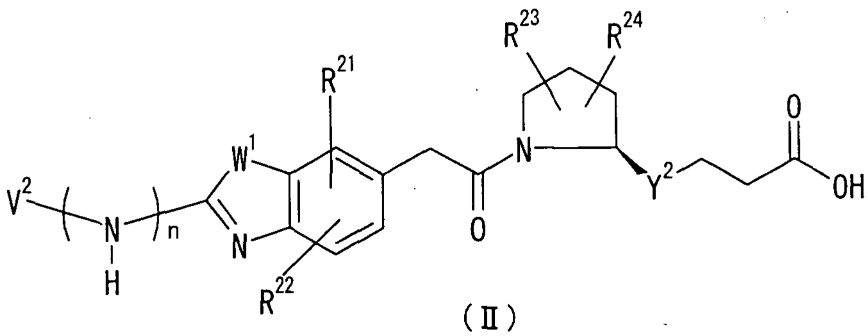

(II)

$R^{11}$, $R^{12}$, $R^{21}$ and $R^{22}$ in the formula (I) or (II) each independently represent a hydrogen atom, a hydroxy group, a halogen atom, a lower alkyl group, a lower alkoxy group, or an amino group optionally having a substituent, preferably a hydrogen atom or a halogen atom, particularly preferably a hydrogen atom, a fluorine atom or a chlorine atom. Examples of a group which can be substituted by the amino group include a lower alkyl group, a lower alkoxy group, a halogen atom, an amino group, a hydroxy group and the like, and one or a plurality of them may be substituted.

$R^{13}$ and $R^{14}$ in the formula (I) each independently represent a hydrogen atom, a hydroxy group, a halogen atom, an amino group, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkyl amino group optionally having a substituent, a dialkylamino group optionally having a substituent, a cyclic amino group optionally having a substituent, an alkylsulfonylamino group optionally having a substituent, an arylsulfonylamino group optionally having a substituent, a heteroaryloxy group optionally having a substituent or an aryloxy group optionally having a substituent, preferably a hydrogen atom, a halogen atom, an alkyl group optionally having a substituent, an alkoxy group optionally having a substituent, an alkoxyalkyl group optionally having a substituent, a cycloalkoxy group optionally having a substituent, a heteroaryloxy group optionally having a substituent, or a heterocyclic group optionally having a substituent, particularly preferably a hydrogen atom, a fluorine atom, a methyl group, an ethyl group, a methoxymethyl group, an ethoxymethyl group, a methoxy group, an ethoxy group, an isopropoxy group, a cyclobutoxy group, a benzyloxy group, a pyridyloxy group, a phenoxy group, a dimethylamino group, a 1-piperidinyl group, a 4-morpholinyl group, a cis-2,6-dimethyl-4-morpholinyl group, a 4-methyl-1-piperazinyl group, a 4-(2-fluoroethyl)-1-piperazinyl group, a 4-(2,2,2-trifluoroethyl)-1-piperazinyl group, a 4-thiomorpholinyl group, a 4-thiomorpholinyl-1,1-dioxide group, a N,O-dimethylhydroxylamino group or the like. Examples of a group which can be substituted by the alkyl group, the aryl group, the heterocyclic group, the alkoxyl group, the alkoxylalkyl group, the cycloalkoxy group, the monoalkylamino group, the dialkylamino group, the cyclic amino group, the alkylsulfonylamino group, the arylsulfonylamino group, the heteroaryloxy group, the aryloxy group, and the aryloxy group include a lower alkyl group such as a methyl group, an ethyl group, an isopropyl group

and the like, a lower alkoxy group such as a methoxy group, an ethoxy group, a propoxy group and the like, a halogen atom such as a fluorine atom, a chlorine atom and the like, an amino group, a hydroxy group and the like, and one or a plurality of them may be substituted. Further, $R^{13}$ and $R^{14}$ may form a 3- to 7- membered cyclic hydrocarbon or heterocycle (optionally having 1 to 3 substituents independently selected from a hydroxy group, a halogen atom, an amino group, an alkyl group, an alkoxy group, an aryl group, an aryloxy group, an alkylamino group, a cyclic amino group, a benzyloxy group and a heteroaryl group on the ring) and, as the cyclic carbon, or the heterocycle, a 6-membered cyclic hydrocarbon or a 6-membered heterocycle is preferable, respectively, and a 6-membered cyclic hydrocarbon such as a cyclohexane ring is particularly preferable.

$R^{23}$ and $R^{24}$ in the formula (II) represent a hydrogen atom, a hydroxy group, a halogen atom, an amino group, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkylamino group optionally having a substituent, a dialkylamino group optionally having a substituent, a cyclic amino group optionally having a substituent, an alkylsulfonylamino group optionally having a substituent, an arylsulfonyl amino group optionally having a substituent, a heteroaryloxy group optionally having a substituent, or an aryloxy group optionally having a substituent, preferably a hydrogen atom, a halogen atom, an alkyl group optionally having a substituent, an alkoxy group optionally having a substituent, an alkoxyalkyl group optionally having a substituent, a cycloalkoxy group optionally having a substituent, a heteroaryloxy group optionally having a substituent, or a heterocyclic group optionally having a substituent, particularly preferably a hydrogen atom, a fluorine atom, a methyl group, an ethyl group, a methoxymethyl group, an ethoxymethyl group, a methoxy group, an ethoxy group, an isopropoxy group, a cyclobutoxy group, a benzyloxy group, a pyridyloxy group, a phenoxy group, a dimethylamino group, a 1-piperidinyl group, a 4-morpholinyl group, a cis-2,6-dimethyl-4-morpholinyl group, a 4-methyl-1-piperazinyl group, a 4-(2-fluoroethyl)-1-piperazinyl group, a 4-(2,2,2-trifluoroethyl)-1-piperazinyl group, a 4-thiomorpholinyl group, a 4-thiomorpholinyl-1,1-dioxide group, a N,O-dimethylhydroxylamino group or the like. Examples of a group which can be substituted by the alkyl group, the aryl group, the heterocyclic group, the alkoxy group, the alkoxyalkyl group, the cycloalkoxy group, the monoalkylamino group, the dialkylamino group, the cyclic amino group, the alkylsulfonylamino group, the arylsulfonylamino group, the heteroaryloxy group, and the aryloxy group include a lower alkyl group such as a methyl group, an ethyl group, an isopropyl group and the like, a lower alkoxy group such as a methoxy group, an ethoxy group, a propoxy group and the like, a halogen atom such as a fluorine atom, a chlorine atom, and the like, an amino group, a hydroxy group and the like, and one or a plurality of them may be substituted. Further, $R^{23}$ and $R^{24}$ may form a 3- to 7-membered cyclic hydrocarbon or a heterocyclic group (optionally having 1 to 3 substituents independently selected from a hydroxy group, a halogen atom, an amino group, an alkyl group, an alkoxy group, an aryl group, an aryloxy group, an alkyl amino group, a cyclic amino group, a benzyloxy group and a heteroaryl group on the ring) and, as the cyclic hydrocarbon or the heterocycle, a 6-membered cyclic hydrocarbon or a 6-membered heterocycle is preferable, respectively, and a 6-membered cyclic hydrocarbon such as a cyclohexane ring and the like is particularly preferable.

Examples of $V^1$ in the formula (I) and $V^2$ in the formula (II) are shown below.

[Chem.5]

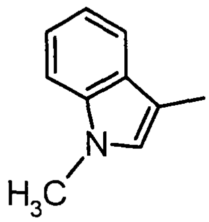
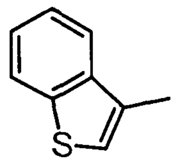
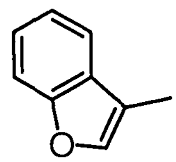
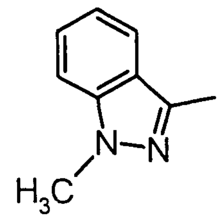
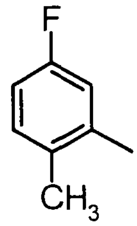
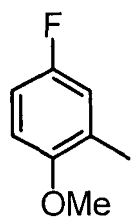
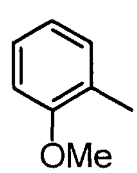
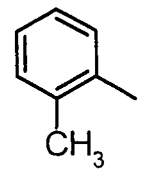
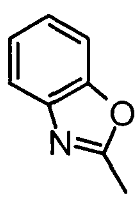
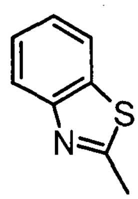
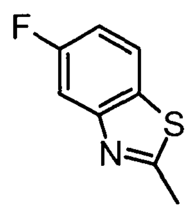
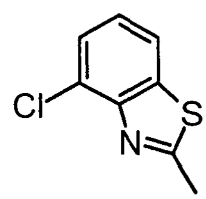

Wherein, $V^1$ and $V^2$ each independently represent an aryl group optionally having a substituent, or a heteroaryl group optionally having a substituent. The aryl group referred herein is as defined above, and is preferably a phenyl group or a naphthyl group, particularly preferably a phenyl group.

The heteroaryl group referred herein is as defined above, a monovalent group formed of indole, isoindole, indazole, benzothiophene, benzofuran, thiazole, pyrimidine, pyrrole, isoquinoline, quinoline, benzoisothiazole, benzoxazole, benzofuran or benzothiazole is preferable, and a monovalent group formed of indole, benzothiophene, benzofuran, benzoxazole, benzothiazole or indazole is particularly preferable. Examples of a group which can be substituted by the aryl group and the heteroaryl group include a lower alkyl group such as a methyl group and the like, a lower alkoxy group such as a methoxy group and the like, a halogen atom, an amino group, a hydroxy group and the like, and one or a plurality of them may be substituted.

$V^1$ in the formula (I) is preferably any one of the following formulas (i-a) to (i-e), particularly preferably (i-a) or (i-e), most preferably (i-a).

[Chem.6]

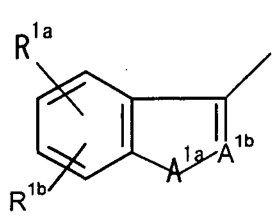

(i-a)

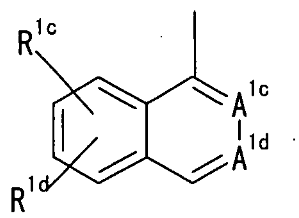

(i-b)

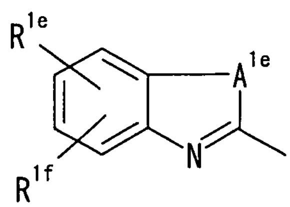

(i-c)

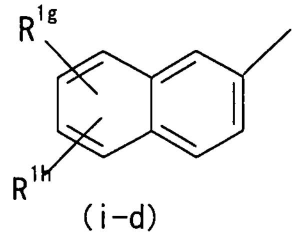

(i-d)

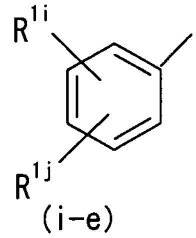

(i-e)

In the formula (i-a), $A^{1a}$ represents an oxygen atom, a sulfur atom or N-$R^{1k}$ (wherein $R^{1k}$ represents a hydrogen atom or a lower alkyl group), preferably a sulfur atom or N-$R^{1k}$. $R^{1k}$ is preferably a hydrogen atom or a lower alkyl group, particularly preferably a hydrogen atom or a methyl group.

In addition, $A^{1b}$ represents a nitrogen atom or C-$R^{1m}$ (wherein $R^{1m}$ represents a hydrogen atom or a lower alkyl group), preferably C-$R^{1m}$. $R^{1m}$ is preferably a hydrogen atom.

In the formula (i-b), $A^{1c}$ represents a nitrogen atom or C-$R^{1n}$ (wherein $R^{1n}$ represents a hydrogen atom or a lower alkyl group), preferably C-$R^{1n}$. $R^{1n}$ is preferably a hydrogen atom. In addition, $A^{1d}$ represents a nitrogen atom or C-$R^{1o}$ (wherein $R^{1o}$ represents a hydrogen atom or a lower alkyl group), preferably C-$R^{1o}$. $R^{1o}$ is preferably a hydrogen atom.

In the formula (i-c), $A^{1e}$ represents an oxygen atom, a sulfur atom or N-$R^{1p}$ (wherein $R^{1p}$ represents a hydrogen atom or a lower alkyl group), preferably N-$R^{1p}$. $R^{1p}$ is preferably a hydrogen atom.

In the formula (i-a), (i-b), (i-c), (i-d) or (i-e), $R^{1a}$, $R^{1b}$, $R^{1c}$, $R^{1d}$, $R^{1e}$, $R^{1f}$, $R^{1g}$, $R^{1h}$, $R^{1i}$ and $R^{1j}$ each independently represent a hydrogen atom, a hydroxy group, an amino group, a halogen atom, an alkyl group optionally having a substituent, an alkoxy group optionally having a substituent, a monoalkylamino group optionally having a substituent, a dialkylamino group optionally having a substituent, an alkylsulfonylamino group optionally having a substituent, an alkylthio group optionally having a substituent, an alkylsulfonyl group optionally having a substituent, preferably a hydrogen atom, a halogen atom, an alkyl group optionally having a substituent, or an alkoxy group optionally having a substituent, particularly preferably a hydrogen atom, a halogen atom, a lower alkyl group such as a methyl group and the like, or a lower alkoxy group such as a methoxy group and the like. Examples of a group which can be substituted by the alkyl group, the alkoxy group, a monoalkylamino group, the dialkylamino group, the alkylsulfonylamino group, the alkylthio group and the alkylsulfonyl group include a lower alkyl group, a lower alkoxy group, a halogen atom, an amino group, a hydroxy group and the like, and one or a plurality of them may be substituted.

$V^2$ in the formula (II) is preferably any one of (ii-a) to (ii-e), particularly preferably (ii-a).

[Chem.7]

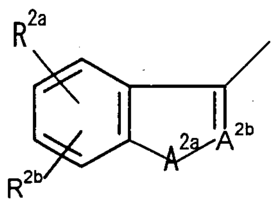

(ii-a)

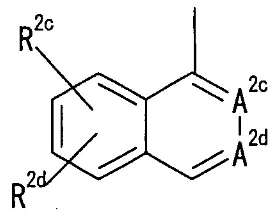

(ii-b)

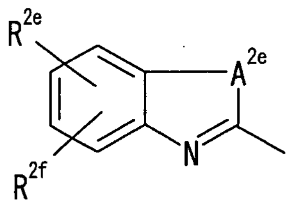

(ii-c)

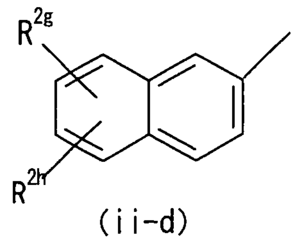

(ii-d)

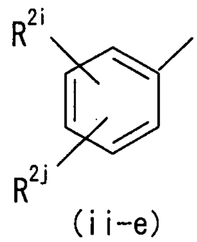

(ii-e)

In the formula (ii-a), $A^{2a}$ represents an oxygen atom, a sulfur atom or N-$R^{2k}$ (wherein $R^{2k}$ represents a hydrogen atom or a lower alkyl group), preferably a sulfur atom or N-$R^{2k}$. $R^{2k}$ is preferably a hydrogen atom or a lower alkyl group, particularly preferably, a hydrogen atom or a methyl group.

In addition, $A^{2b}$ represents a nitrogen atom or C-$R^{2m}$ (wherein $R^{2m}$ represents a hydrogen atom or a lower alkyl group), preferably C-$R^{2m}$. $R^{2m}$ is preferably a hydrogen atom.

In the formula (ii-b), $A^{2c}$ represents a nitrogen atom or C-$R^{2n}$ (wherein $R^{2n}$ represents a hydrogen atom or a lower alkyl group), preferably C-$R^{2n}$. $R^{2n}$ is preferably a hydrogen atom.

In addition, $A^{2d}$ represents a nitrogen atom or C-$R^{2o}$ (wherein $R^{2o}$ represents a hydrogen atom or a lower alkyl group), preferably C-$R^{2o}$. $R^{2o}$ is preferably a hydrogen atom.

In the formula (ii-c), $A^{2e}$ represents an oxygen atom, a sulfur atom or N-$R^{2p}$ (wherein $R^{2p}$ represents a hydrogen atom or a lower alkyl group), preferably an oxygen atom. $R^{2p}$ is preferably a hydrogen atom.

In the formula (ii-a), (ii-b), (ii-c), (ii-d) or (ii-e), $R^{2a}$, $R^{2b}$, $R^{2c}$, $R^{2d}$, $R^{2e}$, $R^{2f}$, $R^{2g}$, $R^{2h}$, $R^{2i}$ and $R^{2j}$ each independently represent a hydrogen atom, a hydroxy group, an amino group, a halogen atom, an alkyl group optionally having a substituent, an alkoxy group optionally having a substituent, a monalkylamino group optionally having a substituent, a dialkylamino group optionally having a substituent, an alkylsulfonylamino group optionally having a substituent, an alkylthio group optionally having a substituent, or an alkylsulfonyl group optionally having a substituent, preferably a hydrogen atom, a halogen atom, an alkyl group optionally having a substituent, or an alkoxy group optionally having a substituent, particularly preferably a hydrogen atom, a halogen atom, a lower alkyl group such as a methyl group and the like, or a lower alkoxy group such as a methoxy group and the like. Examples of a group which can be substituted by the alkyl group, the alkoxy group, the monalkylamino group, the dialkylamino group, the alkylsulfonylamino group, the alkylthio group and the alkylsulfonyl group include a lower alkyl group, a lower alkoxy group, a halogen atom, an amino group, a hydroxy group and the like.

$Y^1$ in the formula (I) and $Y^2$ in the formula (II) each independently represent a divalent aryl group optionally having a substituent, or a divalent heteroaryl group optionally having a substituent, the divalent aryl group referred herein is as defined above, and is preferably a phenylene group or a naphthylene group.

The divalent heteroaryl group referred herein is as defined above, and is preferably a monovalent group formed of pyrrole, imidazole, pyrazole, isothioazole, isoxazole, pyridine, pyrazine, pyrimidine, pyridazine, thiophene, furan, oxazole, tetrazole or thiazole, particularly preferably a monovalent group formed of thiazole, isothiazole, oxazole, tetrazole or isoxazole, most preferably a monovalent group formed of thiazole. In addition, examples of a group which can be substituted by the aryl group and the heteroaryl group include a lower alkyl group such as a methyl group and the like, a lower alkoxy group such as a methoxy group and the like, and a halogen atom such as a fluorine atom and the like.

$Y^1$ in the formula (I) is preferably any one of the following formulas (vii-a) to (vii-f), particularly preferably (vii-a), (vii-b), (vii-c) or (vii-d), most preferably (vii-a).

[Chem.8]

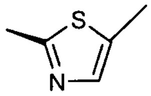
(vii-a)

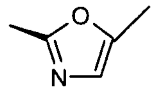
(vii-b)

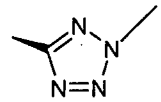
(vii-c)

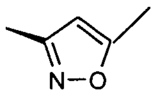
(vii-d)

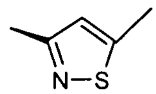
(vii-e)

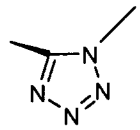
(vii-f)

Herein, a left bond represents that $Y^1$ is bound to a carbon atom of pyrrolidine, and a right bond represents that $Y^1$ is bound to a carbon atom of propionic acid.
In the formula (II), $Y^2$ is preferably any one of the following formulas (viii-a) to (viii-f), particularly preferably (viii-a).
[Chem.9]

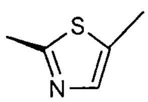
(viii-a)

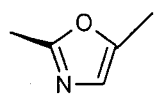
(viii-b)

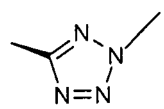
(viii-c)

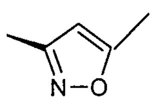
(viii-d)

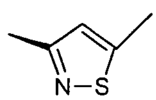
(viii-e)

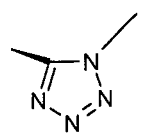
(viii-f)

Herein, a left bond represents that $Y^2$ is bound to a carbon atom of pyrrolidine, and a right bond represents that $Y^2$ is bound to a carbon atom of propionic acid.
Among the compounds represented by the formula (I), a compound in which $V^1$ in the formula (I) is the (i-a), and $Y^1$ in the formula (I) is the (vii-a) is more preferable.
Further, among the present compound, a compound represented by any one of the following formulas (18-1) to (18-133) ispreferable, compounds represented by the following formulas (18-1), (18-36), (18-46) and (18-47) are particularly preferable, and a compound represented the following formula (18-1) is most preferable.
[Chem.10]

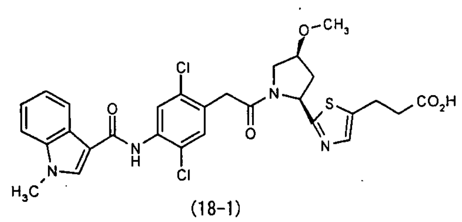

(18-1)

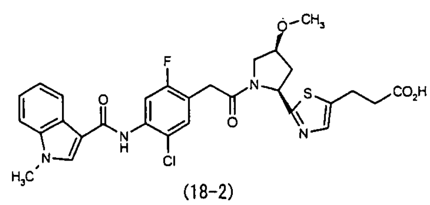

(18-2)

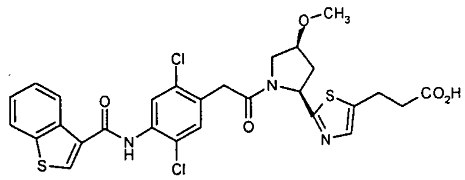

(18-3)

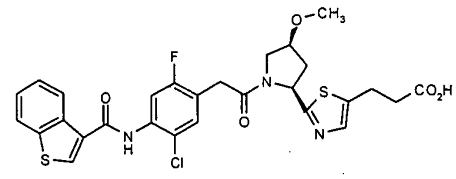

(18-4)

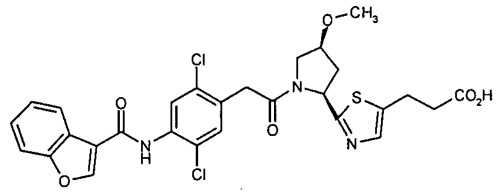

(18-5)

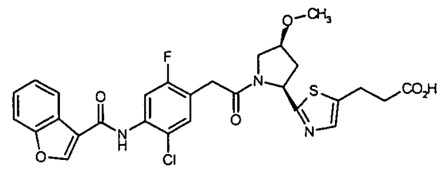

(18-6)

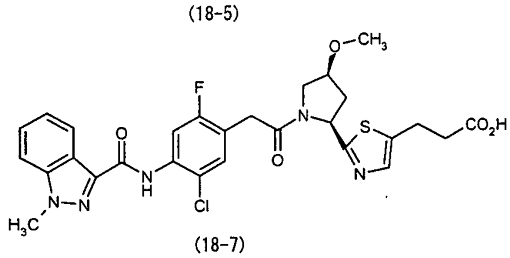

(18-7)

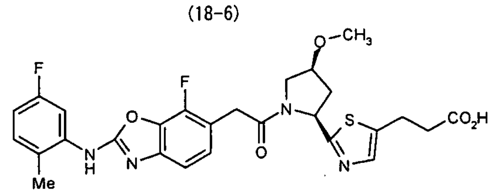

(18-8)

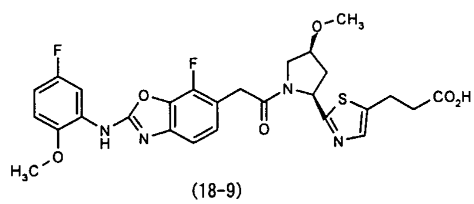

(18-9)

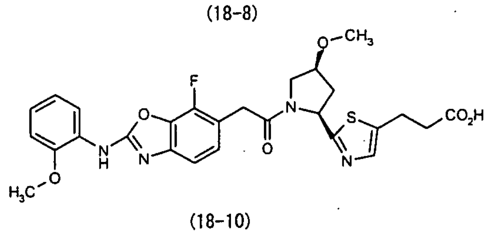

(18-10)

[Chem.11]

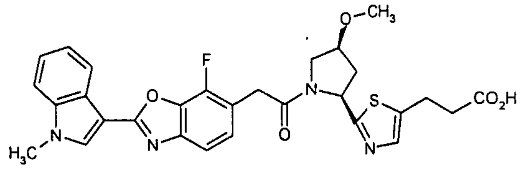

(18-11)

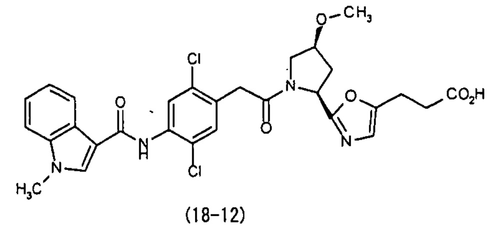

(18-12)

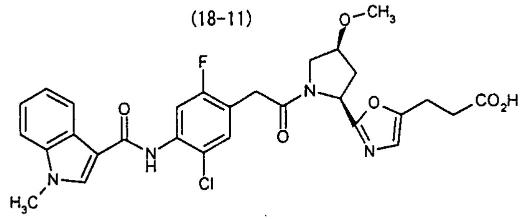

(18-13)

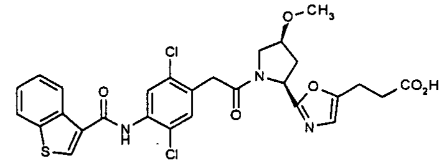

(18-14)

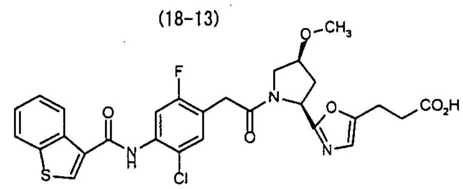

(18-15)

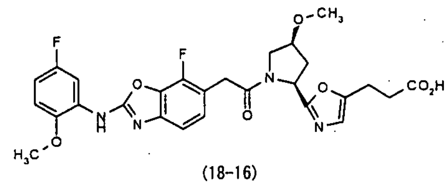

(18-16)

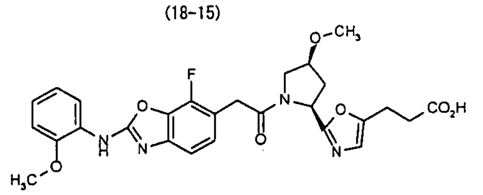

(18-17)

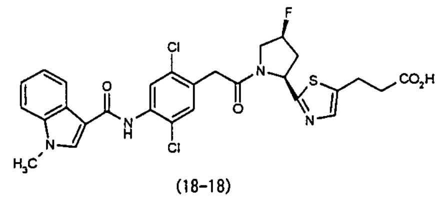

(18-18)

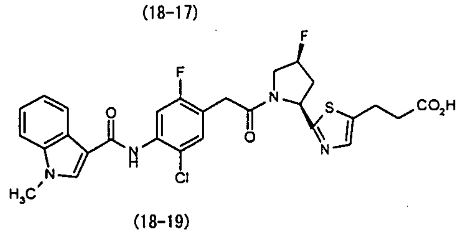

(18-19)

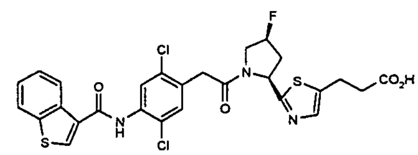

(18-20)

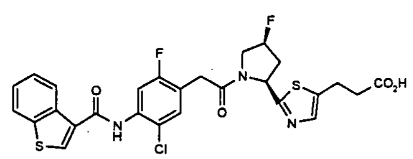

(18-21)

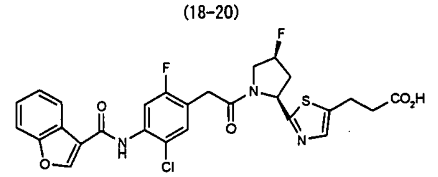

(18-22)

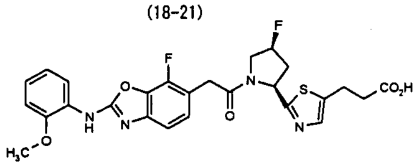

(18-23)

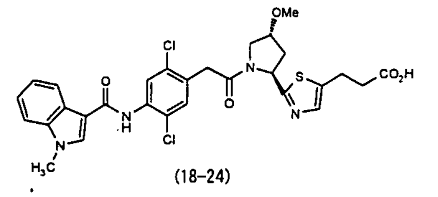

(18-24)

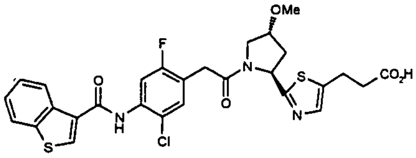

(18-25)

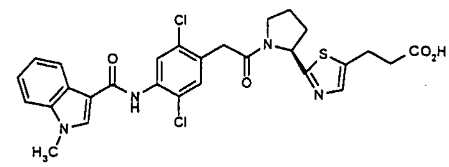

(18-26)

[Chem.12]

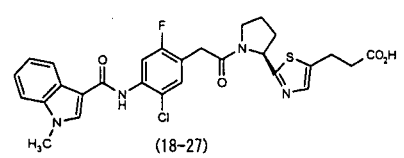

(18-27)

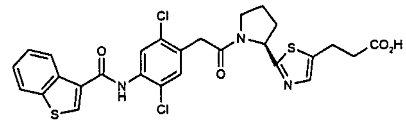

(18-28)

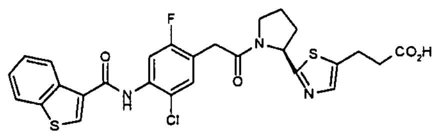

(18-29)

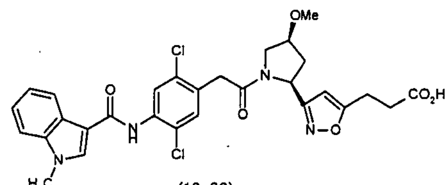

(18-30)

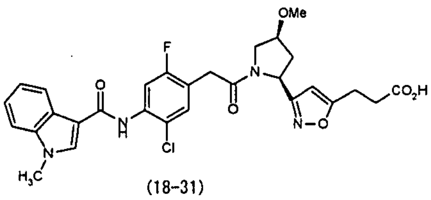

(18-31)

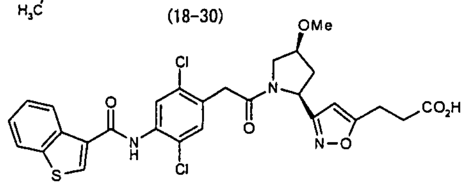

(18-32)

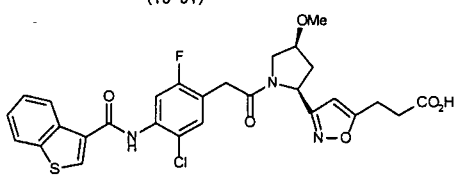

(18-33)

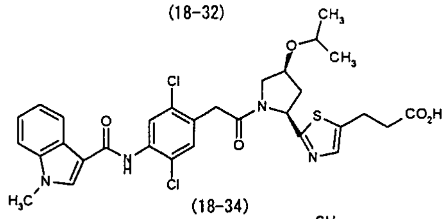

(18-34)

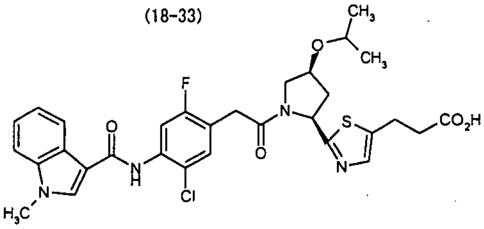

(18-35)

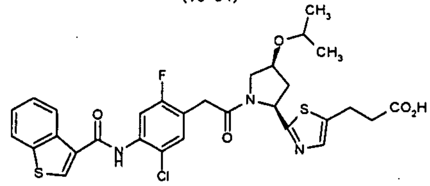

(18-36)

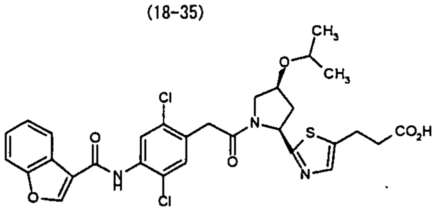

(18-37)

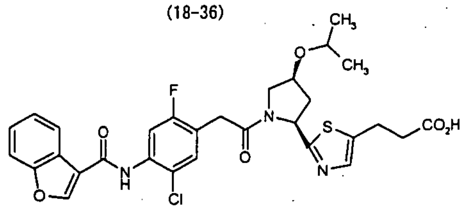

(18-38)

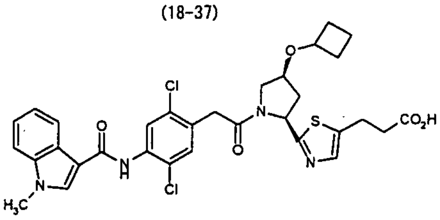

(18-39)

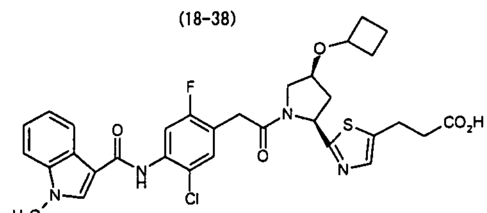

(18-40)

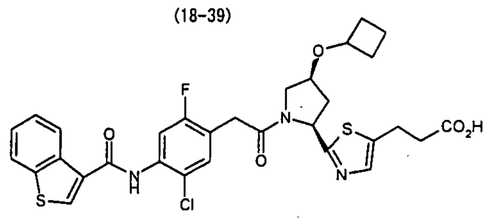

(18-41)

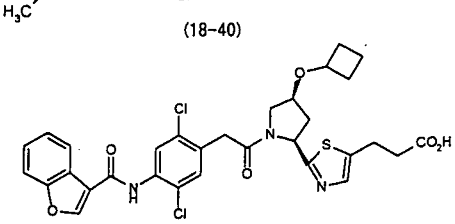

(18-42)

[Chem.13]

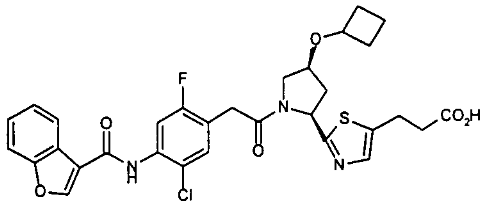

(18-43)

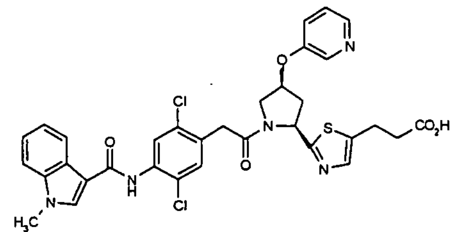

(18-44)

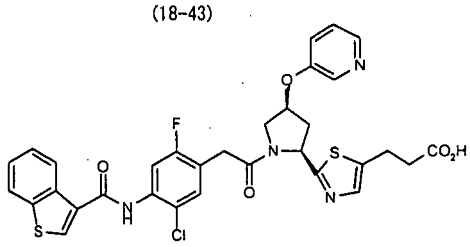

(18-45)

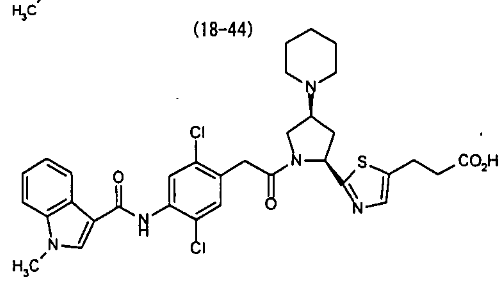

(18-46)

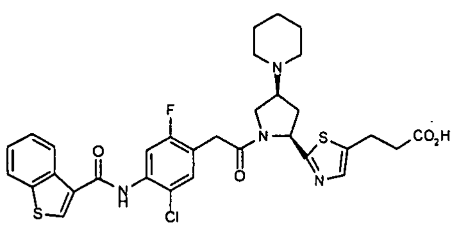

(18-47)

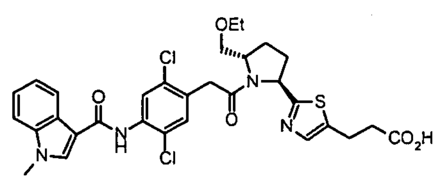

(18-48)

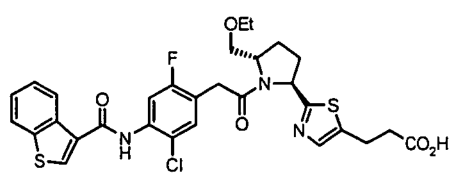

(18-49)

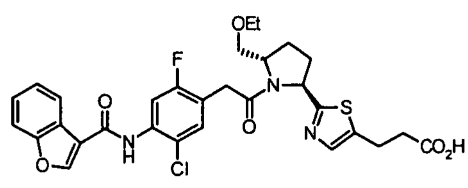

(18-50)

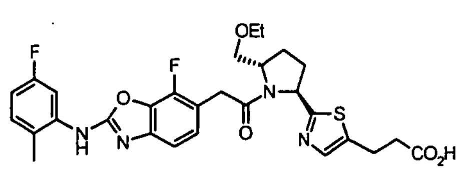

(18-51)

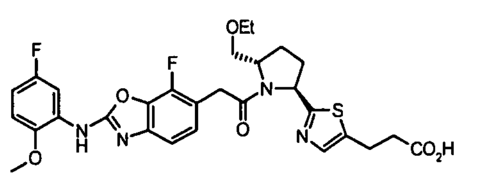

(18-52)

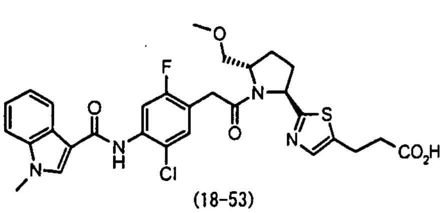

(18-53)

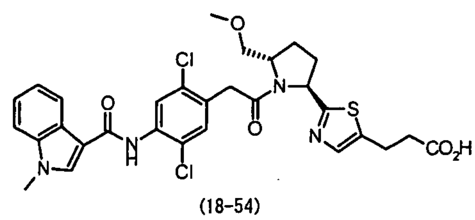

(18-54)

[Chem. 14]

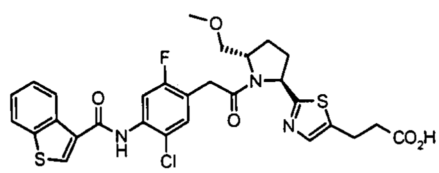

(18-55)

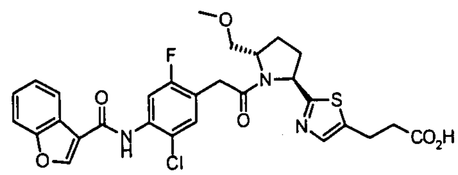

(18-56)

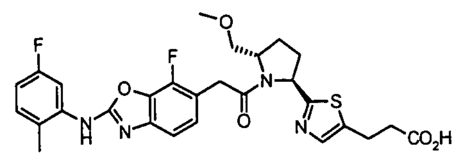

(18-57)

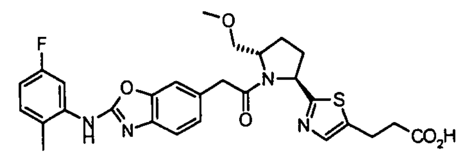

(18-58)

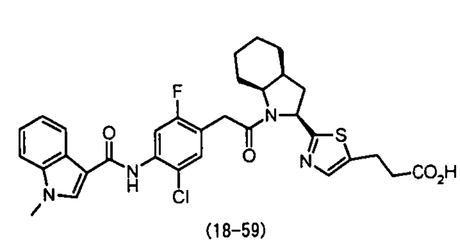

(18-59)

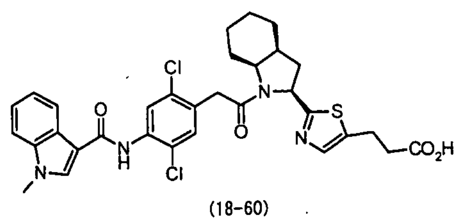

(18-60)

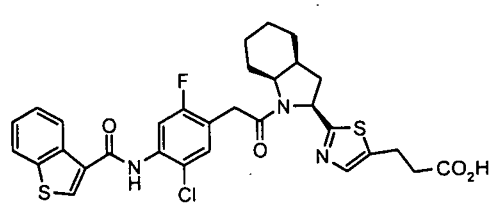

(18-61)

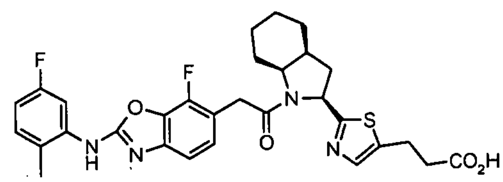

(18-62)

[Chem. 15]

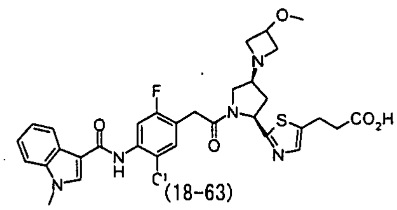

(18-63)

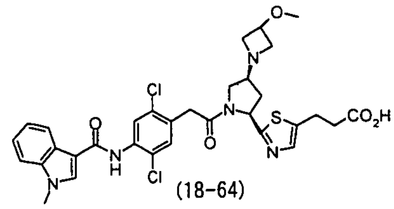

(18-64)

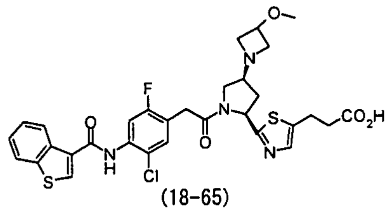

(18-65)

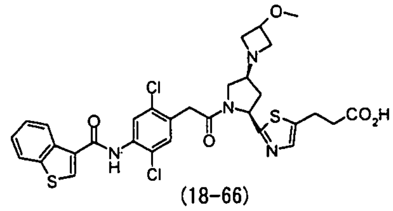

(18-66)

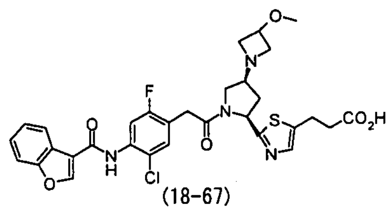

(18-67)

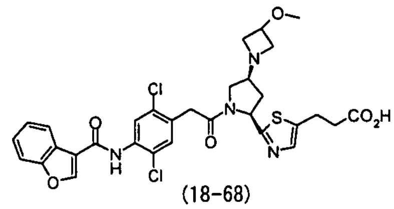

(18-68)

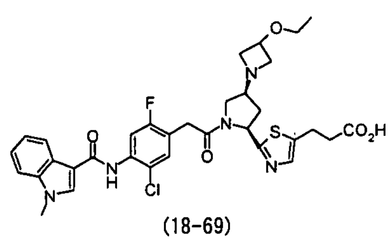

(18-69)

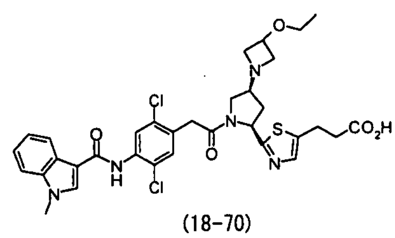

(18-70)

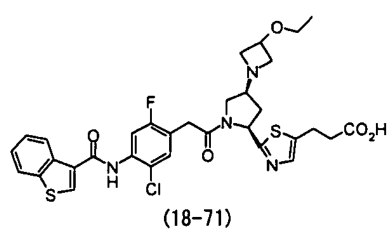

(18-71)

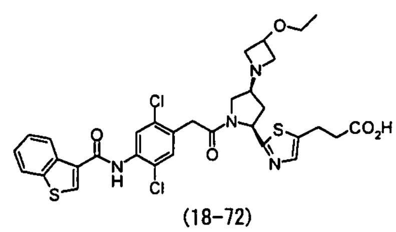

(18-72)

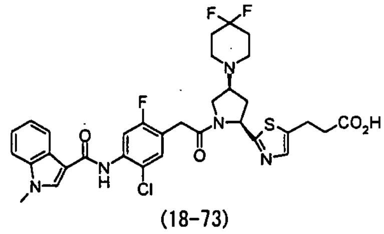

(18-73)

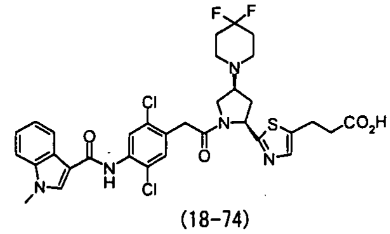

(18-74)

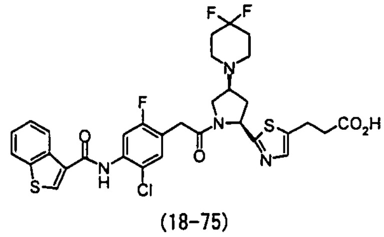

(18-75)

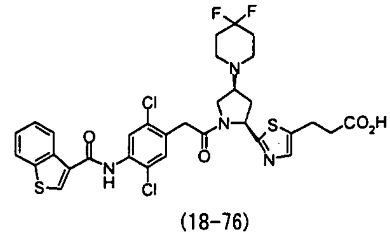

(18-76)

[Chem.16]

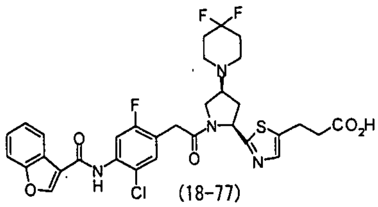
(18-77)

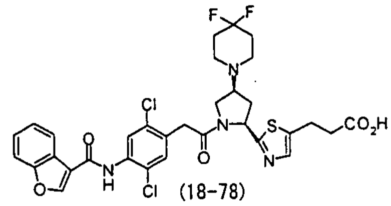
(18-78)

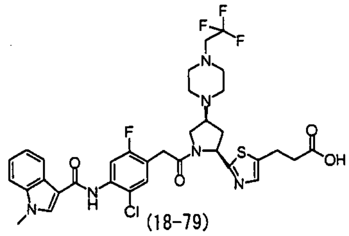
(18-79)

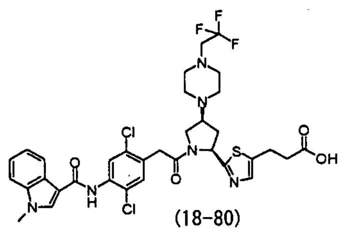
(18-80)

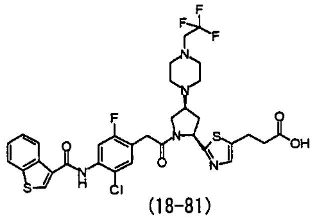
(18-81)

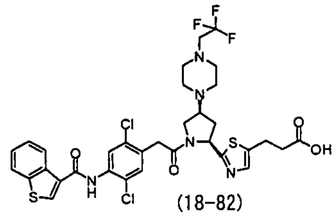
(18-82)

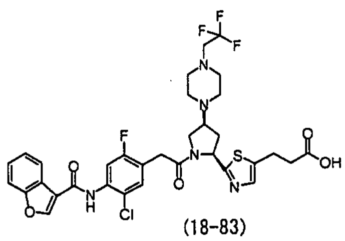
(18-83)

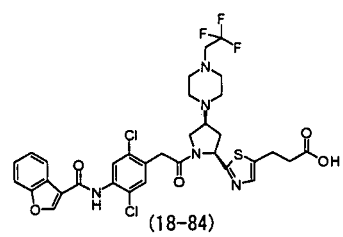
(18-84)

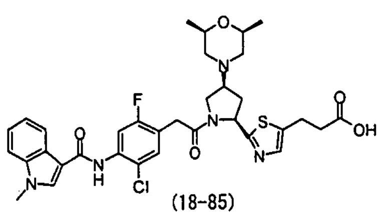
(18-85)

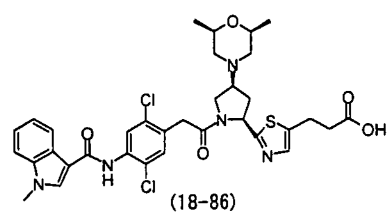
(18-86)

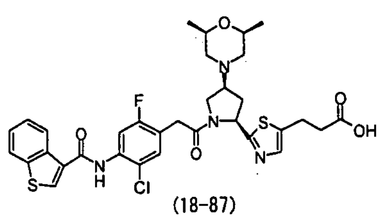
(18-87)

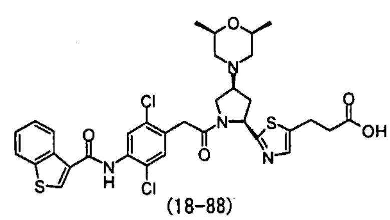
(18-88)

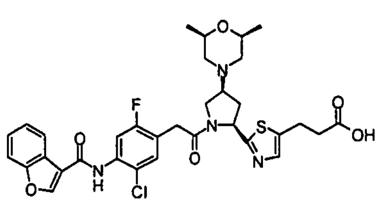
(18-89)

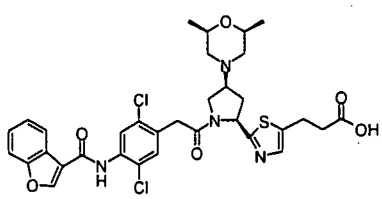
(18-90)

[Chem.17]

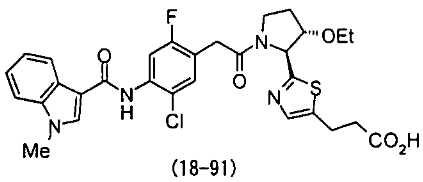
(18-91)

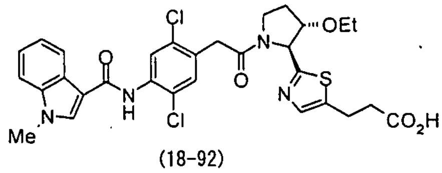
(18-92)

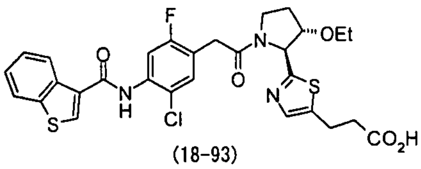
(18-93)

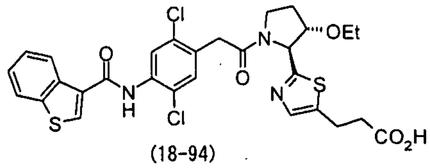
(18-94)

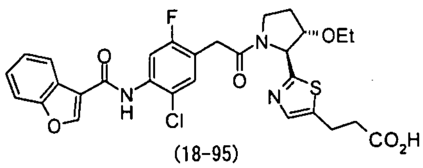
(18-95)

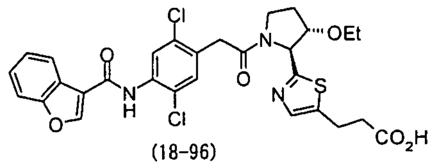
(18-96)

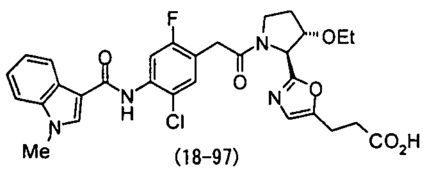
(18-97)

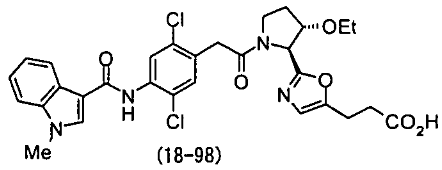
(18-98)

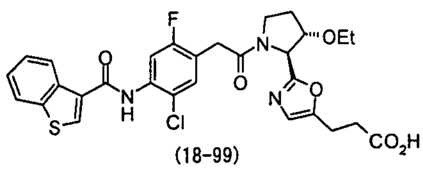
(18-99)

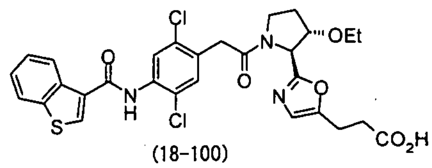
(18-100)

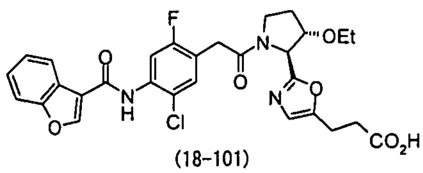
(18-101)

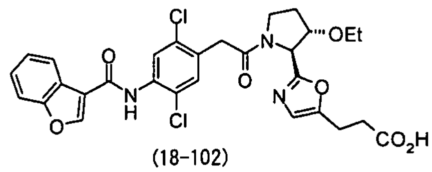
(18-102)

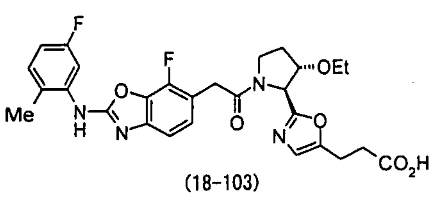
(18-103)

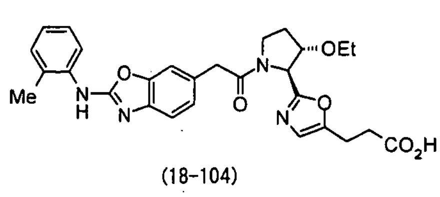
(18-104)

[Chem.18]

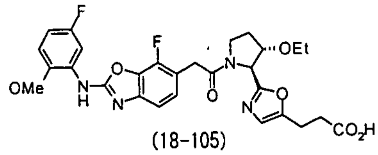

(18-105)

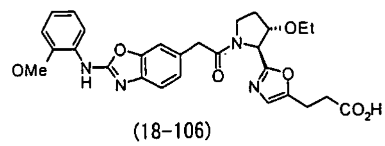

(18-106)

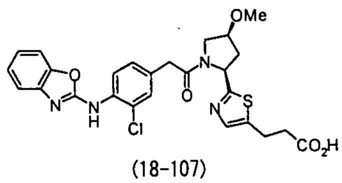

(18-107)

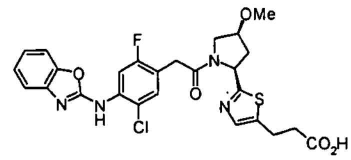

(18-108)

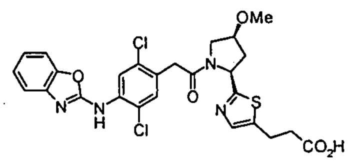

(18-109)

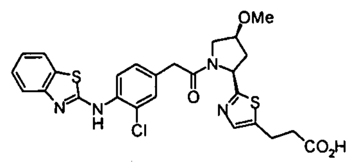

(18-110)

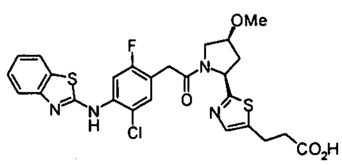

(18-111)

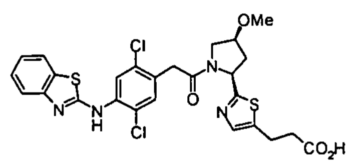

(18-112)

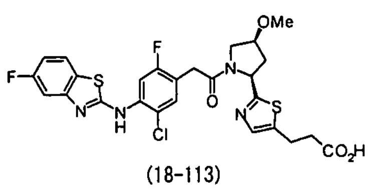

(18-113)

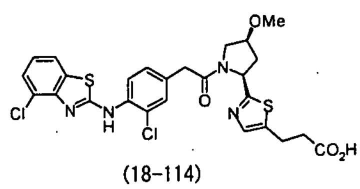

(18-114)

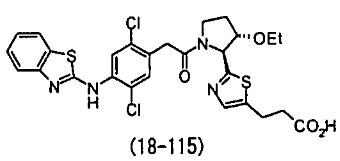

(18-115)

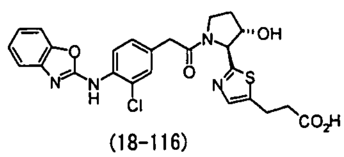

(18-116)

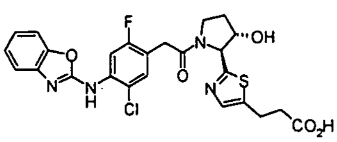

(18-117)

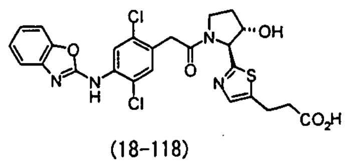

(18-118)

[Chem.19]

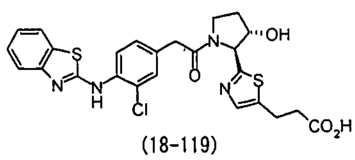

(18-119)

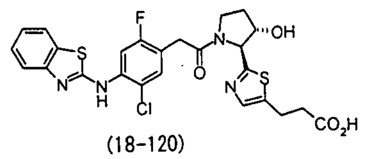

(18-120)

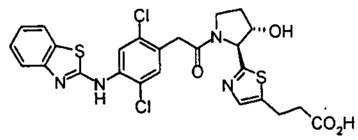

(18-121)

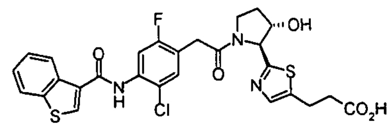

(18-122)

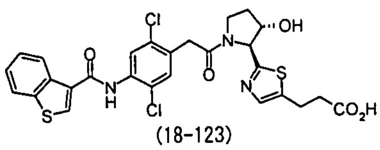

(18-123)

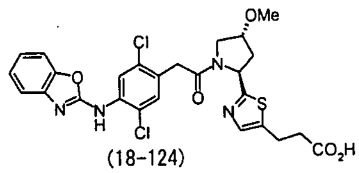

(18-124)

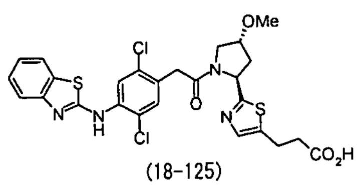

(18-125)

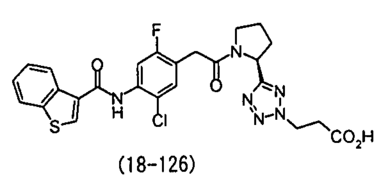

(18-126)

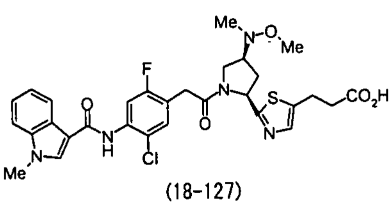

(18-127)

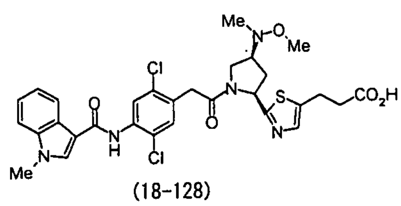

(18-128)

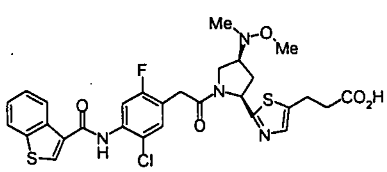

(18-129)

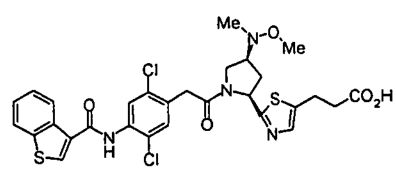

(18-130)

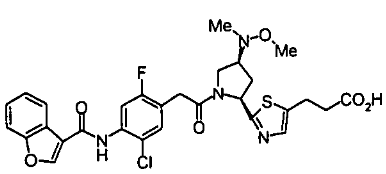

(18-131)

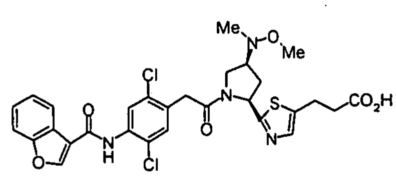

(18-132)

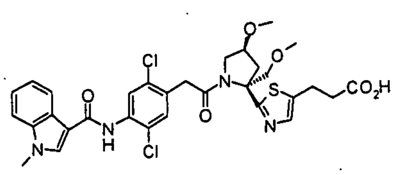

(18-133)

The present compound represented by the formula (I) or (II) can be optionally converted into a physiologically acceptable salt using an inorganic acid such as hydrochloric acid, sulfuric acid, phosphoric acid and the like, or an organic acid such as formic acid, acetic acid, methanesulfonic acid and the like. In addition, when the present compound represented by the formula (I) or (II) has an acidic group such as a carboxyl group and the like, generally, the compound can form a base adduct salt. The physiologically acceptable salt may be any of organic salts and inorganic salts, and preferable examples include alkali metal salts such as a lithium salt, a sodium salt and a potassium salt, alkaline earth metal salts such as a magnesium salt and a calcium salt, an ammonium salt, a triethylamine salt, a N-methylglucamine salt and a tris(hydroxymethyl)aminomethane salt.
In addition, a free compound or a salt of the present compound is present as a solvate in some cases.
The solvate is not particularly limited as far as it is pharmaceutically acceptable, but examples include a hydrate, an ethanolate and the like. In addition, when a nitrogen atom is present in the present compound represented by the formula (I) or (II), the present compound may be N-oxide. These solvate and N-oxide are included in the scope of the present invention.
In addition, various isomers such as a geometrical isomer such as a cis isomer, a trans isomer and the like, and an optical isomer such as a d isomer, a 1 isomer and the like may be present in the present compound represented by the formula (I) or (II) or a salt thereof, depending on a kind and a combination of substituents, and the present compound includes these all steric isomers and a mixture of these steric isomers at any ratio.

The compound of the present invention can be synthesized, for example, according to the following [Scheme 1] to [Scheme 3B].

The present compound represented by the general formula (I) or (II) can be produced by a coupling reaction between a carboxylic acid derivative (represented by the formula (1), (2), (3) or (4)) and cyclic amine derivatives (represented by the formula 5)) or salts of the cyclic amine derivatives, and a subsequent deprotecting step such as hydrolysis of an ester part as shown in [Scheme 1] (in Scheme, $R^{53a}$ represents a hydrogen atom, a lower alkyl group or a lower alkoxymethyl group, $R^{53}$ and $R^{54}$ each independently represent a hydrogen atom, a hydroxy group, a halogen atom, an amino group, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkylamino group optionally having a substituent, a dialkylamino group optionally having a substituent, a cyclic amino group optionally having a substituent, an alkylsulfonylamino group optionally having a substituent, an arylsulfonylamino group optionally having a substituent, a heteroaryloxy group optionally having a substituent, or an aryloxy group optionally having a substituent (examples of a group which can be substituted by the alkyl group, the aryl group, the heterocyclic group, the alkoxy group, the alkoxyalkyl group, the cycloalkoxy group, the monoalkylamino group, the dialkylamino group, the cyclic amino group, the alkylsulfonylamino group, the arylsulfonylamino group, the heteroaryloxy group, the aryloxy group and the aryloxy group include a lower alkyl group, a lower alkoxy group, a halogen atom, an amino group, a hydroxy group and the like, and one or a plurality of them may be substituted), $R^{53}$ and $R^{54}$ may be taken together with a carbon atom constituting a pyrrolidine ring to which $R^{53}$ and $R^{54}$ are bound, to form a 3- to 7-membered cyclic hydrocarbon or a heterocycle (optionally having 1 to 3 substituents independently selected from a hydroxy group, a halogen atom, an amino group, an alkyl group, an alkoxy group, an aryl group, an aryloxy group, an alkylamino group, a cyclic amino group, a benzyloxy group and a heteroaryl group on the ring), $R^{55}$ represents a lower alkyl group, or a benzyl group optionally having a substituent (examples of a group which can be substituted by the benzyl group include a lower alkyl group, a lower alkoxy group, a halogen atom, an amino group, a hydroxy group and the like), and $R^{11}$, $R^{12}$, $R^{21}$, $R^{22}$, $V^1$, $V^2$, $Y^1$, $Y^2$ and $W^1$ are as defined above).
The ester derivative represented by the formula (6), (7), (8) or (9) can be converted into a compound represented by the general formula (I) or (II) which is a free carboxylic acid-type compound, by alkali hydrolysis after the aforementioned condensing reaction when $R^{55}$ is a lower alkyl group, or by debenzylation by catalytic hydrogenation when $R^{55}$ is a benzyl group optionally having a substituent.

[Chem.20]

[Scheme 1]

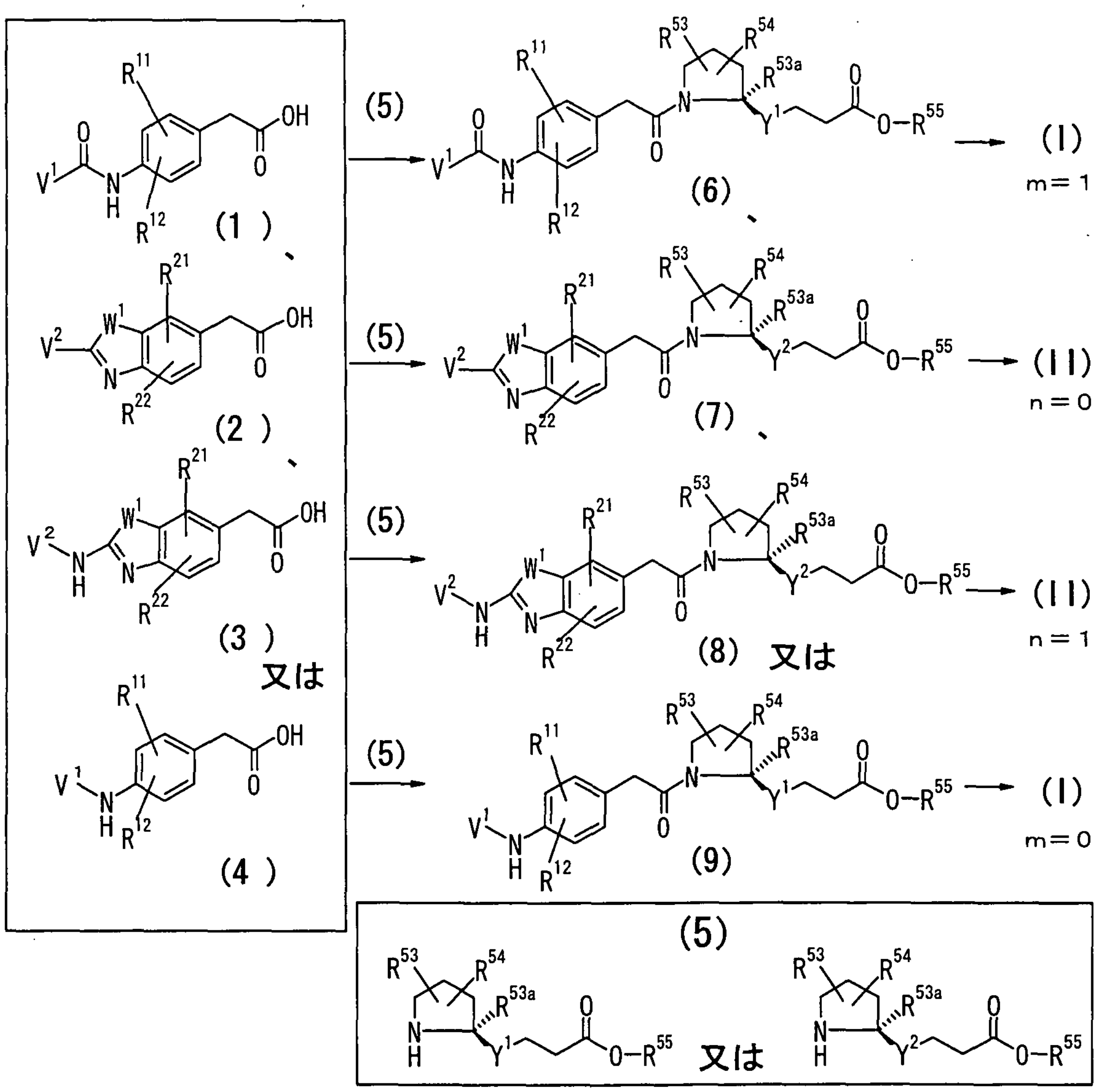

(1 )、(2 )、(3 ) 又は (4 )

(5) → (6)、→ (I) m= 1

(5) → (7)、→ (II) n = 0

(5) → (8) 又は → (II) n = 1

(5) → (9) → (I) m= 0

(5) 又は

The compound represented by the general formula (I) or (II) shown in [Scheme 1] can be produced by the known method. A compound represented by the formula (6), the formula (7), the formula (8) or the formula (9) can be produced depending on a starting material, by reacting carboxylic acids (represented by the formula (1), the formula (2), the formula (3) or the formula (4)) and cyclic amine derivatives (represented by the formula (5)) or salts of the cyclic amine derivatives (e.g. hydrochlorides) at a temperature in a range of -20°C to a boiling point of a solvent, preferably in a range of 0°C to room temperature using a condensing agent which is 1-ethyl-3-(3-dimethylaminopropyl)carbodiimide hydrochloride, N,N-dicyclohexylcarbodiimide, N,N-carbonyldiimidazole, or a similar compound thereto, in an inert halogenated hydrocarbon-based solvent such as methylene chloride, an inert hydrocarbon-based solvent such as toluene, an inert ether-based solvent such as tetrahydrofuran, or an inert polar solvent such as N,N-dimethylformamide according to the known condensing method. A reaction time of a condensing reaction is usually around 30 minutes to 24 hours. In addition, in this condensing reaction, it is preferable to use an organic amine-based base such as triethylamine and N,N-diemthylaminopyridine in a range of 1 equivalent to 30 equivalents and, when a salt of cyclic amine derivatives represented by the formula (5) is used, it is necessary to use the base at a stoichiometric amount or more necessary for neutralizing the salt. Alternatively, it is preferable to use 1-hydroxybenzotriazole at 0.2 equivalent to 1.2 equivalents together with the organic amine-based base.

The following [Scheme 1A] to [Scheme 1D] show a method of synthesizing carboxylic acids (represented by the formula (1), the formula (2), the formula (3) or the formula (4)) used in the condensing reaction as a starting material (in Scheme, $R^{11}$, $R^{12}$, $R^{21}$, $R^{22}$, $V^1$ and $V^2$ are as defined above, and $R^{10}$ represents a lower alkyl group).

The following [Scheme 1A] shows a general process for producing carboxylic acids represented by the formula

(1) which is used for derivatizing into a compound represented by the general formula (I).
A compound represented by the formula (1A-5) can be produced by the known method. For example, a compound of the formula (1A-4) can be produced by reacting a compound represented by the formula (1A-2) and a compound represented by the formula (1A-3) at a temperature in a range of -20° C to a boiling point of a solvent, preferably in a range of room temperature to a boiling point of a solvent in the presence of organic bases such as triethylamine in an inert halogenated hydrocarbon-based solvent such as methylene chloride, an inert hydrocarbon-based solvent such as toluene, or an inert ether-based solvent such as tetrahydrofuran. Alternatively, a compound represented by the formula (1A-4) can be produced by reacting a compound represented by the formula (1A-1) and a compound represented by the formula (1A-3) at a temperature in a range of -20°C to a boiling point of a solvent using a condensing agent which is 1-ethyl-3-(3-dimethylaminopropyl)carbodiimide hydrochloride, N,N-dicyclohexylcarbodiimide, N,N-carbonyldiimidazole, or a similar compound thereto in an inert halogenated hydrocarbon-based solvent such as methylene chloride, an inert hydrocarbon-based solvent such as toluene, an inert ether-based solvent such as tetrahydrofuran, or an inert polar solvent such as N,N-dimethylformamide. Alternatively, this reaction may be performed in the presence of an organic amine-based base such as triethylamine and N,N-diemthylaminopyridine, or inorganic bases and 1-hydroxybenzotriazole. Further, a carboxylic acid derivative can be synthesized represented by the formula (1A-5) by alkali-hydrolyzing an ester part of an ester compound represented by the formula (1A-4) according to the known method. Compounds represented by the formulas (1A-1), (1A-2) and (1A-3) are commercially available, or can be easily prepared according to the known method.

[Chem.21]

[Scheme 1A]

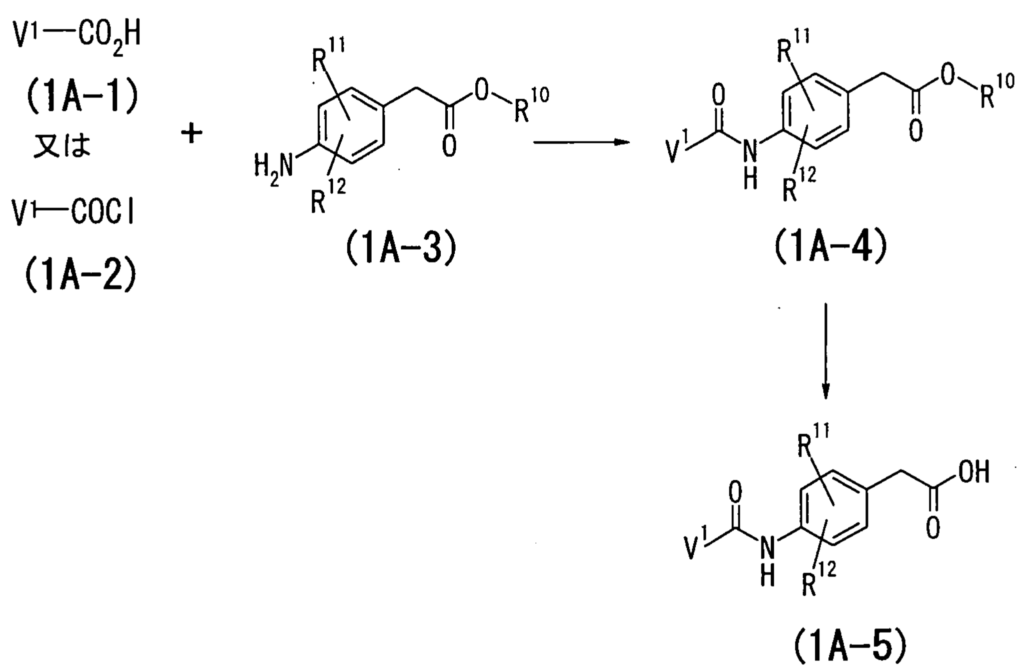

(1A-1) 又は (1A-2) (1A-3) (1A-4) (1A-5)

The following [Scheme 1B] shows a general process for producing a benzoxazole derivative in which $W^1$ is an oxygen atom, among carboxylic acids represented by the formula (2) which is used for derivatizing into a compound (n = 0) represented by the general formula (II) (in Scheme, $R^{21}$, $R^{22}$, $V^2$ and $R^{10}$ are as defined above).

[Chem.22]

[Scheme 1B]

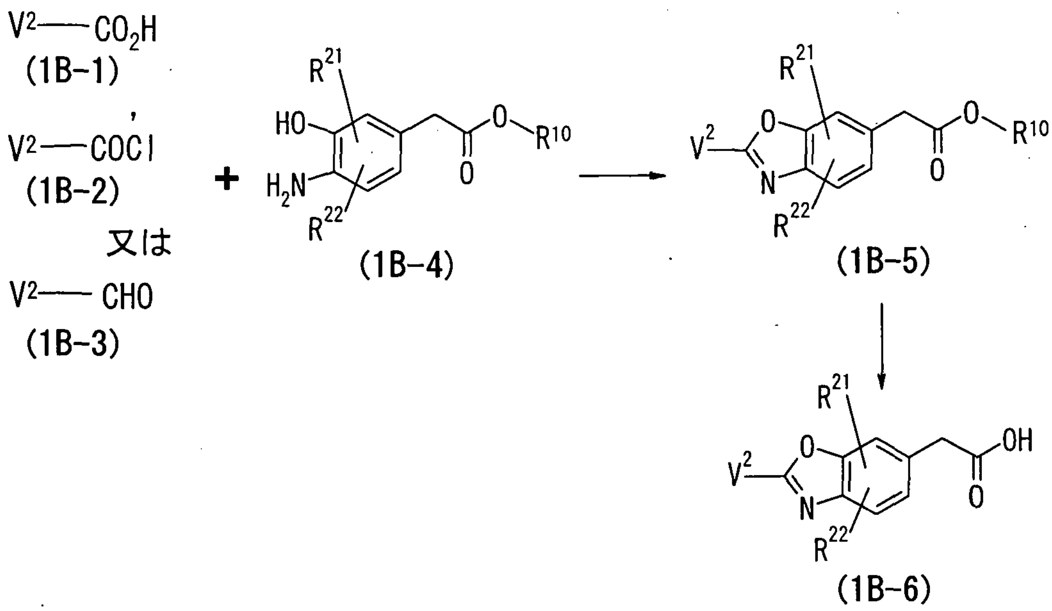

(1B-1), (1B-2) 又は (1B-3) + (1B-4) → (1B-5) → (1B-6)

The compound represented by the formula (1B-6) can be produced by the known method such as the method of Nakayama et al. (WO 2002/053534). For example, a banzoxazole derivative represented by the formula (1B-5) can be obtained by heating at reflux a carboxylic acid chloride compound (1B-2) prepared from a carboxylic acid compound represented by the formula (1B-1) which is commercially available, or can be prepared by the known method by acting oxalyl chloride, thionyl chloride, phosphorus trichloride, or phosphorus pentachloride, preferably oxalyl chloride, or thionyl chloride, and a catalytic amount of N,N-dimethylformamide at a temperature in a range of -20˚C to a boiling point of a solvent in an inert halogenated hydrocarbon solvent such as methylene chloride, an inert hydrocarbon solvent such as toluene, or an inert ether-based solvent such as tetrahydrofuran, and an aniline compound represented by the formula (1B-4) in the presence of acids such as boric acid in an inert hydrocarbon solvent such as xylene. In addition, when produced from a compound represented by the formula (1B-3) and an aniline compound represented by the formula (1B-4), a compound represented by the formula (1B-5) can be obtained by an oxidative cycling reaction using Ph-I $(OAc)_2$ (M. H. Jung et al., J.Med.Chem., 9,56 (1999)). Further, carboxylic acid (1B-6) can be produced by alkali-hydrolyzing an ester part of a compound (1B-5) according to the known method. A compound represented by the formula (1B-3) is commercially available or can be easily prepared by the known method.

The following [Scheme 1C] shows a general process for producing a benzoxazole derivative in which $W^1$ is an oxygen atom, among carboxylic acids represented by the formula (3) which is used for derivatizing into a compound (n = 1) represented by the general formula (II) (in Scheme, $R^{21}$, $R^{22}$, $V^2$, and $R^{10}$ are as defined above).

The compound represented by the formula (1C-5) in [Scheme 1C] can be produced by the known method. For example, a thiourea compound represented by the formula (1C-3) can be prepared by treating an aminophenol derivative (1C-2) which is known per se, or can be produced by the known method, and an isothiocyanate compound (1C-1) which is sold or can be prepared by the known method at a temperature in a range of -20˚C to a boiling point of a solvent, preferably in a range of 0˚ C to room temperature in an inert halogenated hydrocarbon-based solvent such as methylene chloride, an inert hydrocarbon-based solvent such as toluene, an inert ether-based solvent such as tetrahydrofuran, or an alcohol-based solvent such as ethanol, then, a ring-closure reaction product represented by the formula (1C-4) is obtained by treatment with $KO_2$ or $Ni_2O_3$, preferably mercuric oxide (yellow) at a temperature in a range of -20˚C to a boiling point of a solvent, preferably in a range of room temperature to a boiling point of a solvent, for example, in an inert hydrocarbon-based solvent such as toluene, an inert ether-based solvent such as tetrahydrofuran, an inert polar solvent such as N,N-dimethylformamide, or an alcohol-based solvent such as ethanol and, further, an ester part can be converted into carboxylic acid represented by the formula (1C-5) by the known alkali hydrolysis. As the known alternative

method of producing the compound represented by the formula (1C-4), a method of derivatizing a 2-mercaptobenzoxazole derivative represented by the formula (1C-6) into a 2-chlorobenzoxazole derivative represented by the formula (1C-7), and treating this with an aniline derivative (1C-8) can be also applied. First, a 2-mercaptobenzoxazole derivative of the formula (1C-6) can be produced by a method of treating an o-aminophenol derivative represented by the formula (1C-2) with commercially available potassium ethyl xanthate, or treating with a xanthate sodium salt produced from carbon disulfide and sodium ethoxide in situ. As a solvent used, an alcohol-based solvent such as methanol and ethanol is preferable, a reaction temperature is in a range of room temperature to a boiling point of solvent, or a reaction may be performed in a sealed reaction vessel (sealed tube). A reaction is usually completed in 10 minutes to 2 hours. After completion of the reaction, a 2-mercaptobenzoxazole derivative represented by the formula (1C-6) can be produced by treatment with concentrated sulfuric acid. Then, a 2-mercaptobenzoxazole derivative represented by the formula (1C-6) can be converted into a 2-chlorobenzoxazole derivative represented by the formula (1C-7) by treating with 1 equivalent to 20 equivalents of a chlorinating agent such as thionyl chloride at a temperature in a range of room temperature to a boiling point of a solvent, preferably in a room temperature to 80°C in the presence of a catalytic amount of N,N-dimethylformamide. As a reaction solvent, an inert hydrocarbon-based solvent such as toluene and benzene, or an inert halogenated solvent such as 1,2-dichloroethane may be used. A reaction is usually completed in around 1 hour to 3 hours. Regarding conversion of the resulting 2-chlorobenzoxazole derivative represented by the formula (1C-7) into a compound of the formula (1C-4), a compound of the formula (1C-4) can be produced by treatment with an aniline derivative represented by the formula (1C-8) at a temperature in a range of room temperature to a boiling point of a solvent for 1 hour to 30 hours, preferably under heat refluxing condition in an inert hydrocarbon-based solvent such as toluene and benzene, or an inert ether-based solvent such as tetrahydrofuran.

[Chem.23]

[Scheme 1C]

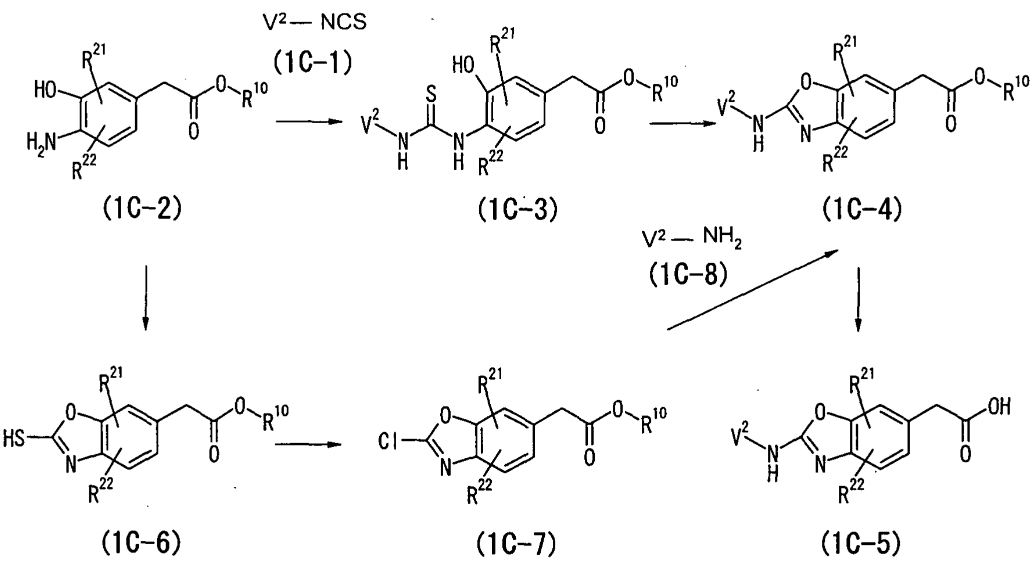


The following [Scheme 1D] shows a general process for producing a compound wherein $V^1$ is a benzoxazol-2-yl derivative or a benzothiazol-2-yl derivative among carboxylic acids represented by the formula (4) which is used for derivatizing into a compound (m = 0) represented by the general formula (I) (in Scheme, $W^3$ represents an oxygen atom or a sulfur atom, $X^a$ represents a chlorine atom or a bromine atom, $R^{45}$, $R^{46}$ and $R^{47}$ each independently represent a halogen atom, a lower alkyl group or a lower alkoxy group, and $R^{11}$, $R^{12}$ and $R^{10}$ are as defined above).

A 2-chlorobenzoxazole derivative represented by the formula (1D-1) is commercially available per se, or can be easily prepared by the known method. On the other hand, a 2-halogenobenzothiazole derivative represented by the formula (1D-3) is commercially available per se, or can be easily prepared from a 2-aminobenzothiazole derivative represented by the formula (1D-2) which is commercially available per se, or can be obtained by the known method. For example,

a 2-chlorobenzothiozole derivative or a 2-bromobenzothiazole derivative represented by the formula (1D-3) can be prepared by adding copper (II) chloride, or copper (II) bromide, and alkyl nitrite such as isoamyl nitrite, and tert-butyl nitrite to a 2-aminobenzothiazole derivative represented by the formula (1D-2) at room temperature in an inert polar solvent such as acetonitrile according to the method of Doyle et al. [Doyle, M.P., et al., J.Org.Chem.1977, 42,2426], or the method of Laurence [Laurence E. Burgess, Synth. Communication, 27 2181-2191 (1997)], warming the reaction mixture to 65°C, adding a 2-aminobenzothiazole derivative represented by the formula (1D-2), and performing a reaction and post-treatment according to the method described in the above document. Then, a2-chlorobenzoxazole derivative represented by the formula (1D-1), or a 2-halogenobenzothiazole derivative represented by the formula (1D-3) can be converted into benzoxazole-2-ylaminophenylacetic acid ester or a benzothiazole-2-ylaminophenylacetic acid ester derivative represented by the formula (1D-5) by the known condensing reaction with an aniline derivative represented by the formula (1D-4). The derivative can be produced by heating at reflux under stirring in an inert hydrocarbon-based solvent such as benzene, toluene, xylene and o-dichlorobenzene, or an inert high boiling point ether-based solvent such as diphenyl ether, preferably an inert hydrocarbon solvent such as xylene as a solvent used in the present condensation. In the present reaction, it is preferable to add pyridinium p-toluenesulfonate (PPTS) in a range of 0.1 equivalent to 1 equivalent as a catalyst for promoting the reaction. A reaction is usually completed in 1 hour to around 24 hours. Then, an ester compound represented by the formula (1D-5) can be converted into carboxylic acid represented by the formula (1D-6) by performing the known alkali hydrolysis.

[Chem.24]

[Scheme 1D]

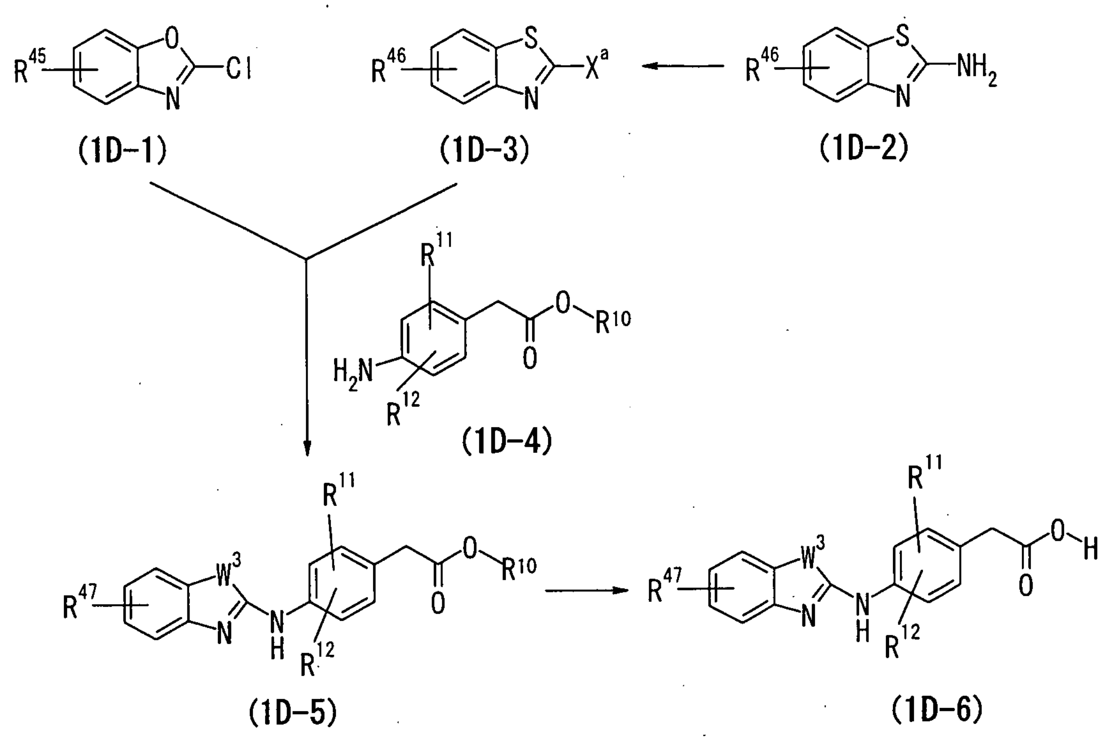

The following [Scheme 2] shows a process for producing compounds in which a substituent has been introduced into a 4-position of pyrrolidine (in Scheme, $R^{51}$ represents a lower alkyl group, P represents a general protecting group for an amino group and, as such a protecting group, groups described in "Protective Groups in Organic Synthesis, eds. by T. W. Greene and P. G. Wuts, John Wiley & Sons, Inc., New York, 1991" are preferable, and a tert-butyloxycarbonyl group (Boc group) is further preferable. X represents a halogen atom such as a fluorine atom, a phenyloxy group, a pyridyloxy group, a 1-piperidinyl group or an azide group, $R^{52a}$ represents a lower alkyl group optionally having a substituent

or a benzyl group optionally having a substituent, and $R^{53a}$ is as defined above).

Regarding 4-hydroxyproline represented by the formula (2-1) which is commercially available (preferably trans-4-hydroxy-L-proline), an esterification of its carboxylic acid part and introduction of a protecting group for a nitrogen atom can be performed according to the known method. For example, an ester compound represented by the formula (2-2) can be produced by treatment at a temperature in a range of room temperature to a boiling point of a solvent in the presence of mineral acids such as concentrated sulfuric acid in an inert alcohol-based solvent such as methanol, and a compound represented by the formula (2-3) can be produced, for example, by treatment in the presence of acid anhydrides such as di-tert-butyl bicarbonate, or acyl-halides such as benzyloxycarbonyl chloride and organic amine-based bases such as triethylamine, or inorganic bases such as potassium carbonate in a mixed solution of water and an inert polar solvent such as acetonitrile. Further, a secondary hydroxy group of an alcohol compound (2-3) can be converted into a secondary hydroxy group having reverse conformation according to the known method. For example, a compound represented by the formula (2-4) can be produced by subjecting an alcohol compound (2-3) to Mitsunobu reaction with formic acid or phenol in an inert ether-based solvent such as tetrahydrofuran to formyloxidize or benzoyloxidize a secondary hydroxy part, and alkali-hydrolyzing this using inorganic bases such as potassium carbonate, preferably sodium bicarbonate.

[Chem.25]

[Scheme 2]

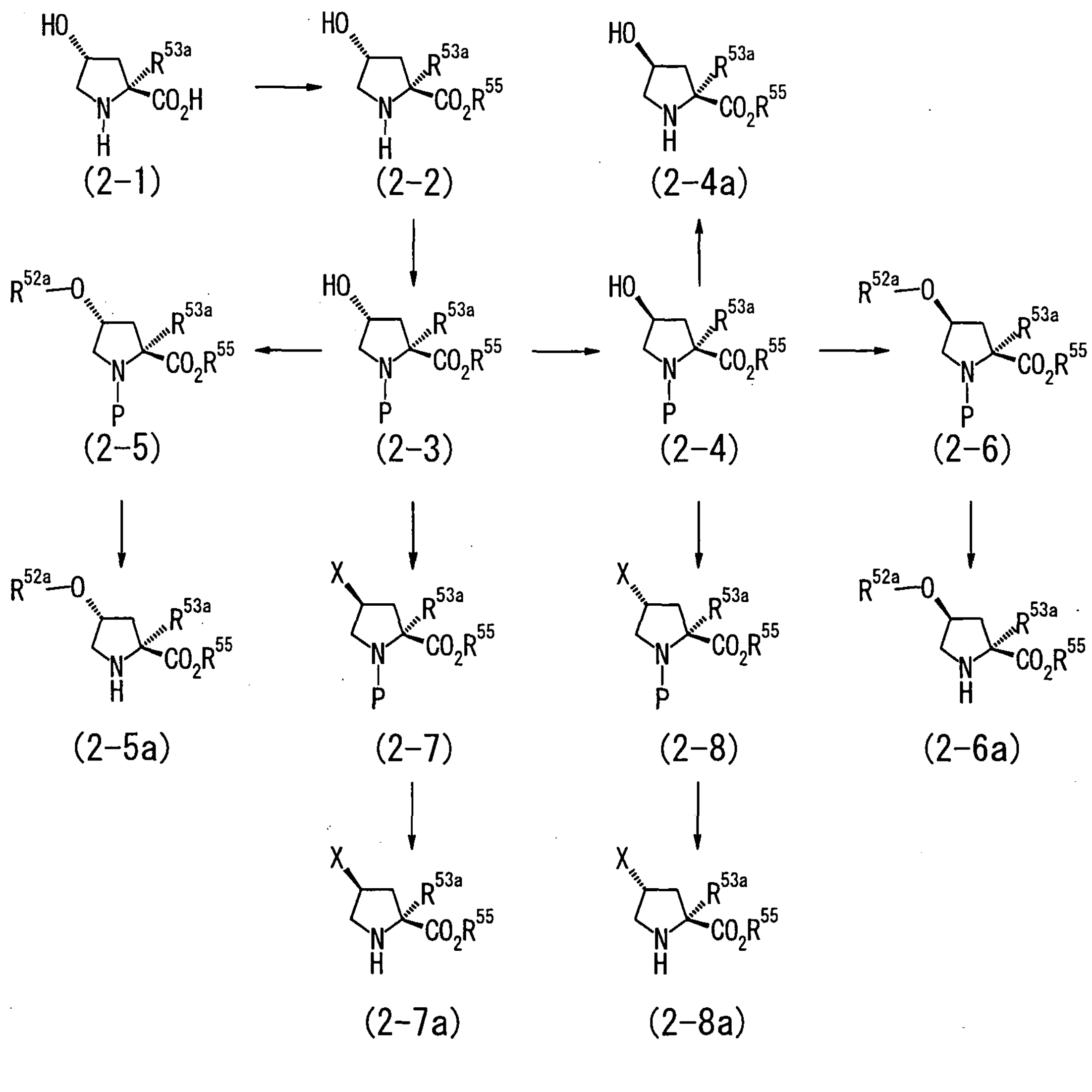

(2-1) (2-2) (2-4a)

(2-5) (2-3) (2-4) (2-6)

(2-5a) (2-7) (2-8) (2-6a)

(2-7a) (2-8a)

On the other hand, secondary hydroxy group parts of an alcohol compound represented by the formula (2-3) and an alcohol compound represented by the formula (2-4) can be converted into respective alkoxy derivatives (2-5) and (2-6) while retaining stereochemistry by the known method. For example, derivatives can be produced by treatment of alkyl halide such as methyl iodide and benzyl bromide at a temperature in a range of -20˚C to a boiling point of a solvent, preferably in a range of -20˚C to room temperature in the presence of a base such as sodium hydride in an inert ether-based solvent such as tetrahydrofuran, or an inert polar solvent such as N,N-dimethylformamide. Alternatively, parts can be converted into an isopropoxy compound or a cyclobutoxy compound by an alkylating reaction of treating corresponding ketone and bismuth tribromide and triethylsilane at a temperature in a range of -20˚C to a boiling point of a solvent, preferably in a range of 0˚C to room temperature in an inert polar solvent such as acetonitrile, after derivatization of a hydroxy group into a silyloxy group such as a tert-butyldimethylsilyloxy group.

Secondary hydroxy groups of compounds represented by the formula (2-3) and a compound represented by the formula (2-4) can be converted into a compound represented by the formula (2-7) and a compound represented by the formula (2-8) in which X is a halogen atom, a phenyloxy group, a pyridyloxy group, a 1-piperidinyl group or an azido group, according to the known SN2-type nucleophilic substitution reaction. For example, as an example of a process for producing a compound represented by the formula (2-7) and a compound represented by the formula (2-8) in which X is a halogen atom, a fluorine atom can be introduced accompanying steric inversion by treating a compound represented by the formula (2-3) with fluorinating agents such as diethylaminosulfur trifluoride at a temperature in a range of -78˚C to a boiling point of a solvent, preferably in a range of 0˚C to room temperature in an inert halogenated hydrocarbon-based solvent such as methylene chloride. In addition, as an alternative method of a process for producing a compound in which X is a halogen atom, or as an example of a process for producing a compound represented by the formula (2-7) or the formula (2-8) in which X is a 1-piperidinyl group or an azido group, a fluorine atom can be also introduced by converting each secondary hydroxy group of a compound represented by the formula (2-3) and a compound represented by the formula (2-4) into a leaving group of an alkyl or arylsulfonyloxy group such as a mesyloxy group, a trifluoromethanesulfonyloxy group or a p-toluenesulfonyloxy group, and treating this with fluorides of an alkali metal such as potassium fluoride, or fluorides of an alkaline earth metal such as cesium fluoride in an inert polar solvent such as N,N-dimethylformamide, and a compound represented by the formula (2-7) or the formula (2-8) can be produced by using piperidine, sodium azide, or lithium azide in place of a halogenating agent. Further, in production of a compound represented by the formula (2-7) or the formula (2-8) in which X is a phenyloxy group or a pyridyloxy group, each secondary hydroxy group of a compound represented by the formula (2-3) and a compound represented by the formula (2-4) can be converted into a phenoxy group or a pyridyloxy group by using an optical extending reaction.

A protecting group for a nitrogen atom of a pyrrolidine ring of the formula (2-5), the formula (2-6), the formula (2-7) or the formula (2-8) produced by the aforementioned proces0s can be converted into a compound represented by the formula (2-4a), the formula (2-5a), the formula (2-6a), the formula (2-7a) or the formula (2-8a), which is free amine or hydrochloride or trifluoroacetate thereof according to the above method described in Reference Document.

The following [Scheme 2A] explains a process for producing a proline derivative in which $R^{53a}$ in the [Scheme 2] has a lower alkyl group or a lower alkoxymethyl group other than a hydrogen atom (in Scheme, $R^{57}$ represents a lower alkyl group or a lower alkoxymethyl group, $R^{58}$ represents a trichloromethyl group or a phenyl group, and $R^{52a}$ represents a lower alkyl group optionally having a substituent, or a benzyl group optionally having a substituent).

[Chem.26]

[Scheme 2A]

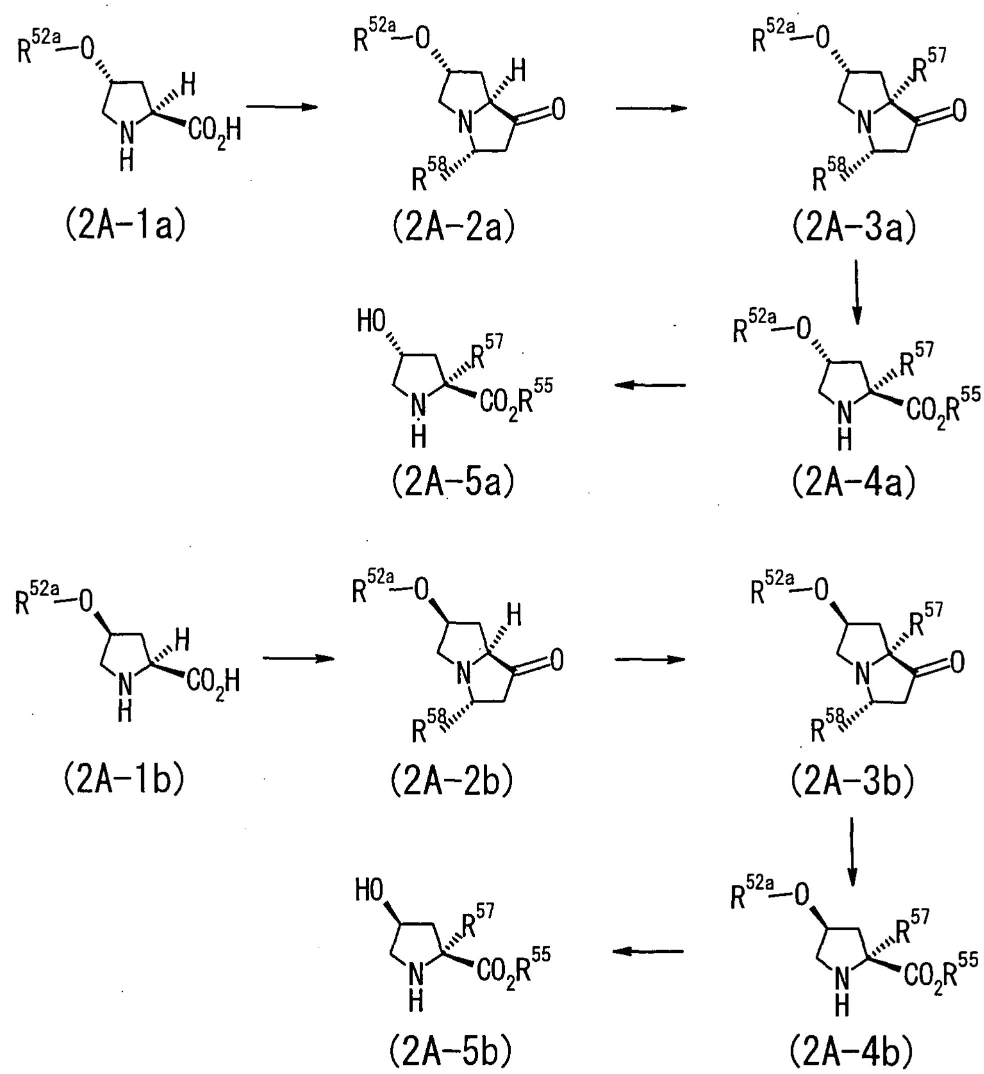

(2A-1a) (2A-2a) (2A-3a) (2A-4a) (2A-5a)

(2A-1b) (2A-2b) (2A-3b) (2A-4b) (2A-5b)

A cis or trans 4-hydroxy-L-proline derivative represented by the formula (2A-1) which is commercially available can be converted into a compound represented by the formula (2A-2) which is an oxabicyclo[3.3.0]octane derivative by a condensing reaction with chloral or benzaldehyde by the known method described, for example, in H. Wang et al. (Synlett (1999), (1), 33-36.), M. Amedjkouh et al. (Tetrahedron: Asymmetry (2002), 13 (20), 2229-2234) or documents cited in these documents. Examples of a solvent used include an inert polar solvent such as acetonitrile, and an inert hydrocarbon-based solvent such as benzene, toluene and xylene. A reaction temperature can be in a range of room temperature to a boiling point of a solvent and, when heating at reflux is performed at a boiling point of a solvent, it is preferable to use a Dean-Stark water-removing device in order to remove water produced by condensation to the outside of a reaction system. In addition, as a catalyst for accelerating a condensing reaction, p-toluenesulfonic acid may be added in a range of 0.1 equivalent to 1.0 equivalent. A reaction is completed in a range of 1 hour to 24 hours. Then, as a method converting steric-specifically a compound of the formula (2A-2) into a compound represented by the formula (2A-3) in which $R^{57}$ is a lower alkyl group or a lower alkoxymethyl group, the compound can be produced by capturing lithium carbonion produced in situ with lower alkyl halide such as methyl iodide, and chloromethyl methyl ether using a nucleophilic base such as lithium diisopropylamide and lithium hexamethyldisilazide in an aprotic ether-based solvent such as tetrahydrofuran according to H. Wang et al., or M. Amedj kouh et al. supra, or the document cited in these documents. Further, according to the aforementioneddocuments, a dicyclic derivative represented by the formula (2A-3) can be converted into a L-proline derivative represented by the formula (2A-4). A lower alkyl group optionally having a substituent represented by the formula (2A-4) can be also implemented. Further, a L-proline derivative represented by the formula (2A-3) includes a free base, and a salt such as hydrochloride and trifluoroacetate. Further, when $R^{52a}$ of

a L-proline derivative represented by the formula (2A-3) is a benzyl group optionally having a substituent, the compound can be converted into a 4-hydroxy-L-proline ester derivative represented by the formula (2A-5) according to ("Protective Groups in Organic Synthesis, eds. by T. W. Greene and P. G. Wuts, John Wiley & Sons, Inc., New York, 1991") in which the benzyl group optionally having a substituent is deprotected to remove.

On the other hand, an example of a SN2-type 1-piperidinyl group accompanying inversion of a hydroxy group shown in [Scheme 2] has been shown, and the following [Scheme 2B] shows a process for producing the formula (2B-3) or the formula (2B-4) in which a protecting group therefor has been removed therefrom utilizing a reductive aminating reaction of introducing steric-specifically an amino substituent into a 4-position of pyrrolidine (in Scheme, $R^{56}$ represents a lower alkyl group, $R^{53b}$ and $R^{53c}$ are the same or different, and represent an alkyl group optionally having a substituent, or a lower alkoxy group, or $R^{53b}$ and $R^{53c}$ may be taken together with a nitrogen atom to which $R^{53b}$ and $R^{53c}$ are bound, to form a 4-to 7-membered cyclic amino group (optionally having 1 to 3 substituents independently selected from a hydroxy group, a halogen atom, an amino group, a lower alkyl group, a lower alkoxy group, an aryl group, an aryloxy group, an akylamino group, a cyclic amino group, a benzyloxy group and a heteroaryl group on the ring), and P and $R^{53a}$ are as defined above).

[Chem.27]

[Scheme 2B]

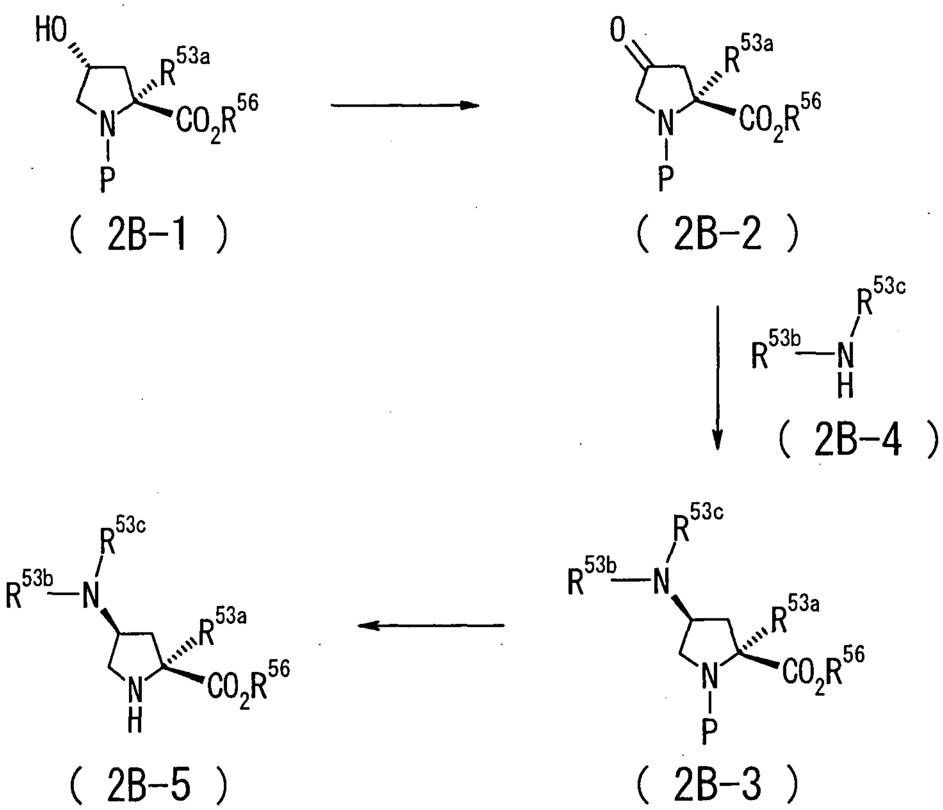

( 2B-1 ) ( 2B-2 ) ( 2B-4 ) ( 2B-5 ) ( 2B-3 )

The known oxidizing reaction of converting a secondary hydroxy group into ketone can be applied to a process of converting a hydroxy-L-proline ester derivative represented by the formula (2B-1) into a 4-oxo-L-proline ester derivative represented by the formula (2B-2), and a mild oxidizing method of preventing isomerization of configuration of a pyrrolidine ring 2-position asymmetric carbon to which an ester group is bound is preferable. For example, an oxidizing method can be performed by applying oxidizing condition such as Swern oxidation (Giordano, C.; Cavicchioli, S.; Levi, S.; Villa, M. ; J Org. Chem. 56 (21), 6114-6118 ((1991), Konradi, A. W.; Pedersen, S. F.; J Org. Chem. 57 (1), 28-32 (1992) etc.), an oxidizing method using hypochlorous acid-tempo, a radical reaction using 2,2,6,6-tetramethyl-1-piperidinyloxy, free radical (Jurczak, J.; Prokopowicz, p.; Golebiowski, A.; Tetrahedron Letters, 34 (44), 7107-7110 (1993) etc.), an oxidizing method using DMSO-cyanyl chloride (De Luca, L.; Giacomelli, G.; Porcheddu, A.; J.Org. Chem., 66 (23), 7907-7909, (2001), De Luca, L.; Giacomelli, G.; Porcheddu, A.; Org Letters, 3 (19), 3041-3043 (2001)), and an oxidizing method

using a sulfur trioxide pyridine complex (Konradi, A. W.; Pedersen, S. F.; J.Org. Chem., 55 (15), 4506-4508, (1990), Takemoto, Y.; Matsumoto, t.; ITO, Y.; Terashima, S.; Chem.Pharm. Bull., 39 (9), 2425-2428, (1991)).
Then, as reaction condition and a reducing agent to be used upon conversion of a 4-oxo-L-proline ester derivative represented by the formula (2B-2) and an amine compound (2B-4) into a proline derivative represented by the formula (2B-3) by introduction of an amino group substituent under stereoselective reducing condition, sodium triacetoxyborohydride, or sodium cyanoborohydride is preferable, a reaction example using sodium triacetoxyborohydride includes Gordon, D. W.; Steele, J.; Bioorg. Med. Chem. Letters, 5 (1), 47-50 (1995), and an example using sodium cyanoborohydride includes Kelley, J. L. ; Mclean, E. W.; Ferris, R. M.; Howard, J. L.; Jmed. Chem., 33 (7), 1910-1914 (1990). Using similar conditions thereto, a proline derivative represented by the formula (2B-3) can be produced. In addition, a protecting group for a nitrogen atom on a pyrrolidine ring of a proline derivative represented by the formula (2B-3) can be converted into free amine, or hydrochloride or trifluoroacetate thereof, or a compound represented by the formula (2B-4).

Compounds shown in [Scheme 3], [Scheme 3A] and [Scheme 3B] are commercially available, or can be produced by the known method (in Scheme, P, $R^{52}$, $R^{53}$, $R^{53a}$ and $R^{56}$ are as defined above).

[Chem.28]

[Scheme 3]

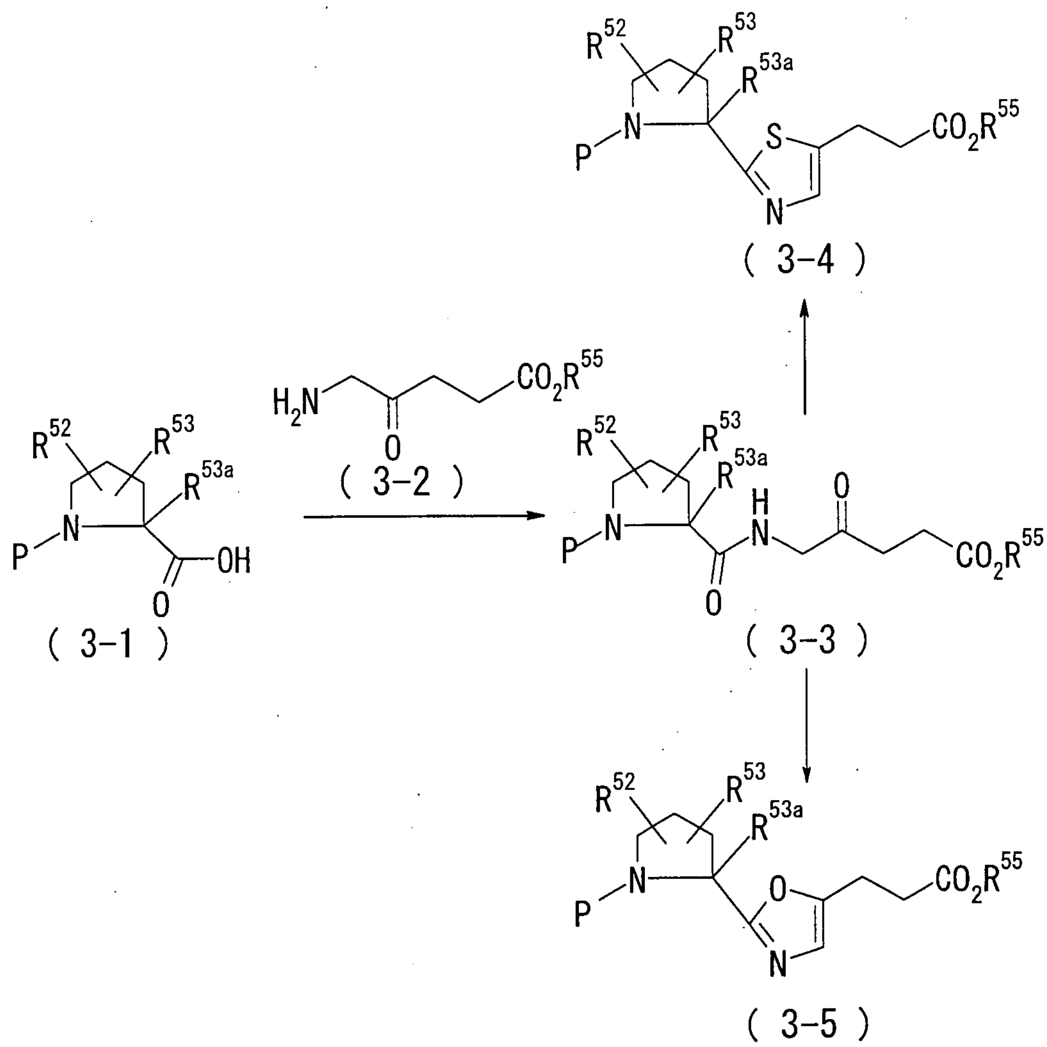

( 3-4 )

( 3-2 )

( 3-1 )

( 3-3 )

( 3-5 )

A compound represented by the formula (3-3) can be obtained by reacting carboxylic acids represented by the

formula (3-1), and amines or their salts represented by the formula (3-2) which is commercially available, or can be produced by the known per se or known method at a temperature in a range of -20˚C to a boiling point of solvent, preferably in a range of 0˚C to room temperature using a condensing agent which is 1-ethyl-3-(3-dimethylaminopropyl) carbodiimide hydrochloride, N,N-dicyclohexylcarbodiimide, N,N-carbonyldiimidazole or a similar compound, in an inert halogenated hydrocarbon-based solvent such as methylene chloride, an inert hydrocarbon-based solvent such as toluene, an inert ether-based solvent such as tetrahydrofuran, or an inert polar solvent such as N,N-dimethylformamide according to the known condensing method.
Further, by treatment with a Lawesson's reagent at a temperature in a range of room temperature to a boiling point of a solvent, preferably in a range of 60˚C to 90˚C in an inert hydrocarbon-based solvent such as toluene according to the known cycling method, a thiazole compound (3-4) can be produced. On the other hand, by treating a compound (3-3) with phosphorus oxychloride, an oxazole compound represented by the formula (3-5) can be also produced.
[Scheme 3A] shows a process for producing (3A-4) below. Carboxylic acids represented by the formula (3-1) can be derivatized into an aldehyde compound represented by the formula (3A-1) by the known per se or known method. That is, carboxylic acids represented by the formula (3-1) can be converted into an alcohol at a temperature in a range of 0˚C to a boiling point of a solvent, preferably at a boiling point of a solvent using borane or a similar reducing agent in an inert ether-based solvent such as tetrahydrofuran, and converted into an aldehyde compound (3A-1) by performing general oxidizing condition, preferably Swern oxidation or the like shown in the previous process of [Scheme 2B]. The aldehyde represented by the formula (3A-1) can be derivatized into an oxime compound represented by the formula (3A-2) by treatment with hydroxylamine hydrochloride and sodium acetate in a mixed solvent of methanol and water. From the oxime compound represented by the formula (3A-2), an isoxazole compound represented by the formula (3A-4) can be produced by treatment with an ester (3A-3) which is commercially available or can be produced by the known method, triethylamine and an aqueous sodium hypochlorite solution at a range of 0˚C to room temperature, preferably at room temperature in an inert halogenated hydrocarbon-based solvent such as methylene chloride.

[Chem.29]

[Scheme 3A]

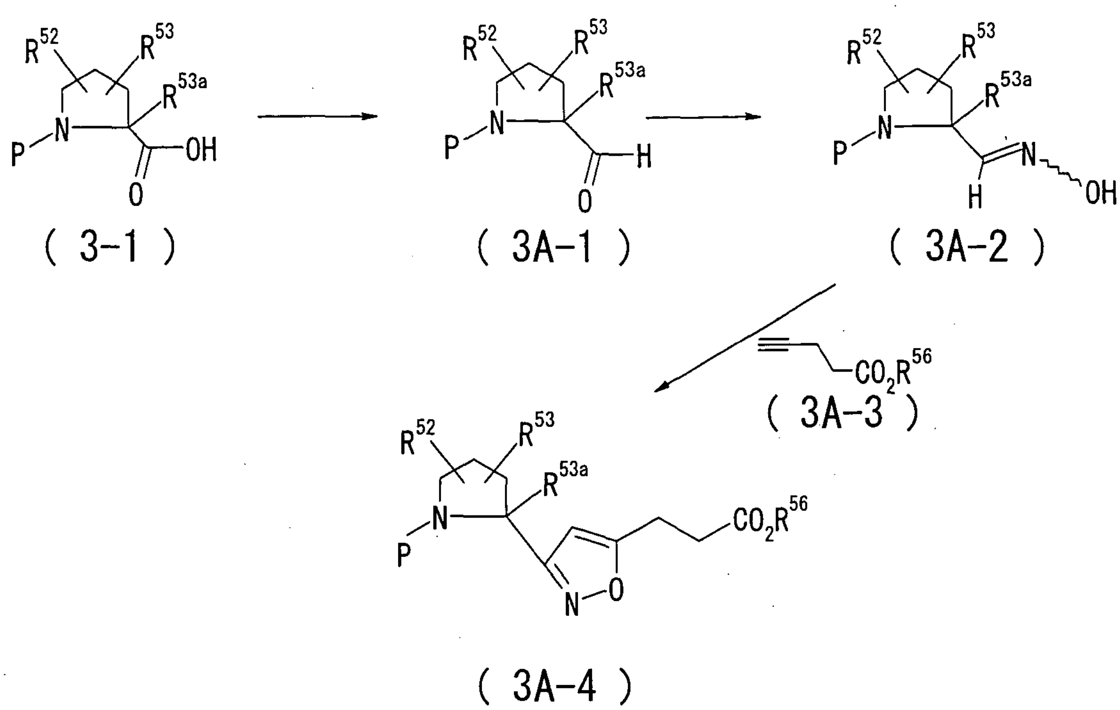

( 3-1 ) ( 3A-1 ) ( 3A-2 ) ( 3A-3 ) ( 3A-4 )

In the following [Scheme 3B], a tetrazolepropionic acid ester derivative represented by the formula (3B-4) can be produced by the known process. A carboxylic acid derivative represented by the formula (3-1) can be converted into an amide derivative represented by the formula (3B-1) by the known method via an active ester. For example, the

carboxylic acid derivative (3-1) can be treated with chloroformic acid methyl ester or chloroformic acid ethyl ester at a temperature in a range of -20°C to room temperature, preferably in a range of 0°C to 10°C in the presence of a stoichiometric amount of organic amine such as triethylamine and N-methylmorpholine in an inert ether-based solvent such as tetrahydrofuran, or an inert hydrocarbon-based solvent such as toluene to convert into a mixed ester derivative, and this can be treated with commercially available concentrated aqueous ammonia (27%) to convert into an objective amide derivative (3B-1). Then, in conversion of the amide derivative (3B-1) into a cyano derivative represented by the formula (3B-2), the known method of converting primary amide into a cyano group by a dehydrating reaction can be applied. For example, an amide derivative (3B-1) can be converted into an objective cyano derivative (3B-2) by treating the amide derivative (3B-1) with p-toluenesulfonyl chloride or trifluoroacetic anhydride as a dehydrating agent at a temperature in a range of 0°C to a boiling point of a solvent in the presence of pyridine, or pyridine at stoichiometric equivalent to 5 equivalents in dioxane. Then, conversion of the cyano derivative (3B-2) into a tetrazole derivative represented by the formula (3B-3) can be performed under the known reaction condition. For example, the cyano derivative (3B-2) is treated with aluminum azide or ammonium azide which has been prepared in situ in a system from sodium azide and aluminum chloride or ammonium chloride in advance, at a temperature in a range of room temperature to 120°C, preferably in a range of 80°C to 110° C usually for 1 hour to 6 hours in an inert polar solvent such as N,N-dimethylformamide and, after completion of the reaction, the produced aluminum salt or ammonium salt of tetrazole can be neutralized with hydrochloric acid to convert into a free tetrazole derivative (3B-3). Alternatively, a tetrazole derivative (3B-3) can be produced by a simple method using an aqueous solvent according to the method of K. B. Sharpless (Organic Letters, 2002, Vol. 4, No. 15, 2525-2527). Then, in conversion into a tetrazolepropionic acid ester derivative (3B-4), the known alkylating reaction can be applied to a free tetrazole derivative (3B-3). For example, the tetrazole derivative (3B-3) can be converted into a tetrazolepropionic acid ester derivative (3B-4) by treatment with a 3-bromopropionic acid ester at a temperature in a range of room temperature to 90°C for 3 hours to 24 hours in the presence of anhydrous potassium carbonate in an inert polar solvent such as N,N-dimethylformamide.

[Chem.30]

[Scheme 3B]

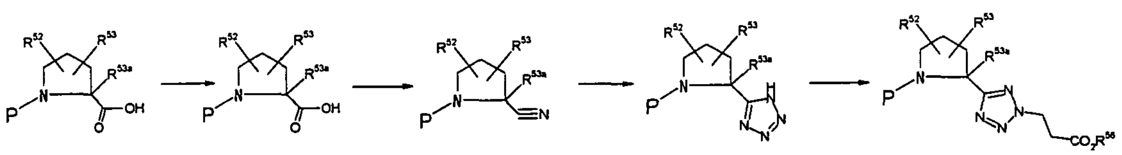

( 3-1 ) ( 3B-1 ) ( 3B-2 ) ( 3B-3 ) ( 3B-4 )

The thus obtained present compound or a salt thereof is useful as a primary component of medicaments for various diseases, for example, a preventive and/or a therapeutic. For example, as shown in Test Examples later, the compound selectively inhibits binding of a cell adhesion molecule to VLA-4 and, at the same time, has the high oral absorbing property. Therefore, the present compound or a salt thereof is useful as a preventive and/or a therapeutic agent for diseases caused by cell adhesion, for example, diseases caused by cell adhesion of VLA-4, that is, various diseases mediated with chemotaxis and adhesion of leukocyte, for example, inflammatory disease, autoimmune disease, cancer metastasis, bronchial asthma, nasal obstruction, diabetes, arthritis, psoriasis, multiple sclerosis, inflammatory bowel disease and rejection reaction at transplantation.

A medicament of the present invention can be administered by various methods including oral administration.

Alternatively, when formulated into an injectable, the medicament can be also administered by any method of intravenous injection, intramuscular injection, subcutaneous injection and the like.

Regarding a method of preparing such preparations, an appropriate preparation is selected depending on an administration method, and can be prepared by various methods for preparing a preparation which are normally used.

As an oral preparation, there can be exemplified tablets, powders, granules, capsules, solutions, syrups, elixirs, oily or aqueous suspensions and the like. As an injectable, a stabilizer, an antiseptic, and a solubilizer may be used in a preparation in some cases, a solution optionally containing these adjuvants is accommodated into a container, and optionally is formulated into a solid preparation with a lyophilizing agent or the like, which is used as a preparation prepared upon use. In addition, an example of a liquid preparation include solutions, suspensions, emulsions and the

like and, when these preparations are prepared, a suspending agent, an emulsifier or the like may be used as an additive.

It is desirable that a medicament containing the present compound is administered as a compound to a human adult once daily, and this is repeated at an appropriate interval. A dose is in a range of 0.01 mg to 2000 mg, preferably in a range of 0.1 mg to 1000 mg.

Further, in the medicament of the present invention, if necessary, an anti-inflammatory agent, an anti-arthritis drug, an adrenal cortical steroid (corticosteroid), an immunosuppressor, an anti-psoriasis drug, a bronchodilator, an anti-bronchial asthma drug or an anti-diabetes drug can be used in combination in such a range that the effect of the present invention is not deteriorated.

The present invention includes a method of preventing and/or treating the aforementioned diseases, comprising administering the present compound or a salt thereof.

Further, the present invention includes use of the present compound or a salt thereof for preparing the aforementioned medicament.

Examples

The present invention will be specifically explained below by way of Examples.

Symbols of "IR", "NMR" and "MS" in Examples mean "infrared absorbing spectrum", "nuclear magnetic resonance spectrum" and "mass spectrometry", respectively. A ratio of elution solvents described in a place of separation and purification by chromatography means a volumetric ratio. "IR" means measurement by a KBr tableting method, or an ATR method. Ameasuring solvent is meant in a parenthesis of "NMR" and, in all cases, TMS "tetramethylsilane" was used as an internal standard substance. In addition, "Anal. Calcd for a rational formula" indicates a calculated value of elementary analysis, a measured value is described after "Found". In the present specification, following abbreviations were used. $BH_3$ DMS: borane dimethyl sulfide complex

Boc: tert-butoxycarbonyl
$Boc_2O$: di-tert-butyl bicarbonate
Bm: benzyl
$CDCl_3$: heavy chloroform
DIAD: diisopropyl azodicarboxylate
DMF: N,N-dimethylformamide
DMSO: dimethyl sulfoxide
EDC: 1-ethyl-3-(3-dimethylaminopropyl)carbodiimide hydrochloride
HOBt: 1-hydroxybenzotriazole
Lawesson's Reagent: 2,4-bis(4-methoxyphenyl)-1,3-dithia-2,4-diphosphoethane-2,4-disulfide
TBDMS: tert-butyl dimethylsilyl
TBDMS-Cl: tert-butyl dimethylsilyl chloride
THF: tetrahydrofuran
TLC: thin-layer chromatography
Z: benzyloxycarbonyl
TEMPO: 2,2,6,6-tetramethyl-1-piperidinyloxy, free radical

Example 1

(1-Benzyloxycarbonyl-(4S)-methoxy-(2S)-pyrrolidinyl)carboxylic acid methyl ester

[Chem.31]

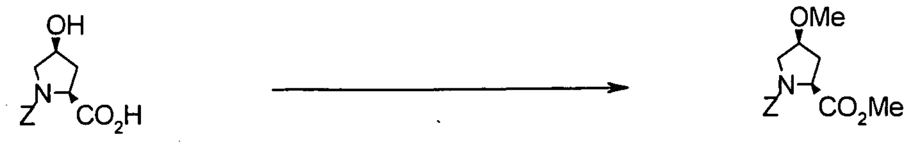

(1-Benzyloxycarbonyl-(4S)-hydroxy-(2S)-pyrrolidinyl)carboxylic acid (25 g, 94.3 mmol) was dissolved in DMF (70 ml), and sodium hydride (60% in oil) (7.73 g, 193.3 mmol) was added at 0°C . After stirred at room temperature for 30 minutes, methyl iodide (12.9 ml, 207. 5 mmol) was added dropwise. After stirred at room temperature for 2 hours,

water was added, followed by extraction with ethyl acetate two times. The combined extracts were dried with anhydrous sodium sulfate, and concentrated under reduced pressure. The resulting residue was purified by column chromatography using silica gel, and the title compound (18g, 65%) was obtained as a colorless oily substance from a hexane: ethyl acetate (3:1-2:3)-eluted part.

$^1$H-NMR ($CDCl_3$) δ: 2.15-2.39 (2H, m), 3.27-3.28 (3H, m), 3.54-3.79 (5H, m), 3.91-4.01 (1H, m), 4.40-4.55 (1H, m), 5.02-5.25 (2H, m), 7.26-7.39 (5H, m).

(1-tert-butoxycarbonyl-(4S)-methoxy-(2S)-pyrrolidinyl)carboxylic acid methyl ester

[Chem. 32]

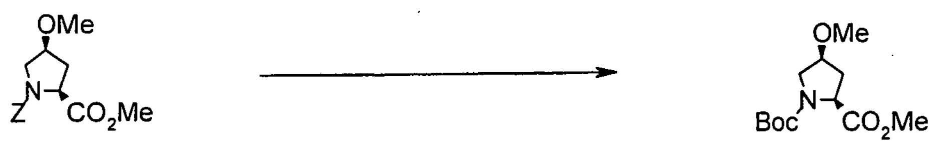

(1-Benzyloxycarbonyl-(4S)-methoxy-(2S)-pyrrolidinyl) carboxylic acid methyl ester (14 g, 47.8 mmol) was dissolved in ethanol (300 ml), 10% Pd-C (1.4 g) was added, and hydrogenation was performed at room temperature overnight. A catalyst was filtered to remove, and the filtrate was concentrated under reduced pressure. The resulting residue was dissolved in methylene chloride (200 ml), and $Boc_2O$ (12.5 g, 57.3 mmol) and triethylamine (13.3 ml, 95.6 mmol) were successively added at room temperature. After stirred for 3 hours and a half, the mixture was concentrated under reduced pressure, and the residue was purified by column chromatography using silica gel to obtain the title compound (11.5 g, 93%) as a pale yellow oily substance from a hexane: ethyl acetate (4:1-1:2)-eluted part.

$^1$H-NMR ($CDCl_3$) δ: 1.42 and 1.48 (total 9H, each s), 2.20-2.36 (2H, m), 3.28 (3H, s), 3.45-3.69 (2H, m), 3.73 (3H, s), 3.90-3.96 (1H, m), 4.45-4.27 (1H, m).

(1-tert-butoxycarbonyl-(4S)-methoxy-(2S)-pyrrolidinyl)carboxylic acid

[Chem. 33]

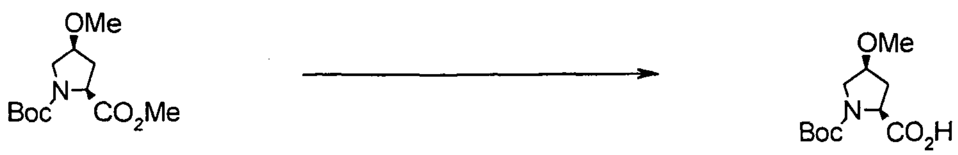

(1-tert-butoxycarbonyl-(4S)-methoxy-(2S)-pyrrolidinyl)carboxylic acid methyl ester (11.5 g, 44.8 mmol) was dissolved in a mixed solution of THF (200 ml) and methanol (100 ml), a 1N aqueous sodium hydroxide solution (90 ml, 90 mmol) was added at room temperature, and the mixture was stirred overnight. THF and methanol were removed under reduced pressure, and this was made weakly acidic with 1N hydrochloric acid, followed by extraction with ethyl acetate two times. The combined extracts were dried with anhydrous sodium sulfate, and concentrated under reduced pressure. The residue was crystallized from hexane and ethyl acetate to obtain the title compound (9.6 g, 87%) as a colorless needle-shaped crystal.

5-[(1-tert-butoxycarbonyl-(4S)-methoxy-(2S)-pyrrolidinyl)carbonylamino]levulinic acid methyl ester

[Chem.34]

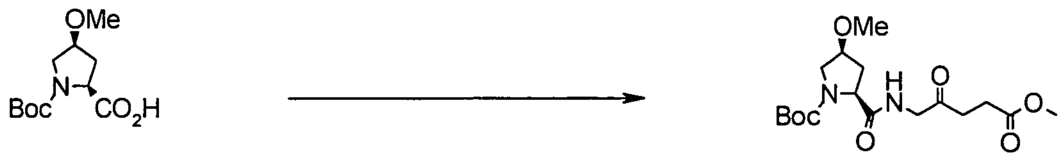

(1-tert-butoxycarbonyl-(4S)-methoxy-(2S)-pyrrolidinyl)carboxylic acid (490 mg, 2.0mmol), 5-aminolevulinic acid methyl ester hydrochloride (364 mg, 2.0 mmol), HOBt (270 mg, 2.0mmol) and EDC (576mg, 3.0mmol) were dissolved in methylene chloride (25 ml), triethylamine (1.6 ml) was added, and this was stirred at room temperature overnight. Water was added to the reaction solution, followed by extraction with methylene chloride two times. The combined extracts were dried with anhydrous sodium sulfate, and concentrated under reduced pressure. The resulting residue was purified by column chromatography using silica gel to obtain the title compound (740 mg, 99%) as a colorless oily substance from a methylene chloride: methanol (97:3)-eluted part.

$^1$H-NMR ($CDCl_3$) δ: 1.47 (9H, br s), 2.02-2.49 (2H, m), 2.60-2.69 (2H, m), 2.72-2.77 (2H, m), 3.29 (3H, s), 3.45-3.53 (1H, m), 3.68 (3H, s), 3.90-3.95 (1H, m), 4.13-4.18 (2H, m), 4.23-4.40 (2H, m).

3-[2-(1-tert-butoxycarbonyl-(4S)-methoxy-(2S)-pyrrolidinyl)-5-thiazolyl]propionic acid methyl ester

[Chem.35]

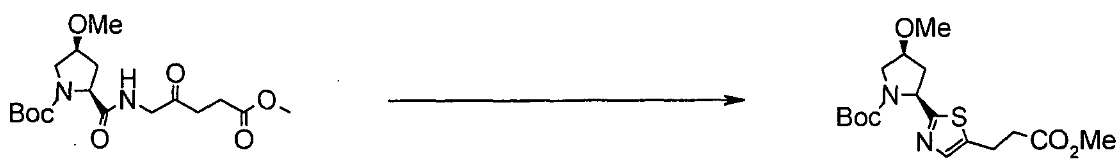

5-[(1-tert-butoxycarbonyl-(4S)-methoxy-(2S)-pyrrolidinyl)carbonylamino]levulinic acid methyl ester (740 mg, 1.99 mmol) was dissolved in toluene (37 ml), a Lawesson's reagent (885 mg, 2.19 mmol) was added, and the mixture was stirred at 90°C for 1 hour. Water was added to the reaction solution, followed by extraction with ethyl acetate two times. The combined extracts were dried with anhydrous sodium sulfate, and concentrated under reduced pressure. The resulting residue was purified by preparative TCL (Merck, Silica gel 60, $F_{254}$, 2mm; developed with methanol: methylene chloride = 5:95, eluted with methanol: methylene chloride =3 : 7) to obtain the title compound (640 mg, 87%) as a pale yellow oily substance.

$^1$H-NMR ($CDCl_3$) δ: 1.33 and 1.49 (total 9H, each br s), 2.32-2.59 (2H, m), 2.65 (2H, t, J = 7.6 Hz), 3.12-3.20 (5H, m), 3.50-3.78 (5H, m), 3.98-4.01 (1H, m), 5.08-5.19 (1H, m), 7.34 (1H, s).

3-[2-((4S)-methoxy-(2S)-pyrrolidinyl)-5-thiazolyl]propionic acid methyl ester hydrochloride

[Chem.36]

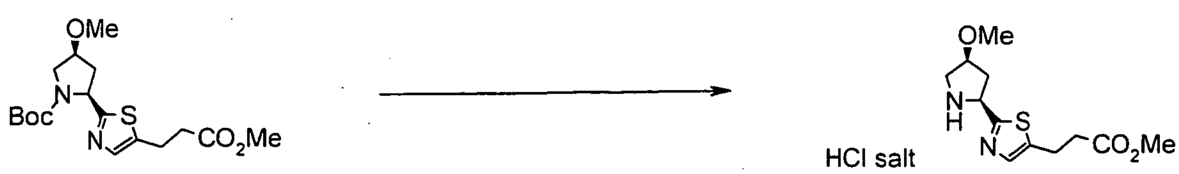

3-[2-(1-tert-butoxycarbonyl-(4S)-methoxy-(2S)-pyrrolidinyl)-5-thiazolyl]propionic acid methyl ester (640 mg, 1.73 mmol) was dissolved in a 4N hydrochloric acid dioxane solution (20ml). The solution was stirred at room temperature for 1 hour and a half. After concentration under reduced pressure, the residue was used in the next reaction without purification.

3-[2-[1-[2,5-Dichloro-4-(1-methyl-3-indolylcarbonylamino)phenylacetyl]-(4S)-methoxy-(2S)-pyrrolidinyl]-5-thiazolyl]propionic acid methyl ester

[Chem. 37]

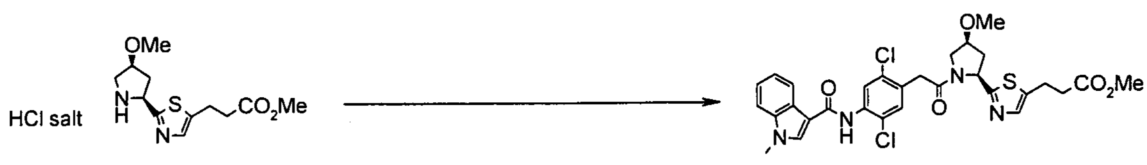


3-[2-((4S)-methoxy-(2S)-pyrrolidinyl)-5-thiazolyl]propionic acid methyl ester hydrochloride (1.3 mmol), 2,5-dichloro-4-(1-methyl-3-indolylcarbonylamino)phenylac etic acid (490 mg, 1.3 mmol), EDC (374 mg, 1.95 mmol), HOBt (175 mg, 1.3 mmol) and triethylamine (1.0 ml, 7.8 mmol) were dissolved in methylene chloride (30 ml), and the solution was stirred at room temperature overnight. The reaction solution was concentrated under reduced pressure, and the residue was purified by preparative TLC (Merck, Silica gel 60, $F_{254}$, 2mm; developed with methanol: methylene chloride = 8.92, eluted with methanol : methylene chloride = 3 : 7) to obtain the title compound (600 mg, 73%) as a faded amorphous substance.

$^1$H-NMR ($CDCl_3$) $\delta$: 2.32-2.75 (4H, m), 3.01-3.17 (2H, m), 3.18 and 3.22 (total 3H, eachs), 3.49-4.13 (11H, m), 5.40-5.52 (1H, m), 7.31-7.44 (4H, m), 7.50 (1H, s), 7.79 and 7. 81 (total 1H, each s), 8.12-8.15 (1H, m), 8.23 and 8.26 (total 1H, each s), 8.75 and 8.82 (total 1H, each s).

3-[2-[1-[2,5-Dichloro-4-(1-methyl-3-indolylcarbonylamino)phenylacetyl]-(4S)-methoxy-(2S)-pyrrolidinyl]-5-thiazolyl]propionic acid

[Chem. 38]

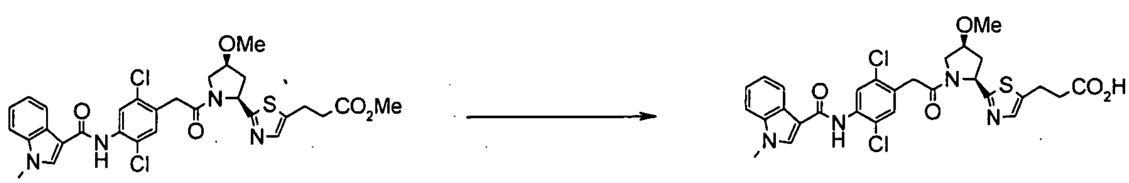

3-[2-[1-[2,5-Dichloro-4-(1-methyl-3-indolylcarbonylamino)phenylacetyl]-(4S)-methoxy-(2S)-pyrrolidinyl]-5-t hiazolyl] propionic acid methyl ester (600 mg, 0.95 mmol) was dissolved in THF (10 ml), 1N aqueous sodium hydroxide solution (2.0 ml, 2.0 mmol) and methanol (5.0 ml) were added, and the mixture was stirred at room temperature overnight. The reaction solution was concentrated under reduced pressure, a small amount of water was added, and the solution was made neutral with 1N hydrochloric acid, followed by extraction with methylene chloride two times. The combined extracts were dried with anhydrous sodium sulfate, and concentrated under reduced pressure. The residue was purified by preparative TLC (Merck, Silica gel 60, $F_{254}$, 2mm; developed with methanol: methylene chloride, eluted with methanol : methylene chloride = 3 : 7) to obtain the title compound (460 mg, 78%) as a white powder.

$^1$H-NMR (DMSO-$d_6$) $\delta$: 2.37-2.61 (4H, m), 2.92-3.03 (2H, m), 3.09 and 3.12 (total 3H, each s), 3.54-4.13 (8H, m), 5.22-5.54 (1H, m), 7.19-7.59 (5H, m), 7.85 and 7.91 (total 1H, each s), 8.13-8.17 (1H, m), 8.30 and 8.31 (total 1H, each s), 9.41 and 9.42 (total 1H, each s).

IR (ATR) $cm^{-1}$ : 2929, 1652, 1500, 1373, 1218, 1099, 1076, 744.

MS (LC-ESI) m/z : 615 ($M^{+}$+1).
Anal. Calcd for $C_{29}H_{28}Cl_2N_4O_5S \cdot 0.5H_2O$: C, 55.77; H, 4.68; N, 8.97. Found: C, 55.53; H, 4.53; N, 8.79.

Compounds of Examples 2 to 11 shown below were produced by the same synthesizing method as that of Example 1.

Example 2

3-[2-[1-[5-Chloro-2-fluoro-4-(1-methyl-3-indolylcarbonyl)aminophenylacetyl]-(4S)-methoxy-(2S)-pyrrolidinyl]-5-thiazolyl]propionic acid

[Chem.39]

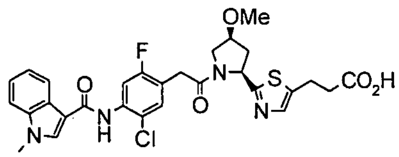

$^{1}$H-NMR (DMSO-$d_6$) δ: 2.37-2.61 (4H, m), 2.90-3.02 (2H, m), 3.10 (3H, s), 3.66-4.17 (8H, m), 5.27-5.54 (1H, m), 7.20-7.73 (6H, m), 8.15 (1H, d, J = 7.8 Hz), 8.32 and 8.33 (total 1H, each s), 9.36 (1H, s).
IR (ATR) $cm^{-1}$ : 2929, 1654, 1513, 1400, 1220, 1182, 744.
MS (LC-ESI) m/z : 599 ($M^{+}$+1).
Anal. Calcd for $C_{29}H_{28}ClFN_4O_5S \cdot 0.5H_2O$: C, 57.28; H, 4.81; N, 9.21. Found: C, 57.07; H, 4.86; N, 9.00.

Example 3

3-[2-[1-[[4-[(3-Benzo[b]thienylcarbonyl)amino]-2,5-dichlorophenyl]acetyl]-(4S)-methoxy-(2S)-pyrrolidinyl]-5-thiazolyl]propionic acid

[Chem.40]

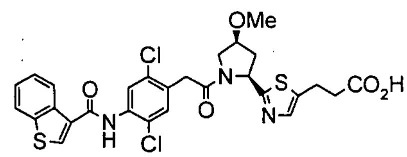

$^{1}$H-NMR (DMSO-$d_6$) δ: 2.37-2.54 (4H, m), 2.93-3.01 (2H, m), 3.10-3.12 (total 3H, eachs), 3.51-4.12 (5H, m), 5.21-5.52 (1H, m), 7.37-7.79 (5H, m), 8.09 (1H, d, J = 8.6 Hz), 8.45 (1H, d, J = 9.3 Hz), 8.67 and 8.69 (total 1H, each s), 10.22 and 10.31 (total 1H, each s).
IR (ATR) $cm^{-1}$ : 2929, 1646, 1502, 1079.
MS (LC-ESI) m/z : 618 ($M^{+}$+1).
Anal. Calcd for $C_{28}H_{25}Cl_2N_3O_5S_2 \cdot 0.5H_2O$: C, 53.59; H, 4.18; N, 6.70. Found: C, 53.50; H, 4.25; N, 6.65.

Example 4

3-[2-[1-[4-[(3-Benzo(b)thienylcarbonyl)amino]-5-chloro-2-fluorophenylacetyl]-(4S)-methoxy-(2S)-pyrrolidinyl]-5-thiazolyl]propionic acid

[Chem. 41]

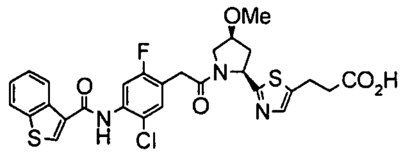

$^1$H-NMR (DMSO-$d_6$) δ: 2.40-2.60 (4H, m), 2.97-3.03 (2H, m), 3.10 (3H, s), 3.44-4.12 (5H, m), 5.21-5.56 (1H, m), 7.34-7.61 (5H, m), 8.09-8.10 (1H, m), 8.44-8.46 (1H, m), 8.64-8.65 (1H, m), 10.09 and 10.12 (total 1H, each s).
IR (ATR) $cm^{-1}$ : 2927, 1650, 1515, 1400, 1216, 765.
MS (LC-ESI) m/z : 602 ($M^{+}$+1).
Anal. Calcd f or $C_{28}H_{25}ClFN_3O_5S_2 \cdot 0.5H_2O$: C, 55.03; H, 4.29; N, 6.88. Found: C, 55.19; H, 4.26; N, 6.72.

Example 5

3-[2-[1-[4-[(1- Benzofuran- 3- ylcarbonyl) amino]- 2,5- dichlorophenylacetyl]-(4S)-methoxy-(2S)-pyrrolidinyl]- 5-thiazolyl]propionic acid
[Chem. 42]

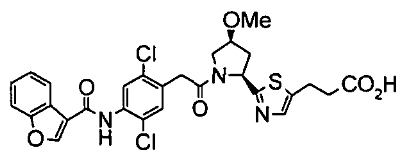

$^1$H-NMR (DMSO-$d_6$) δ: 2.38-2.61 (4H, m), 2.94-3.07 (2H, m), 3.10 and 3.12 (total 3H, each s), 3.50-4.15 (5H, m), 5.20-5.56 (1H, m), 7.35-7.80 (6H, m), 8.08-8.09 (1H, m), 8.81 and 8.83 (total 1H, each s), 10.03 and 10.06 (total 1H, each s).
IR (ATR) $cm^{-1}$ : 2929, 1504, 1122, 1079, 748.
MS (LC-ESI) m/z : 602 ($M^{+}$+1).
Anal. Calcd for $C_{28}H_{25}Cl_2N_3O_6S \cdot 0.3H_2O$: C, 55.32; H, 4.24; N, 6.91. Found: C, 55.25; H, 4.18; N, 6.86.

Example 6

3-[2-[1-[4-[(1-Benzofuran-3-ylcarbonyl)amino]-5-chloro-2-fluorophenylacetyl]-(4S)-methoxy-(2S)-pyrrolidinyl]-5-thiazolyl]propionic acid
[Chem. 43]

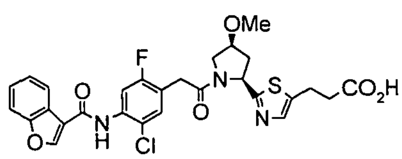

$^1$H-NMR (DMSO-$d_6$) δ: 2.39-2.54 (4H, m), 2.92-3.03 (2H, m), 3.10 (3H, s), 3.50-4.15 (5H, m), 5.22-5.55 (1H, m), 7.34-7.72 (6H, m), 8.09 (1H, dd, J= 6.7, 1.8 Hz), 8.86 (1H, s), 10.18-10.11 (1H, m).
IR (ATR) $cm^{-1}$ : 1648, 1521, 1402, 1122, 746.
MS (LC-ESI) m/z : 586 ($M^{+}$+1).
Anal. Calcd for $C_{28}H_{25}ClFN_3O_6S{\cdot}0.8H_2O$: C, 56.01; H, 4.47; N, 7.00. Found: C, 56.05; H, 4.33; N, 6.94.

Example 7

3-[2-[1-[5-Chloro-2-fluoro-4-(1-methyl-3-indazolylcarbonyl)aminophenylacetyl]-(4S)-methoxy-(2S)-pyrrolidinyl]-5-thiazolyl]propionic acid

[Chem. 44]

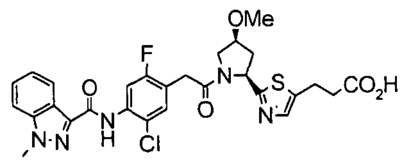

$^1$H-NMR (DMSO-$d_6$) δ: 2.38-2.57 (4H, m), 2.91-3.01 (2H, m), 3.085 and 3.092 (total 3H, each s), 3.67-4.29 (8H, m), 5.24-5.52 (1H, m), 7.30-7.55 (4H, m), 7.81 (1H, d, J = 8.6 Hz), 8.04-8.08 (1H, m), 8.20 (1H, d, J = 8.3 Hz), 9.68 and 9.72 (total 1H, each s).
IR (ATR) $cm^{-1}$ : 2929, 1527, 1403, 1203, 779, 744.
MS (LC-ESI) m/z : 600 ($M^+$+1).
Anal. Calcd for $C_{28}H_{27}ClFN_5O_5S \cdot H_2O$: C, 54.41; H, 4.73; N, 11.33. Found: C, 54.66; H, 4.54; N, 11.24.

Example 8

3-[2-[1-[2-(5-Fluoro-2-methylphenylamino)-7-fluoro-1,3-benzoxazol-6-yl]-(4S)-methoxy-(2S)-pyrrolidinyl]-5-thiazolyl]propionic acid

[Chem. 45]

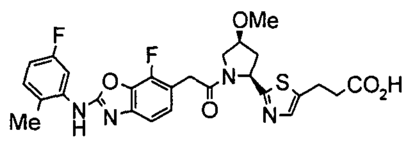

$^1$H-NMR (DMSO-$d_6$) δ: 2.30 (3H, s), 2.38-2.58 (4H, m), 2.92-3.04 (2H, m), 3. 09 (3H, s), 3.46-4.13 (5H, m), 5.22-5.56 (1H, m), 6.85-7.47 (5H, m), 7.89-7.95 (1H, m).
IR (ATR) $cm^{-1}$ : 2935, 1637, 1577, 1452, 1280, 1068.
MS (LC-ESI) m/z : 557 ($M^+$+1).
Anal. Calcd for $C_{27}H_{26}F_2N_4O_5S \cdot 0.2H_2O$: C, 57.89; H, 4.75; N, 10.00. Found: C, 57.82; H, 4.72; N, 9.83.

Example 9

3-[2-[1-[[7-Fluoro-2-(5-fluoro-2-methoxyphenylamino)-1,3-benzoxazol-6-yl]acetyl]-(4S)-methoxy-(2S)-pyrrolidinyl]-5-thiazolyl]propionic acid

[Chem. 46]

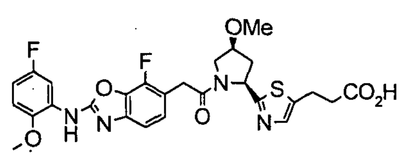

$^1$H-NMR (DMSO-$d_6$) δ: 2.36-2.59 (4H, m), 2.92-3.01 (2H, m), 3.089 and 3.094 (total 3H, each s), 3.48-4.20 (8H, m), 5.25-5.54 (1H, m), 6.86-7.45 (5H, m), 8.16-8.19 (1H, m).
IR (ATR) $cm^{-1}$ : 2935, 1641, 1577, 1214, 711.
MS (LC-ESI) m/z : 573 ($M^+$+1).
Anal. Calcd for $C_{27}H_{26}F_2N_4O_6S \cdot 0.5H_2O$: C, 55.76; H, 4.68; N, 9.63. Found: C, 55.59; H, 4.79; N, 9.59.

Example 10

3-[2-[1-[[7-Fluoro-2-(2-methoxyphenylamino)-1,3-be nzoxazol-6-yl]acetyl]-(4S)-methoxy-(2S)-pyrrolidinyl]-5-thiazolyl]propionic acid
[Chem.47]

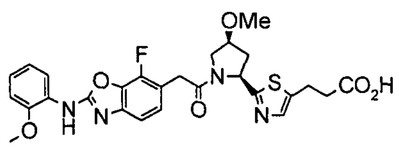

$^1$H-NMR (DMSO-$d_6$) δ: 2.33-2.55 (4H, m), 2.91-3.03 (2H, m), 3.087 and 3.091 (total 3H, each s), 3.46-4.10 (8H, m), 5.22-5.54 (1H, m), 6.90-7.44 (6H, m), 8.11-8.13 (1H, m), 9.86 (1H, br s).
IR (ATR) $cm^{-1}$ : 2933, 1635, 1577.
MS (LC-ESI) m/z : 555 ($M^+$+1).
Anal. Calcd for $C_{27}H_{27}FN_4O_6S \cdot 0.5H_2O$: C, 57.54; H, 5.01; N, 9.94. Found: C, 57.40; H, 5.04; N, 9.78.

Example 11

*3-[2-[1-[7-Fluoro-2-(1-methylindol-3-yl)-1,3-benzoxazol-6-ylacetyl]-(4S)-methoxy-(2S)-pyrrolidinyl]-5-thiazolyl]propionic acid
[Chem.48]

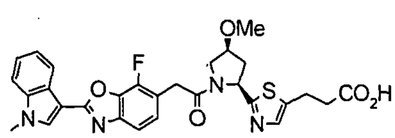

$^1$H-NMR (DMSO-$d_6$) δ: 2.39-2.61 (4H, m), 2.94-3.05 (2H, m), 3.09 and 3.10 (total 3H, each s), 3.48-4.15 (8H, m), 5.22-5.59 (1H, m), 7.04-7.65 (6H, m), 8.27-8.31 (1H, m), 8.43 and 8.45 (total 1H, each s).
IR (ATR) $cm^{-1}$ : 2931, 1625, 1581, 1095, 742.
MS (LC-ESI) m/z : 563 ($M^+$+1).
Anal. Calcd for $C_{29}H_{27}FN_4O_5S \cdot 0.2H_2O$: C, 61.52; H, 4.88; N, 9.89. Found: C, 61.34; H, 4.86; N, 9.77.

Example 12

(1-Benzyloxycarbonyl-(4S)-methoxy-(2S)-pyrrolidinyl)carboxylic acid
[Chem.49]

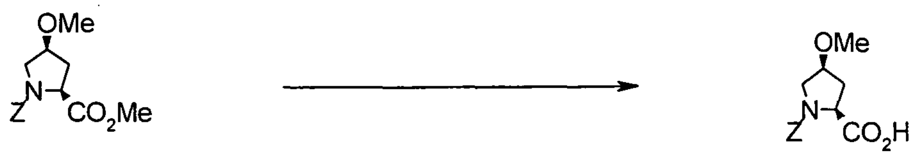

(1-Benzyloxycarbonyl)-(4S)-methoxy-(2S)-pyrrolidinyl)carboxylic acid methyl ester (4.0 g, 13.65 mmol) was dissolved in a mixed solution of THF (100 ml) and methanol (50 ml), a 1N aqueous sodium hydroxide solution (27 ml, 27 mmol) was added at room temperature, and the mixture was stirred overnight. THF and methanol were removed under reduced pressure, and this was made weakly acidic with 1N hydrochloric acid, followed by extraction with ethyl acetate two times. The combined extracts were dried with anhydrous sodium sulfate, and concentrated under reduced pressure to obtain the title compound (3.9 g, 100%) as a colorless oily substance.

5-[(1-Benzyloxycarbonyl-(4S)-methoxy-(2S)-pyrrolidinyl)carbonylamino]levulinic acid methyl ester

[Chem.50]

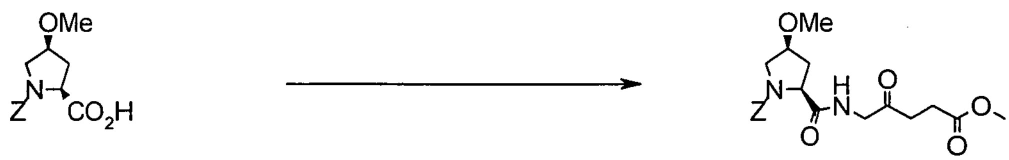

5-(1-Benzyloxycarbonyl-(4S)-methoxy-(2S)-pyrrolidinyl)carboxylic acid (1.4 g, 5.0 mmol), 5-aminolevulinic acid methyl ester hydrochloride (725 mg, 3.98 mmol), HOBt (675 mg, 5.0 mmol) and EDC (1.44 g, 7.5 mmol) were dissolved in methylene chloride (150ml), triethylamine (4.17 ml, 30 mmol) was added, and the mixture was stirred at room temperature overnight. Water was added to the reaction solution, followed by extraction with methylene chloride two times. The combined extracts were dried with anhydrous sodium sulfate, and concentrated under reduced pressure. The resulting residue was purified by column chromatography using silica gel to obtain the title compound (1.3 g, 81%) as a colorless oily substance from a chloroform: methanol (96:4)-eluted part.

$^1$H-NMR ($CDCl_3$) δ: 2.03-2.29 (2H, m), 2.54-2.79 (4H, m), 3.27 (3H, s), 3.49-3.58 (1H, m), 3.69 (3H, s), 3.89-3.99 (1H, m), 4.08-4.23 (2H, m), 4.36-4.46 (1H, m), 5.07-5.29 (3H, m), 7.29-7.44 (5H, m).

3-[2-[1-Benzyloxycarbonyl-(4S)-methoxy-(2S)-pyrrolidinyl]-5-oxazolyl]propionic acid methyl ester

[Chem.51]

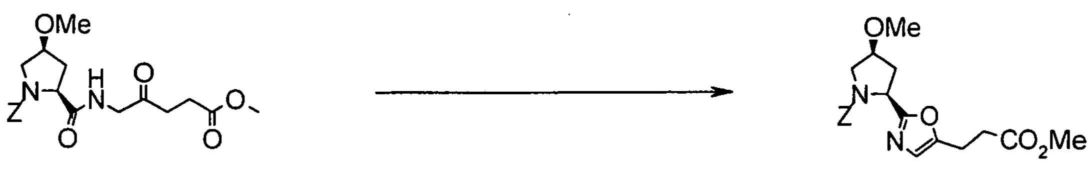

5-[(1-Benzyloxycarbonyl-(4S)-methoxy-(2S)-pyrrolidinyl)carbonylamino]levulinic acid methyl ester (1.3 g, 3.2 mmol) was dissolved in toluene (130 ml), and phosphorus oxychloride (1.8 ml) was added at room temperature. After stirred at 110°C for 3 hours, the reaction solution was poured into ice-water, this was extracted with ethyl acete two times, and combined extracts were washed with an aqueous saturated sodium chloride solution, dried with anhydrous sodium sulfate, and concentrated under reduced pressure. The resulting residue was purified by column chromatography using silica gel to obtain the title compound (1.2 g, 97%) as a pale yellow oily substance from a chloroform: methanol

(.95:5)-eluted part.

$^1H$-NMR ($CDCl_3$) δ: 2.51-2.68 (2H, m), 2.84-2.98 (2H, m), 3.19-3.22 (3H, m), 3.56-4.04 (7H, m), 5.03-5.16 (3H, m), 6.67-6.70 (1H, m), 7.18-7.42 (6H, m).

3-[2-((4S)-methoxy-(2S)-pyrrolidinyl)-5-oxazolyl]propionic acid methyl ester

[Chem.52]

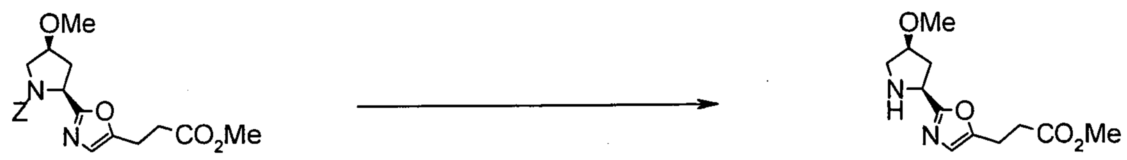

3-[2-[1-Benzyloxycarbonyl-(4S)-methoxy-(2S)-pyrrolidinyl]-5-oxazolyl]propionic acid methyl ester (1.2 g, 3.1 mmol) was dissolved in methanol (100 ml), Pd-C (200 mg) was added, and hydrogenation was performed at room temperature overnight. A catalyst was removed by filtration, and the filtrate was concentrated under reduced pressure. After concentration under reduced pressure, the residue was used in the next reaction without purification.

3-[2-[1-[2,5-Dichloro-4-(1-methyl-3-indolylcarbonylamino)phenylacetyl]-(4S)-methoxy-(2S)-pyrrolidinyl]-5-oxazolyl]propionic acid methyl ester

[Chem.53]

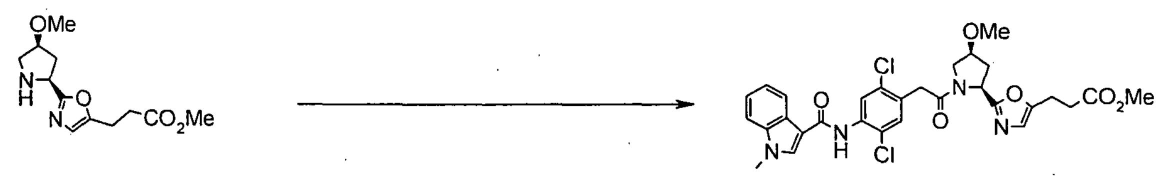

3-[2-((4S)-methoxy-(2S)-pyrrolidinyl)-5-oxazolyl]propionic acid methyl ester (0.5 mmol), 2,5-dichloro-4-(1-methyl-3-indolylcarbonylamino)phenylac etic acid (188 mg, 0.5 mmol), EDC (144 mg, 0.75 mmol), HOBt (68 mg, 0.5 mmol) and triethylamine (0.21 ml, 1.5 mmol) were dissolved in methylene chloride (15 ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 9 : 91, eluted with methanol : methylene chloride = 3 : 7) to obtain the title compound (200 mg, 65%) as a colorless oily substance.

$^1H$-NMR ($CDCl_3$) δ: 2.17-2.55 (2H, m), 2.60-2.71 (2H, m), 2.94-3.01 (2H, m), 3.21 and 3.22 (total 3H, each s), 3.35-4.13 (11H, m), 5.13-5.34 (1H, m), 6.70-6.73 (1H, m), 7.26-7.54 (4H, m), 7.79-7.82 (1H, m), 8.09-8.17 (1H, m), 8.24-8.27 (1H, m), 8.75 and 8.80 (total 1H, each s).

3-[2-[1-[2,5-Dichloro-4-(1-methyl-3-indolylcarbonylamino)phenylacetyl]-(4S)-methoxy-(2S)-pyrrolidinyl]-5-oxazolyl]propionic acid

[Chem. 54]

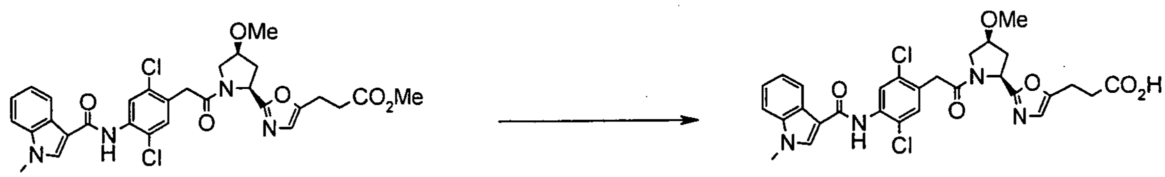

3-[2-[1-[2,5-Dichloro-4-(1-methyl-3-indolylcarbonylamino)phenylacetyl]-(4S)-methoxy-(2S)-pyrrolidinyl]-5-oxazolyl]propionic acid methyl ester (200 mg, 0.32 mmol) was dissolved in THF (7.0 ml), a 1N aqueous sodium hydroxide solution (0.65ml, 0.65mmol) and methanol (3.0ml) were added, and the mixture was stirred at room temperature overnight. The reaction solution was concentrated under reducedpressure, a small amount of water was added, and this was made neutral with 1N hydrochloric acid, followed by extraction with methylene chloride. The combined extracts were dried with anhydrous sodium sulfate, and concentrated under reduced pressure. The residue was purified by preparative TLC (Merck, Silica gel 60, $F_{254}$, 2mm; developed with methanol: methylene chloride = 1:9, eluted with methanol: methylene chloride = 3 : 7) to obtain the title compound (140 mg, 73%) as a white powder.

$^1$H-NMR (DMSO-$d_6$) δ: 2.12-2.57 (2H, m), 2.80-2.84 (2H, m), 3.09 and 3.14 (total 3H, each s), 3.63-4.11 (10H, m), 5.05-5.44 (1H, m), 6.70 and 6.80 (total 1H, each s), 7.22-7.27 (2H, m), 7.48-7.59 (2H, m), 7.88 and 7.90 (total 1H, each s), 8.15 (1H, d, J = 7.8 Hz), 8.32 (1H, s), 9.43 (1H, s). IR (ATR) $cm^{-1}$ : 2935, 1650, 1500, 1373, 1099, 744.
MS (LC-ESI) m/z : 599 ($M^+$+1).
Anal. Calcd for $C_{29}H_{28}Cl_2N_4O6$. $H_2O$: C, 56.41; H, 4.90; N, 9.07. Found: C, 56.50; H, 4.73; N, 9.00.
Compounds of Examples 13 to 17 shown below were produced by the same synthesizing method as that of Example 12.

Example 13

3-[2-[1-[5-Chloro-2-fluoro-4-(1-methyl-3-indolylcarbonyl)aminophenylacetyl]-(4S)-methoxy-(2S)-pyrrolidinyl]-5-oxazolyl]propionic acid

[Chem. 55]

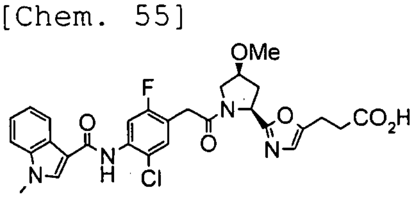

$^1$H-NMR (DMSO-$d_6$) δ: 2.12-2.57 (2H, m), 2.77-2.89 (2H, m), 3.08 and 3.14 (total 3H, each s), 3.60-4.11 (10H, m), 5.07-5.42 (1H, m), 6.69-6.80 (1H, m), 7.20-7.71 (5H, m), 8.15 (1H, d, J = 7.8 Hz), 8.33 and 8.34 (total 1H, each s), 9.37-9.40 (1H, m).
IR (ATR) $cm^{-1}$ : 2931, 1654, 1513, 1402, 1218, 1099, 744.
MS (LC-ESI) m/z : 583 ($M^+$+1).
Anal. Calcd for $C_{29}H_{28}ClFN_4O_6 \cdot H_2O$: C, 57.95; H, 5.03; N, 9.32. Found: C, 58.24; H, 4.90; N, 9.24.

Example 14

3-[2-[1-[[4-[(3-Benzo [b] thienylcarbonyl) amino]- 2,5- dichlorophenyl] acetyl]-(4S)-methoxy-(2S)-pyrrolidinyl] 5-oxazolyl]propionic acid

[Chem. 56]

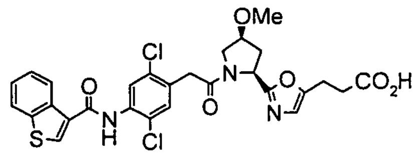

$^1$H-NMR (DMSO-$d_6$) δ: 2.14-2.55 (2H, m), 2.80-2.84 (2H, m), 3.10 and 3.14 (total 3H, each s), 3.64-4.12 (7H, m), 5.03-5. 44 (1H, m), 6.70 and 6.80 (total 1H, each s), 7.44-7.77 (4H, m), 8.10 (1H, d, J = 4.3 Hz), 8.45 (1H, d, J = 7.4 Hz), 8.68 (1H, s), 10.23 (1H, br s).
IR (ATR) $cm^{-1}$ : 2927, 1648, 1504, 1375, 1213, 1079, 765.
MS (LC-ESI) m/z : 602 ($M^+$+1).
Anal. Calcd for $C_{28}H_{25}Cl_2N_3O_6S \cdot H_2O$: C, 54.20; H, 4.39; N, 6.77. Found: C, 54.37; H, 4.22; N, 6.81.

Example 15

3-[2-[1-[[4-[(3-Benzo[b]thienylcarbonyl)amino]-5-chloro-2-fluorophenyl]acetyl]-(4S)-methoxy-(2S)-pyrrolidinyl]-5-oxazolyl]propionic acid
[Chem. 57]

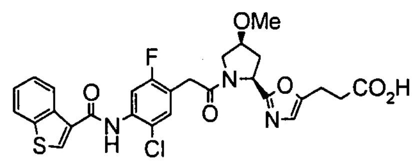

$^1$H-NMR (DMSO-$d_6$) δ: 2.10-2.59 (2H, m), 2.73-2.86 (2H, m), 3.08 and 3.14 (total 3H, each s), 3.63-4.11 (7H, m), 5.03-5.44 (1H, m), 6.69 and 6.79 (total 1H, each s), 7.44-7.58 (4H, m), 8.10 (1H, d, J = 7.0 Hz), 8.46 (1H, d, J = 3.7 Hz), 8.70 and 8.71 (total 1H, each s), 10.21-10.22 (1H, m).
IR (ATR) $cm^{-1}$ : 2929, 1648, 1517, 1402, 1216, 765. MS (LC-ESI) m/z : 586 ($M^+$+1).
Anal. Calcd for $C_{28}H_{25}ClFN_3O_6S \cdot H_2O$: C, 55.68; H, 4.51; N, 6.96. Found: C, 55.86; H, 4.33; N, 6.95.

Example 16

3-[2-[1-[[7-Fluoro-2-(5-fluoro-2-methoxyphenylamino)-1,3-benzoxazol-6-yl]acetyl]-(4S)-methoxy-(2S)-pyrrolidinyl]-5-oxazolyl]propionic acid
[Chem. 58]

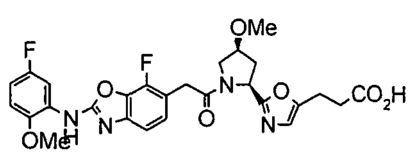

$^1$H-NMR (DMSO-$d_6$) δ: 2.05-2.52 (2H, m), 2.71-2.89 (2H, m), 3.07 and 3.14 (total 3H, each s), 3.49-4.19 (10H, m), 5.01-5.44 (1H, m), 6.69 and 6.80 (total 1H, eachs), 6.85-7.28 (4H, m), 8.17-8.21 (1H, m).
IR (ATR) $cm^{-1}$ : 2940, 1641, 1577, 1214, 711.
MS (LC-ESI) m/z : 557 ($M^+$+1).
Anal. Calcd for $C_{27}H_{26}F_2N_4O_7 \cdot H_2O$: C, 56.44; H, 4.91; N, 9.75. Found: C, 56.53; H, 4.72; N, 9.73.

Example 17

3-[2-[1-[[7-Fluoro-2-(2-methoxyphenylamino)-1,3-benzoxazol-6-yl]acetyl]-(4S)-methoxy-(2S)-pyrrolidinyl]-5-oxazolyl]propionic acid

[Chem. 59]

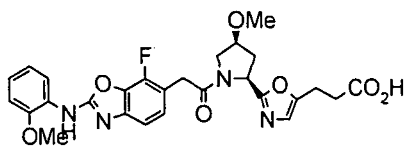

$^1$H-NMR (DMSO-$d_6$) δ: 2.10-2.55 (2H, m), 2.76-2.88 (2H, m), 3.07 and 3.13 (total 3H, each s), 3.62-4.19 (10H, m), 5.01-5.46 (1H, m), 6. 69 and 6.80 (total 1H, each s), 6.98-7.19 (5H, m), 8.13 (1H, d, J = 7.4 Hz), 9.87 (1H, br s).
IR (ATR) $cm^{-1}$ : 2937, 1637, 1577, 748.
MS (LC-ESI) m/z : 539 ($M^+$+1).
Anal. Calcd for $C_{27}H_{27}FN_4O_7 \cdot H_2O$: C, 58.27; H, 5. 25; N, 10.07. Found: C, 58.35; H, 5.04; N, 9.99.

Example 18

5-(1-Benzyloxycarbonyl-(4R)-hydroxy-(2S)-pyrrolidinyl)carboxylic acid methyl ester

[Chem. 60]

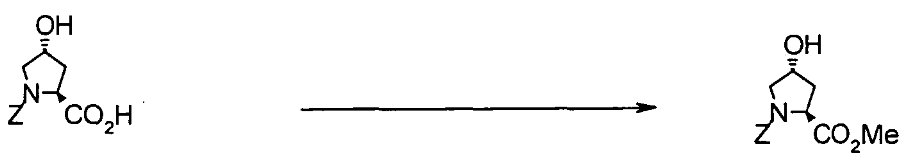

(1-Benzyloxycarbonyl-(4R)-hydroxy-(2S)-pyrrolidinyl)-carboxylic acid (15 g, 56.6 mmol) was dissolved in DMF (40 ml), potassium carbonate (11.7g, 84. 5 mmol) and methyl iodide (5.3 ml, 84.5 mmol) were added, and the mixture was stirred at 60°C for 1 hour. To the reaction solution was added water, followed by extraction with ethyl acetate two times. The combined extracts were washed with an aqueous saturated sodium chloride solution, dried with anhydrous sodium sulfate, and concentrated under reduced pressure. The resulting residue was purified by column chromatography using silica gel to obtain the title compound (16 g, 100%) as a colorless oily substance from a hexane: ethyl acetate (1: 1-1:5) -eluted part.

$^1$H-NMR ($CDCl_3$) δ: 2.04-2.32 (2H, m), 3.51-3.80 (5H, m), 4.47-4.52 (2H, m), 4.97-5.21 (2H, m), 7.26-7.36 (5H, m). 1-(Benzyloxycarbonyl-(4S)-fluoro-(2S)-pyrrolidinyl)carboxylic acid methyl ester

[Chem. 61]

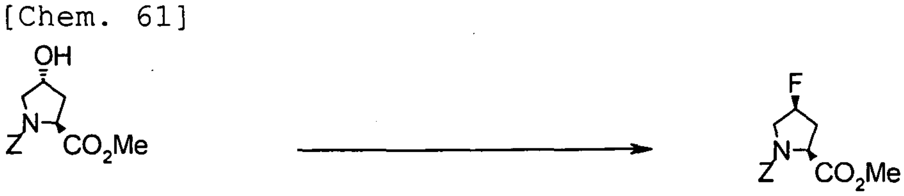

5-(1-Benzyloxycarbonyl-(4R)-hydroxy-(2S)-pyrrolidinyl)carboxylic acid methyl ester (15.7 g, 56.56 mmol) was dissolved in methylene chloride (200 ml), and bismethoxyethylaminosulfur trifluoride (15.6 ml, 84.8 mmol) was added dropwise at -78°C. After stirred at room temperature overnight, the reaction solution was poured into ice-water, followed

by extraction with methylene chloride two times. The combined extracts were washed with an aqueous saturated sodium chloride solution, dried with anhydrous sodium sulfate, and concentrated under reduced pressure. The resulting residue was purified by column chromatography using silica gel to obtain the title compound (14.9 g, 94%) as a pale yellow oily substance from a hexane: ethyl acetate (1:1-1:2)-eluted part.

$^1$H-NMR ($CDCl_3$) δ: 2.23-2.60 (2H, m), 3.61-3.94 (5H, m), 4.54-4.62 (1H, m), 5.09-5.29 (3H, m), 7.33-7.37 (5H, m).

(1-tert-butoxycarbonyl-(4S)-fluoro-(2S)-pyrrolidinyl)carboxylic acid methyl ester

[Chem. 62]

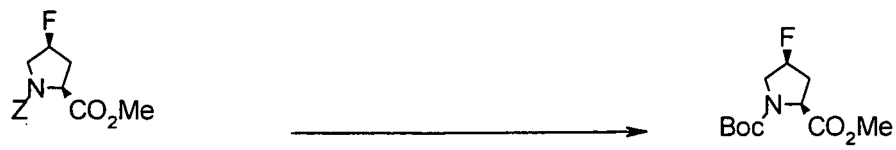

(1-Benzyloxycarbonyl-(4S)-fluoro-(2S)-pyrrolidinyl) carboxylic acid methyl ester (12 g, 42.7 mmol) was dissolved in ethanol (200 ml), Pd-C (1.2 g) was added, and hydrogenation was performed at room temperature overnight. A catalyst was removed by filtration, and the filtrate was concentrated under reduced pressure. The resulting residue was dissolved in methylene chloride (200 ml), and $BoC_2O$ (11.2 g, 51.2 mmol) and triethylamine (12 ml, 85.4 mmol) were successively added at room temperature. After stirred for 3 hours, this was concentrated under reduced pressure, and the residue was purified by column chromatography using silica gel to obtain the title compound (7.3 g, 69%) as a colorless oily substance from a hexane: ethyl acetate (4:1-2:3)-eluted part.

(1-tert-butoxycarbonyl-(4S)-fluoro-(2S)-pyrrolidinyl)carboxylic acid

[Chem. 63]

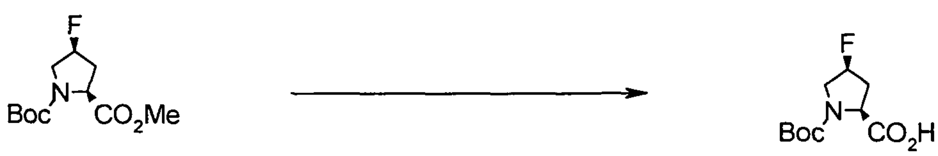

(1-tert-butoxycarbonyl-(4S)-fluoro-(2S)-pyrrolidinyl)carboxylic acid methyl ester (7.3 g, 29.5 mmol) was dissolved in a mixed solution of THF (120 ml) and methanol (60 ml), a 1N aqueous sodium hydroxide solution (60 ml, 60 mmol) was added at roomtemperature, and the mixture was stirred overnight. THF and methanol were removed under reduced pressure, and this was made weakly acidic with 1N, followed by extraction with ethyl acetate two times. The combined extracts were washed with an aqueous saturated sodium chloride solution, dried with anhydrous sodium sulfate, and concentrated under reduced pressure to obtain the title compound (6.1 g, 89%) as a white solid.

5-[(1-tert-butoxycarbonyl-(4S)-fluoro-(2S)-pyrrolidinyl)carbonylamino]levulinic acid methyl ester

[Chem. 64]

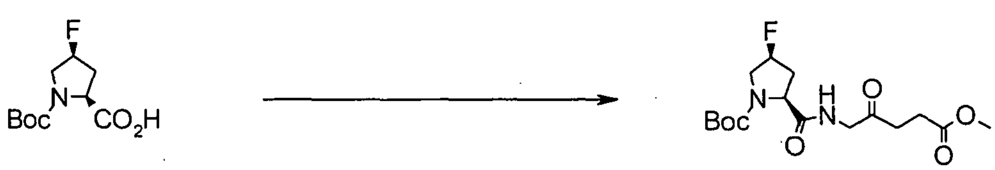

(1-tert-butoxycarbonyl-(4S)-fluoro-(2S)-pyrrolidinyl) carboxylic acid (942 mg, 4.0 mmol), 5-aminolevulinic acid

methyl ester hydrochloride (728 mg, 4.0 mmol), HOBt (540 mg, 4.0 mmol) and EDC (1.2 g, 6.0 mmol) were dissolved in methylene chloride (50 ml), triethylamine (3.3 ml, 24 mmol) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.43 g, 100%) as a colorless oily substance from a methylene chloride: methanol (97: 3) -eluted part.

$^1$H-NMR ($CDCl_3$) δ: 1.45 (9H, br s), 2.39-2.20 (2H, m), 2.65-2.66 (2H, m), 2.73-2.76 (2H, m), 3.48-3.80 (5H, m), 4.11-4.51 (3H, m), 5.13-5.30 (1H, m). 3-[2-[1-tert-butoxycarbonyl-(4S)-fluoro-(2S)-pyrrolidinyl]-5-thiazolyl]propionic acid methyl ester

[Chem. 65]

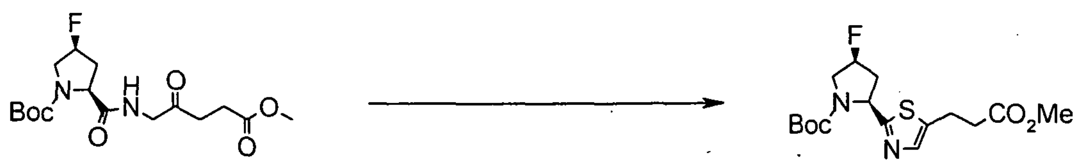

5-[(1-tert-butoxycarbonyl-(4S)-fluoro-(2S)-pyrrolidinyl)carbonylamino]levulinic acid methyl ester (1.43g, 4.0 mmol) was dissolved in toluene (72 ml), a Lawesson's reagent (1.77 g, 4.4 mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride = 5 : 95, eluted with methanol : methylene chloride = 3 : 7) to obtain the title compound (1.35 g, 95%) as a yellow oily substance.

$^1$H-NMR ($CDCl_3$) δ: 1.32-1.55 (9H, m), 2.41-2.81 (4H, m), 3.07-3.14 (2H, m), 3.64-3.93 (5H, m), 5.20-5.32 (2H, m), 7.35 (1H, s).

3-[2-((4S)-fluoro-(2S)-pyrrolidinyl)-5-thiazolyl]propionic acid methyl ester

[Chem. 66]

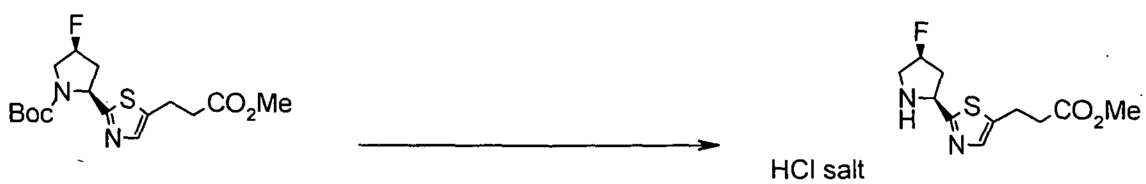

3-[2-[1-tert-butoxycarbonyl-(4S)-fluoro-(2S)-pyrrolidinyl]-5-thiazolyl]propionic acid methyl ester (1.35 g, 3.77 mmol) was dissolved in a 4N hydrochloric acid dioxane solution (35 ml), and the solution was stirred at room temperature for 1 hour and a half. After concentrated under reduced pressure, the residue was used in the next reaction without purification. 3-[2-[1-[2,5-Dichloro-4-(1-methyl-3-indolylcarbonylamino)phenylacetyl]-(4S)-fluoro-(2S)-pyrrolidinyl]-5-thiazolyl]propionic acid methyl ester

[Chem. 67]

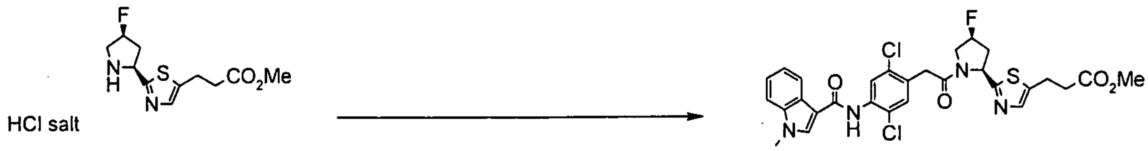

3-[2-((4S)-fluoro-(2S)-pyrrolidinyl)-5-thiazolyl]propionic acid methyl ester hydrochloride (0.62 mmol), 2,5-dichloro-4-(1-methyl-3-indolylcarbonylamino)phenylac etic acid (234 mg, 0.62 mmol), EDC (180 mg, 0.93 mmol), HOBt (84 mg, 0.62 mmol) and triethylamine (0.52 ml, 3. 7 mmol) were dissolved in methylene chloride (15 ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 7 : 93, eluted with methanol : methylene chloride = 3 : 7) to obtain the title compound (300 mg, 78%) as a colorless oily substance.

$^1$H-NMR ($CDCl_3$) δ: 2.41-2.94 (4H, m), 3.10-3.14 (2H, m), 3.49-4.13 (10H, m), 5.25-5.47 (1H, m), 5.53-5.66 (1H, m), 7.30-7.52 (5H, m), 7.80 and 7.81 (total 1H, each s), 8.12-8.15 (1H, m), 8.24 and 8.27 (total 1H, each s), 8.76 and 8.83 (total 1H, each s).

3-[2-[1-[2,5-Dichloro-4-(1-methyl-3-indolylcarbonylamino) phenylacetyl]-(4S)-fluoro-(2S)-pyrrolidinyl]-5-thiazolyl]propionic acid

[Chem. 68]

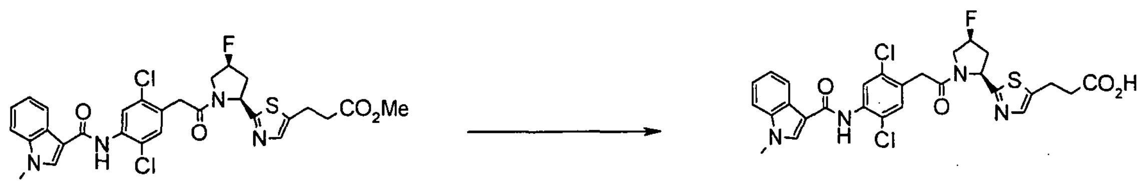

3-[2-[1-[2,5-Dichloro-4-(1-methyl-3-indolylcarbonylamino) phenylacetyl]-(4S)-fluoro-(2S)-pyrrolidinyl]-5-thiazolyl]propionic acid methyl ester (300 mg, 0.48 mmol) was dissolved in THF (10 ml), a 1N aqueous sodium hydroxide solution (2.0 ml, 2.0 mmol) and methanol (5.0 ml) were added, and the mixture was stirred at room temperature overnight. The reaction solution was concentrated under reduced pressure, a small amount of water was added, and this was made acidic with 1N hydrochloric acid, followed by extraction with methylene chloride two times. The combined extracts were dried with anhydrous sodium sulfate, and concentrated under reduced pressure. The residue was purified by preparative TLC (Merck, Silica gel 60, $F_{254}$, 2 mm; developed with methanol : methylene chloride = 9 : 91, eluted with methanol : methylene chloride = 3 : 7) to obtain the title compound (240 mg, 82%) as a white powder.

$^1$H-NMR (DMSO-$d_6$) δ: 2.45-2.83 (4H, m), 2.93-3.03 (2H, m), 3.57-4.11 (7H, m), 5.33-5.77 (2H, m), 7.19-7.58 (5H, m), 7.86-7.93 (1H, m), 8.14-8.18 (1H, m), 8.31 (1H, s), 9.42-9.44 (1H, m).
IR (ATR) $cm^{-1}$ : 1654, 1218, 744.
MS (LC-ESI) m/z : 603 ($M^+$+1).
Anal. Calcd for $C_{28}H_{25}Cl_2FN_4O_4S \cdot 0.8H_2O$: C, 54.43; H, 4.34; N, 9.07. Found: C, 54.46; H, 4.20; N, 9.04.

Compounds of Examples 19 to 23 shownbelow canbe produced by the same synthesizing method as that of Example 19.

Example 19

3-[2-[1-[5-Chloro-2-fluoro-4-(1-methyl-3-indolylcarbonyl)aminophenylacetyl]-(4S)-fluoro-(2S)-pyrrolidinyl]-5-

thiazolyl]propionic acid

[Chem. 69]

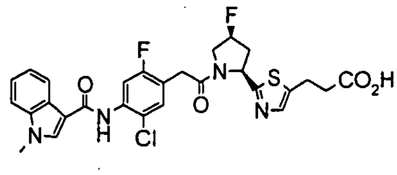

$^1$H-NMR (DMSO-$d_6$) δ: 2.43-2.70 (4H, m), 2.92-3.03 (2H, m), 3.49-4.09 (7H, m), 5.27-5.68 (2H, m), 7.20-7.76 (6H, m), 8.15 (1H, d, J = 7.8 Hz), 8.33 (1H, s), 9.37 (1H, s).
IR (ATR) $cm^{-1}$ : 2925, 1654, 1513, 1400, 1222, 744.
MS (LC-ESI) m/z : 587 ($M^+$+1).
Anal. Calcd for $C_{28}H_{25}ClF_2N_4O_4S \cdot 0.8H_2O$: C, 55.92; H, 4.46; N, 9.32. Found: C, 55.97; H, 4.34; N, 9.22.

Example 20

3-[2-[1-[[4-[(3-Benzo[b]thienylcarbonyl)amino]-2,5-dichlorophenyl]acetyl]-(4S)-fluoro-(2S)-pyrrolidinyl]-5-thiazolyl]propionic acid

[Chem. 70]

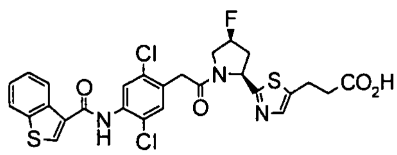

$^1$H-NMR (DMSO-$d_6$) δ: 2.39-2.77 (4H, m), 2.94-3.02 (2H, m), 3.62-4.11 (4H, m), 5.32-5.69 (2H, m), 7.38-7.81 (5H, m), 8.10 (1H, dd, J = 6.7, 1.3 Hz), 8.45 (1H, d, J = 7. 6 Hz), 8.67 and 8.69 (toal 1H, each s), 10.22-10.30 (1H, m).
IR (ATR) $cm^{-1}$ : 1654, 1504, 1213, 765.
MS (LC-ESI) m/z : 606 ($M^+$+1).
Anal. Calcd for $C_{27}H_{22}Cl_2FN_3O_4S_2 \cdot 0.5H_2O$: C, 52.69; H, 3.77; N, 6.83. Found: C, 52.62; H, 3.85; N, 6.81.

Example 21

3-[2-[1-[[4-[(3-Benzo[b]thienylcarbonyl)amino]-5-chloro-2-fluorophenyl]acetyl]-(4S)-fluoro-(2S)-pyrrolidinyl]-5-thiazolyl]propionic acid

[Chem. 71]

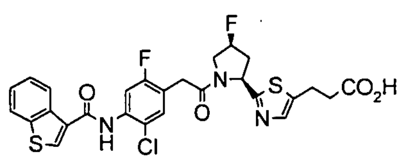

$^1$H-NMR (DMSO-$d_6$) δ: 2.48-2.76 (4H, m), 2.93-3.03 (2H, m), 3.52-4.14 (4H, m), 5.25-5.71 (2H, m), 7.38-7.61 (5H, m), 8.10 (1H, d, J = 8.1 Hz), 8.45 and 8.47 (total 1H, each s), 8.68-8.70 (total 1H, each s), 10.18-10.31 (1H, m).
IR (ATR) $cm^{-1}$ : 1654, 1517, 1402, 1218, 765.

MS (LC-ESI) m/z : 590 ($M^+$+1).
Anal. Calcd for $C_{27}H_{22}ClF_2N_3O_4S_2 \cdot 0.5H_2O$: C, 54.13; H, 3. 87; N, 7.01. Found: C, 53.99; H, 3.92; N, 6.95.

Example 22

3-[2-[1-[[4-[(1-Benzofuran-3-ylcarbonyl)amino]-5-chloro-2-fluorophenyl]acetyl]-(4S)-fluoro-(2S)-pyrrolidinyl]-5-thiazolyl]propionic acid

[Chem. 72]

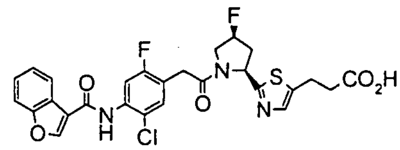

$^1$H-NMR (DMSO-$d_6$) δ: 2.39-2.68 (4H, m), 2.91-3.01 (2H, m), 3.54-4.10 (4H, m), 5.32-5.69 (2H, m), 7.38-7.72 (6H, m), 8.09 (1H, dd, J = 6.5, 1.3 Hz), 8.87 (1H, s), 10.13-10.19 (1H, m).
IR (ATR) $cm^{-1}$ : 1652, 1521, 1402, 746.
MS (LC-ESI) m/z : 574 ($M^+$+1).
Anal. Calcd for $C_{27}H_{22}ClF_2N_3O_5S \cdot 0.8H_2O$: C, 55.11; H, 4.04; N, 7.14. Found: C, 55.07; H, 4.00; N, 7.14.

Example 23

3-[2-[1-[[7-Fluoro-2-(2-methoxyphenylamino)-1,3-benzoxazol-6-yl]acetyl]-(4S)-fluoro-(2S)-pyrrolidinyl]-5-thiazolyl]propionic acid

[Chem. 73]

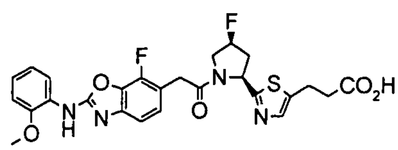

$^1$H-NMR (DMSO-$d_6$) δ: 2.33-2.80 (4H, m), 2.95-3.00 (2H, m), 3.52-4.09 (7H, m), 5.31-5.68 (2H, m), 6.93-7.21 (5H, m), 7.37 and 7.49 (total 1H, each s), 8.12-8.13 (1H, m), 9.87 (1H, br s).
IR (ATR) $cm^{-1}$ : 1639, 1579.
MS (LC-ESI) m/z : 543 ($M^+$+1).
Anal. Calcd for $C_{26}H_{24}F_2N_4O_5S \cdot H_2O$: C, 55.71; H, 4.67; N, 9.99.
Found: C, 55.72; H, 4.56; N, 9.93.

Example 24

(1-Benzyloxycarbonyl-(4R)-methoxy-(2S)-pyrrolidinyl)carboxylic acid methyl ester

[Chem. 74]

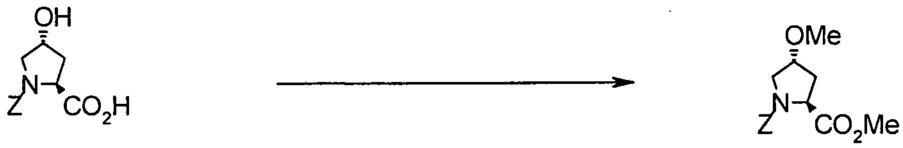

(1-Benzyloxycarbonyl-(4R)-hydroxy-(2S)-pyrrolidinyl)carboxylic acid (4.0 g, 15.1 mmol) was dissolved in DMF (50 ml), and sodium hydride (60% in oil) (1.26 g, 31.6 mmol) was added at 0˚C. After stirred at room temperature for 30 minutes, methyl iodide (2.0 ml, 33.2 mmol) was added dropwise. After stirred at room temperature for 2 hours, water was added, followed by extraction with ethyl acetate two times. The combined extracts were dried with anhydrous sodium sulfate, and concentrated under reduced pressure. The resulting residue was purified by column chromatography using silica gel to obtain the title compound (2.3 g, 52%) as a colorless oily substance from a hexane: ethyl acetate (3:2-2:3) -eluted part.

$^1$H-NMR ($CDCl_3$) δ: 2.04-2.10 (1H, m), 2.27-2.43 (1H, m), 3. 31 and 3.32 (total 3H, each s), 3.51-3.80 (5H, m), 3.94-4.04 (1H, m), 4.39-4.47 (1H, m), 4.99-5.24 (2H, m), 7.29-7.34 (5H, m) .

(1-tert-butoxycarbonyl-(4R)-methoxy-(2S)-pyrrolidinyl)carboxylic acid methyl ester

[Chem. 75]

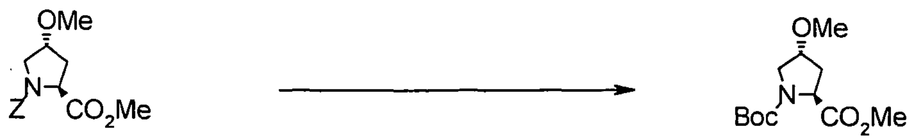

(1-Benzyloxycarbonyl-(4R)-methoxy-(2S)-pyrrolidinyl) carboxylic acidmethyl ester (2. 3 g, 7 . 85 mmol) was dissolved in ethanol (50 ml), Pd-C (230 mg) was added, and hydrogenation was performed at room temperature overnight. A catalyst was removed by filtration, and the filtrate was concentrated under reduced pressure. The resulting residue was dissolved in methylene chloride (40 ml), and $Boc_2O$ (2.1 g, 9.4 mmol) and triethylamine (2.2 ml, 15.7 mmol) were successively added at room temperature. After stirred overnight, this was concentrated under reduced pressure, and the residue was purified by column chromatography using silica gel to obtain the title compound (1.8g, 89%) as a pale yellow oily substance from a hexane: ethyl acetate (4.1-1.1)-eluted part.

$^1$H-NMR ($CDCl_3$) δ: 1.41 and 1.46 (total 9H, each s), 2.01-2.08 (1H, m), 2.27-2.41 (1H, m), 3.32 (3H, s), 3.48-3.67 (2H, m), 3.73 and 3.74 (total 3H, each s), 3.94-4.02 (1H, m), 4.15-4.42 (1H, m).

(1-tert-butoxycarbonyl-(4R)-methoxy-(2S)-pyrrolidinyl)carboxylic acid

[Chem. 76]

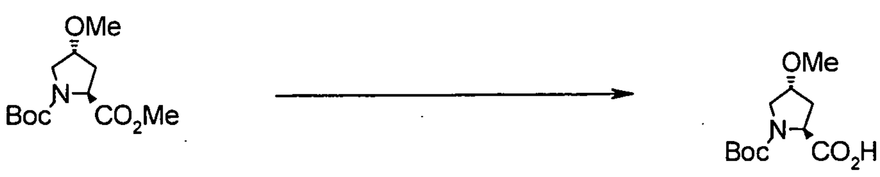

(1-tert-butoxycarbonyl-(4R)-methoxy-(2S)-pyrrolidinyl)carboxylic acid methyl ester (1.8 g, 6.9 mmol) was dissolved in a mixed solution of THF (50 ml) and methanol (25 ml), a 1N aqueous sodium hydroxide solution (14 ml, 14 mmol) was added at roomtemperature, and the mixture was stirred overnight. THF and methanol were removed under reduced pressure, and this was made weakly acidic with 1N hydrochloric acid, followed by extraction with ethyl acetate two times. The combined extracts were dried with anhydrous sodium sulfate, and concentrated under reduced pressure to obtain the title compound (1.66 g, 98%) as a colorless oily substance.

5-[(1-tert-butoxycarbonyl-(4R)-methoxy-(2S)-pyrrolidinyl)carbonylamino]levulinic acid methyl ester

[Chem. 77]

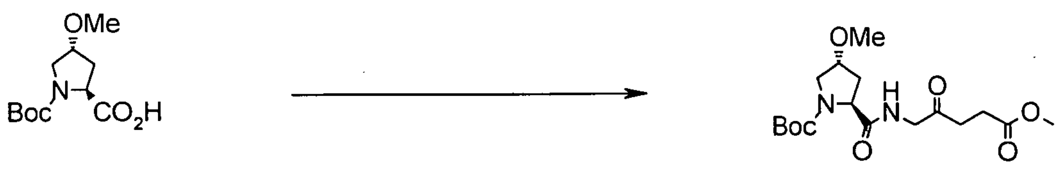

(1-tert-butoxycarbonyl-(4R)-methoxy-(2S)-pyrrolidinyl) carboxylic acid (735 mg, 3.0 mmol), 5-aminolevulinic acid methyl ester hydrochloride (546 mg, 3.0 mmol), HOBt (405 mg, 3.0mmol) andEDC (864 mg, 4.5mmol) were dissolved in methylene chloride (35 ml), triethylamine (2.5 ml, 18 mmol) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.14 g, 100%) as a colorless oily substance from a methylene chloride: methanol (97:3)-eluted part.

$^1$H-NMR ($CDCl_3$) δ: 1.38-1.51 (9H, m), 2.07-2.43 (2H, m), 2.57-2.77 (4H, m), 3.32 (3H, s), 3.47-3.52 (2H, m), 3.67 (3H, s), 3.71-4.44 (4H, m), 6.65 (1H, br s).

3-[2-(1-tert-butoxycarbonyl-(4R)-methoxy-(2S)-pyrrolidinyl)-5-thiazolyl]propionic acid methyl ester

[Chem. 78]

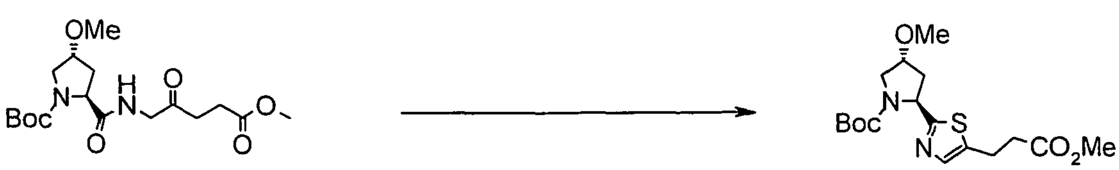

5-[(1-tert-butoxycarbonyl-(4R)-methoxy-(2S)-pyrrolidinyl)carbonylamino]levulinic acid methyl ester (1.11 g, 3.0 mmol) was dissolved in toluene (50 ml), a Lawesson's reagent (1.34 g, 3.3 mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol: methylene chloride = 5 : 95, eluted with methanol : methylene chloride = 3 : 7) to obtain the title compound (950 mg, 85%) as a yellow oily substance.

$^1$H-NMR ($CDCl_3$) δ: 1.31-1.47 (9H, m), 2.23-2.53 (2H, m), 2.65 (2H, t, J = 7.3 Hz), 3.08-3.18 (2H, m), 3.34 (3H, s), 3.49-3.87 (5H, m), 4.04 (1H, br s), 5.11-5.22 (1H, m), 7.37 (1H, s).

3-[2-((4R)-methoxy-(2S)-pyrrolidinyl)-5-thiazolyl]propionic acid methyl ester hydrochloride

[Chem. 79]

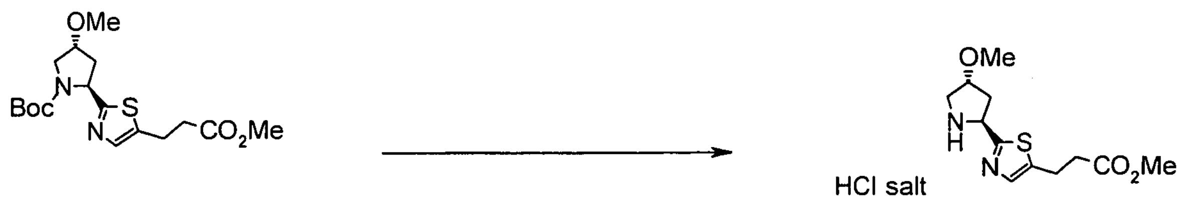


3-[2-(1-tert-butoxycarbonyl-(4R)-methoxy-(2S)-pyrrolidinyl)-5-thiazolyl]propionic acid methyl ester (950 mg, 2.56 mmol) was dissolved in a 4N hydrochloric acid dioxane solution (30 ml), and the solution was stirred at room temperature for 1 hour and a half. After concentrated under reduced pressure, the residue was used in the next reaction without purification.

3-[2-[1-[2,5-Dichloro-4-(1-methyl-3-indolylcarbonylamino)phenylacetyl]-(4R)-methoxy-(2S)-pyrrolidinyl]-5-thiazolyl]propionic acid methyl ester

[Chem. 80]

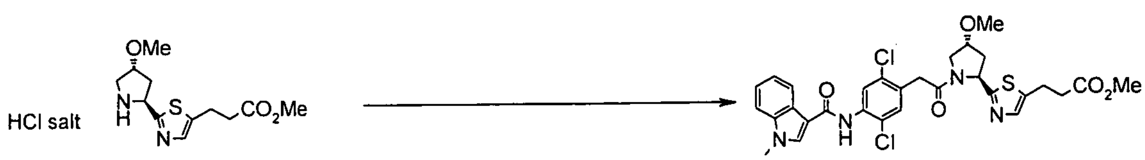


3-[2-((4R)-methoxy-(2S)-pyrrolidinyl)-5-thiazolyl]propionic acid methyl ester hydrochloride (0.5 mmol), 2,5-dichloro-4-(1-methyl-3-indolylcarbonylamino)phenylac etic acid (189 mg, 0.5 mmol), EDC (144 mg, 0.75 mmol), HOBt (68 mg, 0.5 mmol) and triethylamine (0.42 ml, 3.0 mmol) were dissolved in methylene chloride (20 ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 8 : 92, eluted with methanol : methylene chloride = 3 : 7) to obtain the title compound (260 mg, 83%) as a colorless oily substance.

$^1$H-NMR ($CDCl_3$) δ: 2.35-2.72 (4H, m), 3.10-3.18 (2H, m), 3.33-3.41 (3H, m), 3.49-4.27 (11H, m), 5.38-5.55 (1H, m), 7.23-7.46 (5H, m), 7.82-7.86 (1H, m), 8.10-8.30 (2H, m), 8.71-8.87 (1H, m).

3-[2-[1-[2,5-Dichloro-4-(1-methyl-3-indolylcarbonylamino)phenylacetyl]-(4R)-methoxy-(2S)-pyrrolidinyl]-5-thiazolyl]propionic acid

[Chem. 81]

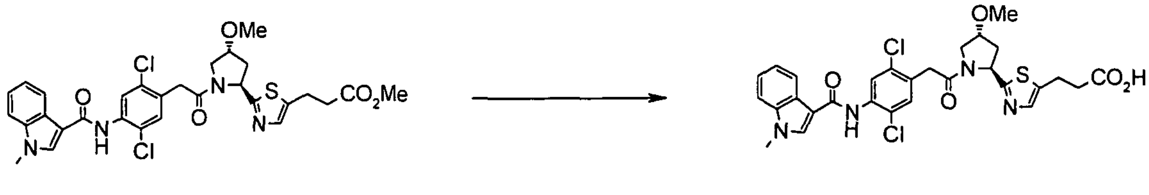

3-[2-[1-[2,5-Dichloro-4-(1-methyl-3-indolylcarbonylamino)phenylacetyl]-(4R)-methoxy-(2S)-pyrrolidinyl]-5-thiazolyl]propionic acid methyl ester (260 mg, 0.41 mmol) were dissolved in THF (10ml), a 1N aqueous sodium hydroxide solution (1.5ml, 1.5 mmol) and methanol (5.0 ml) were added, and the mixture was stirred at room temperature overnight. The reaction solution was concentrated under reduced pressure, a small amount of water was added, this was made neutral with 1N hydrochloric acid, and a precipitated solid was collected by filtration to obtain the title compound (220 mg, 87%) as a white powder.

$^1$H-NMR (DMSO-$d_6$) δ: 2.27-2.61 (4H, m), 2.96-3.07 (2H, m), 3.26-3.31 (3H, m), 3.56-4.20 (8H, m), 5.22-5.52 (1H, m), 7.18-7.30 (2H, m), 7.41 and 7.42 (total 1H, each s), 7. 52-7. 57 (2H, m), 7.85 and 7.90 (total 1H, each s), 8.15 (1H, d, J = 7.6 Hz), 8.29 and 8.30 (total 1H, each s), 9.35 and 9.38 (total 1H, each s).
IR (ATR) cm$^{-1}$ : 2927, 1648, 1500, 1373, 1099, 742.
MS (LC-ESI) m/z : 615 (M$^+$+1).
Anal. Calcd for $C_{29}H_{28}Cl_2N_4O_5S \cdot 0.5H_2O$: C, 55.77; H, 4.68; N, 8.97. Found: C, 55.81; H, 4.67; N, 8.98.
A compound of Example 25 shown below was produced by the same synthesizing method as that of Example 24.

Example 25

3-[2-[1-[4-[(3-Benzo[b]thienylcarbonyl)amino]-5-chloro-2-fluorophenylacetyl]-(4R)-methoxy-(2S)-pyrrolidinyl]-5-thiazolyl]propionic acid

[Chem. 82]

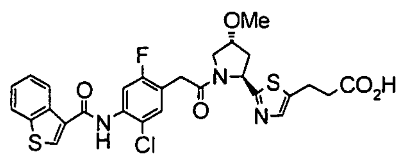

$^1$H-NMR (DMSO-$d_6$) δ: 2.29-2.62 (4H, m), 2.95-3.08 (2H, m), 3.26-3.30 (3H, m), 3.50-4.22 (5H, m), 5.23-5.58 (1H, m), 7.34-7.59 (5H, m), 8.09-8.11 (1H, m), 8.44-8.45 (1H, m), 8.64 and 8.65 (total 1H, each s), 10. 12 and 10.09 (total 1H, each s).
IR (ATR) cm$^{-1}$ : 2929, 1509, 1402, 1216, 1093, 765.
MS (LC-ESI) m/z : 602 (M$^+$+1).
Anal. Calcd for $C_{28}H_{25}ClFN_3O_5S_2 \cdot 0.2H_2O$: C, 55.52; H, 4.23; N, 6.94. Found: C, 55.55; H, 4.35; N, 6.69.

Example 26

5-[(1-tert-butoxycarbonyl-(2S)-pyrrolidinyl)carbonylamino]levulinic acid methyl ester

[Chem. 83]

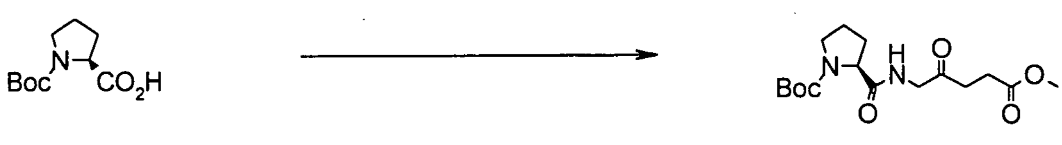

1-tert-butoxycarbonyl-L-proline (645 mg, 3.0 mmol), 5-aminolevulinic acid methyl ester hydrochloride (546 mg, 3.0 mmol), HOBt (405 mg, 3.0 mmol) and EDC (864 mg, 4.5 mmol) were dissolved in methylene chloride (35 ml), triethylamine (2.5 ml, 18 mmol) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.0 g, 100%) as a colorless oily substance from a methylene

chloride: methanol (97:3)-eluted part.

$^1$H-NMR ($CDCl_3$) δ: 1.41-1.51 (9H, m), 1.85-2.28 (4H, m), 2.56-2.81 (4H, m), 3.41-3.51 (2H, m), 3.68 (3H, s), 4.13-4.37 (2H, m).

3-[2-(1-tert-butoxycarbonyl-(2S)-pyrrolidinyl)-5-thiazolyl]propionic acid methyl ester

[Chem. 84]

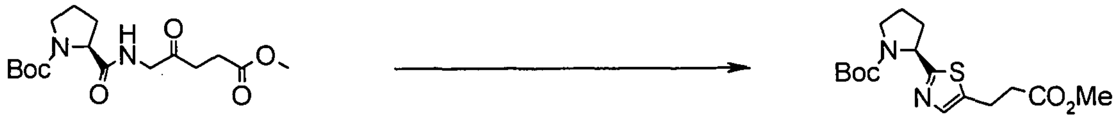

5-[(1-tert-butoxycarbonyl-(2S)-pyrrolidinyl)carbonylamino]levulinic acid methyl ester (1.02 g, 3.0 mmol) was dissolved in toluene (50 ml), a Lawesson's reagent (1.34 g, 3.3 mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride = 5 : 95, eluted with methanol : methylene chloride = 3 : 7) to obtain the title compound (840 mg, 82%) as a yellow oily substance.

$^1$H-NMR ($CDCl_3$) δ: 1.31-1.51 (9H, m), 1.90-2.01 (2H, m), 2.17-2.32 (2H, m), 2.65 (2H, t, J = 7.6 Hz), 3.04-3.15 (2H, m), 3.38-3.71 (5H, m), 5.05-5.16 (1H, m), 7.38 (1H, s).

3-[2-((2S)-pyrrolidinyl)-5-thiazolyl]propionic acid methyl ester hydrochloride

[Chem. 85]

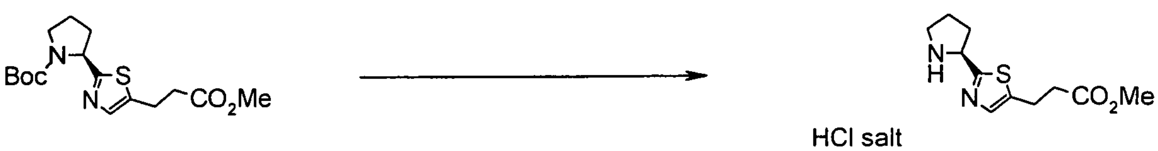

3-[2-(1-tert-butoxycarbonyl-(2S)-pyrrolidinyl)-5-thiazolyl]propionic acid methyl ester (840 mg, 2.46 mmol) was dissolved in a 4N hydrochloric acid dioxide solution (30 ml), and the solution was stirred at room temperature for 1 hour. After concentration under reduced pressure, the residue was used in the next reaction without purification.

3-[3-[1-[2,5-Dichloro-4-(1-methyl-3-indolylcarbonylamino)phenylacetyl]-(2S)-pyrrolidinyl]-5-thiazolyl]propionic acid methyl ester

[Chem. 86]

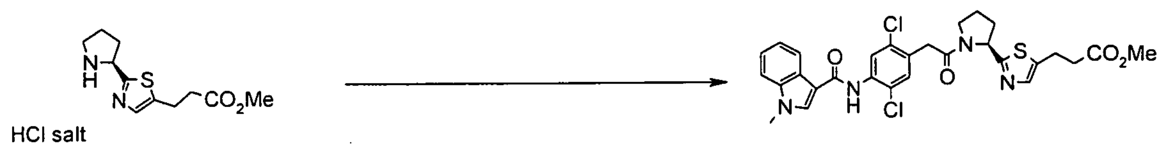

3-[2-(1-tert-butoxycarbonyl-(2S)-pyrrolidinyl)-5-thiazolyl]propionic acid methyl ester hydrochloride (0.61 mmol), 2,5-dichloro-4-(1-methyl-3-indolylcarbonylamino)phenylac etic acid (230 mg, 0.61 mmol), EDC (177 mg, 0.92 mmol), HOBt (82 mg, 0.61 mmol) and triethylamine (0.51 ml, 3.66 mmol) were dissolved in methylene chloride (20 ml), and the mixture was stirred at room temperature overnight. The reaction solution was concentrated under reduced pressure, and the residue was purified by preparative TLC (Merck, silica gel 60, $F_{254}$, 2mm; developed with methanol : methylene chloride = 8 : 92, eluted with methanol : methylene chloride = 3 : 7) to obtain the title compound (300 mg, 82%) as a colorless oily substance.

$^1$H-NMR ($CDCl_3$) δ: 2.00-2.47 (4H, m), 2.64-2.68 (2H, m), 3.10-3.12 (2H, m), 3.49-3.90 (10H, m), 5.32-5.47 (1H, m), 7.33-7.47 (5H, m), 7.79 and 7.80 (total 1H, each s), 8.11-8.15 (1H, m), 8.23 and 8.25 (total 1H, each s), 8.73 and 8.80 (total 1H, each s).

3-[2-[1-[2,5-Dichloro-4-(1-methyl-3-indolylcarbonylamino)phenylacetyl]-(2S)-pyrrolidinyl]-5-thiazolyl]propionic acid

[Chem. 87]

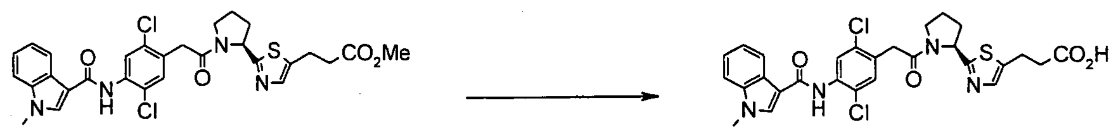

3-[2-[1-[2,5-Dichloro-4-(1-methyl-3-indolylcarbonylamino)phenylacetyl]-(2S)-pyrrolidinyl]-5-thiazolyl]propionic acid methyl ester (300 mg, 0.5 mmol) was dissolved in THF (10 ml), a 1N aqueous sodium hydroxide solution (1.5 ml, 1.5 mmol) and methanol (5.0 ml) were added, and the mixture was stirred at room temperature overnight. The reaction solution was concentrated under reduced pressure, a small amount of water was added, this was made neutral with 1N hydrochloric acid, and a precipitated solid was collected by filtration to obtain the title compound (240 mg, 82%) as a white powder.

$^1$H-NMR (DMSO-$d_6$) δ: 1.86-2.46 (4H, m), 2.53-2.61 (2H, m), 2.96-3.05 (2H, m), 3.45-3.93 (7H, m), 5.24-5.52 (1H, m), 7.19-7.30 (2H, m), 7.42 (1H, d, J= 2.7 Hz), 7.52-7.58 (2H, m), 7.85 and 7. 90 (total 1H, each s), 8.14-8.17 (1H, m), 8.29 and 8.30 (total 1H, each s), 9.36 and 9.39 (total 1H, each s).
IR (ATR) $cm^{-1}$ : 1652, 1506, 1103, 1074, 728.
MS (LC-ESI) m/z : 585 ($M^+$+1).
Anal. Calcd for $C_{28}H_{26}Cl_2N_4O_4S \cdot 0.5H_2O$: C, 56.57; H, 4.58; N, 9.42. Found: C, 56.58; H, 4.57; N, 9.40.
Compounds of Examples 27 to 29 shown below were produced by the same synthesizing method as that of Example 26.

Example 27

3-[2-[1-[5-Chloro-2-fluoro-4-(1-methyl-3-indolylcarbonyl)aminophenylacetyl]-(2S)-pyrrolidinyl]-5-thiazolyl]propionic acid

[Chem. 88]

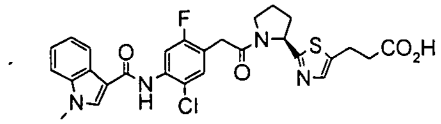

$^1$H-NMR (DMSO-$d_6$) δ: 1.83-2.46 (4H, m), 2.54-2.62 (2H, m), 2.96-3.07 (2H, m), 3.64-4.06 (7H, m), 5.24-5.54 (1H, m), 7.20-7.74 (6H, m), 8.15 (1H, d, J = 7.6 Hz), 8.30 and 8.31 (total 1H, each s), 9.29 and 9.33 (total 1H, each s).
IR (ATR) cm$^{-1}$ : 1650, 1517, 1216, 1101, 728.
MS (LC-ESI) m/z : 569 ($M^+$+1).
Anal. Calcd for $C_{28}H_{26}ClFN_4O_4S \cdot 1.5H_2O$: C, 56.42; H, 4. 90; N, 9.40. Found: C, 56.41; H, 4.77; N, 9.41.

Example 28

3-[2-[1-[[4-[(3-Benzo[b]thienylcarbonyl)amino]-2,5-dichlorophenyl]acetyl]-(2S)-pyrrolidinyl]-5-thiazolyl]propionic acid
[Chem. 89]

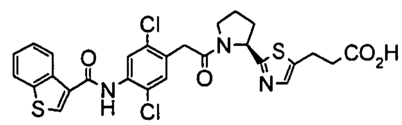

$^1$H-NMR (DMSO-$d_6$) δ: 1.85-2.46 (4H, m), 2.54-2.62 (2H, m), 2.96-3.06 (2H, m), 3.44-3.95 (4H, m), 5.23-5.53 (1H, m), 7.41-7.59 (4H, m), 7.73 and 7.78 (total 1H, each s), 8.10 (1H, dd, J = 6.9, 1.2 Hz), 8.45 (1H, dd, J = 6.7, 1.3 Hz), 8.64 and 8.65 (total 1H, each s), 10.14 and 10.17 (total 1H, each s).
IR (ATR) cm$^{-1}$ : 1643, 1504, 1211, 765.
MS (LC-ESI) m/z : 588 ($M^+$+1).
Anal. Calcd for $C_{27}H_{23}Cl_2N_3O_4S_2 \cdot H_2O$: C, 53.47; H, 4.15; N, 6.93. Found: C, 53.42; H, 4.04; N, 6.83.

Example 29

3-[2-[1-[4-[(3-Benzo[b]thienylcarbonyl)amino]-5-chloro-2-fluorophenylacetyl]-(2S)-pyrrolidinyl]-5-thiazolyl]propionic acid
[Chem. 90]

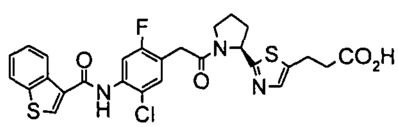

$^1$H-NMR (DMSO-$d_6$) δ: 1.81-2.45 (4H, m), 2.54-2.61 (2H, m), 2.96-3.07 (2H, m), 3.45-3.90 (4H, m), 5.24-5.55

(1H, m), 7.42-7.59 (5H, m), 8.09-8.11 (1H, m), 8.44-8.46 (1H, m), 8.64 and 8.65 (total 1H, each s), 10.09 and 10. 12 (total 1H, each s) .
IR (ATR) $cm^{-1}$ : 1621, 1513, 1400, 1214, 765.
MS (LC-ESI) m/z : 572 ($M^{+}$+1).
Anal. Calcd for $C_{27}H_{23}ClFN_3O_4S_2 \cdot 0.2H_2O$: C, 56.33; H, 4.10; N, 7.30. Found: C, 56.37; H, 4.22; N, 7.07.

Example 30

4-Pentynoic acid benzyl ester
[Chem. 91]

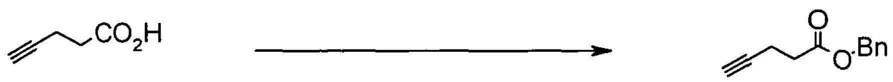

4-Pentynoic acid (1.0 g, 10.2 mmol) was dissolved in DMF (20 ml), potassium carbonate (2.8 g, 20.4 mmol) and benzyl bromide (1.82 ml, 15.3 mmol) were added, and the mixture was stirred at 60°C for 1 hour. To the reaction solution was added water, this was extracted with ethyl acetate two times, and combined extracts were dried with anhydrous sodium sulfate, and concentrated under reduced pressure. The resulting residue was purified by column chromatography using silica gel to obtain the title compound (1.85 g, 96%) as a colorless oily substance from a hexane: ethyl acetate (95:5 = 85:15)-eluted part.
$^1$H-NMR ($CDCl_3$) δ: 1.97 (1H, t, J = 2.6 Hz), 2.51-2.55 (2H, m), 2.59-2.63 (2H, m), 5.15 (2H, s), 7.32-7.40 (5H, m). (1-tert-butoxycarbonyl-(4S)-methoxy-(2S)-pyrrolidinyl)methanol
[Chem. 92]

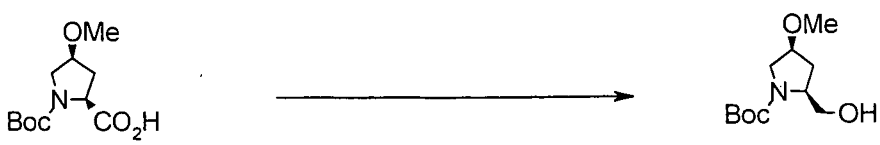

(1-tert-butoxycarbonyl-(4S)-methoxy-(2S)-pyrrolidi nyl)carboxylic acid (2.45 g, 10 mmol) was dissolved in THF (25 ml), $BH_3$-DMS (2.0M in THF) (7.5 ml, 15 mmol) was added, and the mixture was stirred at 60°C for 1 hour and a half. To the reaction solution was added water, followed by extraction with ethyl acetate two times. The combined extracts were dried with anhydrous sodium sulfate, and concentrated under reduced pressure. The resulting residue was purified by column chromatography using silica gel to obtain the title compound (2.3 g, 100%) as a colorless oily substance from a methylene chloride: methanol (85:15)-eluted part.
(1-tert-butoxycarbonyl-(4S)-methoxy-(2S)-pyrrolidinyl)carbaldehyde
[Chem. 93]

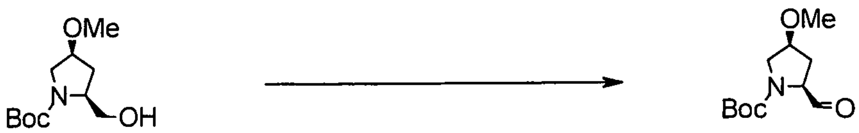

Oxalyl chloride (1.0 ml, 11 mmol) was dissolved in methylene chloride (25 ml), the solution was cooled to -76° C, and dimethyl sulfoxide (1.7 ml, 22 mmol) was added. After stirred for 10 minutes, a solution of (1-tert-butoxycarbonyl-(4S)-methoxy-(2S)-pyrrolidinyl)methanol (2.3 g, 9.9 mmol) in methylene chloride (20 ml) was added. After stirred for 20 minutes, triethylamine (7 ml, 50 mmol) was added, a temperature was returned to room temperature, and the mixture was stirred for 10 minutes. To the reaction solution was added water, this was extracted with methylene chloride two times, and combined extracts were dried with anhydrous sodium sulfate, and concentrated under reduced pressure. The resulting residue was purified by column chromatography using silica gel to obtain the title compound (2.1 g, 92%) as a yellow oily substance from a hexane: ethyl acetate (9:1-1:1)-eluted part.
(1-tert-butoxycarbonyl-(4S)-methoxy-(2S)-pyrrolidinyl)carbaldehyde oxime
[Chem. 94]

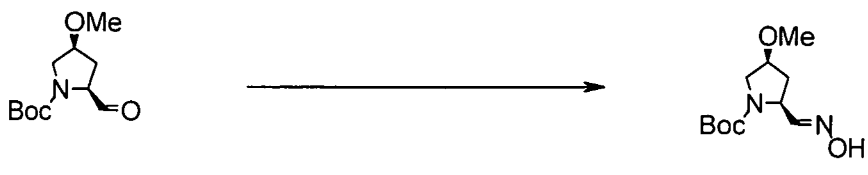

(1-tert-butoxycarbonyl-(4S)-methoxy-(2S)-pyrrolidinyl)carbaldehyde (2.1 g, 9.1 mmol), hydroxylamine hydrochloride (761 mg, 11 mmol) and sodium acetate (2.2 g, 26.5 mmol) were dissolved in a mixed solution of methanol (20 ml) and water (20 ml), and the solution was stirred at room temperature overnight. To the reaction solution was added water, followed by extraction with methylene chloride two times. The combined extracts were dried with anhydrous sodium sulfate, and concentrated under reduced pressure to obtain the title compound (2.23 g, 100%) as a pale yellow oily substance.
3-[3-(1-tert-butoxycarbonyl-(4S)-methoxy-(2S)-pyrrolidinyl)-5-isoxazolyl]propionic acid benzyl ester
[Chem. 95]

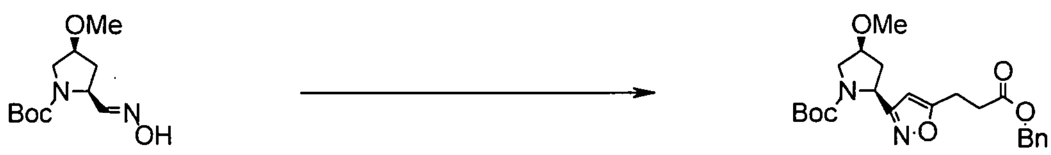

4-Pentynoic acid benzyl ester (1.46 g, 7.8 mmol), (1-tert-butoxycarbonyl-(4S)-methoxy-(2S)-pyrrolidinyl)carbaldehyde oxime (2.28 g, 9.34 mmol) and triethylamine (0.065 ml) were dissolved in methylene chloride (40 ml), a 4% aqueous sodium hypochlorite solution (40ml) was added, and the mixture was vigorously stirred for 4 hours in a heterogeneous system. The reaction solution was extracted with methylene chloride two times, combined extracts were washed with an aqueous saturated sodium chloride solution, dried with anhydrous sodium sulfate, and concentrated under reduced pressure. The resulting residue was purified by column chromatography using silica gel to obtain the title compound (1.0 g, 30%) as a brown oily substancefrom a hexane: ethyl acetate (3:1-1:1)-eluted part.
$^1$H-NMR ($CDCl_3$) δ: 1.36-1.53 (9H, m), 2.21-2.47 (2H, m), 2.75 (2H, t, J = 7.6 Hz), 3.04-3.10 (2H, m), 3.17-4.04 (6H, m), 4.88-5.07 (1H, m), 5.13 (2H, s), 6.01 (1H, s), 7.33-7.37 (5H, m).
3-[3-((4S)-methoxy-(2S)-pyrrolidinyl)-5-isoxazole]propionic acid benzyl ester hydrochloride
[Chem. 96]

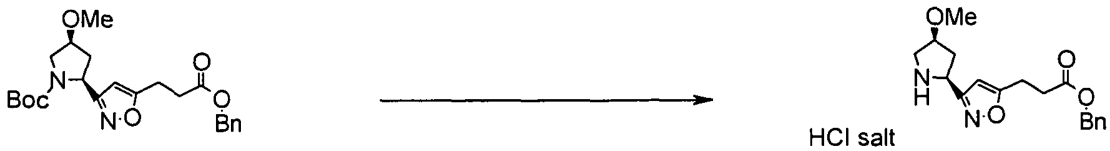



3-[3-(1-tert-butoxycarbonyl-(4S)-methoxy-(2S)-pyrrolidinyl)-5-isoxazole]propionic acid benzyl ester (1.0 g, 2.32 mmol) was dissolved in a 4N hydrochloric acid dioxane solution (25 ml) and the solution was stirred at room temperature for 1 hour and a half. After concentrated under reduced pressure, the residue was used in the next reaction without purification.

3-[3-[1-[2,5-Dichloro-4-(1-methyl-3-indolylcarbonylamino)phenylacetyl]-(4S)-methoxy-(2S)-pyrrolidinyl]-5-isoxazole]propionic acid benzyl ester

[Chem. 97]

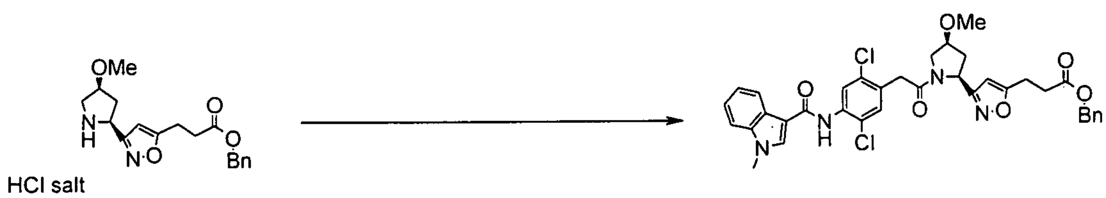



3-[3-((4S)-methoxy-(2S)-pyrrolidinyl)-5-isoxazole] propionic acid benzyl ester hydrochloride (0.58 mmol), 2,5-dichloro-4-(1-methyl-3-indolylcarbonylamino)phenylac etic acid (209 mg, 0.58 mmol), EDC (167 mg, 0.87 mmol), HOBt (78 mg, 0.58 mmol) and triethylamine (0.48 ml, 3.5 mmol) were dissolved in methylene chloride (15 ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 8 : 92, eluted with methanol : methylene chloride = 3 : 7) to obtain the title compound (140 mg, 36%) as a brown oily substance.

$^1$H-NMR ($CDCl_3$) δ: 2.17-2.79 (4H, m), 3.02-3.12 (2H, m), 3.21-4.11 (11H, m), 5.12 and 5.13 (total 2H, each s), 5.24-5.34 (1H, m), 5.97 and 6.15 (total 1H, each s), 7.30-7.44 (9H, m), 7.79 (1H, d, J = 4.4 Hz), 8.11-8.15 (1H, m), 8.22-8.24 (1H, m), 8.75-8.81 (1H, m).

3-[3-[1-[2,5-Dichloro-4-(1-methyl-3-indolylcarbonylamino)phenylacetyl]-(4S)-methoxy-(2S)-pyrrolidinyl]-5-isoxazole]propionic acid

[Chem. 98]

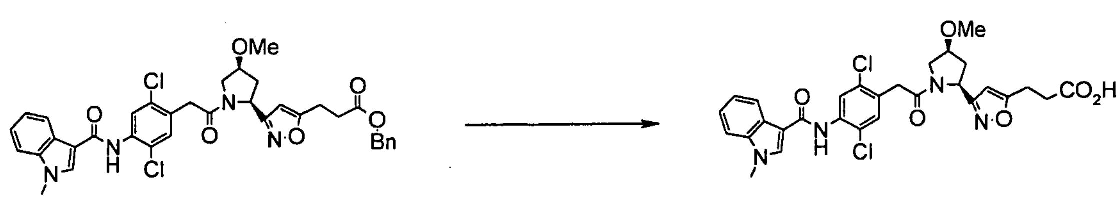

3-[3-[1-[2,5-Dichloro-4-(1-methyl-3-indolylcarbonylamino)phenylacetyl]-(4S)-methoxy-(2S)-pyrrolidinyl]-5-isoxazole]propionic acid benzyl ester (210 mg, 0.30 mmol) was dissolved in THF (10ml), a 1N aqueous sodiumhydroxide solution (1.5 ml, 1.5 mmol) and methanol (5.0 ml) were added, and the mixture was stirred at room temperature overnight. The reaction solution was concentrated under reduced pressure, a small amount of water was added, this was made neutral with 1N hydrochloric acid, and this was extracted with methylene chloride two times. The combined extracts were dried with anhydrous sodium sulfate, and concentrated under reduced pressure. The residue was purified by preparative TLC (Merck, Silica gel 60, $F_{254}$, 2mm; developed with methanol : methylene chloride = 8 : 92, eluted with methanol : methylene chloride = 3 : 7) to obtain the title compound (90 mg, 49%) as a white powder.

$^1$H-NMR (DMSO-$d_6$) δ: 2.14-2.61 (4H, m), 2.87-2.99 (2H, m), 3.14 and 3.17 (total 3H, each s), 3.22-4.12 (8H, m), 5.08-5.36 (1H, m), 6.03 and 6.28 (total 1H, each s), 7.17-7.58 (4H, m), 7.85-7.89 (1H, m), 8.14-8.16 (1H, m), 8.30-8.31 (1H, m), 9.38-9.41 (1H, m).
IR (ATR) $cm^{-1}$ : 1648, 1500, 1375, 1101, 1076, 744.
MS (LC-ESI) m/z : 599 ($M^+$+1).
Anal. Calcd for $C_{29}H_{28}Cl_2N_4O_6 \cdot H_2O$: C, 56.41; H, 4.90; N, 9.07. Found: C, 56.67; H, 4.81; N, 8.92.
Compounds of Examples 31 to 33 shown below were produced by the same synthesizing method as that of Example 30.

Example 31

3-[3-[1-[5-Chloro-2-fluoro-4-(1-methyl-3-indolylcarbonyl)aminophenylacetyl]-(4S)-methoxy-(2S)-pyrrolidinyl]-5-isoxazolyl]propionic acid
[Chem. 99]

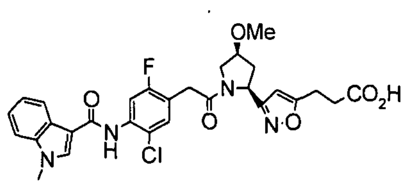

$^1$H-NMR (DMSO-$d_6$) δ: 2.12-2.59 (4H, m), 2.85-2.97 (2H, m), 3.15 (3H, s), 3.43-4.14 (8H, m), 5.07-5.37 (1H, m), 5.99 and 6.25 (total 1H, each s), 7.19-7.72 (5H, m), 8.13-8.18 (1H, m), 8.30-8.34 (1H, m), 9.29-9.42 (1H, m).
IR (ATR) $cm^{-1}$ : 2937, 1648, 1513, 1402, 1222, 1099, 744.
MS (LC-ESI) m/z : 583 ($M^+$+1).
Anal. Calcd for $C_{29}H_{28}ClFN_4O_6 \cdot H_2O$: C, 57.95; H, 5.03; N, 9.32. Found: C, 58.05; H, 5.03; N, 9.14.

Example 32

3-[3-[1-[[4-[(3-Benzo[b]thienylcarbonyl)amino]-2,5-dichlorophenyl]acetyl]-(4S)-methoxy-(2S)-pyrrolidinyl]-5-isoxazolyl]propionic acid
[Chem. 100]

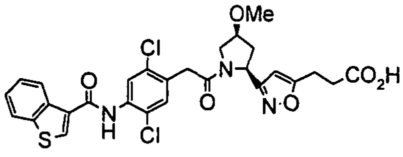

$^1$H-NMR (DMSO-$d_6$) δ: 2.13-2.58 (4H, m), 2.85-2.93 (2H, m), 3.14 and 3.17 (total 3H, each s), 3.22-4.13 (5H, m), 5.06-5.37 (1H, m), 6.00 and 6.25 (total 1H, each s), 7.44-7.77 (4H, m), 8.10 (1H, d, J=6.7Hz), 8.45-8.46 (1H, m), 8.66-8.68 (1H, m), 10.26 (1H, br s).
IR (ATR) $cm^{-1}$ : 1646, 1504, 1079, 765.
MS (LC-ESI) m/z : 602 ($M^+$+1).

Anal. Calcd for $C_{28}H_{25}Cl_2N_3O_6S \cdot H_2O$: C, 54.20; H, 4.39; N, 6.77. Found: C, 54.22; H, 4.24; N, 7.04.

Example 33

3-[3-[1-[[4-[(3-Benzo[b]thienylcarbonyl)amino]-5-chloro-2-fluorophenyl]acetyl]-(4S)-methoxy-(2S)-pyrrolidinyl]-5-isoxazolyl]propionic acid
[Chem. 101]

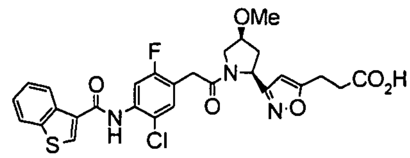

$^1$H-NMR (DMSO-$d_6$) δ: 2.11-2.56 (4H, m), 2.85-2.95 (2H, m), 3.15 (3H, s), 3.23-4..14 (5H, m), 5.07-5.39 (1H, m), 5.97 and 6.24 (total 1H, each s), 7.37-7.58 (4H, m), 8.09-8.10 (1H, m), 8.44-8.51 (1H, m), 8.65-8.69 (1H, m), 10.16-10.22 (1H, m).
IR (ATR) $cm^{-1}$ : 1644, 1515, 1402, 1218, 765.
MS (LC-ESI) m/z : 586 ($M^+$+1).
Anal. Calcd for $C_{28}H_{25}ClFN_3O_6S \cdot H_2O$: C, 55.68; H, 4.51; N, 6.96. Found: C, 55.78; H, 4.44; N, 7.17.

Example 34

(1-Benzyloxycarbonyl-(4S)-tert-butyldimethylsilyloxy-(2S)-pyrrolidinyl)carboxylic acid methyl ester
[Chem. 102]

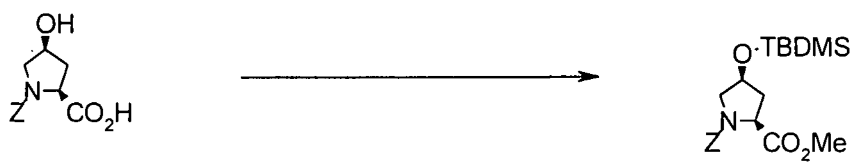

(1-Benzyloxycarbonyl-(4S)-hydroxy-(2S)-pyrrolidinyl) carboxylic acid (5.3 g, 20 mmol) was dissolved in DMF (50 ml), potassium carbonate (5.5 g, 40 mmol) and methyl iodide (1.86 ml, 30 mmol) were added, and the mixture was stirred at 70˚C for 1 hour. To the reaction solution was added water, followed by extraction with ethyl acetate two times. The combined extracts were dried with anhydrous sodium sulfate, and concentrated under reduced pressure. The resulting residue was dissolved in DMF (50 ml), TBDMS-Cl (4.5 g, 30 mmol) and imidazole (4.0 g, 60 mmol) were added, and the mixture was stirred at 50˚C overnight. To the reaction solution was added water, this was extracted with ethyl acetate two times, and combined extracts were dried with anhydrous sodium sulfate, and concentrated under reduced pressure. The residue was purified by column chromatography using silica gel to obtain the title compound (7.1 g, 90%) as a colorless oily substance from a hexane: ethyl acetate (9:1-4:1)-eluted part.
MS (LC-ESI) m/z : 394 ($M^+$+1).
(1-Benzyloxycarbonyl-(4S)-isopropoxy-(2S)-pyrrolidinyl)carboxylic acid methyl ester
[Chem. 103]

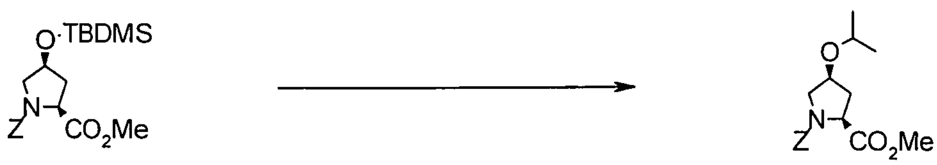

Bismuth tribromide (673 mg, 1.5 mmol) was suspended in acetonitrile (30 ml), triethylsilane (1.2 ml, 7.5 mmol) was added at 0˚C, the mixture was stirred for 5 minutes, and a solution (15 ml) of (1-benzyloxycarbonyl-(4S)-tert-butyldimethylsilyloxy-(2S) -pyrrolidinyl)carboxylic acidmethyl ester (1.97g, 5.0 mmol) and acetone (1.83 ml, 25 mmol) in acetonitrile was added. After stirred at 0˚C for 1 hour, this was stirred at room temperature for 6 hours. An aqueous saturated sodium bicarbonate solution was added to the reaction solution, this was extracted with ethyl acetate two times, and combined extracts were dried with anhydrous sodium sulfate, and concentrated under reduced pressure. The residue was purified by column chromatography using silica gel to obtain the title compound (730 mg, 46%) as a colorless oily substance from a hexane: ethyl acetate (9:1-3:1)-eluted part.

$^1$H-NMR ($CDCl_3$) δ: 1.08 (3H, d, J = 6.1 Hz), 1.10 (3H, d, J=6.1Hz), 2.24-2.27 (2H, m), 3.48-3.73 (6H, m), 4.10-4.13 (1H, m), 4.50-4.40 (1H, m), 5.05-5.21 (2H, m), 7.26-7.37 (5H, m) .

(1-tert-butoxycarbonyl-(4S)-isopropoxy-(2S)-pyrrolidinyl)carboxylic acid methyl ester

[Chem. 104]

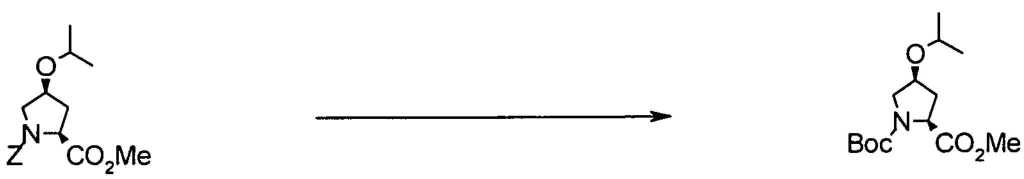

(1-Benzyloxycarbonyl-(4S)-isopropoxy-(2S)-pyrrolid inyl)carboxylic acid methyl ester (730 mg, 2.3 mmol) was dissolved in ethanol (30 ml), Pd-C (200 mg) was added, and hydrogenation was performed at room temperature overnight. The catalyst was removed by filtration, and the filtrate was concentrated under reduced pressure. The resulting residue was dissolved in methylene chloride (30 ml), and $Boc_2O$ (594 mg, 2.7 mmol) and triethylamine (0.63 ml, 4.5 mmol) were successively added at room temperature. After stirred for 3 hours, the mixture was concentrated under reduced pressure, and the residue was purified by column chromatography using silica gel to obtain the title compound (460 mg, 71%) as a colorless oily substance from a hexane: ethyl acetate (9:1-2:1)-eluted part.

$^1$H-NMR ($CDCl_3$) δ: 1.09-1.12 (6H, m), 1.42 and 1.47 (total 9H, each s), 2.14-2.32 (2H, m), 3.39-3.68 (3H, m), 3.71 (3H, s), 4.07-4.12 (1H, m), 4.25-4.43 (1H, m). (1-tert-butoxycarbonyl-(4S)-isopropoxy-(2S)-pyrrolidinyl)carboxylic acid

[Chem. 105]

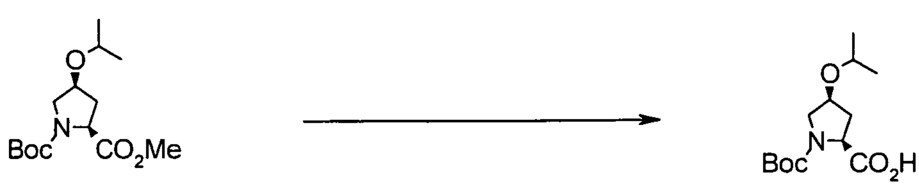

(1-tert-butoxycarbonyl-(4S)-isopropoxy-(2S)-pyrrol idinyl)carboxylic acid methyl ester (460 mg, 1.6 mmol) was

dissolved in a mixed solution of THF (10 ml) and methanol (5.0 ml), a 1N aqueous sodium hydroxide solution (3.0 ml, 3.0 mmol) was added at room temperature, and the mixture was stirred overnight. THF and methanol were removed under reduced pressure, this was made weakly acidic with 1N hydrochloric acid, and extracted with ethyl acetate two times. The combined extracts were washed with an aqueous saturated sodium chloride solution, dried with anhydrous sodium sulfate, and concentrated under reduced pressure to obtain the title compound (9.6 g, 87%) as a colorless oily substance.

5-[1-tert-butoxycarbonyl-(4S)-isopropoxy-(2S)-pyrrolidinylcarbonylamino]levulinic acid methyl ester

[Chem. 106]

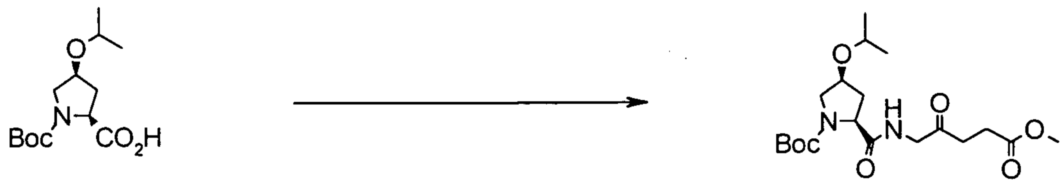

(1-tert-butoxycarbonyl-(4S)-isopropoxy-(2S)-pyrrolidinyl) carboxylic acid (820 mg, 3.0 mmol), 5-aminolevulinic acid methyl ester hydrochloride (546 mg, 3.0 mmol), HOBt (405 mg, 3.0 mmol) and EDC (864 mg, 4.5 mmol) were dissolved in methylene chloride (50 ml), triethylamine (2.5 ml) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.2 g, 100%) as a colorless oily substance from a methylene chloride: methanol (97 : 3) -eluted part.

$^1$H-NMR ($CDCl_3$) δ: 1.04 (3H, d, J = 6.1 Hz), 1.08 (3H, d, J= 6.1 Hz), 1.44 (9H, br s), 2.07-2.51 (2H, m), 2.62-2.67 (2H, m), 2.74 (2H, t, J = 6.5 Hz), 3.46-3.65 (3H, m), 3.67 (3H, s), 3.98-4.13 (2H, m), 4.19-4.39 (2H, m), 7.00-7.18 (1H, m) .
3-[2-(1-tert-butoxycarbonyl-(4S)-isopropoxy-(2S)-pyrrolidinyl)-5-thiazolyl]propionic acid methyl ester

[Chem. 107]

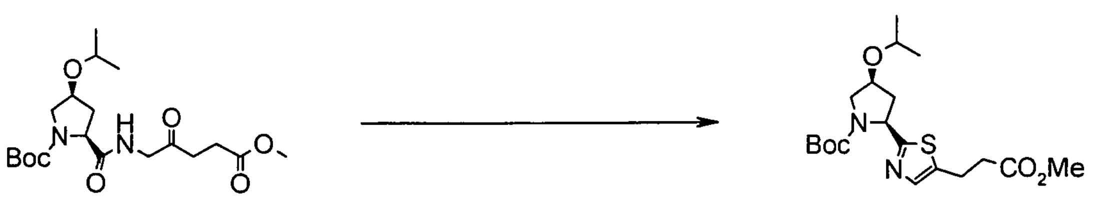

5-[1-tert-butoxycarbonyl-(4S)-isopropoxy-(2S)-pyrrolidinylcarbonylamino]levulinic acid methyl ester (1.2 g, 3.0 mmol) was dissolved in toluene (50ml), a Lawesson's reagent (1.34 g, 3.3 mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, silica gel 60, $F_{254}$, 2mm; developed with methanol : methylene chloride = 5 : 95, eluted with methanol : methylene chloride = 3 : 7) to obtain the title compound (1.0 g, 84%) as a yellow oily substance.

$^1$H-NMR ($CDCl_3$) δ: 0.76-1.10 (6H, m), 1.29-1.51 (9H, m), 2.30-2.57 (2H, m), 2.64 (2H, t, J = 7.3 Hz), 3.04-3.14 (2H, m), 3.46-3.76 (6H, m), 4.14-4.19 (1H, m), 5.03-5.20 (1H, m), 7.35 (1H, s).

3-[2-((4S)-isopropoxy-(2S)-pyrrolidinyl)-5-thiazolyl]propionic acid methyl ester hydrochloride

[Chem. 108]

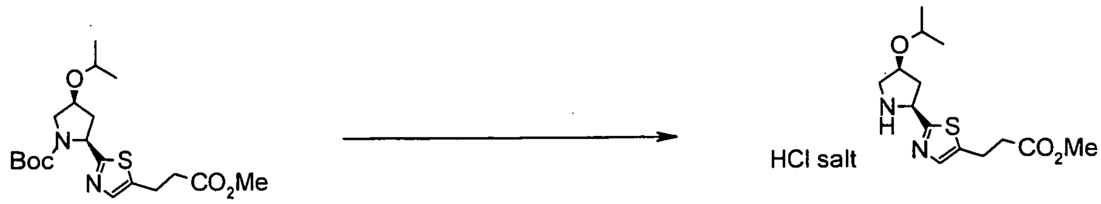

3-[2-(1-tert-butoxycarbonyl-(4S)-isopropoxy-(2S)-pyrrolidinyl)-5-thiazolyl]propionic acid methyl ester (1.0 g, 2.5 mmol) was dissolved in a 4N hydrochloric acid dioxane solution (25 ml), and the solution was stirred at room temperature for 1 hour and a half. After concentrated under reduced pressure, the residue was used in the next reaction without purification.

3-[2-[1-[2,5-Dichloro-4-(1-methyl-3-indolylcarbonylamino)phenylacetyl]-(4S)-isopropoxy-(2S)-pyrrolidinyl]-5-thiazolyl]propionic acid methyl ester

[Chem. 109]

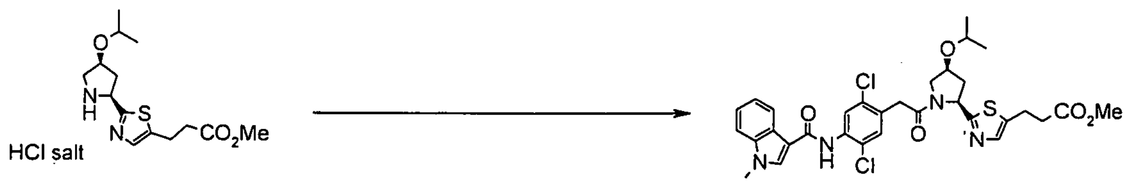

3-[2-((4S)-isopropoxy-(2S)-pyrrolidinyl)-5-thiazolyl]propionic acid methyl ester hydrochloride (0.5 mmol), 2,5-dichloro-4-(1-methyl-3-indolylcarbonylamino)phenylac etic acid (188 mg, 0.5 mmol), EDC (144 mg, 0.75 mmol), HOBt (68 mg, 0.5 mmol) and triethylamine (0.42 ml, 3.0 mmol) were dissolved in methylene chloride (15 ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 9 : 91, eluted with methanol : methylene chloride = 3 : 7) to obtain the title compound (270 mg, 84%) as a colorless oily substance.

$^1$H-NMR ($CDCl_3$) δ: 0.84 and 0.92 (total 3H, each d, J = 6.1 Hz), 1.06 and 1.09 (total 3H, each d, J=6.1 Hz), 2.31-2.67 (4H, m), 3.06-3.16 (2H, m), 3.44-3.94 (11H, m), 4.20-4.31 (1H, m), 5.38-5.50 (1H, m), 7.33-7.50 (5H, m), 7.80 and 7.81 (total 1H, each s), 8.11-8.15 (1H, m), 8.22-8.26 (1H, m), 8.74 and 8.82 (total 1H, each s).

3-[2-[1-[2,5-Dichloro-4-(1-methyl-3-indolylcarbonylamino)phenylacetyl]-(4S)-isopropoxy-(2S)-pyrrolidinyl]-5-thiazolyl] propionic acid

[Chem. 110]

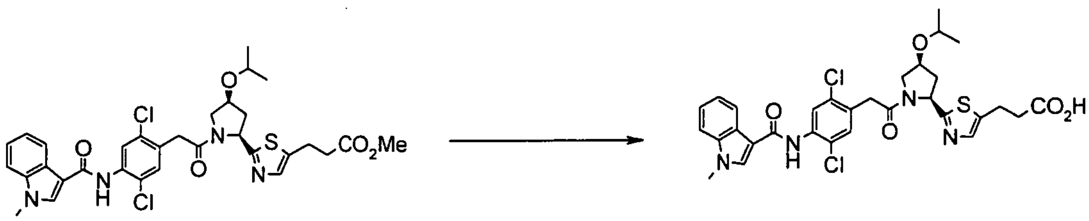

3-[2-[1-[2,5-Dichloro-4-(1-methyl-3-indolylcarbonylamino)phenylacetyl]-(4S)-isopropoxy-(2S)-pyrrolidinyl]-5-thiazolyl]propionic acid methyl ester (260 mg, 0.4 mmol) was dissolved in THF (10 ml), a 1N aqueous sodium hydroxide solution (1.5 ml, 1.5 mmol) and methanol (5.0 ml) were added, and the mixture was stirred at room temperature overnight. The reaction solution was concentrated under reducedpressure, a small amount of water was added, this was made neutral with 1N hydrochloric acid, and a precipitated solid was collected by filtration to obtain the title compound (200 mg, 79%) as a white powder.

$^1$H-NMR (DMSO-$d_6$) δ: 0.77 and 0.83 (total 3H, each d, J = 6.1 Hz), 1.01 (3H, d, J = 5.9 Hz), 2.29-2.58 (4H, m), 2.93-3.03 (2H, m), 3.32-3.95 (8H, m), 4.19-4.35 (1H, m), 5.21-5.54 (1H, m), 7.19-7.57 (5H, m), 7.87 and 7.92 (total 1H, each s), 8.15 (1H, d, J = 7.8 Hz), 8.29 and 8.30 (total 1H, each s), 9.35-9.40 (1H, m).
IR (ATR) $cm^{-1}$ : 1654, 1500, 1375, 1218, 1101, 1078, 744.
MS (LC-ESI) m/z : 643 ($M^+$+1).
Anal. Calcd for $C_{31}H_{32}Cl_2N_4O_5S \cdot 0.5H_2O$: C, 57.06; H, 5.10; N, 8.59. Found: C, 57.18; H, 5.01; N, 8.62.
Compounds of Examples 35 to 38 shown below were produced by the same synthesizing method as that of Example 34.

Example 35

3-[2-[1-[5-Chloro-2-fluoro-4-(1-methyl-3-indolylcarbonyl)aminophenylacetyl]-(4S)-isopropoxy-(2S)-pyrrolidinyl]-5-thiazolyl]propionic acid

[Chem. 111]

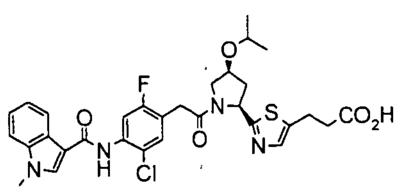

$^1$H-NMR (DMSO-$d_6$) δ: 0.77 and 0.80 (total 3H, each d, J = 6.1 Hz), 0.99-1.01 (3H, m), 2.36-2.55 (4H, m), 2.93-3.05 (2H, m), 3.39-4.01 (8H, m), 4.21-4.34 (1H, m), 5.19-5.57 (1H, m), 7.19-7.76 (6H, m), 8.15 (1H, d, J = 7.6 Hz), 8.30 and 8.31 (total 1H, each s), 9.29 and 9.32 (total 1H, each s). IR (ATR) $cm^{-1}$ : 1654, 1513, 1402, 1218, 1101, 744.
MS (LC-ESI) m/z : 627 ($M^+$+1)
Anal. Calcd for $C_{31}H_{32}ClFN_4O_5S \cdot 0.5H_2O$: C, 58.53; H, 5.23; N, 8.81. Found: C, 58.79; H, 5.12; N, 8.82.

Example 36

3-[2-[1-[4-[(3-Benzo[b]thienylcarbonyl)amino]-5-chloro-2-fluorophenylacetyl]-4S-isopropoxy-(2S)-pyrrolidinyl]-5-thiazolyl]propionic acid

[Chem. 112]

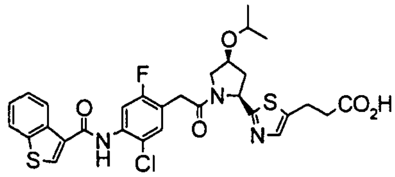

$^1$H-NMR (DMSO-$d_6$) δ: 0.75-0.81 (3H, m), 0.96-1.03 (3H, m), 2.32-2.58 (4H, m), 2.92-3.04 (2H, m), 3.41-3.99 (5H, m), 4.20-4.35 (1H, m), 5.20-5.55 (1H, m), 7.34-7.61 (5H, m), 8.10 (1H, d, J = 8.1 Hz), 8.45 (1H, d, J = 7.3 Hz), 8.64-8.66 (1H, m), 10.08-10.12 (1H, m).
IR (ATR) $cm^{-1}$ : 2969, 1513, 1400, 1216, 765.
MS (LC-ESI) m/z : 630 ($M^+$+1).
Anal. Calcd for $C_{30}H_{29}ClFN_3O_5S_2 \cdot 0.2H_2O$: C, 56.86; H, 4.68; N, 6.63. Found: C, 56.87; H, 4.87; N, 6.47.

Example 37

3-[2-[1-[4-[(1-Benzofuran-3-ylcarbonyl)amino]-2,5-dichlorophenylacetyl]-(4S)-isopropoxy-(2S)-pyrrolidinyl]5-thiazolyl]propionic acid
[Chem. 113]

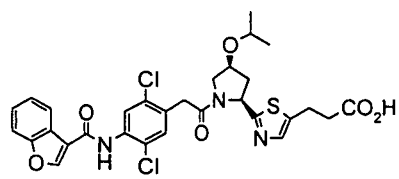

$^1$H-NMR (DMSO-$d_6$) δ: 0.77 and 0.83 (total 3H, each d, J = 6.1 Hz), 1.01 (3H, d, J = 5.9 Hz), 2.32-2.58 (4H, m), 2.93-3.03 (2H, m), 3.38-3.99 (5H, m), 4.22-4.35 (1H, m), 5.21-5.54 (1H, m), 7.34-7.82 (6H, m), 8.08-8.09 (1H, m), 8.81 and 8.82 (total 1H, each s), 10.01 and 10.05 (total 1H, each s), 12.25 (1H, br s). IR (ATR) $cm^{-1}$ : 1646, 1508, 1376, 1122, 1079, 748.
MS (LC-ESI) m/z : 630 ($M^+$+1).
Anal. Calcd for $C_{30}H_{29}Cl_2N_3O_6S \cdot 0.5H_2O$: C, 56.34 ; H, 4.73; N, 6.57. Found: C, 56.35; H, 4.65; N, 6.50.

Example 38

3-[2-[1-[4-[(1-Benzofuran-3-ylcarbonyl) amino]-5-chloro-3-fluorophenylacetyl] (4S)-ispropoxy-(2S)-pyrrolidinyl]-5-thiazolyl]propionic acid
[Chem. 114]

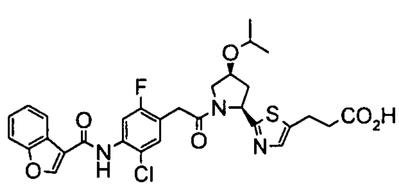

$^1$H-NMR (DMSO-$d_6$) δ: 0.75-0.82 (3H, m), 0.96-1.03 (3H, m), 2.36-2.55 (4H, m), 2.93-3.04 (2H, m), 3.38-3.99

(5H, m), 4.21-4.34 (1H, m), 5.21-5.56 (1H, m), 7.36-7.72 (6H, m), 8.08-8.09 (1H, m), 8.82-8.83 (1H, m), 9.98-10.01 (1H, m). IR (ATR) $cm^{-1}$ : 1521, 1402, 1120, 746.
MS (LC-ESI) m/z : 614 ($M^{+}$+1).
Anal. Calcd for $C_{30}H_{29}ClFN_3O_6S \cdot 0.4H_2O$: C, 58.00; H, 4.83; N, 6.76. Found: C, 58.15; H, 4.81; N, 6.68.

Example 39

(1-Benzyloxycarbonyl-(4S)-cyclobutoxy-(2S)-pyrrolidinyl)carboxylic acid methyl ester
[Chem. 115]

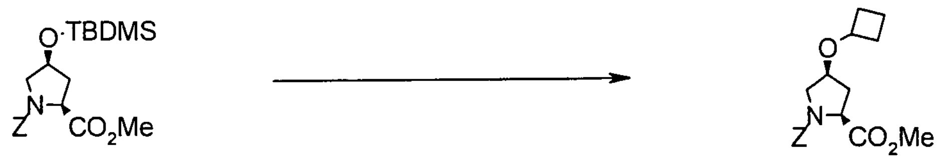

Bismuth tribromide (673mg, 1.5mmol) was suspended in acetonitrile (30ml), triethylsilane (1.6ml, 10mmol) was added at 0˚C, the mixture was stirred for 5 minutes, and a solution (15ml) of (1-benzyloxycarbonyl-(4S)-tert-butyldimethylsilyloxy-(2S) -pyrrolidinyl) carboxylic acid methyl ester (1. 97g, 5.0mmol) and cyclobutanone (1.1ml, 15mmol) in acetonitrile was added, and mixture was stirred at 0˚C for 1 hour, and stirred at room temperature for 6 hours. To the reaction solution was added an aqueous saturated sodium bicarbonate solution, this was extracted with ethyl acetate two times, and combined extracts were dried with anhydrous sodium sulfate, and concentrated under reduced pressure. The residue was purified by column chromatography using silica gel to obtain the title compound (1.26g, 76%) as a colorless oily substance from a hexane: ethyl acetate (7:3-4:1)-eluted part.
$^1$H-NMR ($CDCl_3$) δ: 1.39-1.53 (1H, m), 1.59-1.70 (1H, m), 1.80-1.94 (2H, m), 2.10-2.33 (4H, m), 3.46-3.52 (1H, m), 3.57-4.15 (6H, m), 4.35-4.47 (1H, m), 5.04-5.20 (2H, m), 7.28-7.38 (5H, m).
(1-tert-butoxycarbonyl-4S-cyclobutoxy-(2S)-pyrrodinyl-carboxylic acid methyl ester
[Chem. 116]

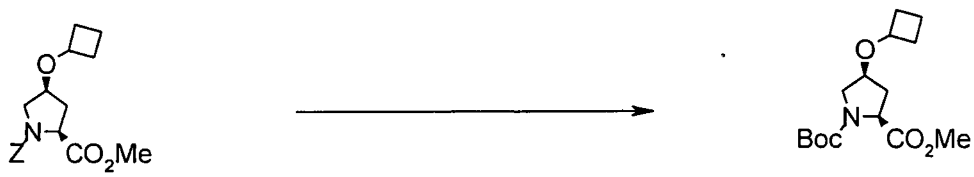

(1-Benzyloxycarbonyl-(4S)-cyclobutoxy-(2S)-pyrroli dinyl)carboxylic acid methyl ester (1.26g, 3.78mmol) was dissolved in methanol (15ml), $Pd(OH)_2$-C (300mg) was added, and hydrogenation wasperformedatroomtemperature overnight. A catalyst was removed by filtration, and the filtrate was concentrated under reduced pressure. The resulting residue was dissolved in methyl chloride (20ml), and $Boc_2O$ (1.1g, 4.9mmol) and triethylamine (3.2ml, 227mmol) were successively added at room temperature. After stirred for 3 hours, this was concentrated under reduced pressure, and the residue was purified by column chromatography using silica gel to obtain the title compound (1.0g, 88%) as a colorless oily substance from a hexane: ethyl acetate (9:1-7:3)-eluted part.
$^1$H-NMR ($CDCl_3$) δ: 1.39-1.51 (10H, m), 1.60-1.71 (1H, m), 1.83-1.95 (2H, m), 2.08-2.37 (4H, m), 3.37-3.42 (1H, m), 3.54-3.69 (1H, m), 3.72 (3H, s), 3.90-3.98 (2H, m), 4.23-4.41 (1H, m).
(1-tert-butoxycarbonyl-(4S)-cyclobutoxy-(2S)-pyrrolidinyl)carboxylic acid
[Chem. 117]

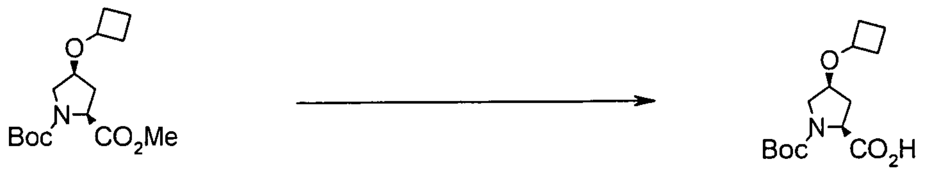

(1-tert-butoxycarbonyl-(4S)-cyclobutoxy-(2S)-pyrrolidinyl)carboxylic acid methyl ester (1.0g, 3.34mmol) was dissolved in a mixed solution of THF (20ml) and methanol (10ml), a 1N aqueous sodium hydroxide solution(6.0ml, 6.0mmol) was added at room temperature, and the mixture was stirred overnight. THF and methanol were removed under reduced pressure, this was made weakly acidic with 1N hydrochloric acid, followed by extraction with ethyl acetate two times. The combined extracts were washed with an aqueous saturated sodium chloride solution, dried with anhydrous sodium sulfate solution, and concentrated under reduced pressure to obtain the title compound (1.0g, 100%) as a colorless oily substance.

5-[1-tert-butoxycarbonyl-(4S)-cyclobutoxy-(2S)]-pyrrolidinylcarbonyl amino]levulinic acid methyl ester

[Chem. 118]

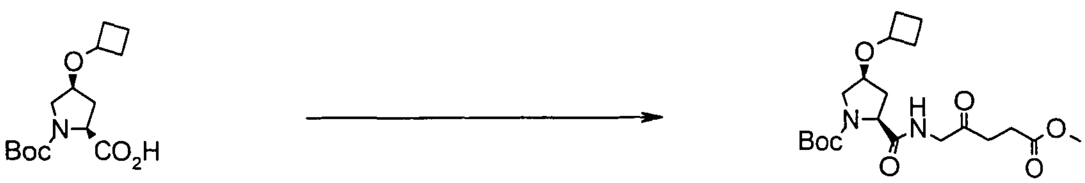

(1-tert-butoxycarbonyl-(4S)-cyclobutoxy-(2S)-pyrrolidinyl)carboxylic acid (1.0g, 3.0mmol), 5-aminolevulinic acidmethylesterhydrochloride (546mg, 3.0mmol), HOBt (405mg, 3.0mmol), and EDC (864mg, 4.5mmol) were dissolved in methylene chloride (50ml), trietyhlamine (2.5ml) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.2g, 100%) as a yellow oily substance from a methylene chloride: methanol (97:3)-eluted part.

$^1$H-NMR ($CDCl_3$) δ: 1.46 (10H, br s), 1.60-1.68 (1H, m), 1.80-1.89 (2H, m), 2.06-2.47 (4H, m), 2.64-2.76 (4H, m), 3.46-3.59 (3H, m), 3.67 (3H, s), 3.85-4.36 (4H, m). 3-[2-(1-tert-butoxycarbonyl-(4S)-cyclobutoxy-(2S)-pyrrolidinyl)-5-thiazolyl]propionic acid methyl ester

[Chem. 119]

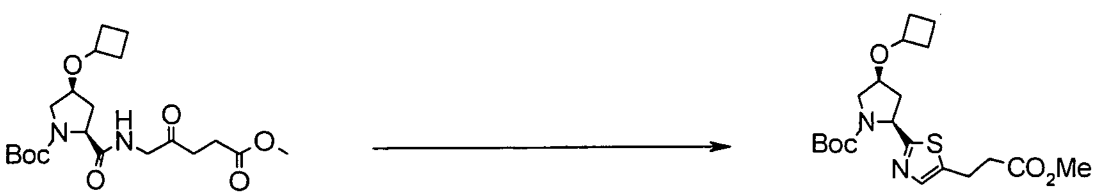

5-[1-tert-butoxdycarbonyl-(4S)-cyclobutoxy-(2S)-pyrrolidinylcarbonyl amino] levulinic acid methyl ester (1.2g, 3.0mmol) was dissolved in toluene (50ml), a Lawesson' s reagent (1.34g, 3.3mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride = 5 : 95, eluted with methanol : methylene chloride = 3 : 7) to obtain the title compound (1.04g, 85%) as a yellow oily substance.

$^1$H-NMR ($CDCl_3$) δ: 1.27-2.49 (17H, m), 2.65 (2H, t, J = 7.4 Hz), 3.11 (2H, br s), 3.46-3.87 (6H, m), 4.01-4.07 (1H, m), 5.00-5.17 (1H, m), 7.34 (1H, s). 3-[2-((4S)-cyclobutoxy-(2S)-pyrrolidinyl)-5-thiazolyl]propionic acid methyl ester hydrochloride

[Chem. 120]

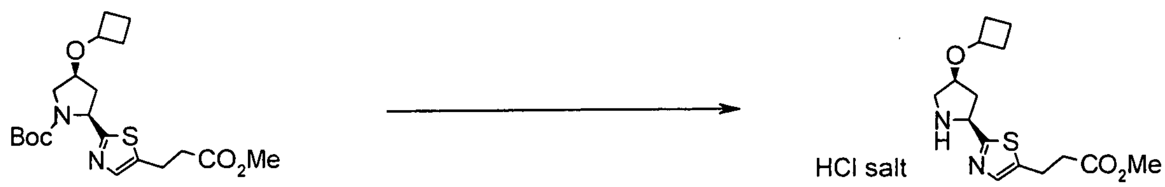

3-[2-(1-tert-butoxycarbonyl-(4S)-cyclobutoxy-(2S)-pyrrolidinyl)-5-thiazolyl][propionic acid methyl ester (1.04g, 2.53mmol) was dissolved in a 4N hydrochloric acid dioxane solution (30ml), and the solution was stirred at room temperature for 1 hour. After concentrated under reduced pressure, the residue was used in the next reaction without purification.

3-[2-[1-[2,5-Dichloro-4-(1-methyl-3-indolylcarbonylamino)phenylacetyl]-(4S)-cyclobutoxy-(2S)-pyrrolidinyl]-5-thiazolyl]propionic acid methyl ester

[Chem. 121]

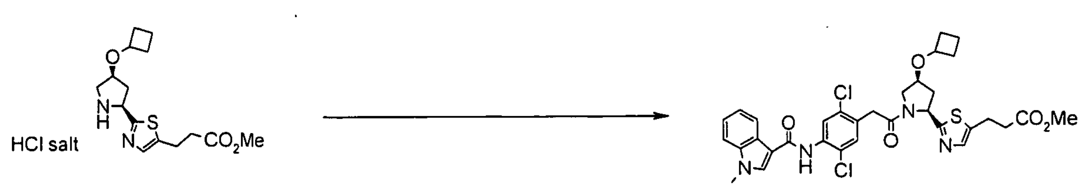

3-[2-((4S)-cyclobutoxy-(2S)-pyrrolidinyl)-5-thiazolyl]propionic acid methyl ester hydrochloride (0.5mmol), 2,5-dichloro-4-(1-methyl-3-indolylcarbonylamino)phenylac eticacid (188mg, 0.5mmol), EDC (144mg, 0.75,mmol), HOBt (68mg, 0.5mmol) and triethylamino (0.42ml, 3.0mmol) were dissolved in methylene chloride (15ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride = 7 : 93, eluted with methanol : methylene chloride = 3 : 7) to obtain the title compound (270mg, 81%) as a colorless oily substance.

$^1$H-NMR ($CDCl_3$) δ: 1.36-2.20 (4H, m), 2.35-2.58 (2H, m), 2.63-2.67 (2H, m), 3.09-3.13 (2H, m), 3.45-3.92 (11H, m), 4.09-4.17 (1H, m), 5.38-5.45 (1H, m), 7.26-7.52 (5H, m), 7.80 and 7.81 (total 1H, each s), 8.13-8.14 (1H, m), 8.23 and 8.26 (total 1H, each s), 8.74 and 8.82 (total 1H, each s).

3-[2-[1-[2,5-Dichloro-4-(1-methyl-3-indolylcarbonylamino)phenylacetyl]-(4S)-cyclobutoxy-(2S)-pyrrolidinyl]-5-thiazolyl]propionoic acid

[Chem. 122]

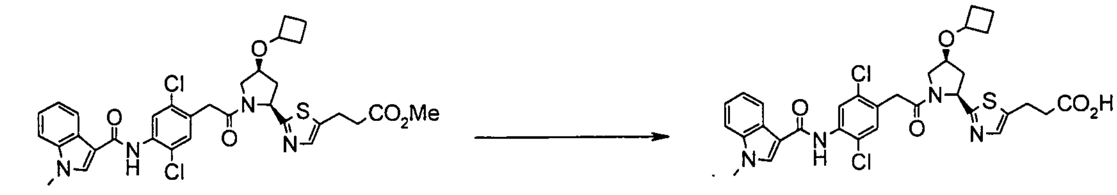

3-[2-[1-[2,5-Dichloro-4-(1-methyl-3-indolylcarbonylamino)phenylacetyl]-(4S)-cyclobutoxy-(2S)-pyrrolidinyl] -5-thiazolyl]propionic acid methyl ester (270mg, 0.4mmol) was dissolved in THF (10ml), a 1N aqueous sodium hydroxide solution(1.5ml, 1.5mmol) and methanol (5.0ml) were added, and the mixture was stirred at room temperature overnight. The reaction solution was concentrated under reduced pressure, a small amount of water was added, this was made neutral with 1N hydrochloric acid, and a precipitated solid was collected by filtration to obtain the title compound (210mg, 80%) as a white powder.

$^1$H-NMR (DMSO-$d_6$) δ: 1.32-2.44 (8H, m), 2.49-2.62 (2H, m), 2.94-3.04 (2H, m), 3.42-3.97 (8H, m), 4.10-4.21 (1H, m), 5.21-5.52 (1H, m), 7.19-7.57 (5H, m), 7.86 and 7.91 (total 1H, each s), 8.13-8.16 (1H, m), 8.29 and 8.30 (total 1H, each s), 9.35 and 9.39 (total 1H, each s). IR (ATR) $cm^{-1}$ : 1654, 1502, 1375, 1218, 1101, 744.
MS (LC-ESI) m/z : 655 ($M^+$+1).
Anal. Calcd for $C_{32}H_{32}Cl_2N_4O_5S \cdot 0.5H_2O$: C, 57.83; H, 5.00; N, 8.43. Found: C, 57.80; H, 4.96; N, 8.43.
Compounds of Examples 40 to 43 shown below were produced by the same synthesizing method as that of Example 39.

Example 40

3-[2-[1-[5-Chloro-2-fluoro-4-(1-methyl-3-indolylcarbonyl)aminophenylacetyl]-(4S)-cyclobutoxy-(2S)-pyrrolidinyl]-5-thiazolyl]propionic acid

[Chem. 123]

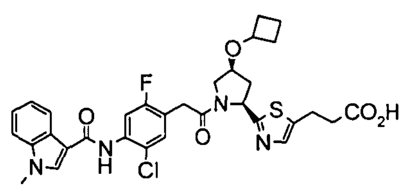

$^1$H-NMR (DMSO-$d_6$) δ: 1.30-2.45 (8H, m), 2.50-2.59 (2H, m), 2.94-3.06 (2H, m), 3.41-3.96 (8H, m), 4.10-4.21 (1H, m), 5.19-5.53 (1H, m), 7.20-7.74 (6H, m), 8.15 (1H, d, J = 7.8 Hz), 8.30 and 8.31 (total 1H, each s), 9.29 and 9.32 (total 1H, each s), 12.25 (1H, br s).
IR (ATR) $cm^{-1}$ : 1656, 1515, 1402, 1220, 1101, 744.
MS (LC-ESI) m/z : 639 ($M^+$+1).
Anal. Calcd for $C_{32}H_{32}ClFN_4O_5S \cdot 0.5H_2O$: C, 59.30; H, 5.13; N, 8.64. Found: C, 59.48; H, 5.07; N, 8.68.

Example 41

3-[2-[1-[4-[(3-Benzo[b]thienylcarbonyl)amino]-5-chloro-2-fluorophenylacetyl[(4S)-cyclobutoxy-(2S)-pyrrolidinyl]-5-thiazolyl]propionic acid

[Chem. 124]

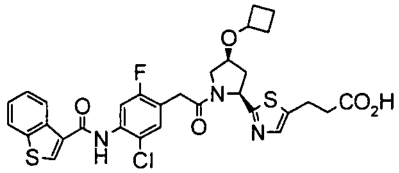

$^1$H-NMR (DMSO-$d_6$) δ: 1.30-2.45 (8H, m), 2.50-2.60 (2H, m), 2.95-3.04 (2H, m), 3.39-3.97 (5H, m), 4.10-4.21 (1H, m), 5.21-5.53 (1H, m), 7.34-7.61 (5H, m), 8.10 (1H, d, J = 8.1 Hz), 8.43-8.47 (1H, m), 8.64 and 8.65 (total 1H, each s), 10.09 and 10.12 (total 1H, each s).
IR (ATR) $cm^{-1}$ : 2937, 1652, 1515, 1402, 1216, 765.
MS (LC-ESI) m/z : 642 ($M^+$+1). Anal. Calcd for $C_{31}H_{29}ClFN_3O_5S_2 \cdot 0.4H_2O$: C, 57.34; H, 4.63; N, 6.47. Found: C, 57.50; H, 4.64; N, 6.48.

Example 42

3-[2-[1-[4-[(1-Benzofuran-3-ylcarbonyl)amino]-2,5-dichlorophenylacetyl]-(4S)-cyclobutoxy-(2S)-pyrrolidinyl]-5-thiazolyl]propionic acid
[Chem. 125]

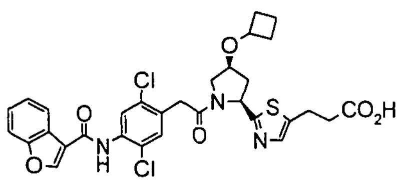

$^1$H-NMR (DMSO-$d_6$) δ: 1.33-2.44 (8H, m), 2.49-2.59 (2H, m), 2.96-3.05 (2H, m), 3.37-3.99 (5H, m), 4.11-4.21 (1H, m), 5.21-5.52 (1H, m), 7.33-7.80 (6H, m), 8.07-8.10 (1H, m), 8.81 and 8.82 (total 1H, each s), 10.02 and 10.05 (total 1H, each s), 12.27 (1H, br s).
IR (ATR) $cm^{-1}$ : 2937, 1646, 1571, 1506, 1103, 856, 748.
MS (LC-ESI) m/z : 642 ($M^+$+1). Anal. Calcd for $C_{31}H_{29}Cl_2N_3O_6S \cdot 0.5H_2O$: C, 57.15; H, 4.64; N, 6.45. Found: C, 57.19; H, 4.64; N, 6.47.

Example 43

3-[2-[1-[4-[(1-Benzofuran-3-ylcarbonyl)amino]-5-chloro-2-fluorophenylacetyl]-(4S)-cyclobutoxy-(2S)-pyrrolidinyl]-5-thiazolyl]propionic acid
[Chem. 126]

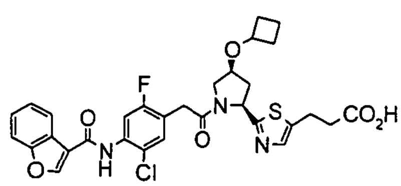

$^1$H-NMR (DMSO-$d_6$) δ: 1.31-2.45 (8H, m), 2.51-2.60 (2H, m), 2.94-3.05 (2H, m), 3.40-3.97 (5H, m), 4.12-4.20

(1H, m), 5.20-5.54 (1H, m), 7.35-7.61 (5H, m), 7.71 (1H, d, J = 8.1 Hz), 8.07-8.10 (1H, m), 8.82 and 8.83 (total 1H, each s), 9.98 and 10.01 (total 1H, each s).
IR (ATR) $cm^{-1}$ : 2937, 1648, 1521, 1402, 1103, 746.
MS (LC-ESI) m/z : 626 ($M^{+}$+1). Anal. Calcd for $C_{31}H_{29}ClFN_3O_6S \cdot 0.5H_2O$: C, 58.63; H, 4.76; N, 6.62. Found: C, 58.75; H, 4.77; N, 6.60.

Example 44

(1-tert-butoxycarbonyl-(4R)-hydroxy-(2S)-pyrrolidinyl)carboxylic acid benzyl ester
[Chem. 127]

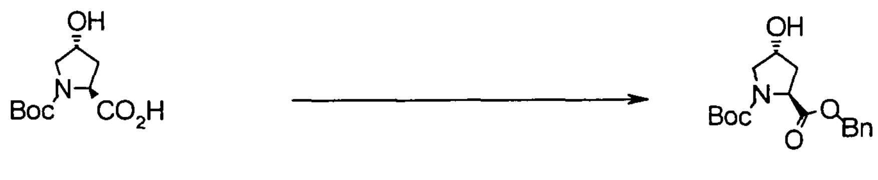

(1-tert-butoxycarbonyl-(4R)-hydroxy-(2S)-pyrrolidinyl)carboxylic acid (6.93g, 30mmol) was dissolved in DMF (100ml), potassium carbonate (8.28g, 60mmol) and benzyl bromide (4.27ml, 36mmol) were added, and the mixture was stirred at 80˚C for 1 hour. To the reaction solution was added water, followed by extraction with ethyl acetate two times. The combined extracts were dried with anhydrous sodium sulfate, and concentrated under reduced pressure. The resulting residue was purified by column chromatography using silica gel to obtain the title compound (9.0g, 93%) as a colorless oily substance from a hexane-ethyl acetate (3:1-1:1) -eluted part.
$^1$H-NMR ($CDCl_3$) δ: 1.31-1.45 (9H, m), 2.03-2.10 (1H, m), 2.22-2.33 (1H, m), 3.46-3.65 (2H, m), 4.41-4.51 (2H, m), 5.07-5.28 (2H, m), 7.28-7.39 (5H, m).
((4R)-acetoxy-1-tert-butoxycarbonyl-(2S)-pyrrolidinyl)carboxylic acid benzyl ester
[Chem. 128]

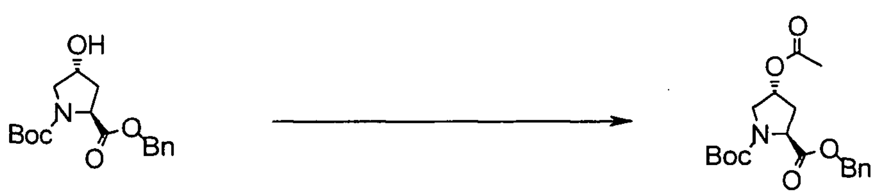

(1-tert-butoxycarbonyl-(4R)-hydroxy-(2S)-pyrrolidinyl) carboxylic acid benzyl ester (3.21g, 10mmol) was dissolved in methylene chloride (50ml), acetic anhydride (1.1ml, 12mmol), pyridine (1.2ml, 15mmol), and dimethylaminopyridine (100mg) were added, and the mixture was stirred at room temperature for 2 hours. The reaction solution was concentrated under reduced pressure, and water was added, followed by extraction with ethyl acetate two times. The combined extracts were dried with anhydrous sodium sulfate, and concentrated under reduced pressure. The resulting residue was purified by column chromatography using silica gel to obtain the title compound (3.48g, 96%) as a colorless oily substance from a hexane: ethyl acetate (7:1-3:1)-eluted part.
$^1$H-NMR ($CDCl_3$) δ: 1.35 and 1.45 (total 9H, each s), 2.05 (3H, s), 2.16-2.42 (2H, m), 3.56-3.69 (2H, m), 4.36-4.51 (1H, m), 5.08-5.29 (3H, m), 7.29-7.41 (5H, m).
((4R)-acetoxy-1-tert-butoxycarbonyl-(2S)-pyrrolidinyl)carboxylic acid
[Chem. 129]

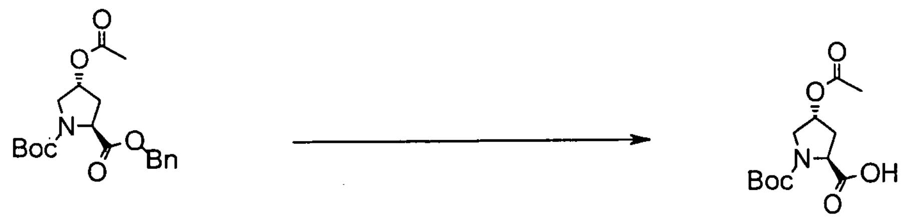

((4R)-acetoxy-1-tert-butoxycarbonyl-(2S)-pyrrolidinyl)carboxylic acid benzyl ester (3.48g, 9.57mmol) was dissolved in ethanol (35ml), $Pd(OH)_2$-C (500mmg) was added, and hydrogenation was performed at room temperature overnight. A catalyst was removed by filtration, and the filtrate was concentrated under reduced pressure to obtain the title compound (2.5g, 96%) as a white solid.

$^1$H-NMR ($CDCl_3$) δ: 1.44 and 1.49 (total 9H, each s), 2.07 (3H, s), 2.24-2.48 (2H, m), 3.54-3.76 (2H, m), 4.37-4.47 (1H, m), 5.26-5.31 (1H, m).

5-[(4R)-acetoxy-1-tert-butoxycarbonyl-(2S)-pyrrolidinylcarbonylamino]levulinic acid methyl ester

[Chem. 130]

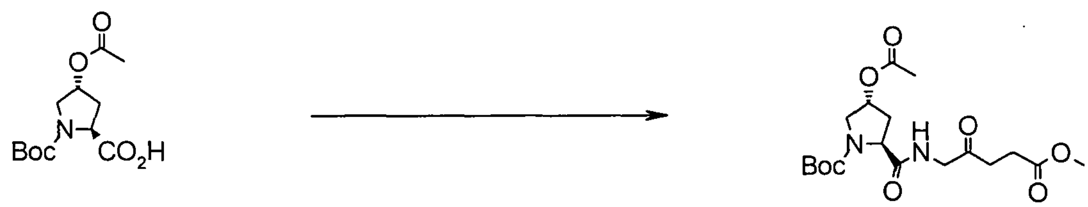

((4R)-acetoxy-1-tert-butoxycarbonyl-(2S)-pyrrolidinyl)carboxylic acid (1.36, 5.0mmol), 5-aminolevulinic acid methyl ester hydrochloride (908mg, 5.0mmol), HOBt (675mg, 5.0mmol) and EDC (1.44g, 7.5mmol) were dissolved in methylene chloride (50ml), triethylamine (4.2ml, 30mmol) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (2.0g, 100%) as a yellow oily substance from a methylene chloride: methanol (97: 3)-eluted part.

$^1$H-NMR ($CDCl_3$) δ: 1.46 (9H, br s), 2.05 (3H, s), 2.18-2.53 (2H, m), 2.59-2.77 (4H, m), 3.55-3.66 (3H, m), 3.68 (3H, s), 4.16-4.50 (3H, m), 5.26-5.29 (1H, m).

3-[2-((4R)-acetoxy-1-tert-butoxycarbonyl-(2S)-pyrrolidinyl)-5-thiazolyl]propionic acid methyl ester

[Chem. 131]

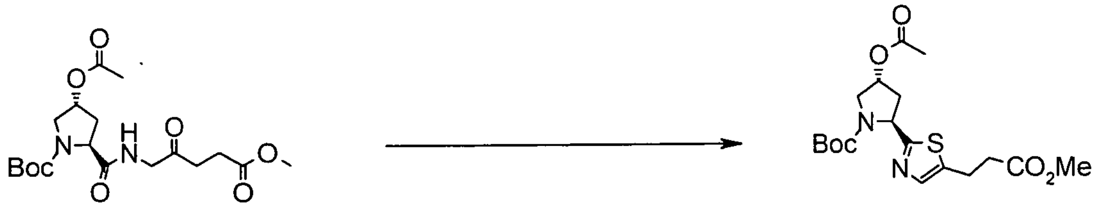

5-[(4R)-acetoxy-1-tert-butoxycarbonyl-(2S)-pyrrolidinylcarbonylamino]levulinic acid methyl ester (2.1g, 5.0mmol) was dissolved in toluene (100ml), a Lawesson's reagent (2.2g, 5.5mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride= 5 : 95, eluted with methanol : methylene chloride= 3 : 7) to obtain the title compound (1.7g, 85%) as a yellow oily substance.

$^1$H-NMR ($CDCl_3$) δ: 1.31-1.46 (9H, m), 2.07 (3H, s), 2.37-2.54 (2H, m), 2.66 (2H, t, J = 7.4 Hz), 3.13 (2H, br s), 3.59-3.81 (5H, m), 5.10-5.37 (2H, m), 7.38 (1H, s). 3-[2-(1-tert-butoxycarbonyl-(4R)-hydroxy-(2S)-pyrrolidinyl)-5-thiazolyl]propionic acid methyl ester

[Chem. 132]

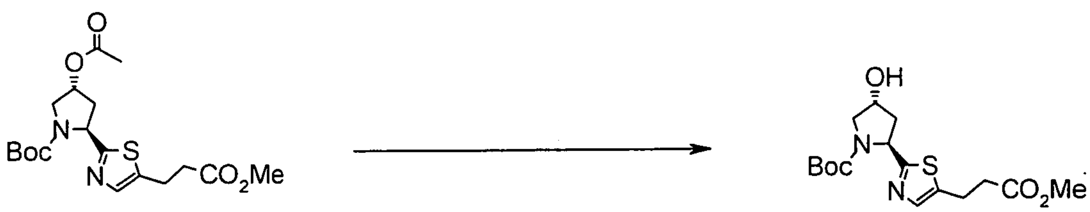

3-[2-((4R)-acetoxy-1-tert-butoxycarbonyl-(2S)-pyrrolidinyl)-5-thiazolyl]propionic acid methyl ester (1.7g, 4.3mmol) was dissolved inmethanol (45ml), potassium carbonate (1.2g, 8.5mmol) was added, and the mixture was stirred at room temperature for 1 hour. To the reaction solution was added water, followed by extraction with ethyl acetate two times. The combined extracts were washed with an aqueous saturated sodium chloride solution, dried with anhydrous sodium sulfate, and concentrated under reduced pressure to obtain the title compound (1.44g, 95%) as a brown oily substance.

$^1$H-NMR ($CDCl_3$) δ: 1.25-1.50 (9H, m), 2.26-2.49 (2H, m), 2.65 (2H, t, J = 7.4 Hz), 3.09-3.16 (2H, m), 3.65-3.71 (5H, m), 4.53-4.60 (1H, m), 5.15-5.29 (1H, m), 7.37 (1H, s).

3-[2-(1-tert-botoxycarbonyl-(4S)-(3-pyridyloxy)-2S)-pyrrolidinyl]-5-thiazolyl]propionic acid methyl ester

[Chem. 133]

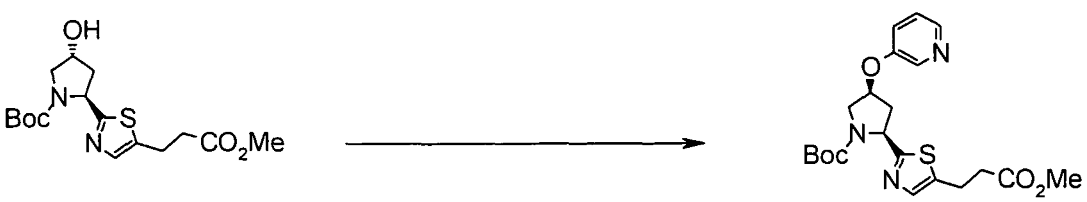

3-[2-(1-tert-butoxtycarbonyl-(4R)-hydroxy-(2S)-pyrrolidinyl)-5-thiazolyl]propionic acid methyl ester (712mg, 2.0mmol), 3-hydroxypyridine (228mg, 2,4mmol) and triphenylphosphine (681mg, 2.6mmol) were dissolved in THF (25ml), DIAD (0.5ml, 2.6mmol) was added, and the mixture was stirred at room temperature overnight. Since the reaction was not completed, triphenylphosphine (255mg, 1.2mmol) and DIAD(0.34ml, 1.2mmol) were further added, and the mixture was stirred at 60˚C for 1 hour. The reaction solution was concentrated under reduced pressure, and the resulting residue was purified by preparative TLC (Merck, Silica gel 60, $F_{254}$, 2mm; developed with methanol : methylene chloride= 6 : 94, eluted with methanol : methylene chloride = 3 : 7) to obtain a mixture (900mg) of the title compound and impurities.

3-[2-((4S)-(3-pyridyloxy)-(2S)-pyrrolidinyl)-5-thiazolyl]propionic acid methyl ester

[Chem. 134]

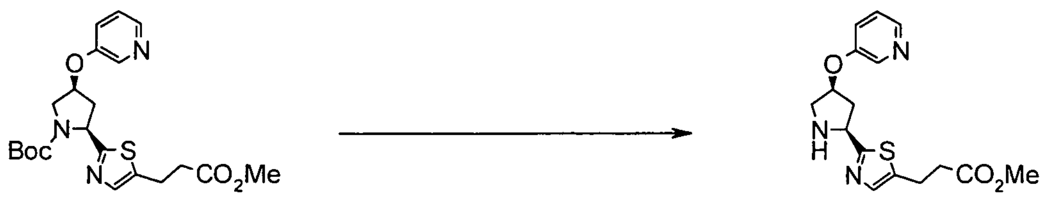

A mixture (900mg) of 3-[2-(1-tert-butoxycarbonyl-(4S)-(3-pyridyloxy)-(2S)-pyrrolidinyl)-5-thiazolyl]propionic acid methyl ester and impurities was dissolved in methanol (10ml), a 4N hydrochloric acid dioxane solution (30ml) was added, and the mixture was stirred at room temperature for 1 hour and a half. The reaction solution was concentrated under reduced pressure, and water was added, followed by washing with ethyl acetate two times. The aqueous layer was made alkaline with an aqueous saturate sodium bicarbonate solution, followed by extraction with methylene chloride two times. The combined extracts were dried with anhydrous sodium sulfate, and concentrated under reduce pressure, and the residue was used in the next reaction.

3-[2-[1-[2,5-Dichloro-4-(1-methyl-3-indolylcarbonylamino)phenylacetyl]-(4S)-(3-pyridyloxy-(2S)-pyrrolidinyl)-5-thiazolyl]propionic acid methyl ester

[Chem. 135]

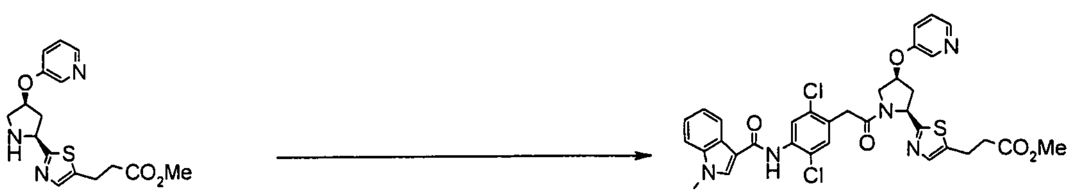

3-[2-((4S)-(3-pyridyloxy)-(2S)-pyrrolidinyl)-5-thiazolyl]propionic acid methyl ester (0.3mmol), 2,5-dichloro-4-(1-methyl-3-indolylcarbonylamino)phenylacetic acid (113mg, 0.3mmol), EDC (87mg, 0.45mmol), HOBt (41mg, 0.3mmol) and triethylamine (0.25ml, 1.8mmol) were dissolved in methylene chloride (15ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride= 8 : 92, eluted with methanol : methylene chloride= 3 : 7) to obtain the title compound (140mg, 68%) as a colorless oily substance.

$^1$H-NMR ($CDCl_3$) δ: 2.53-2.89 (4H, m), 3.10-3.19 (2H, m), 3.47-4.11 (10H, m), 5.04-5.15 (1H, m), 5.54-5.66 (1H, m), 6.92-7.04 (1H, m), 7.16-7.22 (1H, m), 7.31-7.53 (5H, m), 7.80 and 7.82 (total 1H, each s), 8.01-8.27 (4H, m), 8.77 and 8.82 (total 1H, each s).

3-[2-[1-[2,5-Dichloro-4-(1-methyl-3-indolylcarbonylamino)phenylacetyl]-(4S)-(3-pyridyloxy)-(2S)-pyrrolidinyl]-5-thiazolyl]propionic acid

[Chem. 136]

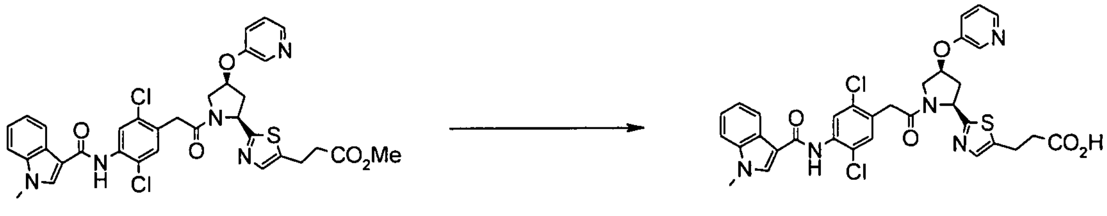

3-[2-[1-[2,5-Dichloro-4-(1-methyl-3-indolylcarbonylamino)phenylacetyl]-(4S)-(3-pyridyloxy)-(2S)-pyrrolidinyl]-5-thiazolyl]propionic acid methyl ester (140mg, 0.2mmol) was dissolved in THF (10ml), a 1N aqueous sodium hydroxide solution (1.5ml, 1.5mmol) and methanol (5.0ml) were added, and the mixture was stirred at room temperature overnight. The reaction solution was concentrated under reduced pressure, a small amount of water was added, this was made neutral with 1N hydrochloric acid, and a precipitate solid was collected by filtration to obtain the title compound (110mg, 80%) as a white powder.

$^1$H-NMR (DMSO-$d_6$) δ: 2.51-2.86 (4H, m), 2.97-3.08 (2H, m), 3.54-4.22 (7H, m), 5.25-5.69 (2H, m), 7.17-7.59 (7H, m), 7.86-8.04 (2H, m), 8.15-8.16 (2H, m), 8.29 and 8.31 (total 1H, each s), 9.37 and 9.39 (total 1H, each s).
IR (ATR) $cm^{-1}$ : 1652, 1500, 1373, 1220, 1101, 1076, 742.
MS (LC-ESI) m/z : 678 ($M^+$+1).
Anal. Calcd for $C_{33}H_{29}Cl_2N_5O_5S \cdot H_2O$: C, 56.90; H, 4.49; N, 10.05. Found: C, 57.02; H, 4.50; N, 9.98. Compound of Example 45 shown below was produced by the same synthesizing method as that of Example 44.

Example 45

3-[2-[1-[4-[(3-Benzo[b]thienylcarbonyl)amino]-5-chloro-2-fluorophenylacetyl]-(4S)-(3-pyridyloxy)-(2S)-pyrrolidinyl]-5-thiazolyl]propionic acid

[Chem. 137]

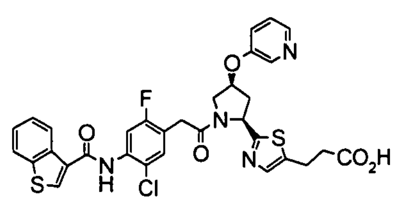

$^1$H-NMR (DMSO-$d_6$) δ: 2.53-2.86 (4H, m), 2.97-3.08 (2H, m), 3.49-4.23 (4H, m), 5.25-5.70 (2H, m), 7.16-7.62 (7H, m), 8.00 (1H, s), 8.10 (1H, d, J = 7.8 Hz), 8.15 (1H, d, J = 4.6 Hz), 8.45 (1H, d, J = 7.3Hz), 8.65 and 8.66 (total 1H, each s), 10.11 and 10.13 (total 1H, each s).
IR (ATR) $cm^{-1}$ : 1654, 1517, 1402, 1218, 765.
MS (LC-ESI) m/z : 665 ($M^+$+1). Anal. Calcd for $C_{32}H_{26}ClFN_4O_5S_2 \cdot 0.5H_2O$: C, 57.01; H, 4.04; N, 8.31. Found: C, 56.83; H, 4.15; N, 8.20.

Example 46

3-[2-(1-tert-butoxycarbonyl-(4S)-(1-piperidinyl)-(2S)-pyrrolidinyl)-5-thiazolynyl]propionic acid methyl ester

[Chem. 138]

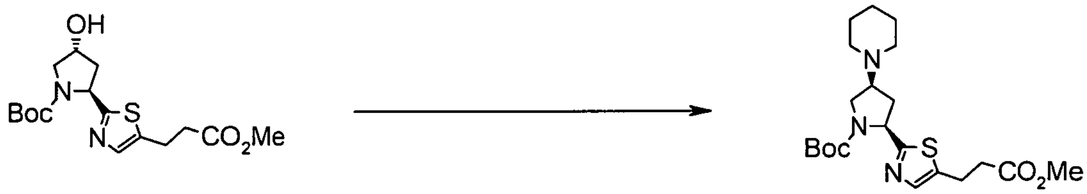

3-[2-(1-tert-butoxycarbonyl-(4R)-hydroxy-(2S)-pyrrolidinyl)-5-thiazolyl]propionic acid methyl ester (740mg, 2.1mmol) and diisopropylethylamine (1.43ml, 8.48mmol) were dissolved in methylene chloride (20ml), trifluoromethanesulfonic anhydride (0.7ml, 4.1mmol) was added at -76˚C, the mixture was stirred for 1 hour, piperidine (0.6ml, 6.2mmol) was added, and the mixture was stirred for 1 hour, and further stirred at -15˚C for 2 hours. To the reaction solution were added water and an aqueous saturated bicarbonate solution, followed by extraction with methylene chloride two times. The combined extracts were dried with anhydrous sodium sulfate, and concentrated under reduced pressure. The resulting residue was purified by preparative TLC (Merck, Silica gel 60, $F_{254}$, 2mm; developed with hexane : ethyl acetate = 1 : 9,elutedwithmethanol:methylenechloride= 3 : 7) to obtain the title compound (390mg, 44%) as a brown oily substance.

$^1$H-NMR ($CDCl_3$) δ: 1.19-1.64 (15H, m), 2.30-2.49 (4H, m), 2.61-2.78 (4H, m), 3.06-3.15 (3H, m), 3.23-3.32 (1H, m), 3.69 (3H, s), 3.90-4.09 (1H, m), 4.95-5.12 (1H, m), 7. 32 (1H, s).

3-[2-((4S)-(1-piperidinyl)-(2S)-pyrrolidinyl)-5-thiazolyl]propionic acid methyl ester

[Chem. 139]

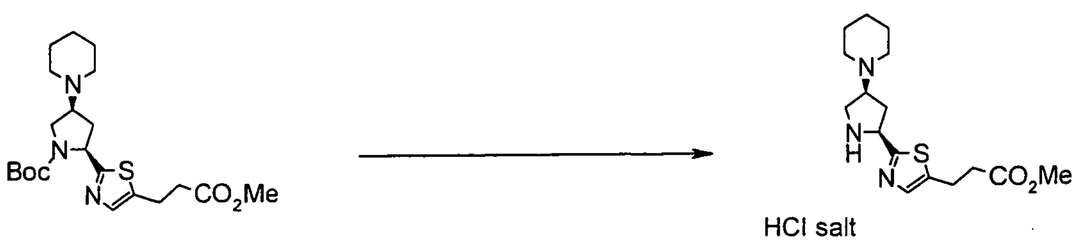

3-[2-(tert-Butoxycarbonyl-(4S)-(1-piperidinyl)-(2S)-pyrrolidinyl)-5-thiazolyl]propionic acid methyl ester (390mg, 0.92mmol) was dissolved in a 4N hydrochloric acid dioxane solution (25ml), and the solution was stirred at room temperature for 1 hour and a half. After concentrated under reduced pressure, the residue was used in the next reaction without purification.

3-[2-[1-[2,5-Dichloro-4-(1-methyl-3-indolylcarbonylamino)phenylacetyl]-(4S)-(1-piperidinyl)-(2S)-pyrrolidinyl]-5-thiazolyl]propionic acid methyl ester

[Chem. 140]

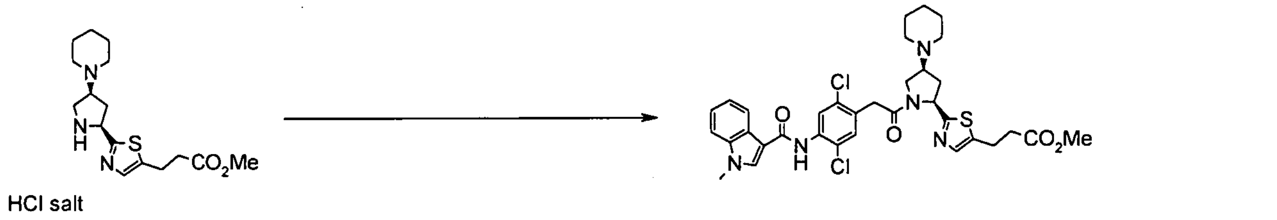

3-[2-((4S)-(1-piperidinyl)-(2S)-pyrrolidinyl)-5-thiazolyl]propionic acid methyl ester (0.46mmol), 2,5-dichloro-4-(1-methyl-3-indolylcarbonylamino)phenylac eticacid (167ml, 0.46mmol), EDC (132mg, 0.69mmol), HOBt (62mg, 0.46mmol) and triethylamine (0.38ml, 0.28mmol) were dissolved in methylene chloride (20ml), and the solution was stirred at room temperature overnight, The reaction solution was concentrated under reduced pressured, and the residue was purified by preparative TLC (Merck, Silica gel 60, $F_{254}$, 2mm; developed with methanol : methylene chloride =8 : 92, eluted with ethanol : methylene chloride = 3 : 7) to obtain the title compound (230mg, 75%) as a colorless oily substance.

$^1$H-NMR ($CDCl_3$) δ: 1.41-1.62 (6H, m), 2.03-2.50 (4H, m), 2.61-2.90 (4H, m), 3.07-3.16 (2H, m), 3.26-4.48 (11H, m), 5.29-5.38 (1H, m), 7.33-7.44 (5H, m), 7.79 and 7.81 (total 1H, each s), 8.10-8.16 (1H, m), 8.22 and 8.26 (total 1H, each s), 8.73 and 8.81 (total 1H, each s).

3-[2-[1-[2,5-Dichloro-4-(1-methyl-3-indolylcarbonylamino-)phenylacetyl]-(4S=)=1-piperidinyl-(2S)-pyrrolidinyl]-5-thiazolyl]propionic acid

[Chem. 141]

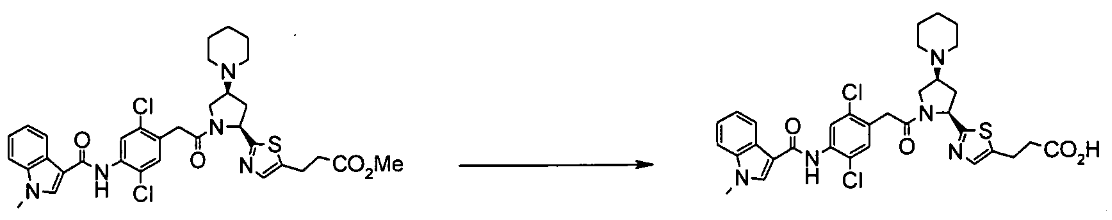

3-[2-[1-[2,5-dichloro-4-(1-methyl-3-indolylcarbonylamino)phenylacetyl]-(4S)-(1-piperidinyl)-(2S)-pyrrolidinyl]-5-thiazolyl]propionic acid methyl ester (210mg, 0.31mmol) was dissolved in THF (10ml), a 1N aqueous sodium hydroxide solution (1.5ml, 1.5mmol) and methanol (5.0ml) were added, and the mixture was stirred at room temperature overnight. The reaction solution was concentrated under reduced pressure, a small amount of water was added, this was made neutral with 1N hydrochloric acid, and concentrated under reduced pressure. The residue was purified by preparative TLC (Merck, Silica gel 60, $F_{254}$, 2mm; developed with methanol : methylene chloride = 12 : 88, eluted with chloroform : methanol : water = 7 : 3 : 1 lower layer) to obtain the title compound (140mg, 68%) as a white powder.

$^1$H-NMR (DMSO-$d_6$) δ: 1.33-1.53 (6H, m), 1.85-1.94 (1H, m), 2.29-2.43 (3H, m), 2.49-2.90 (4H, m), 2.95-3.94 (9H, m), 4.13-4.21 (1H, m), 5.12-5.49 (1H, m), 7.18-7.59 (5H, m), 7.84 and 7.89 (total 1H, each s), 8.15 (1H, d, J = 7.8 Hz), 8.29 and 8.30 (total 1H, each s), 9.37 and 9.39 (total 1H, each s).

IR (ATR) $cm^{-1}$ : 2933, 1654, 1500, 1373, 1218, 1101, 1076, 744.

MS (LC-ESI) m/z : 668 ($M^+$+1).

Anal. Calcd for $C_{33}H_{35}Cl_2N_5O_4S \cdot 1.5H_2O$: C, 56.98; H, 5.51; N, 10.07. Found: C, 56.95; H, 5.73; N, 9.96. Compound of Example 47 shown below was produced by the same synthesizing method as that of Example 46.

Example 47

3-[2-[1-[4-[(3-Benzo[b]thienylcarbonyl)amino]-5-chloro-2-fluorophenylacetyl]-(4S)-(1-piperidinyl)-(2S)-pyrrolidinyl]-5-thiazolyl]propionic acid

[Chem. 142]

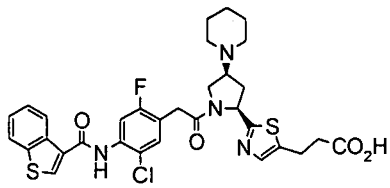

$^1$H-NMR (DMSO-$d_6$) δ: 1.33-1.52 (6H, m), 1.86-1.95 (1H, m), 2.26-2.44 (3H, m), 2.48-2.89 (4H, m), 2.94-3.90 (6H, m), 4.12-4.20 (1H, m), 5.13-5.52 (1H, m), 7.36-7.57 (5H, m), 8.08-8.10 (1H, m), 8.44 and 8.45 (total 1H, each s), 8.65 and 8.66 (total 1H, each s), 10.11-10.15 (1H, m).
IR (ATR) $cm^{-1}$ : 2933, 1652, 1517, 1402, 1214, 765.
MS (LC-ESI) m/z : 655 ($M^+$+1). Anal. Calcd for $C_{32}H_{32}ClFN_4O_4S_2 \cdot H_2O$: C, 57.09; H, 5.09; N, 8.32. Found: C, 56.91; H, 5.32; N, 8.32.

Example 48

[Step 1]

((5S)-benzyloxymethyl-1-tert-butoxycarbonyl-(2S)-pyrrolidinyl)carboxylic acid

[Chem. 143]

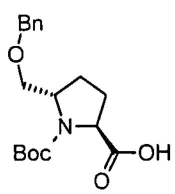

((5S)-benzyloxymethyl-1-tert-butoxycarbonyl-(2S)-pyrrolidinyl)methanol (10.5g, 32.7mmol) was dissolved in acetone (200ml), an aqueous saturated sodium bicarbonate solution (100ml) was added, and potassium bromide (0.39g, 3.27mmol) and TEMPO (5.96g, 26mmol) were added. Under stirring, a 5% aqueous NaOCl solution (80ml) was added dropwise. After stirred at room temperature for 3 hours, the mixture was concentrated under reduced pressure, and this was made acidic with 1N hydrochloric acid and extracted with ethyl acetate two times. The combined extracts were dried with anhydrous sodium sulfate, and concentrated under reduced pressure. The resulting residue was purified by column chromatography to using silica gel to obtain the title compound (9.6g, 87%) as a pale yellow oily substance from a hexane: ethyl acetate (4:1-9:1)-eluted part.

[Step 2]

((5S)-benzyloxymethyl-1-tert-butoxycarbonyl-(2S)-pyrrolidinyl)carboxylic acid methyl ester

[Chem. 144]

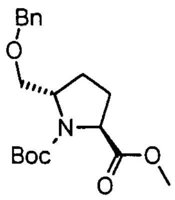

((5S)-benzyloxymethyl-1-tert-butoxycarbonyl-(2S)-pyrrolidinyl)carboxylic acid (9.6g, 28.6mmol) was dissolved in a mixed solution of toluene (160ml) and methanol (40ml), and $TMSCHN_2$ (2.0 M in hexane) (17ml) was added at 0˚C. After stirred at room temperature for 2 hours, this was concentrated under reduced pressure, and the resulting residue was purified by column chromatography using silica gel to obtain the title compound (9.2g, 92%) as a pale brown oily substance from a hexane: ethyl acetate (95:5-7:3)-eluted part.

$^1$H-NMR ($CDC1_3$) δ: 1.38-1.43 (9H, m), 1.83-2.41 (4H, m), 3.38-3.62 (2H, m), 3.71 (3H, s), 4.06-4.34 (2H, m), 4.48-4.57 (2H, m), 7.26-7.33 (5H, m).

[Step 3]

(1-tert-butoxycarbonyl-(5S)-hydroxymethyl-(2S)-pyrrolidinyl)carboxylic acid methyl ester

[Chem. 145]

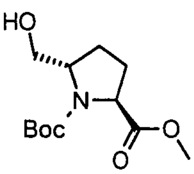

((5S)-benzyloxymethyl-1-tert-butoxycarbonyl-(2S)-pyrrolidinyl)carboxylic acid methyl ester (9.2g, 26.3mmol) was dissolved in ethanol (150ml), $Pd(OH)_2$-C(1g) was added, and hydrogenation was performed at room temperature overnight. Since the reaction did not progress completely, a catalyst was exchanged, and hydrogenation was performed again under the same condition. Further, a catalyst was exchanged, and hydrogenation was performed under the same condition by adding acetic acid (30ml). A catalyst was removed by filtration, and the filtrate was concentrated under the reduced pressure. To the residue was added an aqueous saturated sodium bicarbonate solution, followed by extraction with methylene chloride two times. The combined extracts were dried with anhydrous sodium sulfate, and concentrated under reduced pressure to obtain the title compound (6.8g, 100%) as a pale yellow oily substance.

$^1$H-NMR ($CDCl_3$) δ: 1.41 (9H, s), 1.63-1.67 (1H, m), 1.89-1.94 (1H, m), 2.05-2.29 (2H, m), 3.59-3.68 (2H, m), 3.73 (3H, s), 3.95-3.97 (1H, m), 4.18-4.23 (1H, m), 4.31 (1H, dd, J = 8.7, 2.3 Hz).

[Step 4]

(1-tert-butoxycarbonyl-(5S)-ethoxymethyl-(2S)-pyrrolidinyl)carboxylic acid methyl ester

[Chem. 146]

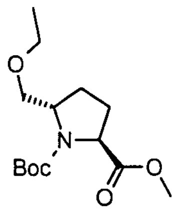

(1-tert-butoxycarbonyl-(5S)-hydroxymethyl-(2S)-pyrrolidinyl) carboxylic acid methyl ester (1.29g, 5.0mmol) and ethyl iodide (804μl, 10mmol) were dissolved in DMF(20ml), and sodium hydride (60% in oil) (260mg, 6.5mmol) was added at 0˚C . After stirred at 0˚C for 1 hour, water was added, and this was extracted with ethyl acetate two times. The combined extracts were dried with anhydrous sodium sulfate, and concentrated under reduced pressure. The resulting residue was purified by column chromatography using silica gel to obtain the title compound (1.0g, 69%) as a colorless oily substance from a hexane: ethyl acetate (98:2-4:1) -eluted part.

$^1$H-NMR ($CDCl_3$) δ: 1.15-1.20 (3H, m), 1.40 and 1.48 (total 9H, each s), 1.88-1.95 (2H, m), 1.99-2.10 (1H, m), 2.22-2.37 (1H, m), 3.27-3.58 (4H, m), 3.71 and 3.72 (total 3H, each s), 4.02-4.34 (2H, m).

[Step 5]

(1-tert-butoxycarbonyl-(5S)-ethoxymethyl-(2S)-pyrrolidinyl)carboxylic acid

[Chem. 147]

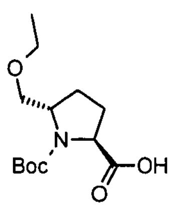

(1-tert-butoxycarbonyl-(5S)-ethoxymethyl-(2S)-pyrrolidinyl)carboxylic acid methyl ester (1.0g, 3.48mmol) was dissolved in THF (20ml), a 1N aqueous sodium hydroxide solution (7.0ml, 7.0mmol) and methanol (10ml) were added, the mixture was stirred at room temperature overnight, a 1N aqueous sodium hydroxide solution (7.0ml, 7.0mmol) was further added, and the mixture was stirred at 50˚C for 1 hour. This was concentrated under reduced pressure, a small amount of water was added, and this was made acidic with 1N hydrochloric acid, followed by extraction with ethyl acetate two times. The combined extracts were dried with anhydrous sodium sulfate, and concentrated under reduced pressure to obtain the title compound (970mg, 100%) as a colorless oily substance.

[Step 6]

5-[(1-tert-butoxycarbonyl-(5S)-ethoxymethyl-(2S)-pyrrolidinyl)carbonylamino]levulinic acid methyl ester

[Chem. 148]

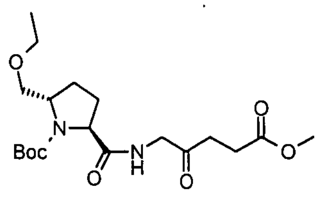

(1-tert-butoxycarbonyl-(5S)-ethoxymethyl-(2S)-pyrrolidinyl)carboxylic acid (951mg, 3.48mmol), 5-aminolevulinic acid methyl ester hydrochloride (633mg, 3.48mmol), HOBt (469mg, 3.48mmol) and EDC (1.0g, 5.2mmol) weredissolvedinmethylchloride (50ml), triethylamino (2.9ml, 20.1mmol) was added, and the mixture was stirred at room temperature overnight. To the reaction solution was added water, followed by extraction wit methylene chloride two times. The combined extracts were dried with anhydrous sodium sulfate, and concentrated under reduced pressure. The resulting residue was purified by column chromatography using silica gel to obtain the title compound (1.3g, 93%) as a pale yellow oily substance from a methylene chloride : methanol (97 : 3)-eluted part.
$^1$H-NMR ($CDCl_3$) δ: 1.15-1.20 (3H, m), 1.39-1.49 (9H, m), 1.89-2.40 (4H, m), 2.62-2.69 (2H, m), 2.72-2.76 (2H, m), 3.34-3.59 (4H, m), 3.68 (3H, s), 3.98-4.31 (4H, m).

[Step7]

3-[2-(1-tert-butoxycarbonyl-(5S)-ethoxymethyl-(2S)-pyrrolidinyl)-5-thiazolyl]propionic acid methyl ester
[Chem. 149]

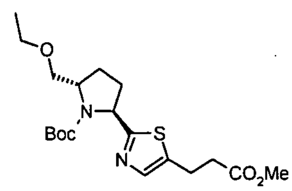

5-[(1-tert-butoxycarbonyl-(5S)-ethoxymethyl-(2S)-pyrrolidinyl)carbonylamino]levulinic acid methyl ester (1.3g, 3.2mmol) was dissolved in toluene (65ml), a Lawesson' s reagent (1.44g, 3.57mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride = 5 : 95, eluted with methanol : methylene chloride = 3 : 7) to obtain the title compound (1.0g, 78%) as a yellow oily substance.
$^1$H-NMR ($CDCl_3$) δ: 1.17-1.22 (3H, m), 1.25 (9H, s), 1.90-2.51 (4H, m), 2.65 (2H, t, J = 7.4 Hz), 3.11 (2H, q, J = 8.3 Hz), 3.34-3.71 (7H, m), 4.04-4.24 (1H, m), 5.04-5.17 (1H, m), 7.37 (1H, s).

[Step 8]

3-[2-((5S)-ethoxymethyl-(2S)-pyrrolidinyl)-5-thiazolyl]propionic acid methyl ester hydrochloride
[Chem. 150]

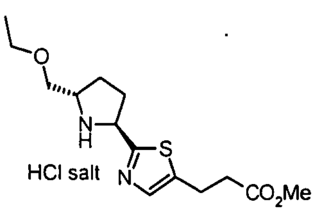

3-[2-(1-tert-butoxycarbonyl-(5S)-ethoxymethyl-(2S) -pyrrolidinyl)-5-thiazolyl]propionic acid methyl ester (1.0g, 2.5mmol) was dissolved in a 4N hydrochloric acid dioxane solution (25ml), and the solution was stirred at room temperature for 30 minutes. After concentration under reduced pressure, the residue was used in the next reaction without purification.

[Step 9]

3-[2-[1-[2,5-Dichloro-4-(1-methyl-3-indolylcarbonylamino)phenylacetyl]-(5S)-ethoxymethyl-(2S)-pyrrolidinyl]-5-thiazolyl]propionic acid methyl ester

[Chem. 151]

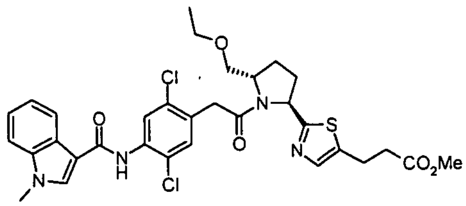

3-[2-((5S)-ethoxymethyl-(2S)-pyrrolidinyl)-5-thiazolyl]propionic acid methyl ester hydrochloride (0.5mmol), 2,5-dichloro-4-(1-methyl-3-indolylcarbonylamino)phenylac eticacid (189mg, 0.5mmol), EDC (144mg, 0.75mmol), HOBt (68mg, 0.5mmol) and triethylamine (0.42ml, 3.0mmol) were dissolved in methylene chloride (15ml), and the mixture was stirred at room temperature overnight. The reaction solution was concentrated under reduced pressure, and the residue was purified by preparative TLC (Merck, Silica gel 60, $F_{254}$, 2mm; developed with methanol : methylene chloride = 7 : 93, eluted with methanol : methylene chloride = 3 : 7) to obtain the title compound (230mg, 70%) as a colorless oily substance.

$^1$H-NMR ($CDCl_3$) δ: 1.15-1.24 (3H, m), 1.97-2.45 (3H, m), 2.61-2.71 (3H, m), 3.05-3.16 (2H, m), 3.30-4.02 (12H, m), 4.35-4.54 (1H, m), 5.34-5.44 (1H, m), 7.27-7.46 (5H, m), 7.79 and 7.80 (total 1H, each s), 8.11-8.15 (1H, m), 8.22-8.25 (1H, m), 8.74 and 8.78 (total 1H, each s).

[Step 10]

3-[2-[1-[2,5-Dichloro-4-(1-methyl-3-indolylcarbonylamino)phenylacetyl]-(5S)-ethoxymethyl-(2S)-pyrrolidinyl]-5-thiazolyl]propionic acid

[Chem. 152]

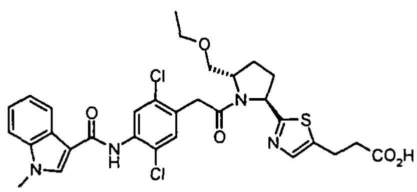

3-[2-[1-[2,5-Dichloro-4-(1-methyl-3-indolylcarbonylamino)phenylacetyl]-(5S)-ethoxymethyl-(2S)-pyrrolidinyl)-5-thiazolyl]propionic acid methyl ester (230mg, 0.35mmol) was dissolved in THF (10ml), a 1N aqueous sodium hydroxide solution (1.5ml, 1.5mmol) and methanol (5.0ml) were added, and the mixture was stirred at room temperature overnight, The reaction solution was concentrated under reducedpressure, a small amount of water was added, this was made neutral with 1 hydrochloric acid, a precipitated solid was collected by filtration to obtain the title compound (180mg, 80%) as a white powder.

$^1$H-NMR (DMSO-d6) δ: 1.07-1.17 (3H, m), 1.77-2.46 (4H, m), 2.50-2.60 (2H, m), 2.93-3.06 (2H, m), 3.23-4.04 (9H, m), 4.21-4.46 (1H, m), 5.19-5.52 (1H, m), 7.18-7.28 (2H, m), 7.35 and 7.36 (total 1H, each s), 7.48-7.56 (2H, m), 7.83 and 7. 87 (total 1H, each s), 8.13 (1H, d, J = 7.8 Hz), 8.28 (1H, s), 9.34-9.36 (1H, m). IR (ATR) $cm^{-1}$ : 2969, 2931, 2873, 1725, 1648, 1500, 1373, 1218, 1101, 1076.
MS (LC-ESI) m/z : 643 ($M^{+}$+1).
Anal. Calcd for $C_{31}H_{32}Cl_2N_9O_5S$: C, 58.85; H, 5.01; N, 8.71. Found: C, 58.11; H, 5.04; N, 8.80.

Compounds of (Example 49) to (Example 52) shown below were synthesized according to the same method as that of [Step 9] and [step 10] of synthesis of the compound (Example 48).

Example49

3-[2-[1-[4-[(3-Benzo[b]thienylcarbonyl)amino]-5-chloro-2-fluorophenylacetyl]-(5S)-ethoxymethyl-(2S)-pyrrolidinyl]5-thiazoly]propionic acid methyl ester

[Chem. 153]

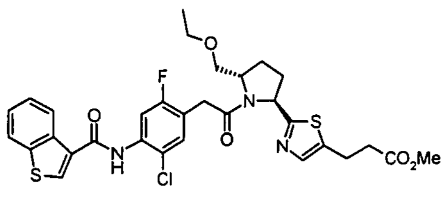

$^1$H-NMR ($CDCl_3$) δ: 1.13-1.25 (3H, m), 2.00-2.42 (3H, m), 2.59-2.70 (3H, m), 3.05-3.16 (2H, m), 3.29-3.90 (9H, m), 4.37-4.52 (1H, m), 5.30-5.43 (1H, m), 7.26-7.54 (4H, m), 7.92 (1H, d, J=7.8Hz), 8.08 and 8.10 (total 1H, eachs), 8.30-8.49 (3H, m).

3-[2-[1-[4-[(3- Benzo [b] thienylcarbonyl) amino]- 5- chloro- 2- fluorophenylacetyl]-(5S)-ethoxymethyl-(2S)-pyrrolidinyl]- 5-thiazolyl]propionic acid

[Chem. 154]

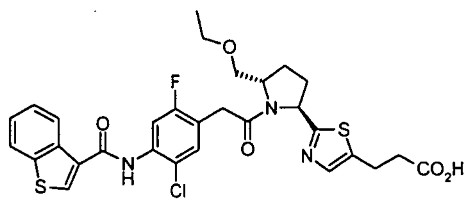

$^1$H-NMR (DMSO-$d_6$) δ: 1.10-1.17 (3H, m), 1.80-2.46 (4H, m), 2.52-2.62 (2H, m), 2.94-3.07 (2H, m), 3.21-3.92 (6H, m), 4.22-4.49 (1H, m), 5.22-5.54 (1H, m), 7.29-7.57 (5H, m), 8.07 and 8.10 (total 1H, each s), 8.44 (1H, d, J = 7.8 Hz), 8.64-8.64 (1H, m), 10.08 and 10.09 (total 1H, each s), 12.29 (1H, br s).

IR (ATR) $cm^{-1}$ : 2971, 2931, 2869, 1513, 1400, 1216, 1108, 765.

MS (LC-ESI) m/z : 630 ($M^+$+1).

Anal. Calcd for $C_{30}H_{29}ClFN_3O_5S_2$ : C, 57.18; H, 4.64; N, 6.67. Found: C, 57.67; H, 4.61; N, 6.72.

Example 50

3-[2-[1-[4-[(1-Benzofuran-3-ylcarbonyl)amino]-5-chloro-2-flurophenylacetyl]-(5S)-ethoxymethyl-(2S)-pyrrolidinyl]-5-thiazolyl]propionic acid methyl ester

[Chem. 155]

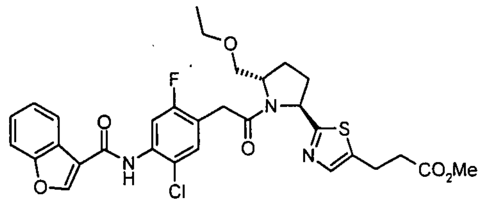

$^1$H-NMR ($CDCl_3$) δ: 1.15-1.25 (3H, m), 2.00-2.42 (3H, m), 2.58-2.69 (3H, m), 3.05-3.17 (2H, m), 3.29-3.90 (9H, m), 4.38-4.52 (1H, m), 5.31-5.43 (1H, m), 7.26-7.61 (5H, m), 8.02-8.07 (1H, m), 8.24-8.46 (3H, m).
3-[2-[1-[4-[(1-Benzofuran-3-ylcarbonyl)amino]-5-chloro-2-fluorophenylacetyl]-(5S)-ethoxymethyl-2(2S)-pyrrolidinyl)-5-thiazolyl]propionic acid
[Chem. 156]

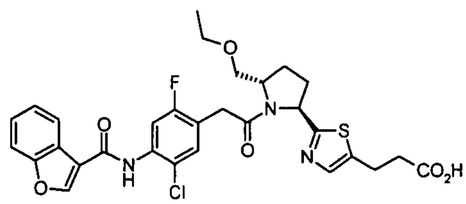

$^1$H-NMR (DMSO-$d_6$) δ: 1.10-1.18 (3H, m), 1.76-2.44 (4H, m), 2.52-2.61 (2H, m), 2.96-3.08 (2H, m), 3.21-3.91 (6H, m), 4.23-4.48 (1H, m), 5.21-5.55 (1H, m), 7.28-7.58 (5H, m), 7.71 (1H, dd, J = 7.1, 1.2 Hz), 8.07-8.08 (1H, m), 8.83 (1H, s), 9.96 and 9.98 (total 1H, each s), 12.29 (1H, br s).
IR (ATR) $cm^{-1}$ : 2973, 2933, 2869, 1724, 1521, 1400, 1103, 746.
MS (LC-ESI) m/z : 614 ($M^{+}$+1).
Anal. Calcd for $C_{30}H_{29}ClFN_3O_6S$: C, 58.68; H, 4.76; N, 6.84. Found: C, 58.90; H, 4.72; N, 6.82.

Example 51

3-[2-[(5S)-ethoxymethyl-1-[7-fluoro-2-(5-fluoro-2-methylphenyl)amino-6-benzoxazolylacetyl]-(2S)-pyrrolidinyl]-5-thiazolyl]propionic acid methyl ester
[Chem. 157]

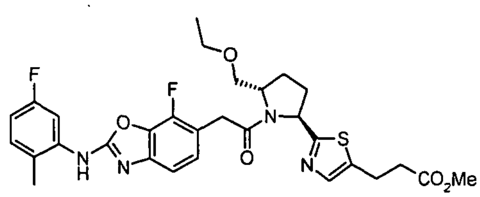

$^1$H-NMR ($CDCl_3$) δ: 1.13-1.27 (3H, m), 2.00-2.42 (6H, m), 2.57-2.71 (3H, m), 3.02-3.18 (2H, m), 3.40-3.95 (9H, m), 4.42-4.52 (1H, m), 5.32-5.42 (1H, m), 6.72-7.32 (4H, m), 7.44 (1H, s), 8.10-8.15 (1H, m).
3-[2-[(5S)-ethoxymethyl-1-[7-fluoro-2-(5-fluoro-2-methylphenyl)amino-6-benzoxazolylacetyl]-(2S)-pyrrolidinyl]-5-thiazolyl]propionic acid
[Chem. 158]

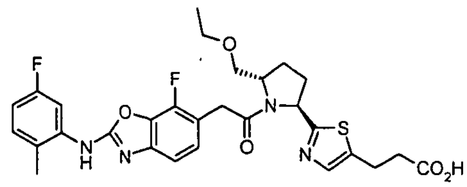

$^1$H-NMR (DMSO-$d_6$) δ: 1.08-1.20 (3H, m), 1.80-2.45 (7H, m), 2.53-2.61 (2H, m), 2.93-3.06 (2H, m), 3.24-4.01 (6H, m), 4.20-4.51 (1H, m), 5.19-5.54 (1H, m), 6.83-7.52 (5H, m), 7.88-7.95 (1H, m), 10.04 (1H, br s), 12.31 (1H, br s).
IR (ATR) $cm^{-1}$ : 2973, 2931, 2871, 1720, 1637, 1577, 1280, 1068, 804.
MS (LC-ESI) m/z : 585 ($M^+$+1).
Anal. Calcd for $C_{29}H_{30}F_2N_4O_5S$: C, 59.58; H, 5.17; N, 9.58. Found: C, 59.99; H, 5.08; N, 9.55.

Example 52

3-[2-[(5S)-ethoxymethyl-1-[7-fluoro-2-(5-fluoro-2-methoxyphenylamino)-6-benzoxazolylacetyl]-(2S)-pyrrolidinyl]-5-thiazolyl]propionic acid methyl ester
[Chem. 159]

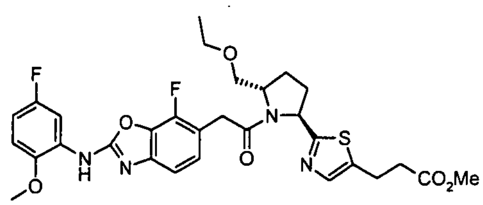

$^1$H-NMR ($CDCl_3$) δ: 1.14-1.26 (3H, m), 1.99-2.43 (3H, m), 2.57-2.71 (3H, m), 3.02-3.16 (2H, m), 3.40-3.94 (12H, m), 4.42-4.52 (1H, m), 5.32-5.42 (1H, m), 6.69-7.32 (4H, m), 7.44 (1H, s), 7.72-7.75 (1H, m), 8.26-8.30 (1H, m).
3-[2-[(5S)-ethoxymethyl-1-[7-fluoro-2-(5-fluoro-2-methoxyphenylamino)-6-benzoxazolylacetyl]-(2S)-pyrrolidinyl]-5-thiazolyl]propionic acid
[Chem. 160]

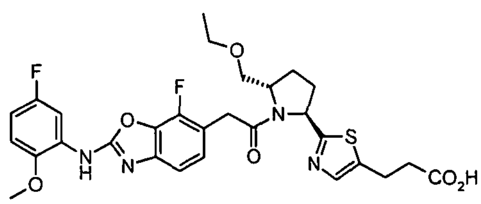

$^1$H-NMR (DMSO-$d_6$) δ: 1.06-1.18 (3H, m), 1.79-2.45 (4H, m), 2.51-2.61 (2H, m), 2.91-3.06 (2H, m), 3.24-4.02 (9H, m), 4.22-4.51 (1H, m), 5.21-5.52 (1H, m), 6.84-7.54 (5H, m), 8.14-8.20 (1H, m), 10.15 (1H, br s), 12.31 (1H, br s).
IR (ATR) $cm^{-1}$ : 2971, 2937, 1724, 1641, 1577.
MS (LC-ESI) m/z : 601 ($M^+$+1).
Anal. Calcd for $C_{29}H_{30}F_2N_4O_6S$: C, 57.99; H, 5.03; N, 9.33. Found: C, 58.26; H, 5.04; N, 9.21.

Example 53

[Step 1]

(1-tert-butoxycarbonyl-(5S)-methoxymethyl-(2S)-pyrrolidinyl)carboxylic acid methyl ester

[Chem. 161]

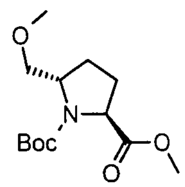

(1-tert-butoxycarbonyl-(5S)-hydroxymethyl-(2S)-pyrrolidinyl) carboxylic acid methyl ester (1.29g, 5.0mmol) and methyl iodide (622μl, 10mmol) were dissolved in DMF (20ml), and sodium hydride (60% in oil) (260mg, 6.5mmol) was added at 0˚C . After stirred at 0˚C for 30 minutes, water was added, followed by extraction with ethyl acetate two times. The combined extracts were dried with anhydrous sodium sulfate, and concentrated under reduced pressure. The resulting residue was purified by column chromatography using silica gel to obtain the title compound (1.2g, 88%) as a colorless oily substance from a hexane-ethyl acetate (98:2 -7 : 3) -eluted part.

$^1$H-NMR ($CDCl_3$) δ: 1.40-1.48 (9H, m), 1.87-1.93 (2H, m), 1.99-2.13 (1H, m), 2.19-2.36 (1H, m), 3.35 (3H, s), 3.39-3.51 (2H, m), 3.71 and 3.72 (total 3H, each s), 4.03-4.20 (1H, m), 4.25-4.33 (1H, m).

[Step 2]

(1-tert-butoxycarbonyl-(5S)-methoxymethyl-(2S)-pyrrolidinyl)carboxylic acid

[Chem. 162]

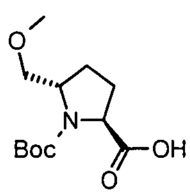

(1-tert-butoxycarbonyl-(5S)-methoxymethyl-(2S)-pyrrolidinyl) carboxylic acid methyl ester (1. 2g, 4.4 mmol) was dissolved in THF (20ml), a 1N aqueous sodiumhydroxide solution (9ml, 9mmol) and methanol (10ml) were added, and the mixture was stirred at room temperature overnight. Since the reaction did not progress completely, the mixture was stirred at 55˚ C for 1 hour. The reaction solution was concentrated under reduced pressure, a small amount water was added, and this was made acidic with 1N hydrochloric acid, followed by extraction with ethyl acetate two times. The combined extracts were dried with anhydrous sodium sulfate, and concentrated under reduced pressure to obtain the title compound (1.1g, 100%) as a colorless oily substance.

[Step 3]

5-[(1-tert-butoxycarbonyl)-(5S)-methoxymethyl-(2S)-pyrrolidinyl]carbonylamino]levulinic acid methyl ester

[Chem. 163]

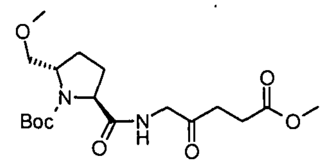

(1-tert-butoxycarbonyl-(5S)-methoxymethyl-(2S)-pyrrolidinyl) carboxylic acid (1.1g, 4.2mmol), 5-aminolevulinic acidmethyl ester hydrochloride (772mg, 4.2mmol), HOBt (572mg, 4.2mmol) and EDC (1.2g, 6.4mmol) were dissolved in methyl chloride (50ml), triethylamine (3.5ml, 25.4mmol) was added, and the mixture was stirred at room temperature overnight. To the reaction solution was added water, followed by extraction with methylene chloride two times. The combined extracts were dried with anhydrous sodium sulfate, and concentrated under reduced pressure. The resulting residue was purified by column chromatography using silica gel to obtain the title compound (1.6g, 100%) as a yellow solid from a methylene chloride: methanol (97:3)-eluted part.

$^1$H-NMR ($CDCl_3$ ) δ: 1.39-1.49 (9H, m), 1.89-2.37 (4H, m), 2.61-2.76 (4H, m), 3.34 (3H, s), 3.41-3.57 (2H, m), 3.67 (3H, s), 3.98-4.30 (4H, m).

[Step 4]

3-[2-(1-tert-butoxycarbonyl-(5S)-methoxymethyl-(2S)-pyrrolidinyl)-5-thiazolyl]propionic acid methyl ester

[Chem. 164]

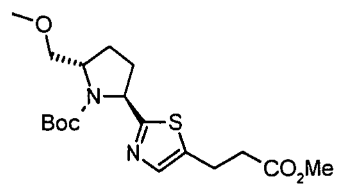

5-[(1-tert-butoxycarbonyl-(5S)-methoxymethyl-(2S)-pyrrolidinyl)carbonylamino]levulinic acid methyl ester (1. 6g, 4.1mmol) was dissolved in toluene, a Lawesson' s reagent (1.8g, 4.6mmol) was added, and the mixture was stirred at 90˚Cfor 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride= 5 : 95, eluted with methanol : methylene chloride = 3 : 7) to obtain the title compound (1.4g, 88%) as a yellow oily substance.

$^1$H-NMR ($CDCl_3$) δ: 1.26 and 1.48 (total 9H, each s), 1.91-2.49 (4H, m), 2.65 (2H, t, J = 7.4 Hz), 3.07-3.15 (2H, m), 3.33-3.88 (7H, m), 4.05-4.25 (1H, m), 5.04-5.16 (1H, m), 7.37 (1H, s).

[Step 5]

3-[2-((5S)-methylmethyl-(2S)-pyrrolidinyl)-5-thiaz olyl]propionic acid methyl ester hydrochloride

[Chem. 165]

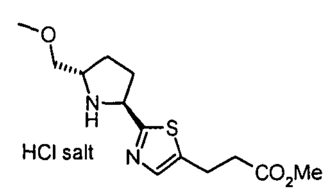

3-[2-(1-tert-butoxycarbonyl-(5S)-methoxymethyl-(2S)-pyrrolidinyl)-5-thiazolyl]propionic acid methyl ester (1.4g, 3.6mmol) was dissolved in a 4N hydrochloric acid dioxane solution (35ml), and the solution was stirred at room temperature for 1 hour. After concentration under reduced pressure, the residue was used in the next reaction without purification.

[Step 6]

3-[2-[1-[5-Chloro-2-fluoro-4-(1-methyl-3-indolylca rbonyl)aminophenylacetyl]-(5S)-methoxymethyl-(2S)-pyrrol idinyl]-5-thiazolyl]propionic acid methyl ester

[Chem. 166]

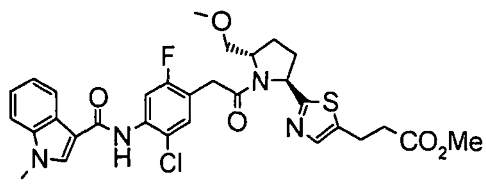

$^1$H-NMR ($CDCl_3$) δ: 2.00-2.41 (3H, m), 2.59-2.70 (3H, m), 3.04-3.17 (2H, m), 3.27-3.90 (13H, m), 4.35-4.52 (1H, m), 5.30-5.42 (1H, m), 7.25-7.46 (5H, m), 7.80 and 7.81 (total 1H, each s), 8.12-8.14 (1H, m), 8.28 and 8.30 (total 1H, each s), 8.43-8.53 (1H, m).

[Step 7]

3-[2-[1-[5-Chloro-2-fluoro-4-(1-methyl-3-indolylcarbonyl)aminophenylacetyl]-(5S)-methoxymethyl-(2S)-pyrrolidinyl]-5-thiazolyl]propionic acid

[Chem. 167]

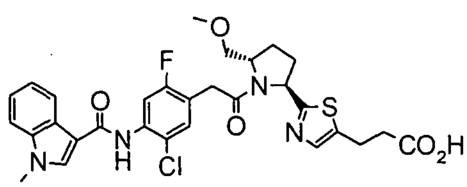

$^1$H-NMR (DMSO-$d_6$) δ: 1.78-2.47 (4H, m), 2.51-2.62 (2H, m), 2.95-3.08 (2H, m), 3.18-3.92 (10H, m), 4.22-4.50 (1H, m), 5.20-5.55 (1H, m), 7.21-7.68 (6H, m), 8.14 (1H, d, J = 7.6 Hz), 8.30 (1H, s), 9.28 (1H, s), 12.29 (1H, br s).
IR (ATR) $cm^{-1}$ : 3421, 2931, 1724, 1650, 1513, 1398, 1099, 742.
MS (LC-ESI) m/z : 613 ($M^+$+1).
Anal. Calcd for $C_{30}H_{30}ClFN_4O_5S$: C, 58.77; H, 4.93; N, 9.14. Found: C, 58.50; H, 4.94; N, 9.04.

Compounds of (Example 54) to (Example 58) shown below were synthesized by the same method as that of [Step 6] and [Step 7] of (Example 53)

Example 54

3-[2-[1-[2,5- Dichloro- 4-(1- methyl- 3- indolylcarbonylamino) phenylacetyl]-(5S)-methoxymethyl-(2S)-pyrrolidinyl]-5-thazolyl]propionic acid methyl ester

[Chem. 168]

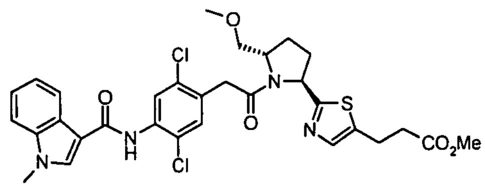

3-[2-((5S)-methoxymethyl-(2S)pyrrolidinyl)-5-thiazolyl]propionic acid methyl ester hydrochloride (0.6mmol), 2,5-dichloro-4-(1-methyl-3-indolylcarbonylamino)phenylac etic acid (226mg, 0.6mmol), EDC (173mg, 0.;9mmol), HOBt (81mg, 0.6mmol) and triethylamine (0.5ml, 3.6mmol) were dissolved in methylene chloride (20ml), and the solution was stirred at room temperature overnight. The reaction solution was concentrated under reduced pressure, and the residue was purified by preparative TLC (Merck, Silica gel 60, $F_{254}$, 2mm; developed with methanol : methylene chloride= 6 : 94, eluted with methanol : methylene chloride = 3 : 7) to obtain the title compound (240mg, 62%) as a colorless oily substance.

$^1$H-NMR ($CDCl_3$) δ: 1.98-2.44 (3H, m), 2.59-2.70 (3H, m), 3.06-3.16 (2H, m), 3.32-3.98 (13H, m), 4.34-4.54 (1H, m), 5.33-5.43 (1H, m), 7.26-7.45 (5H, m), 7.80 (1H, s), 8.11-8.13 (1H, m), 8.21-8.25 (1H, m), 8.74 and 8.78 (total 1H, each s).

3-[2-[1-[2,5-Dichloro-4-(1-methyl-3-indolylcarbonylamino)phenylacetyl]-(5S)-methoxymethyl-(2S)-pyrrolidinyl]-5-thiazolyl]propionic acid

[Chem. 169]

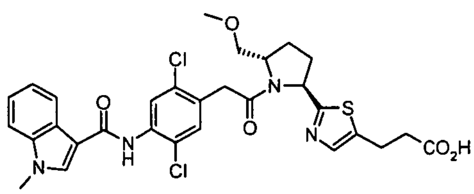

3-[2-[1-[2,5-Dichloro-4-(1-methyl-3-indolylcarbonylamino)phenylacetyl]-(5S)-methoxymethyl-(2S)-pyrrolidinyl]-5-thiazolyl]propionic acid methylester (240mg, 0.37mmol) was dissolved in THF (10ml), a 1N aqueous sodium hydroxide solution (1.5ml, 1.5mmol) and methanol (5.0ml) were added, and the mixture was stirred at room temperature overnight. The reaction solution wasconcentrated underreduced pressure, a small amount of water was added, this was made neutral with 1N hydrochloric acid, and a precipitated solid was collected by filtration to obtain the title compound (200mg, 85%) as a white powder.

$^1$H-NMR (DMSO-$d_6$) δ: 1.79-2.45 (4H, m), 2.52-2.62 (2H, m), 2.95-3.07 (2H, m), 3.24-4.06 (10H, m), 4.24-4.47 (1H, m), 5.20-5.52 (1H, m), 7.19-7.29 (2H, m), 7.37 (1H, s), 7.50-7.57 (2H, m), 7.85 and 7.89 (total 1H, each s), 8.14 (1H, d, J = 7.8 Hz), 8.29 (1H, s), 9.34 and 9.36 (total 1H, each s), 12.30 (1H, br s).

IR (ATR) $cm^{-1}$ : 3419, 2927, 1725, 1648, 1500, 1373, 1101, 1076.

MS (LC-ESI) m/z : 629 ($M^+$+1).

Anal. Calcd for $C_{30}H_{30}Cl_2N_4O_5S \cdot 0.2H_2O$: C, 56. 91; H, 4.84; N, 8.85. Found: C, 56.76; H, 4.77; N, 8.77.

Example 55

3-[2-[1-[4-[(3-Benzo[b]thienylcarbonyl)amino]-5-chloro-2-fluorophenylacetyl]-(5S)-methoxymethyl-(2S)-pyrrolidinyl]-5-thiazolyl]propionic acid methyl ester

[Chem. 170]

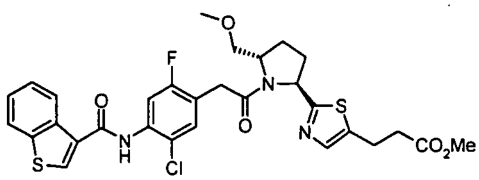

$^1H$-NMR ($CDCl_3$) δ: 1.99-2.41 (3H, m), 2.59-2.71 (3H, m), 3.06-3.16 (2H, m), 3.30-3.87 (10H, m), 4.38-4.52 (1H, m), 5.30-5.42 (1H, m), 7.26-7.54 (4H, m), 7.92 (1H, dd, J = 8.0, 0.6 Hz), 8.08 and 8.09 (total 1H, each s), 8.31-8.49 (3H, m).

3-[2-[1-[4-[(3-Benzo[b]thienylcarbonyl)amino]-5-chloro-2-fluorophenylacetyl]-(5S)-methoxymethyl-(2S)-pyrrolidinyl]-5-thiazolyl]propionic acid

[Chem. 171]

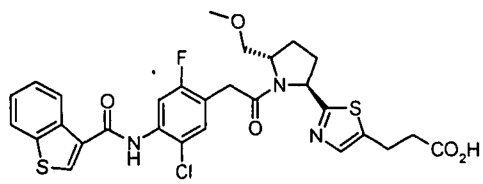

$^1H$-NMR (DMSO-$d_6$) δ: 1.81-2.46 (4H, m), 2.52-2.63 (2H, m), 2.95-3.07 (2H, m), 3.22-3.89 (7H, m), 4.22-4.50 (1H, m), 5.22-5.54 (1H, m), 7.29-7.57 (5H, m), 8.07-8.10 (1H, m), 8.43-8.49 (1H, m), 8.64 (1H, s), 10.08 and 10.09 (total 1H, each s), 12.30 (1H, br s).
IR (ATR) $cm^{-1}$ : 2925, 1724, 1644, 1621, 1513, 1398, 765.
MS (LC-ESI) m/z : 616 ($M^{+}$+1).
Anal. Calcd for $C_{29}H_{27}ClFN_3O_5S_2$ : C, 56. 53; H, 4.42; N, 6. 82 . Found: C, 56.39; H, 4.45; N, 6.71.

Example 56

3-[2-[1-[4-[(1-Benzofuran-3-ylcarbonyl)amino]-5-chloro-2-fluorophenylacetyl]-(5S)-methoxymethyl-(2S)-pyrrolidinyl]-5-thiazolyl]propionic acid methyl ester

[Chem. 172]

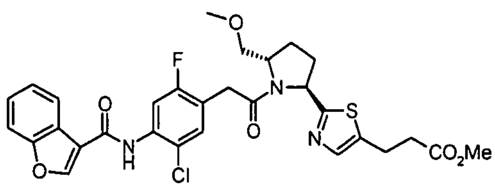

$^1H$-NMR ($CDCl_3$) δ: 1.97-2.43 (3H, m), 2.59-2.70 (3H, m), 3.05-3.16 (2H, m), 3.30-3.87 (10H, m), 4.37-4.51 (1H, m), 5.30-5.43 (1H, m), 7.26-7.62 (5H, m), 8.03-8.07 (1H, m), 8.23-8.47 (3H, m).

3-[2-[1-[4-[(1-Benzofuran-3-ylcarbonyl)amino]-5-chloro-2-fluorophenylacetyl]-(5S)-methoxymethyl-(2S)-pyrrolidinyl]-5-thiazolyl]propionic acid

[Chem. 173]

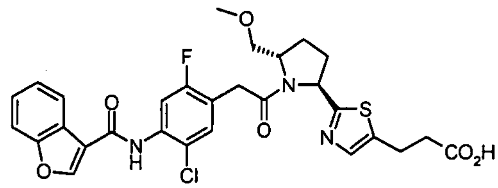

$^1$H-NMR (DMSO-$d_6$) δ: 1.73-2.46 (4H, m), 2.52-2.62 (2H, m), 2.95-3.06 (2H, m), 3.20-3.90 (7H, m), 4.22-4.50 (1H, m), 5.20-5.55 (1H, m), 7.28-7.59 (5H, m), 7.71 (1H, d, J = 7.3 Hz), 8.08 (1H, d, J = 7.6 Hz), 8.82 (1H, s), 9.96 and 9.97 (total 1H, each s), 12.28 (1H, br s).
IR (ATR) $cm^{-1}$ : 2927, 1724, 1643, 1621, 1519, 1400, 1116, 746.
MS (LC-ESI) m/z : 600 ($M^{+}$+1) .
Anal. Calcd for $C_{29}H_{27}ClFN_3O_6S \cdot 0.2H_2O$: C, 57.70; H, 4.57; N, 6.96. Found: C, 57.72; H, 4.57; N, 6.87.

Example 57

3-[2-[1-[7-Fluoro-2-5-fluoro-2-methylphenyl]amino-6-benzoxazolylacetyl]-(5S)-methoxymethyl-(2S)-pyrrolidinyl]-5-thiazolyl]propionic acid methyl ester
[Chem. 174]

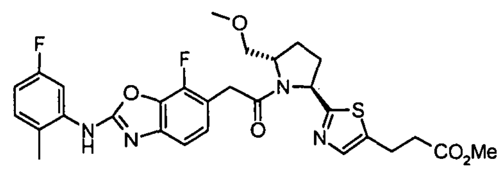

$^1$H-NMR ($CDCl_3$) δ: 2.00-2.42 (6H, m), 2.58-2.71 (3H, m), 3.02-3.18 (2H, m), 3.33-3.94 (10H, m), 4.39-4.53 (1H, m), 5.33-5.44 (1H, m), 6.71-7.45 (6H, m), 8.09-8.15 (1H, m).
3-[2-[1-[7-Fluoro-2-(5-fluoro-2-methylphenyl)-amino-6-benzoxazolylacetyl]-(5S)-methoxymethyl-(2S)-pyrrolidinyl]-5-thiazolyl]propionic acid
[Chem. 175]

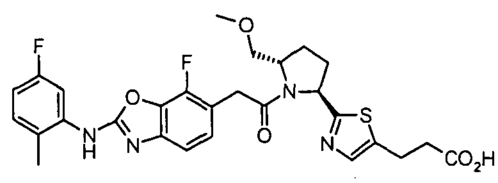

$^1$H-NMR (DMSO-$d_6$) δ: 1.80-2.44 (7H, m), 2.52-2.61 (2H, m), 2.93-3.08 (2H, m), 3.23-3.94 (7H, m), 4.22-4.51 (1H, m), 5.19-5.55 (1H, m), 6.83-6.92 (2H, m), 7.06-7.28 (3H, m), 7.35 and 7.52 (total 1H, each s), 7.90-7.93 (1H, m), 10.04 (1H, br s).
IR (ATR) $cm^{-1}$ : 2927, 1720, 1637, 1577, 1280, 1068.
MS (LC-ESI) m/z : 571 ($M^{+}$+1).
Anal. Calcd for $C_{28}H_{28}F_2N_4O_5S$: C, 58.94; H, 4.95; N, 9.82. Found: C, 58.78; H, 4.91; N, 9.62.

Example 58

3-[2-[1-[2-(5-Fluoro-2-methoxyphenylamino)-6-benzoxazolylacetyl]-(5S)-methoxymethyl-(2S)-pyrrolidinyl]-5-thiazolyl]propionic acid methyl ester

[Chem. 176]

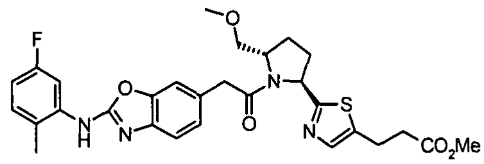

$^1$H-NMR ($CDCl_3$) δ: 1.93-2.37 (6H, m), 2.50-2.67 (3H, m), 3.03-3.15 (2H, m), 3.30-3.90 (10H, m), 4.30-4.53 (1H, m), 5.25-5.44 (1H, m), 6.71-7.44 (7H, m), 8.11-8.14 (1H, m).

3-[2-[1-[2-(5-Fluoro-2-methoxyphenylamino)-6-benzoxazolylacetyl]-(5S)-methoxymethyl-(2S)-pyrrolidinyl]-5-thiazolyl]propionic acid

[Chem. 177]

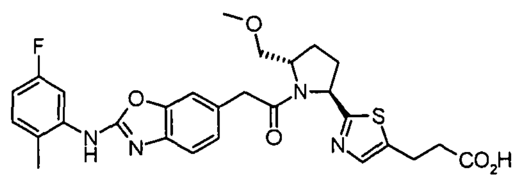

$^1$H-NMR (DMSO-$d_6$) δ: 1.79-2.46 (7H, m), 2.51-2.60 (2H, m), 2.91-3.05 (2H, m), 3.22-3.92 (7H, m), 4.24-4.45 (1H, m), 5.20-5.49 (1H, m), 6.83-6.87 (2H, m), 7.07-7.51 (5H, m), 7.92-7.97 (1H, m), 9.77 (1H, br s).
IR (ATR) $cm^{-1}$ : 2925, 1720, 1637, 1608, 1571, 1240, 1112. MS (LC-ESI) m/z : 553 ($M^+$+1).
Anal. Calcd for $C_{28}H_{29}FN_4O_5S \cdot 0.6H_2O$: C, 59.69; H, 5.40; N, 9.94. Found: C, 59.59; H, 5.18; N, 9.75.

Example 59

[Step 1]

5-[(1-tert-butoxycarbonyl-(2S)-octahydroindolyl)carbonylamino]levulinic acid methyl ester

[Chem. 178]

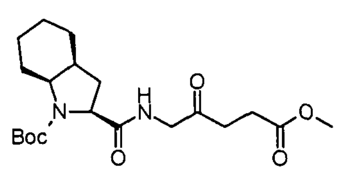

(1-tert-butoxycarbonyl-(2S)-octahydroindolyl)carboxylic acid (650mg, 2.4mmol), 5-aminolevulinic acid methyl ester hydrochloride (439mg, 2.4mmol), HOBt (325mg, 2.4mmol) and EDC (695mg, 3.6mmol) were dissolved in methylene chloride (50ml), triethylamine (2.0ml, 14mmol) was added, and the mixture was stirred at room temperature overnight. To the reaction solution was added water, followed by extraction with methylene chloride two times. The combined

extracts were dried with anhydrous sodium sulfate, and concentrated under reduced pressure. The resulting residue was purified by column chromatography using silica gel to obtain the title compound (1.0g, 100%) as a yellow oily substance from a methylene chloride: methanol (97:3)-eluted part.

$^1$H-NMR ($CDCl_3$) δ: 1.12-1.73 (17H, m), 1.94-2.32 (3H, m), 2.63-2.68 (2H, m), 2.73-2.77 (2H, m), 3.67 (3H, s), 3.81-3.93 (1H, m), 4.18-4.24 (3H, m).

[Step 2]

3-[2-(1-tert-butoxycarbonyl-(2S)-octahydroindolyl)-5-thiazolyl]propionic acid methyl ester

[Chem. 179]

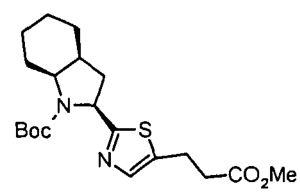

5-[(1-tert-butoxycarbonyl-(2S)-octahydroindoly)carbonylamino]levulinic acid methyl ester (1.0g, 2.4mmol) was dissolved in toluene (50ml), a Lawesson's reagent (1.1g, 2.7mmol) was added, and the mixture was stirred at 90˚C for 1 hour. To the reaction solution was added water, followed by extraction with ethyl acetate two times. The combined extracts were dried with anhydrous sodium sulfate, and concentrated under reduced pressure. The resulting residue was purified by preparative TLC (Merck, Silica gel 60, $F_{254}$, 2mm; developed with methanol : methylene chloride= 5 : 95, eluted with methanol : methylene chloride= 3 : 7) to obtain the title compound (770mg, 81%) as a yellow oily substance.

$^1$H-NMR ($CDCl_3$) δ: 1.15-1.76 (17H, m), 2.12-2.39 (3H, m), 2.66 (2H, t, J = 7.4 Hz), 3.08-3.15 (2H, m), 3.67 and 3.69 (total 3H, each s), 3.84-3.96 (1H, m), 4.94-5.05 (1H, m), 7.34 (1H, s).

[Step 3]

3-[2-((2S)-octahydroindolyl)-5-thiazolyl]propionic acid methyl ester hydrochloride

[Chem. 180]

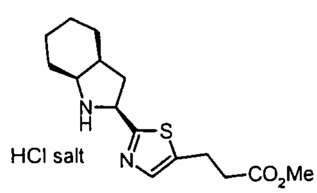

3-[2-(1-tert-butoxycarbonyl-(2S)-octahydroindolyl)-5-thiazolyl]propionic acid methyl ester (770mg, 1.95mmol) was dissolved in a 4N hydrochloric acid dioxane solution (20ml), and the solution was stirred at room temperature for 1 hour. After concentration under reduced pressure, the residue was used in the next reaction without purification.

[Step 4]

3-[2-[1-[5-Chloro-2-fluoro-4-(1-methyl-3-indolylcarbonyl)aminophenylacetyl]-(2S)-octahydroindolyl]-5-thiazolyl]propionic acid methyl ester

[Chem. 181]

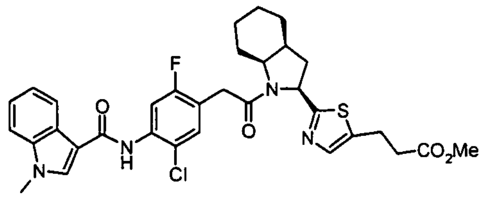

$^1$H-NMR ($CDCl_3$) δ: 1.12-1.97 (8H, m), 2. 21-2. 49 (3H, m), 2.66 (2H, t, J = 7.6 Hz), 3.07-3.21 (3H, m), 3.48-4.37 (8H, m), 5.23-5.32 (1H, m), 7.34-7.45 (5H, m), 7.80 and 7.81 (total 1H, each s), 8.12-8.15 (1H, m), 8.27-8.30 (1H, m), 8.40-8.53 (1H, m).

[Step 5]

3-[2-[1-[5-Chloro-2-fluoro-4-(1-methyl-3-indolylcarbonyl)aminophenylacetyl]-(2S)-octahydroindolyl]-5-thiazolyl]propionic acid

[Chem. 182]

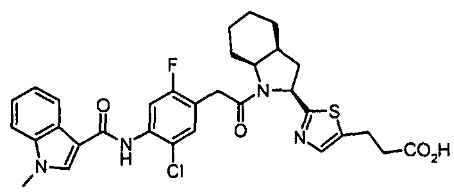

$^1$H-NMR (DMSO-$d_6$) δ: 1.02-2.44 (11H, m), 2.53-2.61 (2H, m), 2.96-3.11 (2H, m), 3.56-4.17 (6H, m), 5.14-5.41 (1H, m), 7.18-7.73 (6H, m), 8.15 (1H, d, J = 7.8 Hz), 8.30 (1H, s), 9.25 and 9.29 (total 1H, each s), 12.27 (1H, br s).
IR (ATR) $cm^{-1}$ : 2929, 1725, 1644, 1513, 1402, 1218, 1101, 744.
MS (LC-ESI) m/z : 623 ($M^+$+1).
Anal. Calcd for $C_{32}H_{32}ClFN_4O_4S \cdot 0.2H_2O$: C, 61.32; H, 5.21; N, 8.94. Found: C, 61.21; H, 5.14; N, 8.93.

Compounds of (Example 60) to (Example 62) shown below were synthesized by the same method as that of [Step 5] and [Step 5] of (Example 59).

Example 60

3-[2-[1-[2,5-Dichloro-4-(1-methyl-3-indolylcarbonyl)aminophenylacetyl]-5-(2S)-octahydroindolyl]-5-thiazoly 1] propionic acid methyl ester

[Chem. 183]

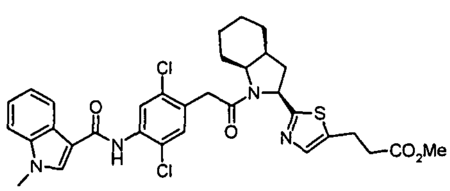

3-[2-((2S)-octahydroindolyl)-5-thiazolyl]propionic acid methyl ester hydrochloride (0.5mmol), 2,5-dichloro-4-(1-methyl-3-indolylcarbonylamino)phenylac etic acid (286mg, 0.5mmol), EDC (144mg, 0.75mmol), HOBt (68mg, 0.5mmol) and triethylamine (0.42ml, 3.0mmol) were dissolved in methylene chloride (20ml), and the solution was stirred at room temperature overnight. The reaction solution was concentrated under reduced pressure, and the residue was purified

by preparative TLC (Merck, Silica gel 60, $F_{254}$, 2mm; developed with methanol : methylene chloride = 5 : 95, eluted with methanol : methylene chloride = 3 : 7 to obtain the title compound (250mg, 77%) as a colorless oily substance.
$^1$H-NMR ($CDCl_3$) δ: 1.21-1.94 (8H, m), 2.23-2.52 (3H, m), 2. 67 (2H, t, J = 7.7 Hz), 3.09-3.26 (3H, m), 3.60-4.41 (8H, m), 5.29-5.31 (1H, m), 7.30-7.45 (5H, m), 7.79 and 7.80 (total 1H, each s), 8.12-8.14 (1H, m), 8.21-8.26 (1H, m), 8.72 and 8.79 (total 1H, each s).

3-[2-[1-[2,5- Dichloro- 4-(1- methyl- 3- indolylcarbonyl) aminophenylacetyl]-(2S)-octahydroindolyl]- 5- thiazolyl] propionic acid

[Chem. 184]

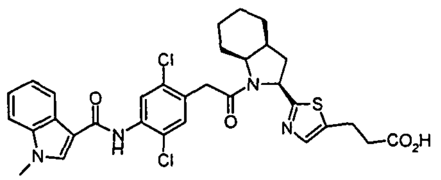

3-[2-[1-[2,5- Dichloro- 4-(1- methyl- 3- indolylcarbonyl) aminophenylacetyl]-(2S)-octahydroindolyl]- 5- thiazolyl] propionic acid methyl ester (250mg, 0.38mmol) was dissolved in THF (10ml), a 1N aqueous sodium hydroxide solution (1.5ml, 1.5mmol) and methanol (5.0mmol) were added, and the mixture was stirred at room temperature overnight. The reaction solution was concentrated under reduced pressure, a small amount of water was added, this was made neutral with 1N hydrochloric acid, and a precipitated solid was collected by filtration to obtain the title compound (190mg, 78%) as a white solid.

$^1$H-NMR (DMSO-$d_6$) δ: 1.17-2.47 (11H, m), 2.52-2.61 (2H, m), 2.94-3.06 (2H, m), 3.66-4.14 (6H, m), 5.12-5.42 (1H, m), 7.19-7.29 (2H,m), 7.38 (1H, s), 7.50-7.55 (2H, m), 7.84 and 7.89 (total 1H, each s), 8.15 (1H, d, J = 7.6 Hz), 8.28 and 8.30 (total 1H, each s), 9.32 and 9.36 (total 1H, each s), 12.26 (1H, br s).
IR (ATR) $cm^{-1}$ : 2931, 1725, 1644, 1502, 1373, 1218, 744.
MS (LC-ESI) m/z : 639 ($M^+$+1).
Anal. Calcd for $C_{32}H_{32}Cl_2N_4O_4S \cdot 0.5H_2O$: C, 59.26; H, 5.13; N, 8.64. Found: C, 59.13; H, 5.15; N, 8.61.

Example 61

3-[2-[1-[4-[(3-Benzo[b]thienylcarbonyl)amino]-5-chloro-2-fluorophenylacetyl]-(2S)-octahydroindolyl]-5-thiazolyl]propionic acid methyl ester

[Chem. 185]

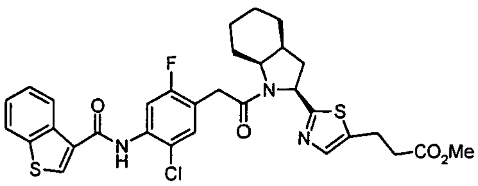

$^1$H-NMR ($CDCl_3$) δ: 1.15-1.95 (8H, m), 2.23-2.47 (3H, m), 2.65-2.69 (2H, m), 3.07-3.24 (3H, m), 3.47-4.38 (5H, m), 5.25-5.30 (1H, m), 7.36-7.55 (4H, m), 7.92 (1H, d, J = 7.8 Hz), 8.08 and 8.09 (total 1H, each s), 8.29-8.50 (3H, m).
3-[2-[1-[4-[(3-Benzo[b]thienylcarbonyl)amino]-5-chloro-2-fluorophenylacetyl]-(2S)-octahydroindolyl]-5-thiazolyl]propionic acid

[Chem. 186]

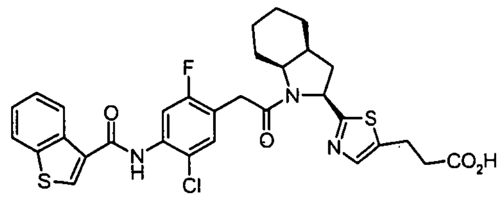

$^1$H-NMR (DMSO-$d_6$) δ: 1.16-2.46 (11H, m), 2.52-2.62 (2H, m), 2.93-3.13 (2H, m), 3.58-3.93 (2H, m), 4.08-4.14 (1H, m), 5.12-5.47 (1H, m), 7.30-7.56 (5H, m), 8.09 (1H, d, J = 6.9 Hz), 8.44-8.45 (1H, m), 8.63 and 8.65 (total 1H, each s), 10.06 and 10.09 (total 1H, each s), 12.28 (1H, br s).
IR (ATR) $cm^{-1}$ : 2929, 1722, 1621, 1515, 1402, 1214, 765.
MS (LC-ESI) m/z : 626 ($M^+$+1).
Anal. Calcd for $C_{31}H_{29}ClFN_3O_4S_2 \cdot 0.2H_2O$: C, 59.12; H, 4.71; N, 6.67. Found: C, 59.06; H, 4.72; N, 6.67.

Example 62

3-[2-[1-[7-Fluoro-2-(5-fluoro-2-methylphenyl)amino-6-benzoxazolylacetyl]-(2S)-octahydroindolyl]-5-thiazolyl] propionic acid methyl ester
[Chem. 187]

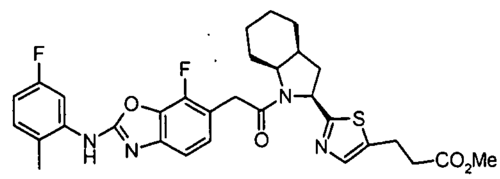

$^1$H-NMR ($CDCl_3$) δ: 1.16-2.02 (8H, m), 2.20-2.52 (6H, m), 2.62-2.71 (2H, m), 3.06-3.18 (3H, m), 3.27-4.38 (5H, m), 5.24-5.33 (1H, m), 6.73-7.41 (6H, m), 8.12 (1H, d, J = 10.7 Hz).
3-[2-[1-[7-Fluoro-2-(5-fluoro-2-methylphenyl)amino-6-benzoxazolylacetyl]-(2S)-octahydroindolyl]-5-thiazolyl] propionic acid
[Chem. 188]

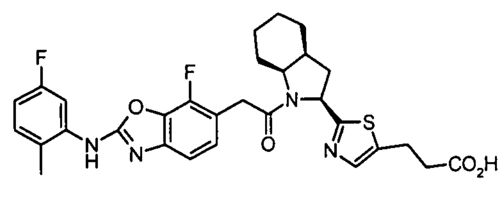

$^1$H-NMR (DMSO-$d_6$) δ: 1.13-2.46 (14H, m), 2.51-2.61 (2H, m), 2.94-3.06 (2H, m), 3.63-3.97 (2H, m), 4.07-4.16 (1H, m), 5.10-5.45 (1H, m), 6.84-7.50 (6H,m), 7.90-7.93 (1H, m), 10.04 (1H, br s).
IR (ATR) $cm^{-1}$ : 2927, 1720, 1639, 1577, 1280, 1203, 1068, 804.
MS (LC-ESI) m/z : 581 ($M^+$+1).
Anal. Calcd for $C_{30}H_{30}F_2N_4O_4S \cdot 0.3H_2O$: C, 61.48; H, 5.26; N, 9.56. Found: C, 61.41; H, 5.18; N, 9.48.

Example 63

-[2-[1-tert-butoxycarbonyl-4-oxo-(2S)-pyrrolidinyl]-5-thiazolyl]propionic acid methyl ester

[Chem. 189]

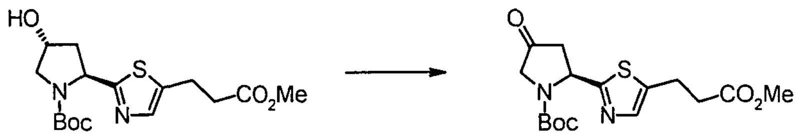

A solution (3ml) of dimethyl sulfoxide (0.85ml, 12.0mmol) in methylene chloride was added dropwise to a solution (50ml) of oxalyl chloride (0.52ml, 6.0mmol) at -78° C under the nitrogen atmosphere while stirring. The reaction solution was further stirred at the same temperature for 50 minutes, a solution of 3-[2-[1-tert-butoxycarbonyl-(4R)-hydroxy-(2S)-pyrrolidin yl]-5-thiazolyl]propionicacidmethyl ester (0.71g, 2.0mmol) in methylene chloride was added dropwise, and the mixture was stirred for 1.5 hours. Further, to this reaction solution was added triethylamine (2.51ml, 18.0mmol) at the same temperature, the mixture was stirred for 30 minutes, a temperature was raised to 0°C, and the mixture was stirred for 30 minutes. To this reaction solution was added an aqueous saturated ammonium chloride solution, and this was stirred for 0.5 hours, followed by extraction with chloroform. The combined extracts were washed with an aqueous saturated sodium chloride solution, and dried with anhydrous sodium sulfate, and a solvent was removed under reduced pressure. The resulting residue was purified by flash chromatography (Boitage flash chromatography system, column size: 40S, eluting solvent: ethyl acetate: n-hexane= 20% - 60%) to obtain the title compound (0.53g, 75%) as a yellow oily substance.

$^1$H-NMR ($CDCl_3$) δ: 1.48 (9H, s), 2.66 (2H, t, J = 7.1 Hz), 2.97-3.06 (2H, m), 3.13 (2H, t, J = 7.1 Hz), 3.69 (3H, s), 3.74 and 3.79 (total 1H, each s, amide isomers), 3.90-4.09 (1H, m), 5.41-5.79 (1H, m), 7.40 (1H, s).
MS (ESI) m/z : 355 ($M^+$+1).

3-[2-[1-tert-butoxycarbonyl-(4S)-[1-(3-methoxy)azetidinyl]-(2S)-pyrrolidinyl]-5-thiazolyl]propionic acid methyl ester

[Chem. 190]

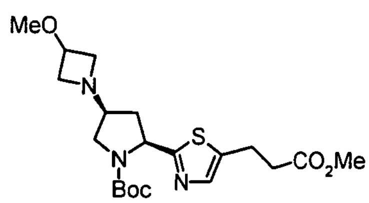

Sodium triacetoxyborohydride (3.67g, 17.32mmol) was added to a solution (100ml) of 3-[2-[1-tert-butoxycarbonyl-4-oxo-(2S)-pyrrolidinyl]-5-t hiazolyl]propionic acid methyl ester (3.07g, 8.66mmol) and 3-methoxyazetidine hydrochloride (2.77g, 22.4mmol) in 1,2-dichloroethane, and this was stirred for 19 hours. To the reaction solution was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size: 40M, eluting solvent: ethyl acetate/methanol= 0% - 10%) to obtain the title compound (2.32g, 63%) as a yellow oily substance.

$^1$H-NMR ($CDCl_3$) δ: 1.29 and 1.45(total 9H, each s, amide isomers), 1.94-2.22 (1H, m), 2.29-2.43 (1H, m), 2.65 (2H, t, J = 7.4 Hz), 2.83 (2H, s), 2.93-3.00 (1H, m), 3.12 (2H, t, J = 6.6 Hz), 3.22 (3H, s), 3.23-3.42 (2H, m), 3.48-3.65 (2H, m), 3.69 (3H, s), 3.83-3.95 (1H, m), 4.96-5.16 (1H, m), 7.30 (1H, s).
MS (ESI) m/z : 426 ($M^+$+1).

3-[2-(4S)-[1-(3-methoxy)azetidinyl]-(2S)-pyrrolidinyl]-5-thiazolyl]propionic acid methyl ester

[Chem. 191]

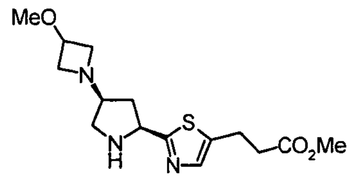

A 4N hydrochloric acid/ dioxane solution (100ml) was added to 3-[2-[1-tert-butoxycarbonyl-(4S)-[1-(3-methoxy) azetidiny 1]-(2S)-pyrrolidinyl]-5-thiazolyl]propionic acid methyl ester (2.32g, 5.45mmol), and the mixture was stirred for 17 hours. The reaction solution was concentrated under reduced pressure. The resulting residue was diluted with methylene chloride, a 1N aqueous sodium hydroxide solution was added to adjust a pH to 10, and this was extracted with methylene chloride. The combined extracts were washed with an aqueous saturated sodium chloride solution, and dried with anhydrous sodium sulfate, and a solvent was removed under reduced pressure. The title compound (1.27g, 72%) was obtained as a yellow oily substance. The present compound was used in the next reaction without further purification.

$^1$H-NMR ($CDCl_3$) δ: 1.68-1.79 (1H, m), 2.35-2.44 (1H, m), 2.65 (2H, t, J = 7.6 Hz), 2.83-3.07 (5H, m), 3.11 (2H, t, J = 6.9 Hz), 3.24 (3H, s), 3.30-3.49 (1H, m), 3.50-3.64 (2H, m), 3.69 (3H, s), 3.95-4.02 (1H, m), 4.45 (1H, t, J = 8.0 Hz), 7.36 (1H, s).
MS (ESI) m/z : 326 ($M^+$+1).

3-[2-[1-[5- Chloro- 2- fluoro- 4-(1- methyl- 3- indolylcarbonylamino) phenylacetyl]-(4S)-[1-(3- methoxy) azetidinyl]-(2S)-pyrrolidinyl]-5-thiazolyl]propionic acid methyl ester

[Chem. 192]

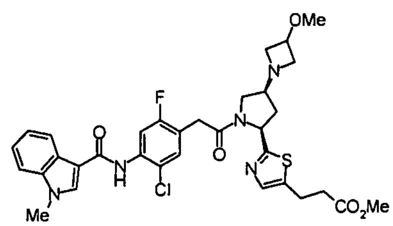

Triethylamine (0.39ml, 2.78mmol) was added to a solution (10ml) of 5-chloro-2-fluoro-4-(1-methyl-3-indolylcarbonylamino)phenylacetic acid (202mg, 0.56mmol), 3-[2-[(4S)-[1-(3-methoxy)azetidinyl]-(2S)-pyrrolidinyl]-5-thiazolyl] propionic acid methyl ester (181mg, 0.56mmol), EDC · HCl (160mg, 0.83mmol) and HOBt (113mg, 0.83mmol) in methylene chloride (10ml) at room temperature, and the mixture was stirred for 2 days. The reaction solution was concentrated under reduced pressure, and the resulting residue was purified by flash chromatography (Biotage flash chromatography system, column size: 25S, eluting solvent: methanol/ethyl acetate= 10% - 30%) to obtain the title compound (238mg, 64%) as a yellow oily substance.

$^1$H-NMR ($CDCl_3$) δ: 2.11-2.54 (2H, m), 2.61-2.71 (2H, m), 2.79-2.88 (2H, m), 3.01-3.39 (7H, m), 3.41-3.76 (7H, m), 3.83-3.94 (4H, m), 5.32 and 5.38 (total 1H, each dd, J = 8.8, 2.9 and 8.3, 4.2 Hz respectively, amide isomers), 7.23 and 7.25 (total 1H, each s, amide isomers), 7.30-7.44 (4H, m), 7.46 and 7.47 (total 1H, each s, amide isomers), 7.80 (1H, d, J = 7.4 Hz), 8.09-8.18 (1H, m), 8.26 and 8.31 (total 1H, each s, amide isomers), 8.45 and 8.53 (total 1H, each d, each J = 12.0 Hz, amide isomers).
MS (ESI) m/z : 668 ($M^+$+1), 670 ($M^+$+3).

3-[2-[1-[5- Chloro- 2- fluoro- 4-(1- methyl- 3- indolylcarbonylamino) phenylacetyl]-(4S)-[1-(3- methoxy) azetidinyl]-(2S)-pyrrolidinyl]-5-thiazolyl]propionic acid

[Chem. 193]

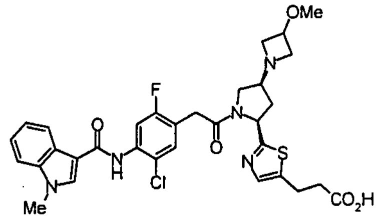

A 1N aqueous sodium hydroxide solution (2ml, 2mmol) was added to a solution of 3-[2-[1-[5-chloro-2-fluoro-4-(1-methyl-3-indolylcarbonyl amino)phenylacetyl]-(4S)-[1-(3-methoxy)azetidinyl]-(2S)-pyrrolidinyl]-5-thiazolyl]propionic acid methyl ester (238mg, 0.36mmol) in tetrahydrofuran/methanol (4ml/2ml) at room temperature, and the mixture was stirred for 4 days. The reaction solution was concentrated under reducedpressure, the resulting residue was adjusted to a pH 7 with 1N hydrochloric acid, and this was extracted with chloroform/ methanol [10/1 (v/v)]. The combined extracts were washed with an aqueous saturated sodium chloride solution. The resulting organic layer was dried with anhydrous sodium sulfate, and a solvent was removed under reduced pressure. The resulting residue was subjected to thin-layer chromatography (chloroform/ethanol= 5/1 (v/v)) to obtain the title compound (217mg) as a colorless glassy solid, which was lyophilized from dioxane to obtain 187mg (79%) of a colorless glassy solid.

$^1$H-NMR ($CDCl_3$) δ: 2.10-2.68 (4H, m), 2.93-3.16 (4H, m), 3.23 and 3.23 (total 3H, each s, amide isomers), 3.34 and 3.44 (total 1H, d and dd, J = 15.7 and 12.3, 5.6 Hz respectively, amide isomers), 3.51-3.79 (6H, m), 3.81 and 3.83 (total 3H, each s, amide isomers), 3.92-4.03 (2H, m), 5.36 and 5.38 (total 1H, each dd, J = 8.6, 4.7and 8.6, 5.1 Hz respectively, amide isomers), 7.20-7.44 (5H, m), 7.75 (1H, d, J=4.2Hz), 8.07-8.14 (1H, m), 8.21 (1H, d, J = 3.4 Hz), 8.37 and 8.47 (total 1H, each d, J = 11.8 and 12.0 Hz respectively, amide isomers). MS (ESI) m/z : 654 ($M^+$+1), 656 ($M^+$+3). IR (ATR) $cm^{-1}$ : 2939, 2833, 1720, 1649, 1583, 1514, 1466.

Compounds of Example 4 to Example 68 shown below were produced by the same method as that of Example 63.

Example 64

3-[2-[1-[2,5- Dichloro- 4-(1- methyl- 3- indolylcarbonylamino) phenylacetyl]-(4S)-[1-(3- methoxy) azetidinyl]-(2S)-pyrrolidinyl]-5-thiazolyl]propionic acid methyl ester

[Chem. 194]

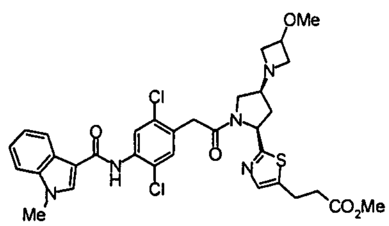

$^1$H-NMR ($CDCl_3$) δ: 2.10-2.54 (2H, m), 2.66 (2H, td, J = 7.5, 2.4 Hz), 2.79-2.91 (2H, m), 2.97-3.18 (3H, m), 3.21 and 3.24 (total 3H, each s, amide isomers), 3.29-3.65 (4H, m), 3.66-3.83 (5H, m), 3.84-3.96 (4H, m), 5.34-5.43 (1H, m), 7.26 (1H, s), 7.40-7.40 (3H, m), 7.49 (1H, s), 7.80 (1H, d, J = 7.1 Hz), 8.10-8.17 (1H, m), 8.22 and 8.25 (total 1H, each s, amide isomers), 8.73 and 8.81 (total 1H, each s, amide isomers).
MS (ESI) m/z : 684 ($M^+$+1), 686 ($M^+$+3).

3-[2-[1-[2,5- Dichloro- 4-(1- methyl- 3- indolylcarbonylamino) phenylacetyl]-(4S)-[1-(3- methoxy)-azetidinyl]-(2S)-pyrrolidinyl]-5-thiazolyl]propionic acid

[Chem. 195]

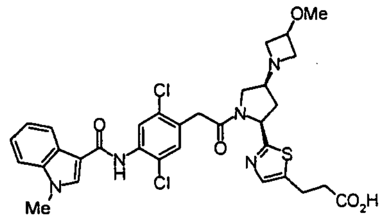

$^1$H-NMR ($CDCl_3$) δ: 2.10-2.51 (2H, m), 2.54-2.71 (3H, m), 2.95-3.18 (4H, m), 3.23 and 3.24 (total 3H, each s, amide isomers), 3.35-3.88 (8H, m), 3.94-4.05 (1H, m), 5.36-5.44 (1H, m), 7.13-7.47 (6H, m), 7.72 and 7.74 (total 1H, each s, amide isomers), 8.07-8.20 (2H, m), 8.64 and 8.73 (total 1H, each s, amide isomers).
MS (ESI) m/z : 670 ($M^+$+1), 672 ($M^+$+3).
IR (ATR) $cm^{-1}$ : 2931, 2831, 1720, 1649, 1568, 1533, 1500, 1468. Anal. Calcd for $C_{32}H_{33}Cl_2N_5O_5S \cdot 1.25H_2O$: C, 55.45; H, 5.16; Cl, 10.23; N, 10.10; S, 4.63. Found: C, 55.64; H, 5.13; Cl, 10.13; N, 9.61; S, 4.52.

Example 65

3-[2-[1-[4-(3-Benzo[b]thiophenylcarbonylamino)-5-chloro-2-fluorophenylacetyl]-(4S)-[1-(3-methoxy)azetidinyl]-(2S)-pyrrolidinyl]-5-thiazolyl]propionic acid methyl ester
[Chem. 196]

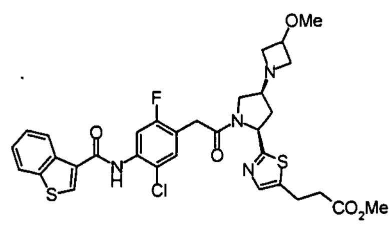

$^1$H-NMR ($CDCl_3$) δ: 2.10-2.54 (2H, m), 2.60-2.72 (2H, m), 2.79-2.89 (2H, m), 3.02-3.19 (3H, m), 3.22 and 3.24 (total 3H, each s, amide isomers), 3.31-3.76 (7H, m), 3.84-3.96 (1H, m), 5.33 and 5.38 (total 1H, each dd, J = 9. 1, 2.9 and 8.6 4.4 Hz respectively, amide isomers), 7.24 (1H, s), 7.32 (1H, s), 7.36 (1H, s), 7.42-7.56 (2H, m), 7.91 and 8.09 (total 1H, dd and d, J = 7.8, 3.4 and 9.6 Hz respectively, amide isomers), 8.30 and 8.35 (total 1H, each s, amide isomers), 8.37 and 8.40 (total 1H, each s, amide isomers), 8.46 and 8.49 (total 1H, each s, amide isomers), 8.47-8.49 (1H, m).
MS (ESI) m/z : 671 ($M^+$+1), 673 ($M^+$+3).
3-[2-[1-[4-(3-Benzo[b]thiophenylcarbonylamino)-5-chloro-2-fluorophenylacetyl]-(4S)-[1-(3-methoxy)azetidinyl]-(2S)-pyrrolidinyl]-5-thiazolyl]propionic acid
[Chem. 197]

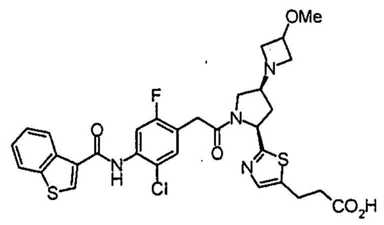

$^1$H-NMR ($CDCl_3$) δ: 2.17-2.41 (1H, m), 2.46-2.79 (6H, m), 3.04-3.34 (3H, m), 3.36-3.67 (4H, m), 3.70-3.78 (1H, m), 3.91-4.24 (1H, m), 5.34-5.43 (1H, m), 7.27-7.58 (4H, m), 7.91 (1H, d, J = 7.8 Hz), 8.09 and 8.10 (total 1H, each s, amide isomers), 8.34 (1H, d, J = 15.9 Hz), 8.41-8.50 (2H, m).
MS (ESI) m/z : 658 ($M^+$+1), 660 ($M^+$+3).
IR (ATR) $cm^{-1}$ : 2956, 2889, 2856, 1720, 1645, 1585, 1516.
Anal. Calcd for $C_{31}H_{30}ClFN_4O_5S \cdot H_2O$: C, 55.15; H, 4.78; Cl, 5.25; F, 2.81; N, 8.30; S, 9.50.
Found: C, 55.37; H, 4.83; Cl, 5.07; F, 2.62; N, 7.96; S, 9.27.

Example 66

3-[2-[1-[4-(3- Benzo [b] thiophenylcarbonylamino)- 2,5- dichlorophenylacetyl]-(4S)-[1-(3- methoxy) azetidinyl]-(2S)-pyrrolidinyl]-5-thiazolyl]propionic acid methyl ester
[Chem. 198]

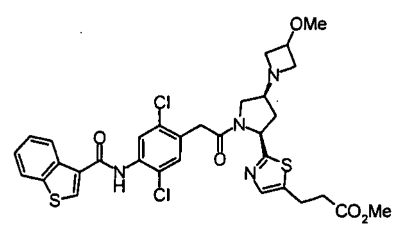

$^1$H-NMR ($CDCl_3$) δ: 2.10-2.56 (2H, m), 2.66 (2H, td, J = 7.5, 4.8 Hz), 2.82-2.90 (2H, m), 3.05-3.17 (3H, m), 3.22 and 3.24 (total 3H, each s, amide isomers), 3.35-3.65 (3H, m), 3.66-3.83 (5H, m), 3.85-3.99 (2H, m), 5. 36 and 5.39 (total 1H, each dd, J = 8.8, 2.9 and 8.6, 4.4 Hz respectively, amide isomers), 7.32 and 7.35 (total 1H, each s, amide isomers), 7.40-7.55 (3H, m), 7.91 (1H, dd, J = 7.8, 3.4 Hz), 8.09 (1H, d, J=8.3Hz), 8.26 and 8.30 (total 1H, each s, amide isomers), 8.48 (1H, dd, J = 8.1, 4.9 Hz), 8.65 and 8.73 (total 1H, each s, amide isomers).
MS (ESI) m/z : 687 ($M^+$+1), 689 ($M^+$+3).
3-[2-[1-[4-(3-Benzo[b]thiophenylcarbonylamino)-2,5-diphenylacetyl]-(4S)-[1,3-(methoxy)azeditinyl]-(2S)-pyrrolidinyl]-5-thiazolyl]propionic acid
[Chem. 199]

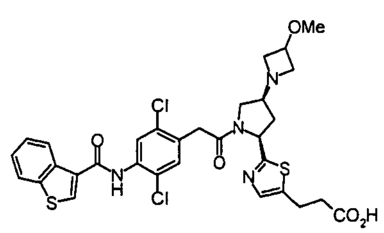

$^1$H-NMR ($CDCl_3$) δ: 2.17-2.41 (1H, m), 2.46-2.79 (6H, m), 3.04-3.34 (3H, m), 3.36-3.67 (4H, m), 3.70-3.78 (1H, m), 3.91-4.24 (1H, m), 5.34-5.43 (1H, m), 7.27-7.58 (4H, m), 7.91 (1H, d, J = 7.8 Hz), 8.09 and 8.10 (total 1H, each s, amide isomers), 8.34 (1H, d, J = 15.9 Hz), 8.41-8.50 (2H, m).
MS (ESI) m/z : 673 ($M^{+}$+1), 675 ($M^{+}$+3).
IR (ATR) $cm^{-1}$ : 2941, 2833, 1712, 1647, 1587, 1558, 1522.

Example 67

3-[2-[1-[4-(3- Benzo [b] furanylcarbonylamino)- 5- chloro- 2- fluorophenylacetyl]-(4S)-[1-(3- methoxy) azetidinyl]-(2S)-pyrrolidinyl]-5-thiazolyl]propionic acid methyl ester
[Chem. 200]

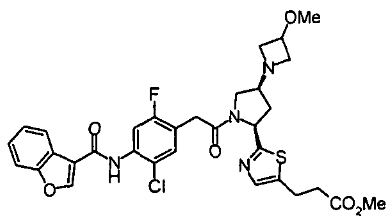

$^1$H-NMR ($CDCl_3$) δ: 2.12-2.56 (3H, m), 2.60-2.71 (2H, m), 2.77-2.99 (2H, m), 3.02-3.19 (3H, m), 3.22 and 3.24 (total 3H, each s, amide isomers), 3.31-3.78 (7H, m), 3.83-4.20 (1H, m), 5.33 and 5.38 (total 1H, each dd, J = 9. 1, 2.9 and 8.3, 4.4 Hz respectively, amide isomers), 7.13-7.19 (1H, m), 7.22-7.28 (1H, m), 7.32 and 7.36 (total 1H, each t, J = 0.7 and 1.0 Hz respectively, amide isomers), 7.40-7.50 (2H, m), 7.56-7.63 (1H, m), 8.01-8.09 (1H, m), 8.24 (1H, s), 8.28 and 8.30 (total 1H, each s, amide isomers), 8.38 and 8.47 (total 1H, each d, each J = 11.8 Hz, amide isomers).
MS (ESI) m/z : 655 ($M^{+}$+1), 657 ($M^{+}$+3) .
3-[2-[1-[4-(3- Benzo [b] furanylcarbonylamino)- 5- chloro- 2- fluorophenylacetyl]-(4S)-[1-(3- methoxy) azetidinyl]-(2S)-pyrrolidinyl]-5-thiazolyl]propionic acid
[Chem. 201]

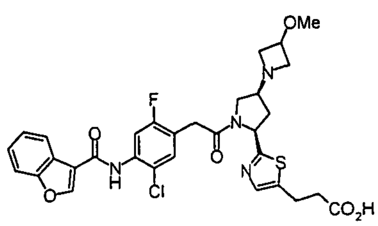

$^1$H-NMR ($CDCl_3$) δ: 2.16 (1H, dt, J=13.4, 5.0Hz), 2.31-2.48 (1H, m), 2.55-2.68 (2H, m), 2.95-3.17 (4H, m), 3.23 and 3.24 (total 3H, each s, amide isomers), 3.30-3.84 (5H, m), 3.92-4.04 (1H, m), 5.36 and 5.38 (total 1H, each dd, J = 8.6, 4.7 and 8.6, 4.9 Hz respectively, amide isomers), 7.24 (1H, d, J = 7.8 Hz), 7.32 (1H, s), 7.35-7.47 (3H, m), 7.52-7.61 (1H, m), 8.00-8.06 (1H, m), 8.23 and 8.27 (total 1H, each s, amide isomers), 8.29 (1H, d, J = 7.8 Hz), 8.32 and 8.26 (total 1H, each d, J = 11.0 and 11.8 Hz respectively, amide isomers). MS (ESI) m/z : 641 ($M^{+}$+1), 643 ($M^{+}$+3).
IR (ATR) $cm^{-1}$: 2941, 2833, 1712, 1647, 1558, 1522, 1448.
Anal. Calcd for $C_{31}H_{30}ClFN_4O_6S \cdot H_2O$: C, 56. 49; H, 4.89; Cl, 5. 38; F, 2.88; N, 8.50; S, 4.86.
Found: C, 56.53; H, 4.90; Cl, 5.19; F, 2.81; N, 8.07; S, 4.67.

Example 68

3-[2-[1-[4-(3- Benzo [b] furanylcarbonylamino)- 2,5- dichlorophenylacetyl]-(4S)-[1-(3- methoxy) azetidinyl]-(2S)-pyrrolidinyl]-5-thiazolyl]propionic acid methyl ester

[Chem. 202]

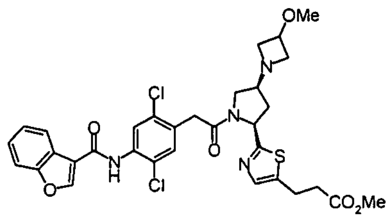

$^1$H-NMR ($CDCl_3$) δ: 2.09-2.56 (2H, m), 2.66 (2H, td, J = 7.5, 3.8 Hz), 2.80-2.89 (2H, m), 3.02-3.18 (3H, m), 3.22 and 3.24 (total 3H, each s, amide isomers), 3.33-3.96 (9H, m), 4.10-4.18 (1H, m), 5.36 and 5.39 (total 1H, each dd, J = 9.1, 2.7 and 8.3, 4.2 Hz respectively, amide isomers), 7.32 and 7.35 (total 1H, each s, amide isomers), 7.39-7.51 (3H, m), 7.55-7.63 (1H, m), 8.01-8.08 (1H, m), 8.20 and 8.24 (total 1H, each s, amide isomers), 8.29 (1H, d, J = 6.9 Hz), 8.63 and 8.71 (total 1H, each s, amid isomers).
MS (ESI) m/z : 670 ($M^+$+1), 672 ($M^+$+3).

3-[2-[1-[4-(3- Benzo [b] furanylcarbonylamino)- 2,5- dichlorophenylacetyl]-(4S)-[1-(3- methoxy) azetidinyl]-(2S)-pyrrolidinyl]-5-thiazolyl]propionic acid

[Chem. 203]

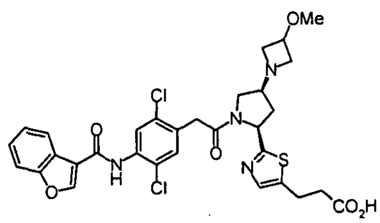

$^1$H-NMR ($CDCl_3$) δ: 2.09-2.68 (4H, m), 2.94-3.20 (4H, m), 3.23 and 3.24 (total 3H, each s, amide isomers), 3.24-3.83 (6H, m), 3.94-4.05 (1H, m), 5.39 (1H, dd, J = 8.5, 5.0 Hz), 7.22 and 7.33 (total 1H, each s, amide isomers), 7.33-7.44 (3H, m), 7.52-7.59 (1H, m), 7.99-8.05 (1H, m), 8.20 and 8.26 (total 1H, each s, amide isomers), 8.28 and 8.31 (total 1H, each s, amide isomers), 8.54 and 8.57 (total 1H, each s, amide isomers).
MS (ESI) m/z : 657 ($M^+$+1), 659 ($M^+$+3).
IR (ATR) $cm^{-1}$: 2939, 2833, 1718, 1643, 1572, 1508, 1427.
Anal. Calcd for $C_{31}H_{30}Cl_2N_4O_6S \cdot H_2O$: C, 55. 11; H, 4.77; C1, 10. 50; N, 8.29; S, 4.75.
Found: C, 55.25; H, 4.78; Cl, 10.22; N, 7.99; S, 4.70.

Example 69

3-[2-[1-teert-Butoxycarbonyl-(4S)-[1-(3-methoxy)azetidinyl]-(2S)-pyrrolidinyl]-5-thiazolyl]propionic acid methyl ester

[Chem. 204]

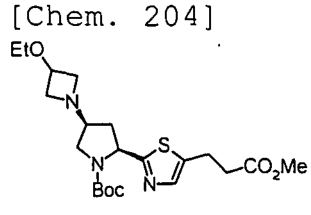

Sodium triacetoxyborohydride (2.69g, 12.70mmol) was added to a solution (50ml) of 3-[2-[1-tert-butoxycarbonyl-4-oxo-(2S)-pyrrolidinyl]-5-t hiazolyl]propionic acid methyl ester (2.25g, 6.35mmol) and 3-ethoxyazetidine hydrochloride (1.75g, 12.7mmol) in 1, 2-dichloroethane, and the mixture was stirred for 17 hours. To the reaction solution was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size: 40M, eluting solvent: ethyl acetate/methanol= 0%-20%) to obtain the title compound (2.80g) as a mixture containing ethyl ester produced by partial transesterification, as a yellow oily substance.

$^1$H-NMR ($CDCl_3$) δ: 1.26 (3H, t, J = 7.1 Hz), 1.56 (9H, s), 2. 64 (3H, dd, J=14.0, 7.4 Hz), 2.78-2.87 (2H, m), 2.92-3.00 (1H, m), 3.06-3.17 (3H, m), 3.34-3.42 (2H, m), 3.51-3.65 (2H, m), 3.69 (3H, s), 4.15 (2H, q, J = 7.1 Hz), 7.30 (1H, s). MS (ESI) m/z : 440 ($M^+$+1), m/z : 454 ($M^+$+1, ethyl ester).

3-[2-[(4S)-[1-(3-ethoxy)azetidinyl]-(2S)-pyrrolidinyl]-5-thiazolyl]propionic acid methyl ester

[Chem. 205]

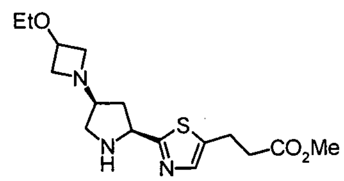

A 4N hydrochloric acid/dioxane solution (50ml) was added to 3-[2-[1-tert-butoxycarbonyl-(4S)-[1-(3-ethoxy)azetidinyl]-(2S)-pyrrolidinyl]-5-thiazolyl]propionic acid methyl ester (2.80g, 6.35mmol), and the mixture was stirred for 24 hours. The reaction solution was concentrated under reduced pressure. The resulting residue was diluted with methylene chloride, and an aqueous saturated sodium bicarbonate solution was added to adjust a pH to 10, followed by extraction with methylene chloride. The combined extracts were washed with an aqueous saturated sodium chloride solution, dried with anhydrous sodium sulfate, and a solvent was removed under reduced pressure. The title compound (1.02g, 47%) was obtained as a pale brown oily substance. The present compound was used in the next reaction without further purification.

3-[2-[1-[5-Chloro-2-fluoro-4-(1-methyl-3-indolylca rbonylamino)phenylacetyl]-(4S)-[1-(3-ethoxy)azetidinyl]-(2S)-pyrrolidinyl]-5-thiazolyl]propionic acid methyl ester

[Chem. 206]

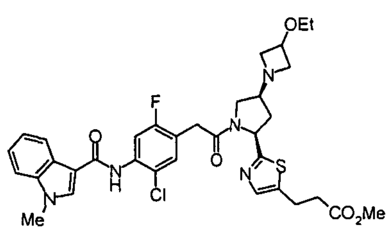

Triethylamine (1.39ml, 10mmol) was added to a solution (10ml) of 5-chloro-2-fluoro-4-(1-methyl-3-indolylcarbonylamino)phenylacetic acid (359mg, 1.0mmol), 3-[2-[(4S)-[1-(3-ethoxy)azetidinyl]-(2S)-pyrrolidinyl]-5-thiazolyl]propionic acid methyl ester (256mg, 0.68mmol), EDC · HCl (288mg, 1.50mmol) and HOBt (203mg, 1.5mmol) in methylene chloride (10 ml) at room temperature, and the mixture was stirred for 2 days. The reaction solution was concentrated under reduced pressure, and the resulting residue was purified by flash chromatography (Biotag flash chromatography system, column size: 25S, eluting solvent: methanol/ethyl acetate= 0-10%) to obtain the title compound (320mg, 69%) as a yellow oily substance.

$^1$H-NMR ($CDCl_3$) δ: 1.13-1.28 (5H, m), 2.10-2.55 (2H, m), 2.61-2.70 (2H, m), 2.83 (2H, q, J = 7.0 Hz), 3.01-3.18 (3H, m), 3.25-3.80 (7H, m), 3.87 and 3.88 (total 3H, each s, amide isomers), 3.92-4.01 (1H, m), 4.09-4.18 (2H, m), 5.32

and 5.37 (total 1H, each dd, J = 9.1, 2.9 and 8.6, 4.4 Hz respectively, amide isomers), 7.20-7.50 (5H, m), 7.79 (1H, d, J = 7.8 Hz), 8.10-8.18 (1H, m), 8.26 and 8.30 (total 1H, each s, amide isomers), 8.44 and 8.53 (total 1H, each d, J = 12.0 and 12.5 Hz respectively, amide isomers).
MS (ESI) m/z : 682 ($M^{+}$+1), 683 ($M^{+}$+3).

3-[2-[1-[5- Chloro- 2- fluoro- 4-(1- methyl- 3- indolylcarbonylamino) phenylacetyl]-(4S)-[1-(3- ethoxy) azeditinyl]-(2S)-pyrroldinyl]-5-thiazolyl]propionic acid

[Chem. 207]

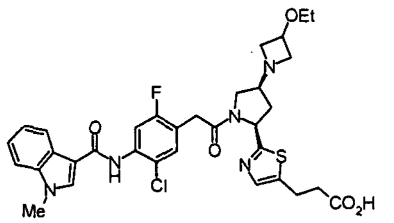

A 1N aqueous sodium hydroxide solution (2ml, 2mmol) was added to a solution of 3-[2-[1-[5-chloro-2-fluoro-4-(1-methyl-3-indolylcarbonylamino)phenylacetyl]-(4S)-[1-(3-ethoxy)azetidinyl]-(2S)-pyrrolidinyl]-5-thiazolyl]propionic acid methyl ester (320mg, 0.47mmol) in a tetrahydrofuran-methanol mixture (4ml/2ml) at room temperature, and the mixture was stirred for 15 hours.
The reaction solution was adjusted to a pH 6 using 1N hydrochloric acid, and concentrated under reduced pressure. The resulting residue was subjected to thin-layer chromatography (eluting solvent: chloroform/ethanol = 10/1 (v/v)) to obtain the title compound (250mg) as a colorless glassy solid, which was lyophilized from dioxane to obtain 228mg (73%) of a colorless glassy solid.

$^{1}$H-NMR ($CDCl_3$) δ: 1.17 (3H, td, J=7.0, 1.6Hz), 2.03-2.20 (2H, m), 2.30-2.70 (3H, m), 2.98-3.19 (4H, m), 3.22-3.49 (3H, m), 3.56-3.88 (7H, m), 3.96-4.16 (2H, m), 5.34-5.44 (1H, m), 7.21-7.45 (5H, m), 7.76 (1H, d, J = 6.4 Hz), 8.07-8.15 (1H, m), 8.22 (1H, s), 8.38 and 8.48 (total 1H, each d, J = 11.8 and 12.0 Hz respectively, amide isomers).
MS (ESI) m/z : 668 ($M^{+}$+1), 670 ($M^{+}$+3).
IR (ATR) $cm^{-1}$ : 2970, 2935, 1718, 1649, 1585, 1516, 1468.

Compounds of Example 70 to Example 72 shown below were produced by the same method as that of Example 69.

Example 70

3-[2-[1-[2,5- Dichloro- 4-(1- methyl- 3- indolylcarbonylamino) phenylacetyl]-(4S)-[1-(3- ethoxy) azetidinyl]-(2S9-pyrrolidinyl)-5-thiazolyl]propionic acid methyl ester

[Chem. 208]

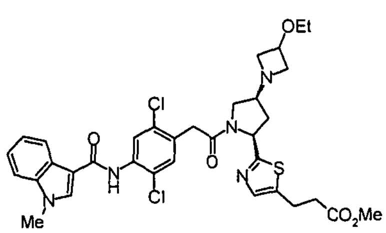

$^{1}$H-NMR ($CDCl_3$) δ: 1.14-1.28 (5H, m), 2.11-2.54 (3H, m), 2.61-2.69 (2H, m), 2.79-2.89 (2H, m), 3.00-3.48 (6H, m), 3.50-3.83 (4H, m), 3.87 and 3.88 (total 3H, each s, amide isomers), 3.91-4.19 (2H, m), 5.34-5.43 (1H, m), 7.23-7.49 (5H, m), 7.79 (1H, d, J = 7.6 Hz), 8.09-8.16 (1H, m), 8.21 and 8.25 (total 1H, each s, amide isomers), 8.72 and 8.80 (total 1H, each s, amide isomers).

MS (ESI) m/z : 698 ($M^+$+1), 700 ($M^+$+3).

3-[2-[1-[2,5-Dichloro-4-(1-methyl-3-indolylcarbonylamino)phenylacetyl]-(4S)-[1-(3-ethoxy)azetidinyl]-(2S)-pyrrolidinyl]-5-thiazolyl]propionic acid

[Chem. 209]

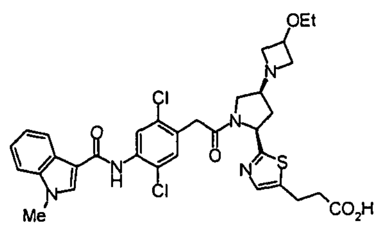

$^1$H-NMR ($CDCl_3$) δ: 1.17 (3H, td, J = 7.0, 2.9 Hz), 2.12-2.21 (1H, m), 2.35-2.73 (4H, m), 2.97-3.50 (7H, m), 3.58-3.88 (7H, m), 4.01-4.15 (2H, m), 5.37-5.45 (1H, m), 7.20-7.46 (5H, m), 7.72 and 7.75 (total 1H, each s, amide isomers), 8.07-8.15 (2H, m), 8.65 and 8.74 (total 1H, each s, amide isomers). MS (ESI) m/z : 684 ($M^+$+1), 686 ($M^+$+3). IR (ATR) $cm^{-1}$: 2970, 2850, 1716, 1649, 1568, 1533, 1500.

Example 71

3-[2-[1-[4-(3- Benzo [b] thiophenylcarbonylamino)- 5- chloro- 2- fluorophenylacetyl]-(4S)- 1-(3- ethoxy) azetidinyl-(2S)-pyrrolidinyl]-5-thiazolyl]propionic acid methyl ester

[Chem. 210]

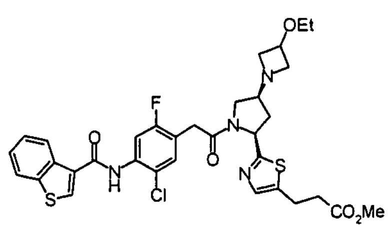

$^1$H-NMR ($CDCl_3$) δ: 1.14-1.28 (6H, m), 1.80-1.92 (1H, m), 2.11-2.53 (3H, m), 2.59-2.90 (2H, m), 3.01-3.18 (2H, m), 3.23-3.85 (8H, m), 3.89-4.22 (3H, m), 5.33 and 5.37 (total 1H, each dd, J = 9.1, 2. 9 and 8.3, 4.4 Hz respectively, amide isomers), 7.22-7.39 (1H, m), 7.42-7.56 (2H, m), 7.91 (1H, dd, J = 7.8, 3.4 Hz), 8.07-8.11 (1H, m), 8.29-8.40 (1H, m), 8.50-8.44 (2H, m).

MS (ESI) m/z : 685 ($M^+$+1), 687 ($M^+$+3).

3-[2-[1-[4-(3- Benzo [b] thiophenylcarbonylamino)- 5- chloro- 2- fluorophenylacetyl]-(4S)- 1-(3- ethoxy) azetidinyl-(2S)-pyrrolidinyl]-5-thiazolyl]propionic acid

[Chem. 211]

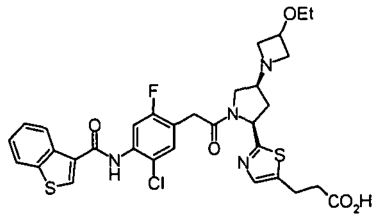

$^1$H-NMR ($CDCl_3$) δ: 1.18 (3H, t, J = 7.1 Hz), 2.00-2.23 (2H, m), 2.31-2.71 (4H, m), 2.97-3.50 (7H, m), 3.58-3.83 (4H, m), 3.96-4.18 (2H, m), 5.34-5.42 (1H, m), 7.21-7.55 (4H, m), 7.89 (1H, t, J = 8.0 Hz), 8.08 (1H, d, J = 3.9 Hz), 8.28-8.49 (3H, m).
MS (ESI) m/z : 671 ($M^+$+1), 673 ($M^+$+3).
IR (ATR) $cm^{-1}$ : 2972, 2852, 1716, 1647, 1585, 1518, 1400.

Example 72

3-[2-[1-[4-(3- Benzo [b] thiophenylcarbonylamino)- 2,5- dichlorophenylacetyl]-(4S)- 1-(3- ethoxy) azetidinyl-(2S)-pyrrolidinyl]-5-thiazolyl]propionic acid methyl ester
[Chem. 212]

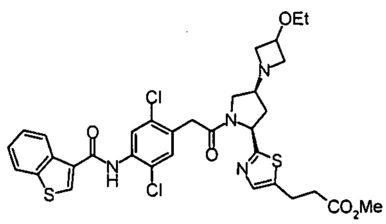

$^1$H-NMR ($CDCl_3$) δ: 1.18 (3H, q, J = 8.1 Hz), 1.22-1.29 (2H, m), 2.10-2.55 (2H, m), 2.61-2.69 (2H, m), 2.81-2.89 (2H, m), 3.04-3.17 (3H, m), 3.32-3.83 (7H, m), 3.92-4.05 (1H, m), 4.08-4.18 (2H, m), 5.34-5.42 (1H, m), 7.32-7.36 (1H, m), 7.42-7.55 (3H, m), 7.91 (1H, q, J = 3.9 Hz), 8.08 (1H, d, J=8.1 Hz), 8.27 and 8.31 (total 1H, each s, amide isomers), 8.45-8.50 (1H, m), 8.65 and 8.72 (total 1H, each s, amide isomers).
MS (ESI) m/z : 701 ($M^+$+1), 703 ($M^+$+3).
3-[2-[1-[4-(3- Benzo [b] thiophenylcarbonylamino)- 2,5- dichlorophenylacetyl]-(4S)- 1-(3- ethoxy) azetidinyl-(2S)-pyrrolidinyl]-5-thiazolyl]propionic acid
[Chem. 213]

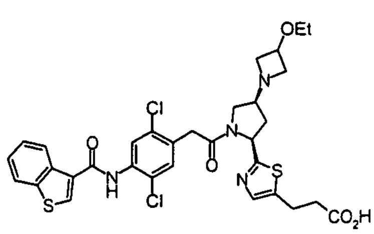

$^1$H-NMR ($CDCl_3$) δ: 1.14-1.22 (3H, m), 2.03-2.21 (2H, m), 2.32-2.75 (4H, m), 2.99-3.51 (6H, m), 3.56-3.87 (4H,

m), 4.00-4.16 (2H, m), 5.40 (1H, dd, J = 8.4, 5.2 Hz), 7.24 (1H, s), 7.30-7.54 (4H, m), 7.88 (1H, t, J = 8.9 Hz), 8.08 (1H, dd, J = 3.9, 0.9 Hz), 8.26 and 8.31 (total 1H, each s, amide isomers), 8.46 (1H, d, J = 8.1 Hz), 8.59 and 8.65 (total 1H, each s, amide isomers).
MS (ESI) m/z : 687 ($M^+$+1), 689 ($M^+$+3).
IR (ATR) $cm^{-1}$ : 2970, 2848, 1716, 1647, 1570, 1504.
Anal. Calcd for $C_{32}H_{32}Cl_2N_4O_5S_2 \cdot 0.5H_2O$: C, 53.78; H, 4.94; Cl, 9.92; N, 7.84; S, 8.97.
Found: C, 54.11; H, 4.78; Cl, 9.58; N, 7.48; S, 8.54.

Example 73

3-[2-[1-tert-butoxycarbonyl-(4S)-[1-(4,4-difluoro)piperidinyl]-(2S)-pyrrolidinyl]-5-thiazolyl]propionic acid methyl ester
[Chem. 214]

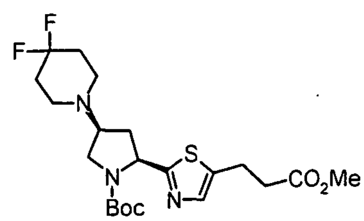

Sodium triacetoxyborohydride (2.69g, 12.69mmol) was added to a solution (50ml) of 3-[2-[1-tert-butoxycarbonyl-4-oxo-(2S)-pyrrolidinyl]-5-t hiazolyl]propionic acid methyl ester (2.25g, 6.35mmol) and 4,4-difluoropiperidine hydrochloride (1.00g, 6.35mmol) in 1,2-dichloroethane, and the mixture was stirred for 20 hours. To the reaction solution was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size : 40M, eluting solvent: n-hexane/ethyl acetate = 50% - 100%) to obtain the title compound (2.22g, 76%) containing ethyl ester as an impurity, as a yellow oily substance.
$^1$H-NMR ($CDCl_3$) δ: 1.20-1.61 (11H, m), 1.86-2.18 (4H, m), 2.46-2.74 (6H, m), 2.83-2.95 (1H, m), 3.06-3.17 (2H, m), 3.28 (1H, t, J = 9.6 Hz), 3.69 (2H, s), 3.98-4.08 (1H, m), 4.09-4.19 (1H, m), 4.99-5.08 (1H, m), 7.35 (1H, s).
MS (ESI) m/z : 460 ($M^+$+1).
3-[2-[(4S)-[1-(4,4-difluoro)piperidinyl]-(2S)-pyrrolidinyl]-5-thiazolyllpropionic acid methyl ester
[Chem. 215]

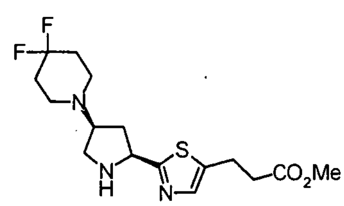

Trifluoroacetic acid (20ml) was added to a solution (100ml) of 3-[2-[1-tert-butoxycarbonyl-(4S)-[1-(4,4-difluoro) piperi dinyl]-(2S)-pyrrolidinyl]-5-thiazolyl]propionic acid methyl ester (2. 92g, 6.35mmol) in methylene chloride at room temperature, and the mixture was stirred for 3 days. The reaction solution was concentrated, an aqueous saturated sodium bicarbonate solution was added to the resulting residue, and the mixture was stirred for 0.5 hour, followed by extraction with methylene chloride. The combined organic layers were washed with an aqueous saturated sodium chloride solution, and dried with anhydrous sodium sulfate. A solvent was removed under reduced pressure to obtain the title compound (2.21g, 94%) containing ethyl ester as an impurity, as a green oily substance. The present compound was used in the next reaction without further purification.

$^1$H-NMR ($CDCl_3$) δ: 1.51 (1H, d, J = 6.6 Hz), 1.80-2.09 (5H, m), 2.48-2.70 (7H, m), 2.93-3.16 (4H, m), 3.24-3.32 (1H, m), 3.70 (2H, s), 3.98 (1H, s), 4.57 (1H, dd, J = 8.8, 7.4 Hz), 7.39-7.37 (1H, m).
MS (ESI) m/z : 360 ($M^+$+1).

3-[2-[1-[5- Chloro- 2- fluoro- 4-(1- methyl- 3- indolylcarbonylamino) phenylacetyl]-(4S)-[1-(4,4- difluoro) piperidinyl]-(2S)-pyrrolidinyl]-5-thiazolyl]propionic acid methyl ester

[Chem. 216]

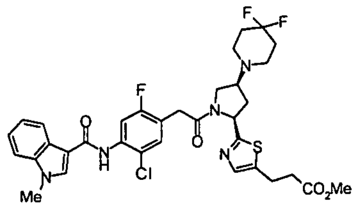

Triethylamine (0.69ml, 10.0mmol) was added to a solution (10ml) of 5-chloro-2-fluoro-4-(1-methyl-3-indolylcarbonylamino)phenylacetic acid (359mg, 1.0mmol), 3-[2-[(4S)-[1-(4,4-difluoro)piperidinyl]-(2S)-pyrrolidin yl] -5-thiazolyl]propionic acid methyl ester (368mg, 1.0mmol), EDC/HCl (286mg, 1.5mmol) and HOBt (202mg, 1.5mmol) in methylene chloride (10ml) at room temperature, and the mixture was stirred for 17 hours. The reaction solution was concentrated under reduced pressure, and the resulting residue was purified by flash chromatography (Biotage flash chromatography system, column size:25S, eluting solvent: n-hexane/ethyl acetate = 50% - 100%) to obtain the title compound (408mg, 58%) containing ethyl ester as an impurity, as a yellow oily substance.

$^1$H-NMR ($CDCl_3$) δ: 1.88-2.31 (5H, m), 2.48-2.75 (6H, m), 2.81-3.52 (4H, m), 3.56-3.74 (4H, m), 3.88 and 3.89 (total 3H, each s, amide isomers), 3.94-4.02 (1H, m), 4.11-4.21 (1H, m), 4.29-4.41 (1H, m), 5.30-5.39 (1H, m), 7.32-7.46 (5H, m), 7.80 and 7.81 (total 1H, each s, amide isomers), 8.10-8.18 (1H, m), 8.27 and 8.31 (total 1H, each s, amide isomers), 8.45 and 8.54 (total 1H, each d, J = 11.8 and 12.0 Hz respectively, amide isomers).

3-[2-[1-[5- Chloro- 2- fluoro- 4-(1- methyl- 3- indolylcarbonylamino) phenylacetyl]-(4S)-[1-(4,4- difluoro) piperidinyl]-(2S)-pyrrolidinyl]-5-thiazolyl]propionic acid

[Chem. 217]

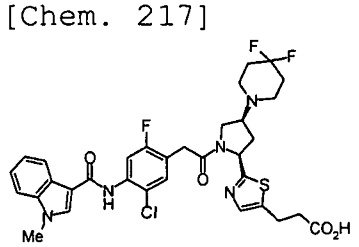

A 1N aqueous sodium hydroxide solution (2ml, 2mmol) was added to a solution of 3-[2-[1-[5-chloro-2-fluoro-4-(1-methyl-3-indolylcarbonylamino)phenylacetyl]-(4S)-[1-(4,4-difluoro)piperidinyl]-(2S)-pyrrolidinyl]-5-thiazolyl]propionic acid methyl ester (408mg, 0.58mmol) in a mixture of tetrahydrofuran/methanol (4ml/2ml) at room temperature, and the mixture was stirred for 14 hours. The reaction solution was added to a pH6 using 1N hydrochloric acid, and a solvent was removed under reduced pressure. The resulting residue was subjected to thin-layer chromatography [eluting solvent: chloroform/methanol = 20/1 (V/V)] to obtain the title compound (214mg) as a colorless glassy solid, which was lyophilized from dioxane to obtain 209mg(79%) as a pale yellow glassy solid.

$^1$H-NMR ($CDCl_3$) δ: 1.91-2.10 (4H, m), 2.15-2.30 (1H, m), 2.51-2.76 (7H, m), 2.81-3.19 (3H, m), 3.29-3.92 (6H, m), 3.91-4.51 (1H, m), 5.31-5.44 (1H, m), 7.27-7.44 (5H, m), 7.72-7.80 (1H, m), 8.05-8.23 (2H, m), 8.63 (1H, s), 8.74 and 8.75 (total 1H, each s, amide isomers).
MS (ESI) m/z : 687 ($M^+$+1), 689 ($M^+$+3).
IR (ATR) $cm^{-1}$ : 1720, 1655, 1626, 1585, 1516, 1468.

Compounds of Example 74 to Example 78 shown below were produced by the same method as that of Example 73.

Example 74

3-[2-[1-[2,5- Dichloro- 4-(1- methyl- 3- indolylcarbonylamino) phenylacetyl]-(4S)-[1-(4,4- difluoro) piperidinyl]-(2S)-pyrrolidinyl]-5-thiazolyl]propionic acid methyl ester

[Chem. 218]

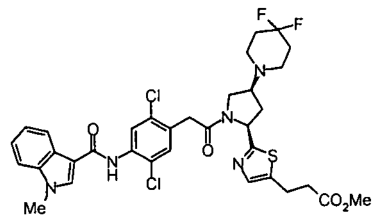

$^1$H-NMR ($CDCl_3$) δ: 1.91-2.08 (5H, m), 2.09-2.34 (1H, m), 2.47-2.73 (5H, m), 2.78-3.20 (3H, m), 3.25-3.54 (1H, m), 3.64-3.82 (4H, m), 3.88 and 3.89 (total 3H, each s, amide isomers), 3.95-4.03 (1H, m), 4.12-4.18 (1H, m), 4.35-4.44 (1H, m), 5.33-5.39 (1H, m), 7.29-7.49 (5H, m), 7.80 (1H, d, J = 6.4 Hz), 8.10-8.17 (1H, m), 8.24 (1H, d, J = 12.3 Hz), 8.74 and 8.81 (total 1H, each s, amide isomers).
MS (ESI) m/z : 717 ($M^+$+1), 719 ($M^+$+3).

3-[2-[1-[2,5- Dichloro- 4-(1- methyl- 3- indolylcarbonylamino) phenylacetyl]-(4S)-[1-(4,4- difluoro) piperidinyl]-(2S)-pyrrolidinyl]-5-thiazolyl]propionic acid

[Chem. 219]

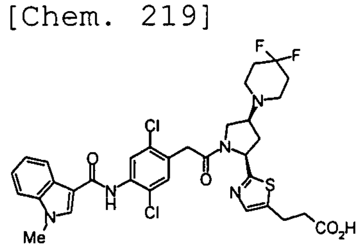

$^1$H-NMR ($CDCl_3$) δ: 1.91-2.10 (4H, m), 2.15-2.30 (1H, m), 2.51-2.76 (7H, m), 2.81-3.19 (3H, m), 3.29-3.92 (6H, m), 3.91-4.51 (1H, m), 5.31-5.44 (1H, m), 7.27-7.44 (5H, m), 7.72-7.80 (1H, m), 8.05-8.23 (2H, m), 8.63 (1H, s), 8.74 and 8.75 (total 1H, each s, amide isomers).
MS (ESI) m/z : 704 ($M^+$+1), 706 ($M^+$+3).
IR (ATR) $cm^{-1}$ : 2964, 2852, 1720, 1653, 1533, 1500, 1469.

Example 75

3-[2-[1-[4-(3-Benzo[b]thiophenylcarbonylamino)-5-chloro-2-fluorophenylacetyl]-(4S)-[1-(4,4-difluoro)piperidinyl]-(2S)-pyrrolidinyl]-5-thiazolyl]propionic acid methyl ester

[Chem. 220]

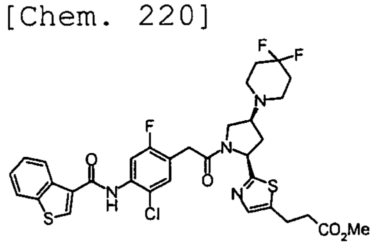

$^1$H-NMR ($CDCl_3$) δ: 1.89-2.34 (5H, m), 2.48-2.72 (7H, m), 2.80-3.54 (4H, m), 3.57-3.75 (4H, m), 3.91-4.44 (2H, m), 5.31-5.37 (1H, m), 7.35-7.57 (4H, m), 7.91 and 7.92 (total 1H, each d, J = 8.1 and 7.8 Hz respectively, amide isomers), 8.09 (1H, d, J = 7.1 Hz), 8.33 and 8.39 (total 1H, each d, J = 14.2 and 11.8 Hz respectively, amide isomers), 8.50-8.45 (2H, m). MS (ESI) m/z : 705 ($M^+$+1), 707 ($M^+$+3).

3-[2-[1-[4-(3-Benzo[b]thiophenylcarbonylamino)-5-chloro-2-fluorophenylacetyl]-(4S)-[1-(4,4-difluoro)piperidinyl]-(2S)-pyrrolidinyl]-5-thiazolyl]propionic acid

[Chem. 221]

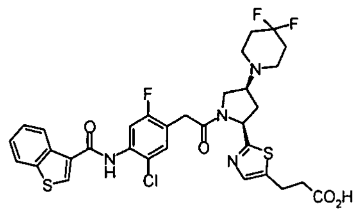

$^1$H-NMR ($CDCl_3$) δ: 2.17-2.41 (1H, m), 2.46-2.79 (6H, m), 3.04-3.34 (3H, m), 3.36-3.67 (4H, m), 3.70-3.78 (1H, m), 3.91-4.24 (1H, m), 5.34-5.43 (1H, m), 7.27-7.58 (4H, m), 7.91 (1H, d, J = 7.8 Hz), 8.09 and 8.10 (total 1H, each s, amide isomers), 8.34 (1H, d, J = 15.9 Hz), 8.41-8.50 (2H, m).
MS (ESI) m/z : 691 ($M^+$+1), 693 ($M^+$+3).
IR (ATR) $cm^{-1}$ : 1718, 1649, 1587, 1518, 1400. Anal. Calcd for $C_{32}H_{30}ClF_3N_4O_4S_2 \cdot 0.5H_2O$: C, 54.89; H, 4.46; Cl, 5.06; F, 8.14; N, 8.00; S, 9.16.
Found: C, 54.88; H, 4.37; Cl, 4.74; F, 7.73; N, 7.70; S, 9.25.

Example 76

3-[2-[1-[4-(3- Benzo [b] thiophenylcarbonylamino)- 2,5- dichlorophenylacetyl-(4S)-[1-(4,4- difluoro) piperidinyl]-(2S)-pyrrolidinyl]-5-thiazolyl]propionicacidmethylester

[Chem. 222]

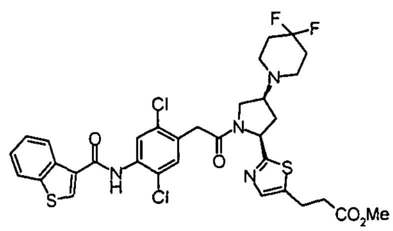

$^1$H-NMR ($CDCl_3$) δ: 1.90-2.05 (5H, m), 2.09-2.35 (1H, m), 2.49-2.71 (5H, m), 2.77-3.56 (5H, m), 3.64-3.83 (4H, m), 3.96-4.43 (2H, m), 5.36 (1H, dd, J = 15.6, 7.8 Hz), 7.34-7.57 (4H, m), 7.91 (1H, dd, J = 8.0, 3.1 Hz), 8.08 (1H, d, J = 5.9 Hz), 8.29 (1H, d, J = 11.5 Hz), 8.47 (1H, dd, J = 8.1, 3.2 Hz), 8.65 and 8.72 (total 1H, each s, amide isomers). MS (ESI) m/z : 721 ($M^+$+1), 723 ($M^+$+3).

3-[2-[1-[4-(3- Benzo [b] thiophenylcarbonylamino)- 2,5- dichlorophenylacetyl-(4S)-[1-(4,4- difluoro) piperidinyl]-(2S)-pyrrolidinyl]-5-thiazolyl]propionic acid

[Chem. 223]

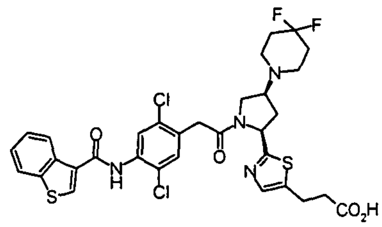

$^1$H-NMR ($CDCl_3$) δ: 2.17-2.41 (1H, m), 2.46-2.79 (6H, m), 3.04-3.34 (3H, m), 3.36-3.67 (4H, m), 3.70-3.78 (1H, m), 3.91-4.24 (1H, m), 5.34-5.43 (1H, m), 7.27-7.58 (4H, m), 7.91 (1H, d, J = 7.8 Hz), 8.09 and 8.10 (total 1H, each s, amide isomers), 8.34 (1H, d, J = 15.9 Hz), 8.41-8.50 (2H, m).
MS (ESI) m/z : 707 ($M^+$+1), 709 ($M^+$+3).
IR (ATR) $cm^{-1}$ : 1718, 1647, 1570, 1423, 1362, 1296.
Anal. Calcd for $C_{32}H_{30}Cl_2F_2N_4O_4S_2 \cdot 0.5H_2O$: C, 53. 63; H, 4.36; Cl, 9.89; F, 5.30; N, 7.82; S, 8.77.
Found: C, 53.31; H, 4.27; Cl, 9.65; F, 5.14; N, 7.57; S, 8.77.

Example 77

3-[2-[1-[4-(3- Benzo [b] furanylcarbonylamino)- 5- chloro- 2- fluorophenylacetyl]-(4S)-[1-(4,4- difluoro) piperidinyl]-(2S)-pyrrolidinyl]-5-thiazolyl]propionic acid methyl ester
[Chem. 224]

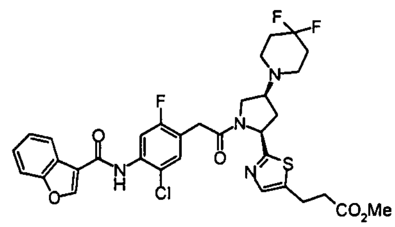

$^1$H-NMR ($CDCl_3$) δ: 1.91-2.33 (5H, m), 2.48-2.71 (6H, m), 2.78-3.05 (2H, m), 3.06-3.66 (4H, m), 3.66-3.71 (3H, m), 3.95-4.43 (2H, m), 5.30-5.37 (1H, m), 7.35-7.48 (4H, m), 7.58-7.64 (1H, m), 8.01-8.08 (1H, m), 8.27 (1H, d, J = 13.7 Hz), 8.30 (1H, d, J = 6.4 Hz), 8.39 and 8.47 (total 1H, each d, J = 11.5 and 11.8 Hz respectively, amide isomers).
MS (ESI) m/z : 688 ($M^+$+1), 690 ($M^+$+3).
3-[2-[1-[4-(3- Benzo [b] furanylcarbonylamino)- 5- chloro- 2- fluorophenylacetyl]-(4S)-[1-(4,4- difluoro) piperidinyl]-(2S)-pyrrolidinyl]-5-thiazolyl]propionic acid
[Chem. 225]

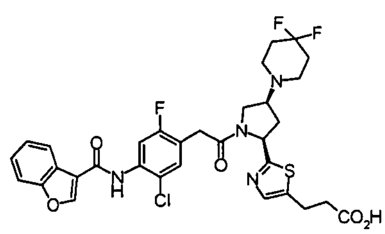

$^1$H-NMR ($CDCl_3$) δ: 1.93-2.30 (5H, m), 2.55-2.76 (7H, m), 2.83-3.19 (3H, m), 3.25-3.47 (1H, m), 3.52-3.75 (2H,

m), 3.90-4.53 (1H, m), 5.32-5.42 (1H, m), 7.24 (1H, s), 7.37-7.45 (4H, m), 7.55-7.62 (1H, m), 7.99-8.07 (1H, m), 8.47-8.19 (3H, m) .
MS (ESI) m/z : 675 ($M^+$+1), 677 ($M^+$+3).
IR (ATR) $cm^{-1}$ : 1720, 1672, 1645, 1587, 1560, 1522, 1448.
Anal. Calcd for $C_{32}H_{30}ClF_3N_4O_5S \cdot 0.5H_2O$: C, 56.18; H, 4.57; Cl, 5.18; F, 8.33; N, 8.19; S, 4.69.
Found: C, 55.90; H, 4.47; Cl, 5.07; F, 8.02; N, 7.91; S, 4.62.

Example 78

3-[2-[1-[4-(3- Benzo [b] furanylcarbonylamino)- 2,5- dichlorophenylacetyl]-(4S)-[1-(4,4- difluoro) piperidinyl]-(2S)-pyrrolidinyl]-5-thiazolyl]propionic acid methyl ester
[Chem. 226]

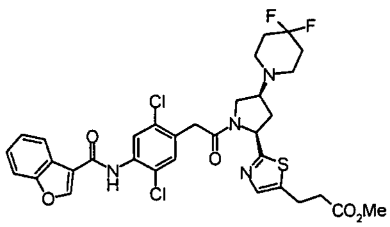

$^1$H-NMR ($CDCl_3$) δ: 1.92-2.34 (5H, m), 2.49-2.72 (5H, m), 2.80-3.58 (5H, m), 3.65-3.85 (4H, m), 3.97-4.44 (3H, m), 5.33-5.39 (1H, m), 7.28 (1H, s), 7.35-7.47 (4H, m), 7.57-7.64 (1H, m), 8.01-8.08 (1H, m), 8.21 (1H, d, J = 11.5 Hz), 8.70 and 8.73 (total 1H, each s, amide isomers).
MS (ESI) m/z : 705 ($M^+$+1), 707 ($M^+$+3).

3-[2-[1-[4-(3- Benzo [b] furanylcarbonylamino)- 2,5- dichlorophenylacetyl]-(4S)-[1-(4,4- difluoro) piperidinyl]-(2S)-pyrrolidinyl]-5-thiazolyl]propionic acid
[Chem. 227]

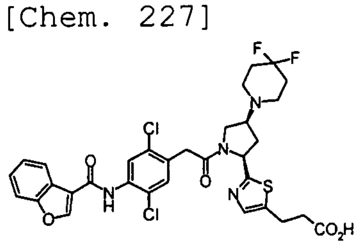

$^1$H-NMR ($CDCl_3$) δ: 1.94-2.34 (6H, m), 2.53-2.77 (6H, m), 2.85-2.97 (1H, m), 3.11 (2H, q, J = 7.7 Hz), 3.33-3.87 (3H, m), 3.93-4.56 (1H, m), 5.39 (1H, dd, J = 17. 4, 9.1 Hz), 7.28 (1H, s), 7.37-7.48 (4H, m), 7.57-7.63 (1H, m), 8.00-8.07 (1H, m), 8.18 and 8.23 (total 1H, each s, amide isomers), 8.286 and 8.294 (total 1H, each s, amide isomers), 8.59 and 8.69 (total 1H, each s, amide isomers).
MS (ESI) m/z : 657 ($M^+$+1), 659 ($M^+$+3).
IR (ATR) $cm^{-1}$ : 2939, 2833, 1718, 1643, 1572, 1508, 1427.
Anal. Calcd for $C_{32}H_{30}Cl_2F_2N_4O_5S \cdot 1.5H_2O$: C, 53.49; H, 4. 63; Cl, 9.87; F, 5.29; N, 7.80; S, 4.46.
Found: C, 53.06; H, 4.20; Cl, 9.59; F, 5.13; N, 7.56; S, 4.42.

Example 79

3-[2-[1-tert-butoxycarbonyl-(4S)-[1-(4-(2,2,2-trifluoro)ethyl)piperazinyl]-(2S)-pyrrolidinyl]-5-thiazolyl]propionic acid methyl ester
[Chem. 228]

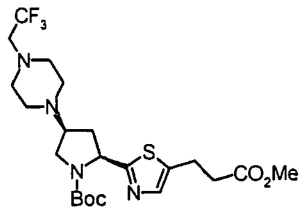

Sodium triacetoxyborohydride (3.59g, 16.93mmol) was added to a solution (50ml) of 3-[2-[1-tert-butoxycarbonyl-4-oxo-(2S)-pyrrolidinyl]-5-thiazolyl]propionic acid methyl ester (2.0g, 5.64mmol) and 4-(2,2,2-trifluoro)ethyl)piperazine di- trifluoroacetate (2.39g, 8.46mmol) in 1,2-dichloroethane, and the mixture was stirred for 32 hours. To the reaction solution was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size:40M, eluting solvent:ethyl acetate/methanol = 0% - 5%) to obtain the title compound (2.55g, 89%) as a yellow oily substance.

$^1$H-NMR ($CDCl_3$) δ: 1.27 (6H, s), 1.43 (9H, s), 1.54 (OH, s), 1.90-2.10 (1H, m), 2.43-2.89 (8H, m), 2.97 (2H, q, J = 9. 5 Hz), 3.12 (2H, t, J = 7.1Hz), 3.27 (1H, t, J = 10.0 Hz), 3. 69 (3H, s), 7.29 and 7.33 (total 1H, each s, amide isomers). MS (ESI) m/z : 507 ($M^+$+1).

3-[2-[(4S)-[1-(4-(2,2,2-trifluoro)ethyl)piperazinyl]-(2S)-pyrrolidinyl]-5-thiazolyl]propionic acid methyl ester dihydrochloride

[Chem. 229]

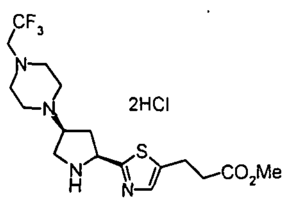

A 4N hydrochloric acid/dioxane solution (50ml) was added to 3-[2-[1-tert-butoxycarbonyl-4S]-[1-(4-(2,2,2-trifluoro)ethyl)piperazinyl]-(2S)-pyrrolidinyl]-5-thiazolyl]propionic acid methyl ester (2.55g, 5.03mmol), and the mixture was stirred for 13 hours. The reaction solution was concentrated under reduced pressure. The resulting residue was diluted with chloroform, ether was further added, and a precipitated solid was collected by filtration. This was dried under reduced pressure to obtain the title compound (3.02g, over yield) as a colorless solid. The present compound was used in the next reaction without further purification.
MS (ESI) m/z: 407 ($M^+$+1).

3-[2-[1-[5-Chloro-2-fluoro-4-(1-methyl-3-indolylcarbonylamino)phenylacetyl]-(4S)-[1-(4-(2,2,2-trifluoro)ethyl) piperazinyl]-(2S)-pyrrolidinyl]-5-thiazolyl]propionic acid methyl ester

[Chem. 230]

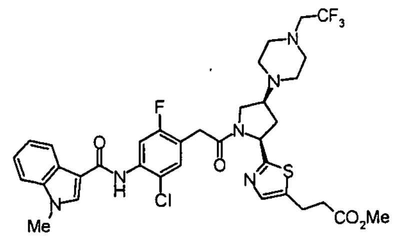

Triethylamine (0.52ml, 3.75mmol) was added to a solution (10ml) of 5-chloro-2-fluoro-4-(1-methyl-3-indolylcarbonylamino)phenylacetic acid (0.27g, 0.75mmol), 3-[2-[(4S)-[1-(4-(2,2,2-trifluoro)ethyl)piperazinyl]-(2S)-pyrrolidinyl]-5-thiazolyl]propionic acid methyl ester hydrochloride (0.39g, 0.75mmol), EDC · HCl (0.22g, 1.13mmol) and HOBt (0.15g, 1.13mmol) in methylene chloride at room temperature, and the mixture was stirred for 15 hours. To the reaction solution was added an aqueous saturated sodium bicarbonate solution, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size: 25M, eluting solvent: ethyl acetate/methanol = 0% - 10%) to obtain the title compound (340mg, 61%) as a brown oily substance.

$^1$H-NMR ($CDCl_3$) δ: 2.01-2.29 (1H, m), 2.42-3.51 (18H, m), 3.55-3.79 (4H, m), 3.82-4.01 (4H, m), 5.25-5.55 (1H, m), 7.23-7.48 (5H, m), 7.80 (1H, s), 7.81 (OH, s), 8.10-8.18 (1H, m), 8.27 and 8.31 (total 1H, each s, amide isomers), 8.45 and 8.54 (total 1H, each d, J = 11.7 and 12.2 Hz respectively, amide isomers).
MS (ESI) m/z : 749 ($M^+$+1), 751 ($M^+$+3).

3-[2-[1-[5-Chloro-2-fluoro-4-(1-methyl-3-indolylcarbonylamino)phenylacetyl]-(4S)-[1-(4-(2,2,2-trifluoro)ethyl)piperazinyl]-(2S)-pyrrolidinyl]-5-thiazolyl]propionic acid

[Chem. 231]

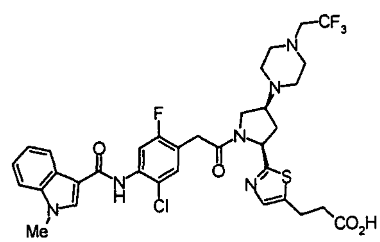

A 1N aqueous sodium hydroxide solution (2ml, 2mmol) was added to a solution of 3-[2-[1-[5-chloro-2-fluoro-4-(1-methyl-3-indolylcarbonylamino)phenylacetyl]-(4S)-[1-(4-(2,2,2-trifluoro)ethyl)piperazinyl]-(2S)-pyrrolidinyl]-5-thiazolyl]propionic acid methyl ester (0.34g, 0.45mmol) in a mixture of tetrahydrofuran/methanol (4ml/2ml) at room temperature, and the mixture was stirred for 3 days. The reaction solution was adjusted to a pH 7 with 1N hydrochloric acid, and concentrated under reduced pressure. The resulting residue was subjected to thin-layer chromatography [eluting solvent: chloroform/ethanol = 5/1 (v/v)], and recrystallized with ether/chloroform/n-hexane to obtain the title compound (218mg, 67%) as a colorless solid.

$^1$H-NMR ($CDCl_3$) δ: 2.11-2.29 (1H, m), 2.48-2.79 (10H, m), 2.81-3.19 (6H, m), 3.30-3.46 (1H, m), 3.50-3.77 (2H, m), 3.85 and 3.87 (total 3H, each s, amide isomers), 4.00 and 4.40 (total 1H, each dd, J = 9.9, 7.2 and 11.3, 6.9 Hz respectively, amide isomers), 5.26-5.60 (1H, m), 7.24-7.26 (1H, m), 7.28-7.45 (3H, m), 7.76 and 7.79 (total 1H, each s, amide isomers), 8.09-8.15 (1H, m), 8.24 and 8.25 (total 1H, each s, amide isomers), 8.36 (1H, d, J = 11.8 Hz), 8.48 (1H, s).
MS (ESI) m/z : 735 ($M^+$+1), 737 ($M^+$+3)
IR (ATR) $cm^{-1}$ : 3435, 3114, 2947, 2883, 2821, 1718, 1644.

Compounds of Example 80 to Example 84 shown below were produced by the same method as that of Example 79.

Example 80

3-[2-[1-[2,5-Dichloro-4-(1-methyl-3-indolylcarbonylamino)phenylacetyl]-(4S)-[1-(4-(2,2,2-trifluoro)ethyl)piperazinyl]-(2S)-pyrrolidinyl]-5-thiazolyl]propionic acid methyl ester

[Chem. 232]

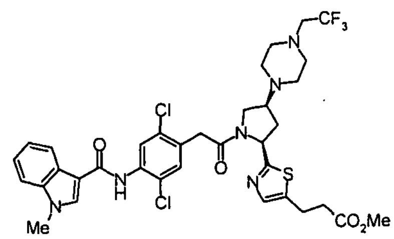

$^1$H-NMR ($CDCl_3$) δ: 2.06-2.27 (1H, m), 2.36-3.52 (16H, m), 3.62-4.02 (10H, m), 5.30-5.39 (1H, m), 7.24-7.46 (5H, m), 7.77-7.82 (1H, m), 8.09-8.17 (1H, m), 8.22 and 8.25 (total 1H, each s, amide isomers) 8.73 and 8.81 (total 1H, each s, amide isomers).

MS (ESI) m/z : 765 ($M^+$+1), 767 ($M^+$+3).

3-[2-[1-[2,5-Dichloro-4-(1-methyl-3-indolylcarbonylamino)phenylacetyl]-(4S)-[1-(4-(2,2,2-trifluoro)ethyl)piperazinyl]-(2S)-pyrrolidinyl]-5-thiazolyl]propionic acid

[Chem. 233]

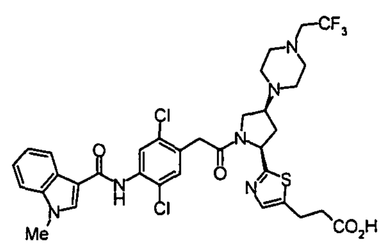

$^1$H-NMR ($CDCl_3$) δ: 1.81-1.90 (1H, m), 2.13-2.32 (1H, m), 2.44-3.17 (14H, m), 3.34-4.51 (8H, m), 5.25-5.59 (1H, m), 7.23-7.44 (5H, m), 7.74 and 7.78 (total 1H, each s, amide isomers), 8.08-8.14 (1H, m), 8.16 and 8.18 (total 1H, each s, amide isomers), 8.63 and 8.67 (total 1H, each s, amide isomers).

MS (ESI) m/z : 751 ($M^+$+1), 753 ($M^+$+3).

IR (ATR) $cm^{-1}$ : 2951, 2823, 1720, 1653, 1568, 1533.

Anal. Calcd for $C_{34}H_{35}Cl_2F_3N_5O_5S \cdot 0.75H_2O$: C, 53.37; H, 4.81; Cl, 9.27; F, 7.45; N, 10.98; S, 4.19.

Found: C, 53.66; H, 4.71; Cl, 9.30; F, 7.04; N, 10.62; S, 4.17.

Example 81

3-[2-[1-[4-(3- Benzo [b] thiophenylcarbonylamino)- 5- chloro- 2- fluorophenylacetyl]-(4S)-[1-(4-(2,2,2- trifluoro) ethyl)piperazinyl]-(2S)-pyrrolidinyl]-5-thiazolyl]propionic acid methyl ester

[Chem. 234]

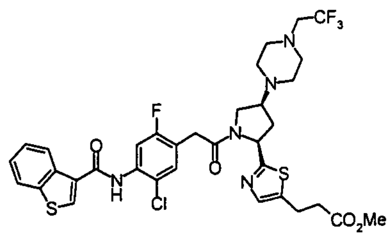

$^1$H-NMR ($CDCl_3$) δ: 1.95-2.31 (2H, m), 2.42-3.20 (14H, m), 3.19-3.65 (3H, m), 3.68 (3H, s), 3.76-4.44 (2H, m), 5.33 (1H, t, J = 8.8 Hz), 7.22-7.56 (4H, m), 7.91 and 7.92 (total 1H, each d, J = 7.8 and 8.1 Hz respectively, amide isomers), 8.08 and 8.10 (total 1H, each s, amide isomers), 8.29-8.53 (3H, m).
MS (ESI) m/z : 752 ($M^+$+1), 754 ($M^+$+3).

3-[2-[1-[4-(3- Benzo [b] thiophenylcarbonylamino)- 5- chloro- 2- fluorophenylacetyl]-(4S)-[1-(4-(2,2,2- trifluoro) ethyl)piperazinyl]-(2S)-pyrrolidinyl]-5-thiazolyl]propionic acid

[Chem. 235]

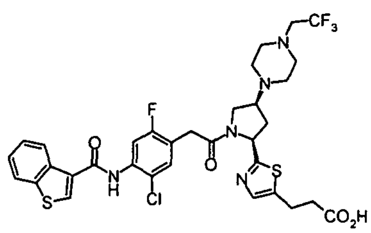

$^1$H-NMR ($CDCl_3$) δ: 2.15-2.28 (1H, m), 2.50-3.21 (13H, m), 3.28-3.48 (1H, m), 3.54-3.77 (2H, m), 4.00 (1H, dd, J = 9.8, 6.9 Hz), 4.41 (0H, dd, J = 11.0, 6.9 Hz), 5.33-5.40 (1H, m), 7.24 and 7.25 (total 1H, each s, amide isomers), 7.34-7.56 (4H, m), 7.85-7.93 (1H, m), 8.08 (1H, d, J = 4.9 Hz), 8.28-8.62 (3H, m), 8.29-8.32 (3H, m).
MS (ESI) m/z : 738 ($M^+$+1), 740 ($M^+$+3). IR (ATR) $cm^{-1}$ : 3413, 2956, 2848, 2823, 1718, 1653, 1585, 1518.

Example 82

3-[2-[1-[4-(3- Benzo [b] thiophenylcarbonylamino)- 2,5- dichlorophenylacetyl]-(4S)-[1-(4-(2,2,2- trifluoro) ethyl) piperazinyl]-(2S)-pyrrolidinyl]-5-thiazolyl]propionic acid methyl ester

[Chem. 236]

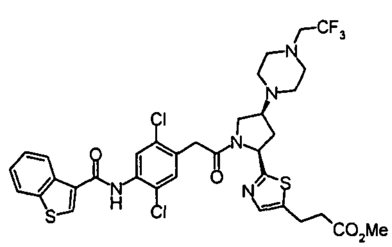

$^1$H-NMR ($CDCl_3$) δ: 2.10-2.56 (2H, m), 2.66 (2H, td, J = 7.5, 4.8 Hz), 2.82-2.90 (2H, m), 3.05-3.17 (3H, m), 3.22 (3H, s), 3.24 (0H, s), 3.35-3.65 (3H, m), 3.66-3.83 (5H, m), 3.85-3.99 (2H, m), 5.36 and 5. 39 (total 1H, each dd, J = 8.8, 2.9 and 8.6, 4.4 Hz respectively, amide isomers), 7.32 and 7.35 (total 1H, each s, amide isomers), 7.40-7.55 (3H, m), 7.91 (1H, dd, J = 7.8, 3.4 Hz), 8.09 (1H, d, J = 8.3 Hz), 8.26 and 8.30 (total 1H, each s, amide isomers), 8.48 (1H,

dd, J = 8.1, 4.9 Hz), 8.65 and 8.73 (total 1H, each s, amide isomers).
MS (ESI) m/z : 768 ($M^+$+1), 770 ($M^+$+3).

3-[2-[1-[4-(3-Benzo[b]thiophenylcarbonylamino)-2,5-dichlorophenylacetyl]-(4S)-[1-(4-(2,2,2-trifluoro)ethyl) piperazinyl[2S]-pyrrolidinyl]-5-thiazolyl]propionic acid

[Chem. 237]

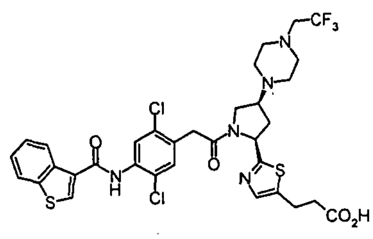

$^1$H-NMR ($CDCl_3$) δ: 1.78-2.28 (1H, m), 2.40-3.20 (13H, m), 3.29-3.52 (1H, m), 3.49-4.44 (6H, m), 5.34 (1H, q, J = 7.9 Hz), 7.25 (1H, s), 7.32-7.55 (4H, m), 7.90 and 7.91 (total 1H, each d, J = 7.8 and 8.3 Hz respectively, amide isomers), 8.07 and 8.09 (total 1H, each s, amide isomers), 8.27 and 8.30 (total 1H, each s, amide isomers), 8.47 and 8.48 (total 1H, each d, J = 7.6 and 7.8 Hz respectively, amide isomers), 8.64 and 8.71 (total 1H, each s, amide isomers).
MS (ESI) m/z : 754 ($M^+$+1), 756 ($M^+$+3).
IR (ATR) $cm^{-1}$ : 2951, 2819, 1718, 1649, 1570, 1502, 1456.
Anal. Calcd for $C_{33}H_{32}Cl_2F_3N_5O_4S \cdot 0.5H_2O$: C, 51.90; H, 4.36; Cl, 9.28; F, 7.46; N, 9.17; S, 4.36.
Found: C, 51.87; H, 4.71; Cl, 9.30; F, 7.04; N, 10.62; S, 4.17.

Example 83

3-[2-[1-[4-(3-Benzo[b]furanylcarbonylamino)-5-chloro-2-fluorophenylacetyl-(4S)-[1-(4-(2,2,2-trifluoro)ethyl) piperazinyl]-(2S)-pyrrolidinyl]-5-thiazolyl]propionic acid methyl ester

[Chem. 238]

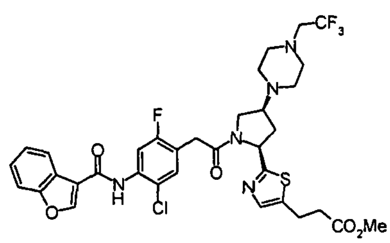

$^1$H-NMR ($CDCl_3$) δ: 1.98-2.29 (2H, m), 2.43-3.18 (13H, m), 3.22-3.40 (1H, m), 3.40-3.71 (6H, m), 3.97 and 4.35 (total 1H, each dd, J = 10.0, 6.6 and 10.7, 6.3 Hz respectively, amide isomers), 5.29-5.36 (1H, m), 7.28 (1H, s), 7.34-7.49 (4H, m), 7.58-7.64 (1H, m), 8.01-8.08 (1H, m), 8.22-8.32 (2H, m), 8.39 and 8.47 (total 1H, each d, each J = 11.8 Hz, amide isomers).
MS (ESI) m/z : 736 ($M^+$+1), 738 ($M^+$+3).

3-[2-[1-[4-(3-Benzo[b]furanylcarbonylamino)-5-chloro-2-fluorophenylacetyl-(4S)-[1-(4-(2,2,2-trifluoro)ethyl) piperazinyl]-(2S)-pyrrolidinyl]-5-thiazolyl]propionic acid

[Chem. 239]

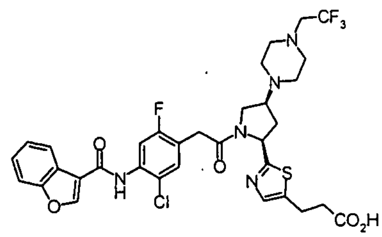

$^1$H-NMR ($CDCl_3$) δ: 2.08-2.31 (1H, m), 2.48-2.79 (11H, m), 2.82-3.18 (5H, m), 3.29-3.77 (3H, m), 4.01 and 4.41 (total 1H, each dd, J = 10.0, 6.8 and 12.1, 6.7 Hz respectively, amide isomers) 5.33-5.41 (1H, m), 7.24-7.49 (4H, m), 7. 52-7. 64 (1H, m), 8.01-8.10 (1H, m), 8.25 (1H, d, J = 13.9 Hz), 8.29 and 8.31 (total 1H, each s, amide isomers), 8.33 and 8.44 (total 1H, each d, J = 10.3 and 11.5 Hz respectively, amide isomers).
MS (ESI) m/z : 722 ($M^+$+1), 724 ($M^+$+3).
IR (ATR) $cm^{-1}$ : 3415, 2956, 2825, 1714, 1649, 1587, 1558, 1522.

Example 84

3-[2-[1-[4-(3-Benzo[b]furanylcarbonylamino)-2,5-dichlorophenylacetyl-(4S)-[1-(4-(2,2,2-trifluoro)ethyl)piperazinyl]-(2S)-pyrrolidinyl]-5-thiazolyl]propionic acid methyl ester
[Chem. 240]

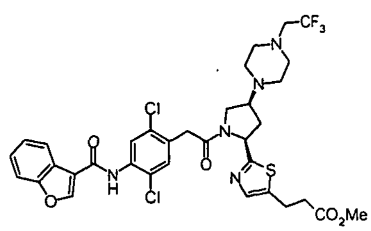

$^1$H-NMR ($CDCl_3$) δ: 1.65-2.29 (2H, m), 2.44-3.20 (14H, m), 3.30-3.53 (2H, m), 3.64-4.20 (6H, m), 4.37 (1H, dd, J = 10.8, 6.4 Hz), 5.35 (1H, dd, J = 15.2, 7.4 Hz), 7.36 and 7.38 (total 1H, each s, amide isomers), 7.39-7.48 (2H, m), 7.57-7.64 (1H, m), 8.01-8.09 (1H, m), 8.21 (1H, d, J = 12.5 Hz), 8.29 (1H, d, J = 5.9 Hz), 8.65 and 8.72 (total 1H, each d, each J = 1.7 Hz, amide isomers).
MS (ESI) m/z : 752 ($M^+$+1), 754 ($M^+$+3).
3-[2-[1-[4-(3-Benzo[b]furanylcarbonylamino)-2,5-dichlorophenylacetyl-(4S)-[1-(4-(2,2,2-trifluoro)-ethyl)piperazinyl]-(2S)-pyrrolidinyl]-5-thiazolyl]propionic acid
[Chem. 241]

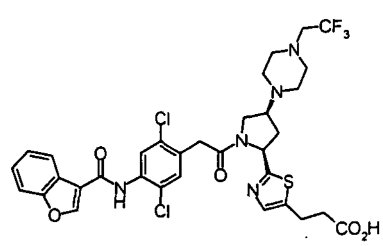

$^1H$-NMR ($CDCl_3$) δ: 2.14-2.31 (1H, m), 2.52-3.16 (15H, m), 3.33-4.10 (4H, m), 4.43 (1H, dd, J = 11.1, 6.5 Hz), 5.38 (1H, dd, J=17.2, 8.2 Hz), 7.34-7.46 (4H, m), 7.51-7.63 (1H, m), 8.00-8.09 (1H, m), 8.19 (1H, d, J = 16.4 Hz), 8.28 and 8.30 (total 1H, each s, amide isomers), 8.58 and 8.68 (total 1H, each s, amide isomers).
MS (ESI) m/z : 738 ($M^+$+1), 740 ($M^+$+3).
IR (ATR) $cm^{-1}$ : 2952, 2823, 1718, 1644, 1570, 1506, 1450.
Anal. Calcd for $C_{33}H_{32}Cl_2F_3N_5O_5S \cdot H_2O$: C, 52.39; H, 4.53; Cl, 9.37; F, 7.53; N, 9.26; S, 4.24.
Found: C, 52.57; H, 4.30; Cl, 9.13; F, 7.26; N, 8.86; S, 4.20.

Example 85

3-[2-[1-tert-butoxycarbonyl-(4S)-[4-(cis-2,6-dimethyl)morpholinyl]-(2S)-pyrrolidinyl]-5-thiazolyl]propionic acid methyl ester

[Chem. 242]

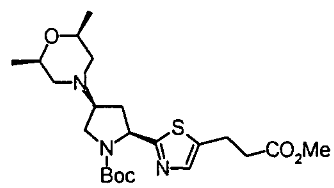

Sodium triacetoxyborohydride (5.38g, 25.39mmol) was added to a solution (50ml of 3-[2-[1-tert-butoxycarbonyl-4-oxo-(2S)-pyrrolidinyl]-5-thiazolyl]propionic acid methyl ester (3.0g, 8.46mmol) and cis-2,6-dimethylmorpholine (Tokyo Chemical Industry Co., Ltd.: catalogue No;D0746) (2.10ml, 16.93mmol) in 1,2-dichloroethane, and the mixture was stirred for 22 hours. To the reaction solution was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size:40M, eluting solvent: ethyl acetate/methanol = 0% - 5%) to obtain the title compound (3.53g, 92%) as a yellow oily substance. $^1H$-NMR ($CDCl_3$) δ: 1.08-1.33 (14H, m), 1.36-1.58 (2H, m), 1.68-1.85 (2H, m), 1.94-2.18 (1H, m), 2.55-2.80 (5H, m), 3.12 (2H, t, J = 5.9 Hz), 3.23-3.34 (1H, m), 3.53-3.67 (2H, m), 3.69 (3H, s), 3.81-4.18 (1H, m), 4.97-5.16 (1H, m), 7.34 (1H, s).
MS (ESI) m/z : 454 ($M^+$+1) .

3-[2-[(4S)-[4-(cis-2,6-dimethyl)morpholinyl]-(2S)-pyrrolidinyl]-5-thiazolyl]propionic acid methyl ester dihydrochloride

[Chem. 243]

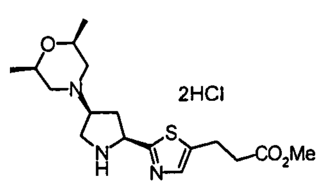

A 4N hydrochloric acid/dioxane solution (50m1) was added to 2-[2-[1-tert-butoxycarbonyl-(4S)-[4-(cis-2,6-dimethyl)morpholinyl]-(2S)-pyrrolidinyl]-5-thiazolyl]propionic acid methyl ester (3.53g, 7.78mmol), and the mixture was stirred for 17 hours. The reaction solution was concentrated under reduced pressure. The resulting residue was diluted with chloroform, ether was further added, and a precipitated solid was collected by filtration. This was dried under reduced pressure to obtain the title compound (3.38g, over yield) as a pale yellow solid. The present compound was used in the next reaction without further purification.
MS (ESI) m/z : 356 ($M^+$+1).

3-[2-[1-[5-Chloro-2-fluoro-4-(1-methyl-3-indolylcarbonylamino)phenylacetyl]-(4S)-[4-(cis-2,6-dimethyl)morpholinyl]-(2S)-pyrrolidinyl]-5-thiazolyl]propionic acid methyl ester

[Chem. 244]

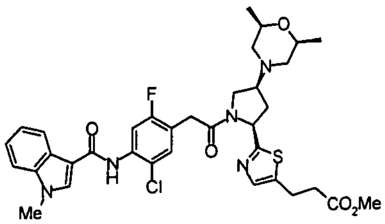

Triethylamine (0.52ml, 3.75mmol) was added to a solution (10ml) of 5-chloro-2-fluoro-4-(1-methyl-3-indolylcarbonylamino)phenylacetic acid (0.27g, 0.75mmol), 3-[2-[(4S)-[4-(cis-2,6-dimethyl)morpholinyl]-(2S)-pyrrolidinyl]-5-thiazolyl]propionic acid methyl ester dihydrochloride (0.29g, 0.75mmol), EDC · HCl (0.22g, 1.13mmol) and HOBt (0.15g, 1.13mmol) in methylene chloride at room temperature, and the mixture was stirred for 3 days. The reaction solution was concentrated under reduced pressure, and the resulting residue was purified by flash chromatography (Biotage flash chromatography system, column size: 25M, eluting solvent: ethyl acetate/methanol = 0% - 10%) to obtain the title compound (410mg, 79%) as a colorless oily substance.

$^1$H-NMR ($CDCl_3$) δ: 1.13 (3H, d, J = 10.0 Hz), 1.15 and 1.17 (total 3H, each d, J = 6.4 and 6. 6 Hz respectively, amide isomers), 1.70-1.90 (2H, m), 2.11-2.30 (1H, m), 2.56-2.92 (6H, m), 3.06-3.72 (9H, m), 3.89 and 3.89 (total 3H, each s, amide isomers), 3.97 and 4.32 (total 1H, each dd, J=10.0, 6.6 and 11.8, 6.6 Hz respectively, amide isomers), 5.29-5. 36 (1H, m), 7.25 (1H, s), 7.32-7.46 (5H, m), 7.80 and 7.81 (total 1H, each s, amide isomers), 8.10-8.17 (1H, m), 8.28 and 8.31 (total 1H, each s, amide isomers), 8.45 and 8.54 (total 1H, each d, J = 11.8 and 12.3 Hz respectively, amide isomers). MS (ESI) m/z : 696 ($M^+$+1), 698 ($M^+$+3).

3-[2-[1-[5-Chloro-2-fluoro-4-(1-methyl-3-indolylcarbonylamino)phenylacetyl]-(4S)-[9-(cis-2,6-dimethyl)morpholinyl]-(2S)-pyrrolidinyl]-5-thiazolyl]propionic acid

[Chem. 245]

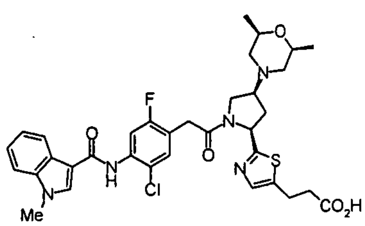

A 1N aqueous sodium hydroxide solution (2ml, 2mmol) was added to a solution of 3-[2-[1-[5-chloro-2-fluoro-4-(1-methyl-3-indolylcarbonyl amino)phenylacetyl]-(4S)-[4-(cis-2,6-dimethyl)morpholiny 1]-(2S)-pyrrolidinyl]-5-thiazolyl] propionic acid methyl ester (0.41g, 0.59mmol) in a mixture of tetrahydrofuran/methanol (4mm/2ml) at room temperature, and the mixture was stirred for 3 days. The reaction solution was adjusted to a pH 7 with 1N hydrochloric acid, and concentrated under reduced pressure. The resulting residue was subjected to thin-layer chromatography [chloroform/ethanol = 5/1 (v/v)], and recrystallized with ether/chloroform/n-hexane to obtain the title compound (245mg, 61%) as a colorless solid.

$^1$H-NMR ($CDCl_3$) δ: 1.14 (3H, d, J = 6.1 Hz), 1.16 and 1. 19 (total 3H, each d, J = 5.1 and 6. 3 Hz respectively, amide isomers), 1.70-2.28 (3H, m), 2.57-2.99 (6H, m), 3.02-3.19 (2H, m), 3.32-3.77 (7H, m), 3.86 and 3.88 (total 3H, each s, amide isomers), 3.97-4.44 (1H, m), 5.37 (1H, t, J = 8.3 Hz), 7.24-7.43 (4H, m), 7.78 and 7.79 (total 1H, each s, amide isomers), 8.08-8.15 (1H, m), 8.25 and 8.33 (total 1H, each d, J = 11.5 and 11.7 Hz respectively, amide isomers), 8.49 (1H, d, J = 12.0 Hz).

MS (ESI) m/z : 682 ($M^+$+1), 684 ($M^+$+3).

IR (ATR) $cm^{-1}$ : 2927, 1674, 1633, 1568, 1508, 1448, 1412.

Anal. Calcd for $C_{34}H_{37}ClFN_5 6_5S \cdot 1.5H_2O$: C, 57.58; H, 5.68; N, 9.87; S, 4.52.
Found: C, 57.17; H, 5.19; N, 10.62; S, 4.51.

Compounds of Example 86 to Example 90 shown below were produced by the same method as that of Example 85.

Example 86

3-[2-[1-[2,5-Dichloro-4-(1-methyl-3-indolylcarbonylamino)phenylacetyl]-(4S)-[4-(cis-2,6-dimethyl)morpholinyl]-(2S)-pyrrolidinyl]-5-thiazolyl]propionic acid methyl ester

[Chem. 246]

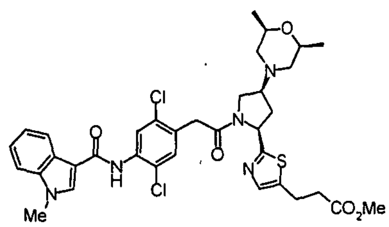

$^1$H-NMR ($CDCl_3$) δ: 1.13 (3H, d, J = 6.4 Hz), 1.16 and 1.17 (total 3H, each d, J = 6.4 and 6.1 Hz respectively, amide isomers), 1.69-1.89 (2H, m), 2.00-2.33 (1H, m), 2.56-2.91 (6H, m), 3.07-3.16 (2H, m), 3.28-3.84 (7H, m), 3.88 and 3.89 (total 3H, each s, amide isomers), 3.97 (1H, dd, J = 10.3, 6.1 Hz, 4.35 (1H, dd, J = 11.4, 6.5 Hz), 5.31-5.39 (1H, m), 7.32-7.47 (5H, m), 7.79 and 7.80 (total 1H, each s, amide isomers), 8.10-8.16 (1H, m), 8.22 and 8.25 (total 1H, each s, amide isomers), 8.74 and 8.81 (total 1H, each s, amide isomers).
MS (ESI) m/z : 712 ($M^+$+1), 714 ($M^+$+3).

3-[2-[1-[2,5-Dichloro-4-(1-methyl-3-indolylcarbonylamino)phenylacetyl]-(4S)-[4-(cis-2,6-dimethyl)morpholinyl]-(2S)-pyrrolidinyl]-5-thiazolyl]propionic acid

[Chem. 247]

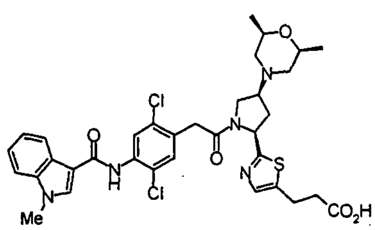

$^1$H-NMR ($CDCl_3$) δ: 1.13-1.21 (6H, m), 1.74-2.36 (3H, m), 2.52-3.18 (9H, m), 3.30-4.51 (10H,m), 5.39 (1H, dd, J =18.5, 8.2 Hz), 7.21-7.46 (5H, m), 7.74 and 7.76 (total 1H, each s, amide isomers), 8.07-8.22 (2H, m), 8.64 and 8.77 (total 1H, each s, amide isomer).
MS (ESI) m/z : 698 ($M^+$+1), 700 ($M^+$+3).
IR (ATR) $cm^{-1}$ : 2970, 2852, 1720, 1655, 1568, 1533.

Example 87

3-[2-[1-[4-(3-Benzo [b] thiophenylcarbonylamino)-5-chloro-2-fluorophenylacetyl]-(4S)-[4-(cis-2,6-dimethyl) morpholinyl]-(2S)-pyrrolidinyl]-5-thiazolyl]propionic acid methyl ester

[Chem. 248]

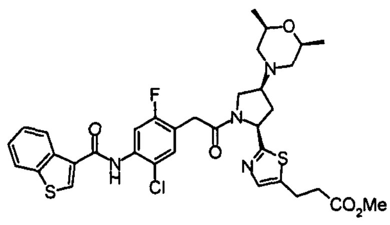

$^1$H-NMR ($CDCl_3$) δ: 1.13 (3H, d, J = 6.1 Hz), 1.15 and 1.18 (total 3H, each d, J = 6.6 and 6.6 Hz respectively, amide isomers), 1.69-1.91 (2H, m), 2.07-2.31 (1H, m), 2.55-2.92 (5H, m), 3.10 and 3.14 (total 2H, each t, J = 7.6 and 7.1 Hz respectively, amide isomers), 3.22-3.67 (5H, m), 3.68 (3H,s), 3.97 and 4.32 (total 1H, each dd, J = 9.6, 6.4 and 11. 5, 7.1 Hz respectively, amide isomers), 5.32 and 5.33 (total 1H, each t, J = 7.8 and 8.3 Hz respectively, amide isomers), 7.25 and 7.27 (total 1H, each s, amide isomers), 7.34-7.55 (4H, m), 7.91 and 7.92 (total 1H, each d, J = 8.0 and 7.8 Hz respectively, amide isomers), 8.08 and 8.10 (total 1H, each s, amide isomers), 8.33 and 8.38 (total 1H, each d, J = 14.2 and 11.5 Hz respectively, amide isomers), 8.44-8.49 (2H, m).
MS (ESI) m/z : 699 ($M^++1$), 701 ($M^++3$).

3-[2-[1-[4-(3- Benzo [b] thiophenylcarbonylamino)- 5- chloro- 2- fluorophenylacetyl]-(4S)-[4-(cis- 2,6- dimethyl) morpholinyl]-(2S)-pyrrolidinyl]-5-thiazolyl]propionic acid

[Chem. 249]

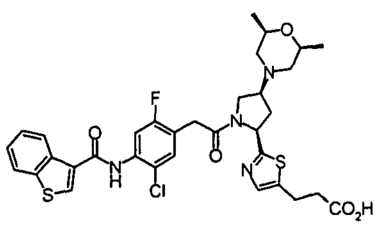

$^1$H-NMR ($CDCl_3$) δ: 1.15 (3H, d, J = 5.4 Hz), 1. 16 and 1.19 (total 3H, each d, J = 5. 4 and 6.3 Hz respectively, amide isomers), 1.77-1.98 (2H, m), 2.17-2.32 (1H, m), 2.58-3.19 (8H, m), 3.24-3.81 (6H, m), 4.02 and 4.42 (total 1H, t and dd, J = 8.2 and 8.9, 4.5 Hz respectively, amide isomers), 5.32-5.40 (1H, m), 7.22-7.30 (1H, m), 7.32-7.56 (3H, m), 7.90 (1H, dd, J = 7.8, 2.9 Hz), 8.08 (1H, s), 8.28-8.37 (1H, m), 8.44 and 8.46 (total 2H, each d, J= 9. 3 and 6. 3 Hz respectively, amide isomers).
MS (ESI) m/z : 685 ($M^++1$), 687 ($M^++3$).
IR (ATR) $cm^{-1}$ : 2972, 2860, 1720, 1644, 1585, 1518, 1402.
Anal. Calcd for $C_{33}H_{34}ClFN_46_5S_2 \cdot H_2O$: C, 56.36; H, 5.16; Cl, 5.04; F, 2.70; N, 7.97; S, 9.12.
Found: C, 56.71; H, 5.01; Cl, 4.79, F; 2.66, N, 7.62; S, 8.96.

Example 88

3-[2-[1-[4-(3- Benzo [b] thiophenylcarbonylamino)- 2,5- dichlorophenylacetyl]-(4S)-[4-(cis- 2,6- dimethyl) morpholinyl]-(2S)-pyrrolidinyl]-5-thiazolyl]propionic acid methyl ester

[Chem. 250]

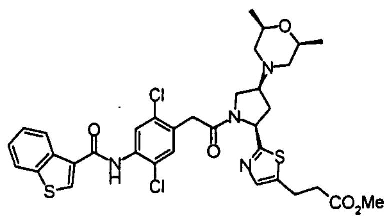

$^1$H-NMR ($CDCl_3$) δ: 1.14 (3H, d, J = 6.9 Hz), 1.16 and 1.17 (total 3H, each d, J = 5. 9 and 6.1 Hz respectively, amide isomers), 1.70-1.92 (2H, m), 2.06-2.44 (1H, m), 2.57-2.93 (7H, m), 3.07-3.16 (2H, m), 3.22-3.88 (7H, m), 3. 99 and 4.34 (total 1H, each dd, J = 10.0, 7.1 and 11.5, 6.4 Hz respectively, amide isomers), 5.34 and 5.36 (total 1H, each t, J = 8.3 and 5.9 Hz respectively, amide isomers), 7.36-7.39 (1H, m), 7.40-7.59 (3H, m), 7.89-7.94 (1H, m), 8.07 and 8.09 (total 1H, each s, amide isomers), 8.27 and 8.30 (total 1H, each s, amide isomers), 8.45-8.50 (1H, m), 8.65 and 8.72 (total 1H, each d, J = 2.0 and 2.2 Hz respectively, amide isomers). MS (ESI) m/z : 715 ($M^+$+1), 717 ($M^+$+3).

3-[2-[1-[4-(3- Benzo [b] thiophenylcarbonylamino)- 2,5- dichlorophenylacetyl]-(4S)-[4-(cis- 2,6- dimethyl) morpholinyl]-(2S)-pyrrolidinyl]-5-thiazolyl]propionic acid

[Chem. 251]

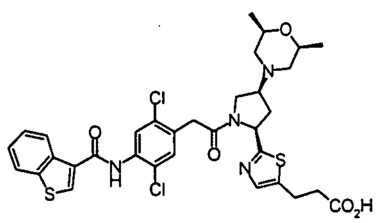

$^1$H-NMR ($CDCl_3$) δ: 1.13-1.21 (6H, m), 1.76-1.99 (2H, m), 2.17-2. 34 (1H, m), 2.54-3.20 (7H, m), 3.26-4.11 (6H, m), 4.44 (1H, dd, J = 11.4, 6.7 Hz), 5.34-5.44 (1H, m), 7.23 (1H, s), 7.34-7.53 (4H, m), 7.89 (1H, t, J = 7.1 Hz), 8.05-8.09 (1H, m), 8.23 and 8.29 (total 1H, each s, amide isomers), 8.46 (1H, d, J = 8.1 Hz), 8.59 and 8.69 (total 1H, each d, J = 1.0 and 0.7Hz respectively, amide isomers). MS (ESI) m/z : 701 ($M^+$+1), 703 ($M^+$+3).
IR (ATR) $cm^{-1}$ : 2970, 2858, 1720, 1647, 1570, 1504, 1425.
Anal. Calcd for $C_{33}H_{34}Cl_2N_4O_5S_2 \cdot H_2O$: C, 55.07; H, 5.04; Cl, 9, 85; N, 7.39; S, 8.91.
Found: C, 55.38; H, 4.85; Cl, 9.45; N, 7.39; S, 8.74.

Example 89

3-[2-[1-[4-(3- Benzo [b] furanylcarbonylamino)- 5- chloro- 2- fluorophenylacetyl-(4S)-[4-(cis- 2,6- dimethyl) morpholinyl]-(2S)-pyrrolidinyl]-5-thiazolyl]propionic acid methyl ester

[Chem. 252]

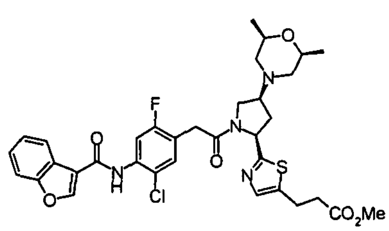

$^1$H-NMR ($CDCl_3$) δ: 1.13 (3H, d, J = 6.6 Hz), 1.15 and 1.18 (total 3H, each d, each J = 6.3 Hz, amide isomers), 1.68-1.92 (2H, m), 1.96-2.32 (2H, m), 2.56-2.94 (5H, m), 3.04-3.79 (9H, m), 3.98 and 4. 32 (total 1H, each dd, J=10.0, 6. 3 and 11.5, 6. 1 Hz respectively, amide isomers), 5. 37-5.28 (1H, m), 7.51-7.17 (4H, m), 7.63-7.57 (1H, m), 8.09-8.01 (1H, m), 8.50-8.23 (3H, m).
MS (ESI) m/z : 683 ($M^+$+1), 685 ($M^+$+3)

3-[2-[1-[4-(3- Benzo [b] furanylcarbonylamino)- 5- chloro- 2- fluorophenylacetyl-(4S)-[4-(cis- 2,6- dimethyl) morpholinyl]-(2S)-pyrrolidinyl]-5-thiazolyl]propionic acid

[Chem. 253]

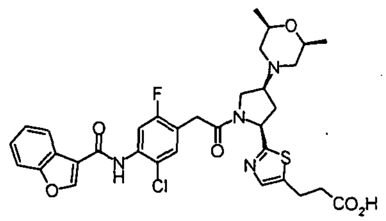

$^1$H-NMR ($CDCl_3$) δ: 1. 12-1.21 (6H, m), 1.74-2.31 (3H, m), 2.56-3.18 (7H, m), 3.24-3.81 (5H, m), 4.01 and 4.40 (total 1H, each dd, J = 9. 8, 6.9 and 11.2, 6. 7 Hz respectively, amide isomers), 5.35 (1H, q, J = 7.8 Hz), 6.98 (1H, s), 7.24 and 7.26 (total 2H, each s, amide isomers), 7.34-7.45 (3H, m), 7.55-7.62 (1H, m), 8.00-8.07 (1H, m), 8.22 and 8.27 (total 1H, each s, amide isomers), 8.29 (1H, d, J = 3.9 Hz), 8.32 and 8.42 (total 1H, each d, each J = 11.5 Hz, amide isomers). MS (ESI) m/z : 669 ($M^+$+1), 671 ($M^+$+3).
IR (ATR) $cm^{-1}$ : 2972, 2937, 2871, 1720, 1649, 1587, 1521, 1448. Anal. Calcd for $C_{33}H_{34}ClFN_4O_6S \cdot 0.75H_2O$: C, 58.06; H, 5.24; Cl, 5.19; F, 2.78; N, 8.21; S, 4.70.
Found: C, 57.94; H, 5.28; Cl, 5.59; F, 2.87; N, 7.97; S, 4.67.

Example 90

3-[2-[1-[4-(3- Benzo [b] furanylcarbonylamino)- 2,5- dichlorophenylacetyl-(4S)-[4-(cis- 2,6- dimethyl) morpholinyl]-(2S)-pyrrolidinyl]-5-thiazolyl]propionic acid methyl ester

[Chem. 254]

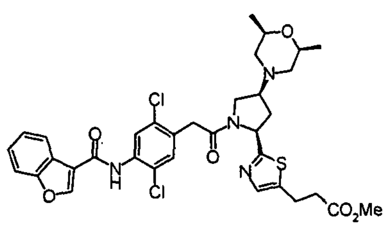

$^1$H-NMR ($CDCl_3$) δ: 1.11-1.19 (6H, m), 1.68-1.94 (2H, m), 2.06-2.32 (2H, m), 2.58-2.93 (5H, m), 3.06-3.17 (2H, m), 3.26-3.88 (6H, m), 3. 99 and 4.34 (total 1H, each dd, J = 9.3, 6.3 and 11.5, 6.6 Hz respectively, amide isomers), 5.30-5.40 (1H, m), 7.28 (1H, s), 7.34-7.48 (4H, m), 7.57-7.64 (1H, m), 8.01-8.08 (1H, m), 8.20 and 8.23 (total 1H, each s, amide isomers), 8.29 (1H, d, J=2.6 Hz), 8.30 (1H, d, J= 2.4 Hz), 8.65 and 8.73 (total 1H, each d, each J = 1. 5 Hz, amide isomers). MS (ESI) m/z : 699 ($M^+$+1), 701 ($M^+$+3).

3-[2-[1-[4-(3- Benzo [b] furanylcarbonylamino)- 2,5- dichlorophenylacetyl-(4S)-[4-(cis- 2,6- dimethyl) morpholinyl]-(2S)-pyrrolidinyl]-5-thiazolyl]propionic acid

[Chem. 255]

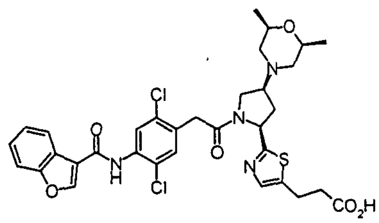

$^1$H-NMR ($CDCl_3$) δ: 1.12-1.21 (6H, m), 1.76-1.97 (2H, m), 2.19-2.32 (1H, m), 2.52-3.00 (5H, m), 3.04-3.16 (2H, m), 3.33-4.07 (5H, m), 4.02 and 4.43 (total 1H, each dd, J = 9.5, 6.6 and 10.5, 6.1 Hz respectively, amide isomers) 5.32-5.44 (1H, m), 7.24 (1H, s), 7.36-7.45 (4H, m), 7.54-7.62 (1H, m), 8.00-8.06 (1H, m), 8.16 and 8.22 (total 1H, each s, amide isomers), 8.276 and 8.284 (total 1H, each s, amide isomers), 8.58 and 8.66 (total 1H, each s, amide isomers).
MS (ESI) m/z : 685 ($M^+$+1), 687 ($M^+$+3).
IR (ATR) $cm^{-1}$ : 2970, 2856, 1720, 1647, 1572, 1508, 1448.

Example 91

(1-tert-butoxycarbonyl-(3S)-hydroxy-(2S)-pyrrolidinyl)carboxylic acid methyl ester
[Chem. 256]

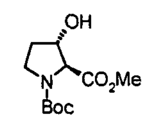

Thionyl chloride (9.20ml, 126mmol) was added dropwise to methanol (100ml) at -10˚C under stirring. After completion of addition, (3S)-hydroxy-L-proline (commercially available, Across) (5.51g, 42.0mmol) was added, and the mixture was stirred for 5 hours while a temperature was raised to room temperature. A solvent of the reaction mixture was removed under reduced pressure, and the resulting residue was recrystallized with a methanol-diethyl ether system to obtain (3S)-hydroxyl-L-proline methyl ester hydrochloride (7.66g). This hydrochloride was suspended in 1,4-dioxane (100ml), triethylamine (17.6ml, 126.3mmol) and di-tert-butyl bicarbonate (11.0g, 50.4mmol) were added, and the mixture was stirred at room temperature for 2 weeks. Precipitated triethylamine hydrochloride was removed by suction filtration, and the filtrate was concentrated. The resulting residue was diluted with ethyl acetate, washed with water and an aqueous saturated sodium chloride solution, and dried with anhydrous sodium sulfate, and a solvent was removed under reduced pressure. The resulting residue was purified by column chromatography (eluting solvent, n-hexane:ethyl acetate = 3: 1-1:1) using silica gel to obtain the title compound (9.00g, 87%) as a colorless oily substance.
$^1$H-NMR ($CDCl_3$) δ: 1.41 and 1.47 (total 9H, each s), 1.88-1.95 (1H, m), 2.06-2.16 (2H, m), 3.53-3.69 (2H, m), 3.75 (3H, s), 4.18 and 4.29 (total 1H, each s), 4.43-4.45 (1H, m).
(1-tert-butoxycarbonyl-(3S)-ethoxy-(2S)-pyrrolidinyl)carboxylic acid
[Chem. 257]

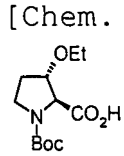

(1-tert-butoxycarbonyl-(3S)-hydroxy-(2S)-pyrrolidinyl)carboxylic acid methyl ester (9.00g, 36.87mmol) and iodoethane (8.80ml, 110mmol) were dissolved in DMF (75ml), sodium hydride (60% in oil, 2.20g, 55.0mmol) was added at 0˚C, after completion of addition, a temperature of the reaction mixture was raised to room temperature, and the mixture was further stirred at room temperature for 5 hours. To the reaction solution was added water, and 1N hydrochloric

acid was added to make the solution weakly acidic, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent, n-hexane:ethyl acetate = 6:1) using silica gel to obtain a mixture of (1-tert-butoxycarbonyl-(3S)-ethoxy-(2S)-pyrrolidinyl)car boxylic acid methyl ester and transesterified (1-tert-butoxycarbonyl-(3S)-ethoxy-(2S)-pyrrolidinyl)carboxylic acid ethyl ester, as a pale yellow oily substance. This mixture was dissolved in tetrahydrofuran (60ml), a 1N aqueous sodium hydroxide solution (61ml, 61mmol) was added, and the mixture was stirred at room temperature for 20 hours. 1N hydrochloric acid was added to the reaction solution to weakly acidic, followed by extraction with ethyl acetate. The extract was washed with an aqueous saturated sodium chloride solution, and dried with anhydrous sodium sulfate, and a solvent was removed under reduced pressure to obtain the title compound (8.31g, 87%) as a colorless oily substance.

$^1$H-NMR ($CDCl_3$) δ: 1.19-1.24 (3H, m), 1.41-1.53 (9H, m), 1.98-2.05 (2H, m), 3.47-3.57 (4H, m), 4.36-4.40 (2H, m).

MS (LC-ESI) m/z: 160 ($M^+$+1-Boc).

5-[(1-tert-butoxycarbonyl-(3S)-ethoxy-(2S)-pyrrolidinyl)carbonylamino]levulinic acid methyl ester

[Chem. 258]

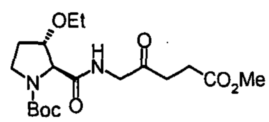

(1-tert-butoxycarbonyl-(3S)-ethoxy-(2S)-pyrrolidinyl) carboxylic acid (2. 03g, 7.83mmol), 5-aminolevulinic acid methyl esterhydrochloride (1. 42g, 7.84mmol), EDC · HCl (2.25g, 11.7mmol), HOBt (1.06g, 7.84mmol) and triethylamine (5.46ml, 39.2mmol) were dissolved in DMF (45ml), and the solution was stirred at room temperature for 15 hours. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by column chromatography (eluting solvent : chloroform:methanol= 50:1) using silica gel to obtain the title compound (2.00g, 66%) as a pale yellow oily substance.

$^1$H-NMR ($CDCl_3$) δ: 1.20 (3H, t, J= 7.1 Hz), 1.47 (9H, broad s), 1.97-2.01 (2H, m), 2.64-2.83 (4H, m), 3.49-3.60 (4H, m), 3.68 (3H, s), 4.13-4.36 (4H, m).

MS (LC-ESI) m/z: 387 ($M^+$+1).

3-[2-(1-tert-butoxycarbonyl-(3S)-ethoxy-(2S)-pyrrolidinyl)-5-thiazolyl]propionic acid methyl ester

[Chem. 259]

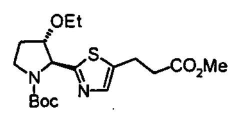

5-[(1-tert-butoxycarbonyl-(3S)-ethoxy-(2S)-pyrrolidinyl)carbonylamino]levulinic acid methyl ester (2.00g, 5.18mmol) was dissolved in toluene (50ml), a Lawesson's reagent (2.30g, 5.69mmol) was added, and the mixture was stirred at 90°C for 3 hours. After allowing to cool to room temperature, water was added to the reaction solution, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent:n-hexane:ethyl acetate= 3:1-2:1) using silica gel to obtain the title compound (1.43g, 72%) as a pale yellow oily substance.

$^1$H-NMR ($CDCl_3$) δ: 1.23 (3H, t, J = 7.1 Hz), 1.35 and 1.49 (total 9H, each s), 1.98-2.08 (2H, m), 2.65 (2H, t, J = 7.5 Hz), 3.09-3.14 (2H, m), 3.50-3.69 (total 7H, series of m, including 3H, s, at δ 3.69), 4.14-4.18 (1H, m), 5.00 and 5.15 (total 1H, each s), 7.40 (1H, s).

MS (LC-ESI) m/z: 385 ($M^+$+1).

3-[2-((3S)-ethoxy-(2S)-pyrrolidinyl)-5-thiazolyl]propionic acid methyl ester

[Chem. 260]

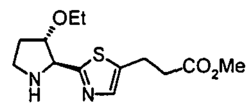

3-[2-(1-tert-butoxycarbonyl-(3S)-ethoxy-(2S)-pyrrolidinyl)-5-thiazolyl]propionic acid methyl ester (1.43g, 3.72mmol) was dissolved in methylene chloride (7ml) trifluoroacetic acid (7ml) was added, and the mixture was stirred at room temperature for 1 hour. To the reaction solution was added an aqueous saturated sodium bicarbonate solution to make the solution weakly alkaline, followed by extraction with chloroform. The extract was washed with an aqueous saturated sodium chloride solution, and dried with anhydrous sodium sulfate, and a solvent was removed under reduced pressure to obtain the title compound (0.96g, 91%) as a yellow oily substance.

$^1$H-NMR ($CDCl_3$) δ: 1.23 (3H, t, J = 7.1 Hz), 1.87-1.95 (2H, m), 2.65 (2H, t, J = 7. 5 Hz), 3.08-3.14 (3H, m), 3.21-3.31 (1H, m), 3.48-3.73 (total 5H, series of m, including 3H, s, at δ 3.69), 4.19-4.21 (1H, m), 4.45-4.46 (1H, m), 7.40-7.41 (1H, m).

3-[2-[1-[[5-Chloro-2-fluoro-4-(1-methyl-3-indolylcarbonylamino)phenyl]acetyl]-(3S)-ethoxy-(2S)-pyrrolidinyl]-5-thiazolyl]propionic acid methyl ester

[Chem. 261]

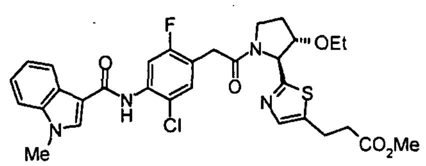

5-Chloro-2-fluoro-4-(1-methyl-3-indolylcarbonylamino)phenylacetic acid (241mg, 0.67mmol), 3-[2-((3S)-ethoxy-(2S)-pyrrolidinyl)-5-thiazolyl]propion ic acid methyl ester (190mg, 0.67mmol), EDC · HCl (192mg, 1.00mmol), HOBt (90mg, 0.67mmol) and triethylamine (140μl, 1.00mmol) were dissolved in DMF(7ml), and the solution was stirred at room temperature for 15 hours. The reaction solution was diluted with water, followed by extracted with ethyl acetate. The extract was washed with an aqueous saturated sodium chloride solution, and dried with anhydrous sodium sulfate, and a solvent was removed under reduced pressure. The resulting residue was purified by column chromatography (eluting solvent : chloroform: methanol= 60:1-30:1) using silica gel to obtain the title compound (397mg, 95%) as a pale yellow amorphous solid.

$^1$H-NMR ($CDCl_3$) δ: 1.20-1.24 (3H, m), 2.07-2.31 (2H, m), 2.63-2.68 (2H, m), 3.08-3.15 (2H, m), 3.46-3.82 (total 9H, series of m, including 3H, s, at δ 3.68), 3.89 (3H, s), 4.15-4.16 and 4.26-4.27 (total 1H, each m), 5.23 and 5.45 (total 1H, each s), 7.34-7.48 (5H, m), 7.80 and 7.81 (total 1H, each s), 8.13-8.15 (1H, m), 8.29-8.31 (1H, m), 8.46 and 8.53 (total 1H, each d, J = 12.3 Hz).
MS (LC-ESI) m/z: 627 ($M^+$+1).

3-[2-[1-[[5-Chloro-2-fluoro-4-(1-methyl-3-indolylcarbonylamino)phenyl]acetyl]-(3S)-ethoxy-(2S)-pyrrolidinyl]-5-thiazolyl]propionic acid

[Chem. 262]

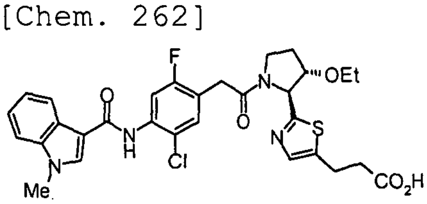

3-[2-[1-[[5-Chloro-2-fluoro-4-(1-methyl-3-indolylcarbonylamino)phenyl]acetyl]-(3S)-ethoxy-(2S)-pyrrolidiny 1]-5-thiazolyl]propionic acid methyl ester (397mg, 0.63mmol) was dissolved in tetrahydrofuran (3ml), a 0.5N aqueous sodium hydroxide solution (2.50ml, 1.25mmol) was added, and the mixture was stirred at room temperature for 15 hours.

1N hydrochloric acid was added to the reaction solution to acidic, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent : chloroform: methanol = 50:1 -15:1) using silica gel to obtain the title compound (231mg, 60%) as a colorless amorphous solid.

$^1$H-NMR (DMSO-$d_6$) δ: 1.17 (3H, q, J=7.1 Hz), 1.98-2.18 (2H, m), 2.54-2.61 (2H, m), 2.98-3.06 (2H, m), 3.38-3.52 (2H, m), 3.62-3.89 (total 7H, series of m, including 3H, s, at δ 3.89), 4.17 (1H,s), 5.19 and 5.46 (total 1H, each s), 7.20-7.30 (2H, m), 7.35-7.57 (3H, m), 7.67-7.74 (1H, m), 8.15 (1H, d, J = 8.1 Hz), 8.31 (1H, s), 9.27 and 9.30 (total 1H, each s), 12.31 (1H, broad s).
IR (ATR) $cm^{-1}$: 1657, 1518, 1402, 1099, 744.
MS (LC-ESI) m/z: 613 ($M^+$+1).
Anal. Calcd for $C_{30}H_{30}ClFN_4O_5S$: C, 58.77; H, 4.93; N, 9.14; S, 5.23; Cl, 5.78; F, 3.10.
Found: C, 58.46; H, 4.93; N, 8.94; S, 5.24; Cl, 5.75; F, 3.05.

Compounds of Example 92 to Example 96 shown below were produced by the same method as that of Example 91.

Example 92

3-[2-[1-[[2,5-Dichloro-4-(1-methyl-3-indolylcarbonylamino)phenyl]acetyl]-(3S)-ethoxy-(2S)-pyrrolidinyl]-5-thiazolyl]propionic acid methyl ester

[Chem. 263]

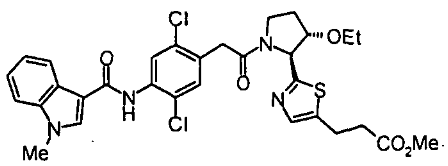

$^1$H-NMR ($CDCl_3$) δ: 1.19-1.25 (3H, m), 2.08-2.31 (2H, m), 2.63-2.67 (2H, m), 3.06-3.15 (2H, m), 3.53-3.85 (total 9H, series of m, including 3H, s, at δ 3.69), 3.89 (3H, s), 4.15-4.16 and 4.27-4.28 (total 1H, each m), 5.25 and 5.47 (total 1H, each s), 7.33-7.47 (5H, m), 7.80 and 7.81 (total 1H, each s), 8.12-8.15 (1H, m), 8.23-8.25 (1H, m), 8.75 and 8.80 (total 1H, each s).
MS (LC-ESI) m/z: 643 ($M^+$+1).

3-[2-[1-[[2,5-Dichloro-4-(1-methyl-3-indolylcarbonylamino)phenyl]acetyl]-(3S)-ethoxy-(2S)-pyrrolidinyl]-5-thiazolyl]propionic acid

[Chem. 264]

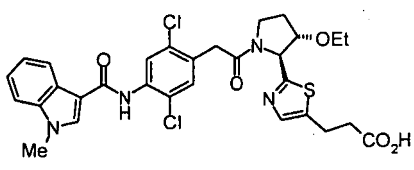

$^1$H-NMR (DMSO-$d_6$) δ: 1.17 (3H, q, J = 6.9 Hz), 1.99-2.17 (2H, m), 2.54-2.61 (2H, m), 2. 98-3.06 (2H, m), 3.45-3.98 (total 9H, series of m, including 3H, s, at δ 3.89), 4.14-4.19 (1H, m), 5.20 and 5.45 (total 1H, each s), 7.20-7.29 (2H, m), 7.47 (1H, s), 7.54-7.58 (2H, m), 7.88 and 7.92 (total 1H, each s), 8.15 (1H, d, J = 7.8 Hz), 8.29 and 8.30 (total 1H, each s), 9.33 and 9.36 (total 1H, each s), 12.29 (1H, broad s).
IR (ATR) $cm^{-1}$: 1653, 1502, 1373, 1101, 1076, 744.
MS (LC-ESI) m/z: 629 ($M^+$+1). Anal. Calcd for $C_{30}H_{30}Cl_2N_4O_5S \cdot 1/2H_2O$: C, 56.43; H, 4.89; N, 8.77; S, 5.02; Cl, 11.10.
Found: C, 56.62; H, 4.86; N, 8.88; S, 5.18; Cl, 10.94.

Example 93

3-[2-[1-[4-(3-Benzo[b]thiophenylcarbonyl)amino-5-chloro-2-fluorophenyl]acetyl]-(3S)-ethoxy-(2S)-pyrrolidinyl]-5-thiazolyl]propionic acid methyl ester

[Chem. 265]

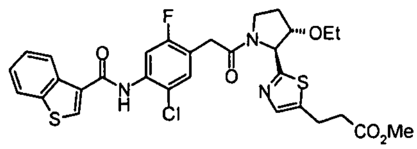

$^1$H-NMR ($CDCl_3$) δ: 1.21-1.25 (3H, m), 2.06-2.30 (2H, m), 2.65 (2H, q, J = 7.5 Hz), 3.08-3.14 (2H, m), 3.51-3.70 (total 7H, series of m, including 3H, s, at δ 3.68), 3.75-3.83 (2H, m), 4.16-4.17 and 4.26-4.27 (total 1H, each m), 5.23 and 5.45 (total 1H, each s), 7.35-7.53 (4H, m), 7.91-7.93 (1H, m), 8.08-8.09 (1H, m), 8.33-8.40 (1H, m), 8.46-8.49 (2H, m).
MS (LC-ESI) m/z: 630 ($M^+$+1) .

3-[2-[1-[4-(3-Benzo[b]thiophenylcarbonyl)amino-5-chloro-2-fluorophenyl]acetyl]-(3S)-ethoxy-(2S)-pyrrolidinyl]-5-thiazolyl]propionic acid

[Chem. 266]

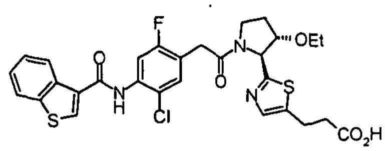

$^1$H-NMR (DMSO-$d_6$) δ: 1.14-1.20 (3H, m), 1.99-2.16 (2H, m), 2.54-2.61 (2H, m), 2.98-3.07 (2H, m), 3.52-3.91 (6H, m), 4.15-4.18 (1H, m), 5.19 and 5.47 (total 1H, each s), 7.39-7.60 (5H, m), 8.10 (1H, d, J = 7.6 Hz), 8.45 (1H, d, J = 7.1 Hz), 8.64 and 8.65 (total 1H, each s), 10.09 and 10.11 (total 1H, each s), 12.30 (1H, broad s).
IR (ATR) $cm^{-1}$: 1649, 1518, 1400, 1215, 1101, 766.
MS (LC-ESI) m/z: 616 ($M^+$+1).
Anal. Calcd for $C_{29}H_{27}ClFN_3O_5S_2$: C, 56.53; H, 4.42; N, 6.82; S, 10.41; Cl, 5.75; F, 3.08.
Found: C, 56.57; H, 4.52; N, 6.70; S, 10.44; Cl, 5.53; F, 2.98.

Example 94

3-[2-[1-[4-(3-Benzo[b]thiophenylcarbonyl)amino-2,5-dichlorophenyl]acetyl]-(3S)-ethoxy-(2S)-pyrrolidinyl]-5-thiazolyl]propionic acid methyl ester

[Chem. 267]

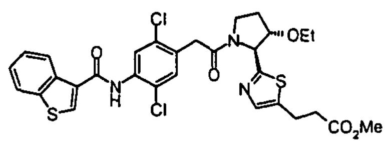

$^1$H-NMR ($CDCl_3$) δ: 1.20-1.30 (3H, m), 2.09-2.32 (2H, m), 2.63-2.68 (2H, m), 3.09-3.16 (2H, m), 3.55-3.90 (total 9H, series of m, including 3H, s, at δ 3.69), 4.16-4.28 (1H, m), 5.25 and 5.47 (total 1H, each s), 7.38-7.54 (4H, m),

7.91-7.93 (1H, m), 8.08-8.09 (1H, m), 8.27-8.29 (1H, m), 8.47-8.49 (1H, m), 8.68 and 8.74 (total 1H, each s).
MS (LC-ESI) m/z: 646 ($M^{+}$+1).

3-[2-[1-[4-(3-Benzo[b]thiophenylcarbonyl)amino-2,5-dichlorophenyl]acetyl]-(3S)-ethoxy-(2S)-pyrrolidinyl]-5-thiazolyl]propionic acid

[Chem. 268]

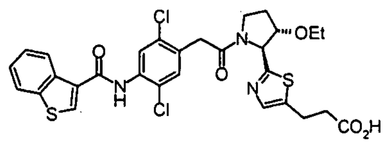

$^1$H-NMR (DMSO-$d_6$) δ: 1.14-1.19 (3H, m), 1.98-2.19 (2H, m), 2.54-2.61 (2H, m), 2.98-3.07 (2H, m), 3.47-4.06 (6H, m), 4.15-4.18 (1H, m), 5.21 and 5.46 (total 1H, eachs), 7.44-7.51 (3H, m), 7.58 and 7. 62 (total 1H, eachs), 7.73 and 7. 78 (total 1H, each s), 8.10 (1H, d, J= 10.0 Hz), 8.45 (1H, d, J = 7.1 Hz), 8.64 and 8.65 (total 1H, each s), 10.13 and 10.15 (total 1H, each s), 12.30 (1H, broad s).
IR (ATR) $cm^{-1}$: 1647, 1504, 1375, 1211, 1080, 766. MS (LC-ESI) m/z: 632 ($M^{+}$+1).
Anal. Calcd for $C_{29}H_{27}Cl_2N_3O_5S_2$: C, 55.06; H, 4.30; N, 6.64; S, 10.14; Cl, 11.21.
Found: C, 54.97; H, 4.48; N, 6.45; S, 9.91; Cl, 10.93.

Example 95

3-[2-[1-[4-(3-Benzo[b]furanylcarbonyl)amino-5-chloro-2-fluorophenyl]acetyl]-(3S)-ethoxy-(2S)-pyrrolidinyl]-5-thiazolyl]propionic acid methyl ester

[Chem. 269]

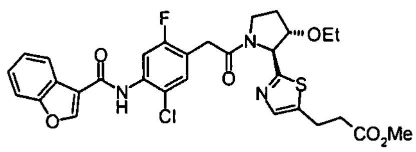

$^1$H-NMR ($CDCl_3$) δ: 1.20-1.25 (3H, m), 2.06-2.35 (2H, m), 2.63-2.68 (2H, m), 3.08-3.15 (2H, m), 3.46-3.81 (total 9H, series of m, including 3H, s, at δ 3.68), 4.17-4.27 (1H, m), 5.23 and 5.45 (total 1H, each s), 7.36-7.52 (4H, m), 7. 59-7. 60 (1H, m), 8.05-8.07 (1H, m), 8.28-8.30 (2H, m), 8.38-8.47 (1H, m).
MS (LC-ESI) m/z: 614 ($M^{+}$+1).

3-[2-[1-[4-(3-Benzo[b]furanylcarbonyl)amino-5-chloro-2-fluorophenyl]acetyl]-(3S)-ethoxy-(2S)-pyrrolidinyl]-5-thiazolyl]propionic acid

[Chem. 270]

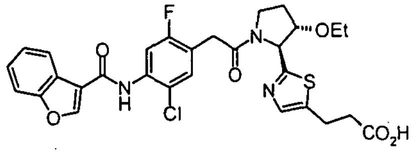

$^1$H-NMR (DMSO-$d_6$) δ: 1.14-1.19 (3H, m), 1.98-2.19 (2H, m), 2.54-2.61 (2H, m), 2.98-3.06 (2H, m), 3.52-3.91 (6H, m), 4.16-4.17 (1H, m), 5.18 and 5.47 (total 1H, each s), 7.37-7.47 (3H, m), 7.53-7.61 (2H, m), 7.70-7.72 (1H, m), 8.08-8.10 (1H, m), 8.82 and 8.83 (total 1H, each s), 9.97 and 9.99 (total 1H, each s), 12.30 (1H, broad s).

IR (ATR) $cm^{-1}$: 1521, 1402, 1103, 748.
MS (LC-ESI) m/z: 600 ($M^{+}$+1).
Anal. Calcd for $C_{29}H_{27}ClFN_3O_6S$: C, 58.05; H, 4.54; N, 7.00; S, 5.34; Cl, 5.91; F, 3.17.
Found: C, 57.75; H, 4.58; N, 6.94; S, 5.39; Cl, 5.82; F, 3.16.

Example 96

3-[2-[1-[4-(3-Benzo[b]furanylcarbonyl)amino-2,5-dichlorophenyl]acetyl]-(3S)-ethoxy-(2S)-pyrrolidinyl]-5-thiazolyl]propionic acid methyl ester
[Chem. 271]

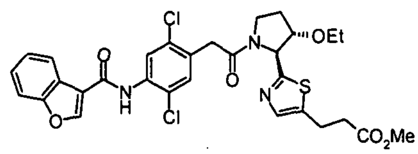

$^1$H-NMR ($CDCl_3$) δ: 1. 20-1. 25 (3H, m), 2.07-2.33 (2H, m), 2.63-2.68 (2H, m), 3.09-3.16 (2H, m), 3.53-3.89 (total 9H, series of m, including 3H, s, at δ 3.69), 4.16-4.28 (1H, m), 5.25 and 5.46 (total 1H, each s), 7.39-7.48 (4H, m), 7.59-7.62 (1H, m), 8.03-8.07 (1H, m), 8.21-8.22 (1H, m), 8.29 and 8.30 (total 1H, each s), 8.68 and 8.74 (total 1H, each s).
MS (LC-ESI) m/z: 630 ($M^{+}$+1).
3-[2-[1-[4-(3-Benzo[b]furanylcarbonyl)amino-2,5-dichlorophenyl]acetyl]-(3S)-ethoxy-(2S)-pyrrolidinyl]-5-thiazolyl]propionic acid
[Chem. 272]

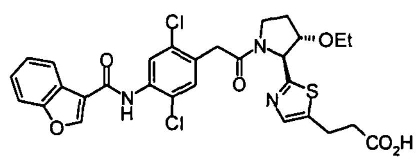

$^1$H-NMR (DMSO-$d_6$) δ: 1.14-1.20 (3H, m), 1.99-2.18 (2H, m), 2.54-2.60 (2H, m), 2.98-3.06 (2H, m), 3.46-4.04 (6H, m), 4.15-4.18 (1H, m), 5.20 and 5.46 (total 1H, each s), 7.37-7.51 (3H, m), 7.58-7.62 (1H, m), 7.72-7.79 (2H, m), 8.07-8.10 (1H, m), 8.81 and 8.82 (total 1H, each s), 10.02 and 10.04 (total 1H, each s), 12.29 (1H, broad s).
IR (ATR) $cm^{-1}$: 1645, 1572, 1508, 1304, 1122, 1103, 1080, 748. MS (LC-ESI) m/z: 616 ($M^{+}$+1). Anal. Calcd for $C_{29}H_{27}Cl_2N_3O_6S$: C, 56.50; H, 4.41; N, 6.82; S, 5.20; Cl, 11.50.
Found: C, 56.25; H, 4.42; N, 6.74; S, 5.30; Cl, 11.28.

Example 97

5-[(1-Benzyloxycarbonyl-(3S)-ethoxy-(2S)-pyrrolidinyl)carbonylamino]levulinic acid methyl ester
[Chem. 273]

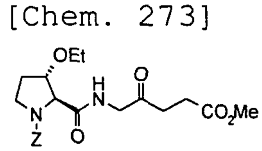

5-[(1-tert-butoxycarbonyl-(3S)-ethoxy-(2S)-pyrrolidinyl)carbonylamino]levulinic acid methyl ester (7.75g, 10.05mmol) was dissolved in 4N hydrochloric acid-1,4-dioxane (70ml), and the solution was stirred at room temperature

for 2 hours. A solvent was removed under reduced pressure to obtain 5-[(3S)-ethoxy-(2S)-pyrrolidinyl]carbonylamino] levulinic acid methyl ester hydrochloride (6.47g) as a pale brown amorphous solid. This crude product was dissolved in acetonitrile (100ml), triethylamine (13.98ml, 100.3mmol) and benzyl chloroformate (5.73ml, 40.1mmol) were added, and the mixture was stirred at room temperature for 1.5 hours. Precipitated triethylamine hydrochloride was removed by suction filteration, and the filtrate was concentrated. The resulting residue was diluted with ethyl acetate, washed with water and an aqueous saturated sodium chloride solution, and dried with anhydrous sodium sulfate, and a solvent was removed under reduced pressure. The resulting residue was purified by flash column chromatography (Biotage flash chromatography system, column size: 40N, eluting solvent: n-hexane: ethyl acetate= 1: 2- 1:9) to obtain the title compound (4.47g, 53%) as a colorless oily substance.

$^1$H-NMR ($CDCl_3$) δ: 1.19 (3H, t, J = 7.1 Hz), 2.00-2.06 (2H, m), 2.61-2.73 (4H, m), 3.47-3.72 (total 7H, series of m, including 3H, s, at δ 3.68), 4.00-4.42 (4H, m), 5.11-5.18 (2H, m), 7.15-7.37 (5H, m).

3-[2-(1-Benzyloxycarbonyl-(3S)-ethoxy-(2S)-pyrrolidinyl)-5-oxazolyl]propionic acid methyl ester

[Chem. 274]

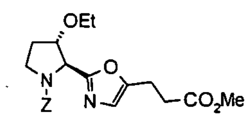

5-[(1-Benzyloxycarbonyl-(3S)-ethoxy-(2S)-pyrrolidinyl)carbonylamino]levlinic acid methyl ester (2.39g, 5.68mmol) was dissolved in toluene (170ml), phosphorus oxychloride (3.18ml, 34.1mmol) was added, and the mixture was stirred at 110°C for 3 hours. After allowing to cool to room temperature, the reaction solution was poured into ice-aqueous saturated sodium bicarbonate solution, and the mixture was stirred for 30 minutes, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent: chloroform: methanol= 60:1-30:1) using silica gel to obtain the title compound (1.97g, 86%) as a pale brown oily substance.

$^1$H-NMR ($CDCl_3$) δ: 1.19-1.23 (3H, m), 2.03-2.23 (2H, m), 2.52-2.69 (2H, m), 2.86-3.01 (2H, m), 3.48-3.73 (total 7H, series of m), 4.04-4.09 (1H, m), 4.95-5.18 (3H, m), 6.68-6.71 (1H, m), 7.16-7.37 (5H, m).
MS (LC-ESI) m/z: 403 ($M^+$+1).

3-[2-((3S)-ethoxy-(2S)-pyrrolidinyl)-5-oxazolyl]propionic acid methyl ester

[Chem. 275]

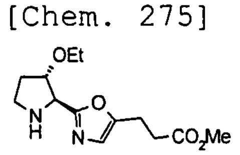

3-[2-(1-Benzyloxycarbonyl-(3S)-ethoxy-(2S)-pyrrolidinyl)-5-oxazolyl]propionic acid methyl ester (1.97g, 4.90mmol) was dissolved in methanol (20ml), 10% palladium hydroxide/carbon (0.40g) was added, and hydrogenation was performed at room temperature for 2 days. A catalyst was removed by suction filtration, and the filtrate was concentrated to obtain the title compound (1.17g, 89%) as a brown oily substance.

$^1$H-NMR ($CDCl_3$) δ: 1.22 (3H, t, J = 7.1 Hz), 1.96-2.03 (1H, m), 2.08-2.17 (1H, m), 2.63-2.69 (3H, m), 2.95-3.01 (2H, m), 3.24-3.34 (2H, m), 3.45-3.61 (2H, m), 3.70 (3H, s), 4.26-4.28 (1H, m), 4.43-4.44 (1H, m), 6.71-6.72 (1H, m).

3-[2-[1-[[5-Chloro-2-fluoro-4-(1-methyl-3-indolylcarbonylamino)phenyl]acetyl]-(3S)-ethoxy-(2S)-pyrrolidiny1]-5-oxazolyl]propionic acid methyl ester

[Chem. 276]

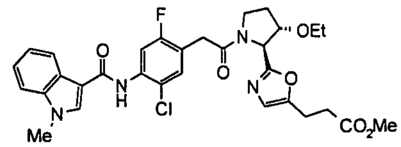

5-Chloro-2-fluoro-4-(1-methyl-3-indolylcarbonylamino)phenylacetic acid (250mg, 0.69mmol), 3-[2-((3S)-ethoxy-(2S)-pyrrolidinyl)-5-oxazolyl]propionic acid methyl ester (186mg, 0.69mmol), EDC · HCl (199mg, 1.04mmol), HOBt (94mg, 0.69mmol) and triethylamine (145μl, 1.04mmol) were dissolved in DMF (7ml), and the solution was stirred at room temperature for 2 days. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by flash column chromatography (Biotage flash chromatography system, column size: 25S, eluting solvent, methylene chloride:methanol= 20:0-20:1) to obtain the title compound (410mg, 97%) as a pale brown oily substance.

$^1$H-NMR ($CDCl_3$) δ: 1.19-1.28 (3H, m), 2.12-2.35 (2H, m), 2.62-2.67 (2H, m), 2.95-3.00 (2H, m), 3.48-3.80 (total 9H, series of m, including 3H, s, at δ 3.69), 3.89 (3H, s), 4.04-4.05 and 4.19-4.20 (total 1 H, each m), 5.04 and 5.25 (total 1H, each s), 6.69 and 6.73 (total 1H, each s), 7.33-7.46 (4H, m), 7.80-7.81 (1H, m), 8.13-8.15 (1H, m), 8.30 (1H, s), 8.48-8.52 (1H, m).
MS (LC-ESI) m/z: 611 ($M^+$+1).

3-[2-[1-[[5-Chloro-2-fluoro-4-(1-methyl-3-indolylcarbonylamino)phenyl]acetyl]-(3S)-ethoxy-(2S)-pyrrolidinyl]-5-oxazolyl]propionic acid

[Chem. 277]

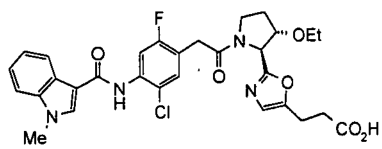

3-[2-[1-[[5-Chloro-2-fluoro-4-(1-methyl-3-indolylcarbonylamino)phenyl]acetyl]-(3S)-ethoxy-(2S)-pyrrolidiny 1]-5-oxazolyl]propionic acid methyl ester (410mg, 0.67mmol) was dissolved in tetrahydrofuran (3ml), a 0.5N aqueous sodium hydroxide solution (2.70ml, 1.35mmol), and the mixture was stirred at room temperature for 16 hours. 1N hydrochloric acid was added to the reaction solution to acidic, and extracted with ethyl acetate. The extract was washed with an aqueous saturated sodium chloride solution, and dried with anhydrous sodium sulfate, and a solvent was removed under reduced pressure. The resulting residue was purified by column chromatography (eluting solvent : chloroform : methanol = 60 : 1 - 30 : 1) using silica gel to obtain the title compound (206mg, 51%) as a colorless amorphous solid.

$^1$H-NMR (DMSO-$d_6$) δ: 1.12-1.18 (3H, m), 1.98-2.21 (2H, m), 2.52-2.59 (2H, m), 2.82-2.90 (2H, m), 3.49-3.88 (total 9H, series of m, including 3H, s, at δ 3.88), 3.99-4.00 and 4.22-4.23 (total 1H, each m), 4.95 and 5.28 (total 1H, each s), 6.77 and 6.88 (total 1H, each s), 7.19-7.28 (2H, m), 7.36-7.48 (1H, m), 7.55 (1H, d, J = 8.1 Hz), 7.66-7.71 (1H, m), 8.14 (1H, d, J = 8.1 Hz), 8.30 (1H, s), 9.27-9.29 (1H, m), 12.27 (1H, broad s).
IR (ATR) $cm^{-1}$: 1655, 1518, 1402, 1099, 744.
MS (LC-ESI) m/z: 597 ($M^+$+1).
Anal. Calcd for $C_{30}H_{30}ClFN_4O_6$ · 1/4$H_2O$: C, 59.90; H, 5.11; N, 9.31; Cl, 5.89; F, 3.16.
Found: C, 60.02; H, 5.10; N, 8.95; Cl, 5.77; F, 3.10.

Compounds of Example 98 to Example 106 shown below were prepared by the same method as that of Example 97.

Example 98

3-[2-[1-[[2,5-Dichloro-4-(1-methyl-3-indolylcarbonylnamino)phenyl]acetyl]-(3S)-ethoxy-(2S)-pyrrolidinyl]-5-oxazolyl]propionic acid methyl ester

[Chem. 278]

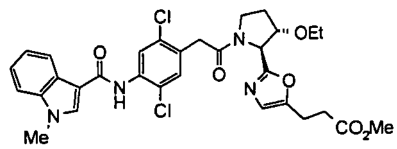

$^1$H-NMR ($CDCl_3$) δ: 1.18-1.25 (3H, m), 2.12-2.34 (2H, m), 2.62-2.66 (2H, m), 2.97-3.01 (2H, m), 3.50-3.80 (total 9H, series of m, including 3H, s, at δ 3.69), 3.89 (3H, s), 4.05-4.06 and 4.19-4.20 (total 1H, each m), 5.04 and 5.28 (total 1H, each s), 6.70 and 6.73 (total 1H, each s), 7.32-7.48 (4H, m), 7.79-7.80 (1H, m), 8.12-8.15 (1H, m), 8.23-8.24 (1H, m), 8.75 and 8.79 (total 1H, each s).

MS (LC-ESI) m/z: 627 ($M^+$+1).

3-[2-[1-[[2,5-Dichloro-4-(1-methyl-3-indolylcarbonylamino)phenyl]acetyl]-(3S)-ethoxy-(2S)-pyrrolidinyl]-5-oxazolyl]propinic acid

[Chem. 279]

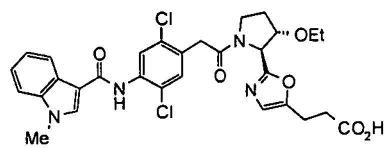

$^1$H-NMR (DMSO-$d_6$) δ: 1.12-1.18 (3H, m), 2.01-2.22 (2H, m), 2.52-2.59 (2H, m), 2.82-2.90 (2H, m), 3.49-4.24 (total 10H, series of m, including 3H, s, at δ 3.88), 4.97 and 5.26 (total 1H, each s), 6.78 and 6.89 (total 1H, each s), 7.18-7.28 (2H, m), 7.46-7.55 (2H, m), 7.86 and 7.89 (total 1H, each s), 8.14 (1H, d, J = 7.6 Hz), 8.29 (1H, s), 9.34-9.35 (1H, m), 12.28 (1H, broad s).

IR (ATR) $cm^{-1}$: 1655, 1502, 1373, 1101, 1076, 744.

MS (LC-ESI) m/z: 613 ($M^+$+1).

Anal. Calcd for $C_{30}H_{30}Cl_2N_4O_6 \cdot 1/4H_2O$: C, 58.31; H, 4.97; N, 9.07; Cl, 11.47.

Found: C, 58.25; H, 4.97; N, 8.87; Cl, 11.40.

Example 99

3-[2-[1-[4-(3-Benzo[b]thiophenylcarbonyl)amino-5-chloro-2-fluorophenyl]acetyl]-(3S)-ethoxy-(2S)-pyrrolidinyl]-5-oxazolyl]propionic acid methyl ester

[Chem. 280]

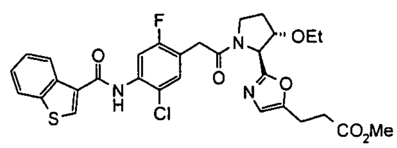

$^1$H-NMR ($CDCl_3$) δ: 1.20-1.25 (3H, m), 2.08-2.37 (2H, m), 2.61-2.67 (2H, m), 2.94-3.00 (2H, m), 3.50-3.87 (total 9H, series of m, including3H, s, at δ 3.69), 4.04-4.05 and 4.20-4.21 (total 1H, each m), 5.04 and 5.25 (total 1H, each s), 6.69-6.74 (1H, m), 7.36-7.54 (3H, m), 7.90-7.92 (1H, m), 8.08-8.09 (1H, m), 8.33-8.49 (3H, m).

MS (LC-ESI) m/z: 614 ($M^{+}$+1).

Example 100

3-[2-[1-[4-(3-Benzo[b]thiophenylcarbonyl)amino-2,5-dichlorophenyl]acetyl]-(3S)-ethoxy-(2S)-pyrrolidinyl]-5-oxazolyl]propionic acid methyl ester

1

[Chem. 281]

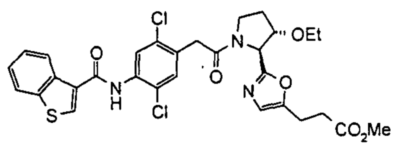

$^{1}$H-NMR ($CDCl_3$) δ: 1.19-1.26 (3H, m), 2.04-2.36 (2H, m), 2.62-2.66 (2H, m), 2.97-2.99 (2H, m); 3.50-3.87 (total 9H, series of m, including 3H, s, at δ 3.69), 4.05-4.06 and 4.20-4.21 (total 1H, each m), 5.05 and 5.28 (total 1H, each s), 6.70 and 6.74 (total 1H, each s), 7.43-7.53 (3H, m), 7.91 (1H, d, J = 8.1 Hz), 8.09 (1H, s), 8.29 (1H, s), 8.48 (1H, d, J = 7.6 Hz), 8.66 and 8.70 (total 1H, each s).

MS (LC-ESI) m/z: 630 ($M^{+}$+1).

3-[2-[1-[4-(3-Benzo[b]thiophenylcarbonyl)amino-2,5-dichlorophenyl]acetyl]-(3S)-ethoxy-(2S)-pyrrolidinyl]-5-oxazolyl]propionic acid

[Chem. 282]

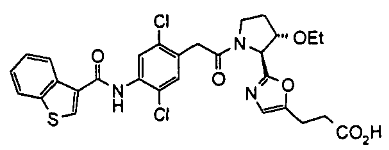

$^{1}$H-NMR (DMSO-$d_6$) δ: 1.13-1.19 (3H, m), 2.04-2.24 (2H, m), 2.53-2.60 (2H, m), 2.83-2.92 (2H, m), 3.49-3.97 (total 6H, series of m), 4.02-4.03 and 4.25-4.26 (total 1H, each m), 4.98 and 5.28 (total 1H, each s), 6.79 and 6.90 (total 1H, each s), 7.44-7.60 (3H, m), 7.74-7.77 (1H, m), 8.09-8.10 (1H, m), 8.44-8.46 (1H, m), 8.64 (1H, s), 10.14-10.15 (1H, m), 12.32 (1H, broad s).

IR (ATR) $cm^{-1}$: 1643, 1504, 1423, 1373, 1103, 1080, 766.

MS (LC-ESI) m/z: 616 ($M^{+}$+1).

Anal. Calcd for $C_{29}H_{27}Cl_2N_3O_6S \cdot 1/4H_2O$: C, 56.09; H, 4.46; N, 6.77; S, 5.16; Cl, 11.42.

Found: C, 56.17; H, 4.46; N, 6.68; S, 5.25; Cl, 10.96.

Example 101

3-[2-[1-[4-(3-Benzo[b]furanylcarbonyl)amino-5-chloro-2-fluorophenyl]acetyl]-(3S)-ethoxy-(2S)-pyrrolidinyl]-5-oxazolyl]propionic acid methyl ester

[Chem. 293]

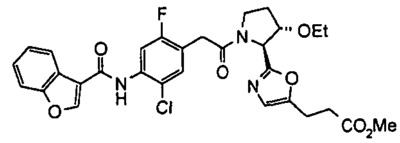

$^1$H-NMR ($CDCl_3$) δ: 1.20-1.25 (3H, m), 2.08-2.37 (2H, m), 2.61-2.67 (2H, m), 2.94-3.00 (2H, m), 3.49-3.84 (total 9H, series of m, including 3H, s, at δ 3.69), 4.04-4.05 and 4.20-4.21 (total 1H, each m), 5.04 and 5.25 (total 1H, each s), 6.69 and 6.74 (total 1H, each s), 7.37-7.49 (3H, m), 7.58-7.62 (1H, m), 8.02-8.08 (1H, m), 8.27-8.30 (2H, m), 8.38-8.45 (1H, m).
MS (LC-ESI) m/z: 598 ($M^+$+1).

3-[2-[1-[4-(3-Benzo[b]furanylcarbonyl)amino-5-chloro-2-fluorophenyl]acetyl]-(3S)-ethoxy-(2S)-pyrrolidinyl]-5-oxazolyl]propionic acid

[Chem. 284]

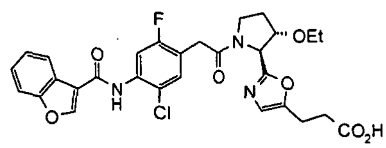

$^1$H-NMR (DMSO-$d_6$) δ: 1.13-1.19 (3H, m), 2.02-2.26 (2H, m), 2.53-2.60 (2H, m), 2.83-2.91 (2H, m), 3.41-3.89 (total 6H, series of m), 4.00-4.02 and 4.24-4.25 (total 1H, each m), 4.96 and 5.30 (total 1H, each s), 6.78 and 6.89 (total 1H, each s), 7.37-7.59 (4H, m), 7.70-7.72 (1H, m), 8.07-8.10 (1H, m), 8.83 (1H, s), 9.97-9.98 (1H, m), 12.31 (1H, broad s) .
IR (ATR) $cm^{-1}$: 1645, 1523, 1404, 1120, 1103, 748.
MS (LC-ESI) m/z: 584 ($M^+$+1) .
Anal. Calcd for $C_{29}H_{27}ClFN_3O_7$: C, 59.64; H, 4.66; N, 7.20; Cl, 6.07; F, 3.25.
Found: C, 59.38; H, 4.66; N, 7.03; Cl, 6.23; F, 3.26.

Example 102

3-[2-[1-[4-(3-Benzo[b]furanylcarbonyl)amino-2,5-dichlorophenyl]acetyl]-(3S)-ethoxy-(2S)-pyrrolidinyl]-5-oxazolyl]propionic acid methyl ester

[Chem. 285]

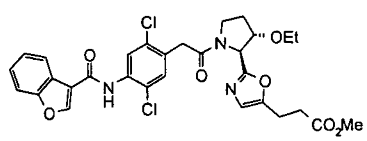

$^1$H-NMR ($CDCl_3$) δ: 1.19-1.25 (3H, m), 2.04-2.36 (2H, m), 2.64 (2H, t, J = 7. 6 Hz), 2.97-2.99 (2H, m), 3.50-3.87 (total 9H, series of m, including 3H, s, at δ 3.69), 4.05-4.06 and 4.20-4.21 (total 1H, each m), 5.05 and 5.28 (total 1H, each s), 6.70 and 6.73 (total 1H, each s), 7.38-7.50 (3H, m), 7.58-7.62 (1H, m), 8.03-8.06 (1H, m), 8.22-8.23 (1H, m), 8.29 (1H, s), 8.66 and 8.70 (total 1H, each s).
MS (LC-ESI) m/z: 614 ($M^+$+1).

3-[2-[1-[4-(3-Benzo[b]furanylcarbonyl)amino-2,5-dichlorophenyl]acetyl]-(3S)-ethoxy-(2S)-pyrrolidinyl]-5-oxazolyl]propionic acid

[Chem. 286]

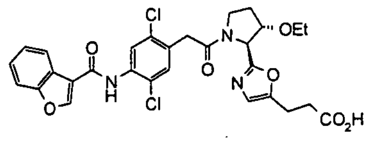

$^1$H-NMR (DMSO-$d_6$) δ: 1.13-1.19 (3H, m), 2.04-2.25 (2H, m), 2.53-2.60 (2H, m), 2.83-2.91 (2H, m), 3.49-3.97 (6H, m), 4.02-4.03 and 4.25-4.26 (total 1H, each m), 4.98 and 5.27 (total 1H, each s), 6. 79 and 6.90 (total 1H, each s), 7.37-7.45 (2H, m), 7.52 and 7.60 (total 1H, each s), 7.70-7.79 (2H, m), 8.08-8.10 (1H, m), 8.82 (1H, s), 10.02-10.03 (1H, m), 12.32 (1H, broad s).
IR (ATR) $cm^{-1}$: 1641, 1508, 1300, 1119, 1103, 1080, 748.
MS (LC-ESI) m/z: 600 ($M^{+}$+1).
Anal. Calcd for $C_{29}H_{27}Cl_2N_3O_7 \cdot 1/4H_2O$: C, 57.58; H, 4.58; N, 6.95; Cl, 11.72.
Found: C, 57.83; H, 4.50; N, 6.73; Cl, 11.34.

Example 103

3-[2-[1-[[7-Fluoro-2-(5-fluoro-2-methylphenyl)amino-6-benzoxazolyl]acetyl]-(3S)-ethoxy-(2S)-pyrrolidinyl]-5-oxazolyl]propionic acid methyl ester
[Chem. 287]

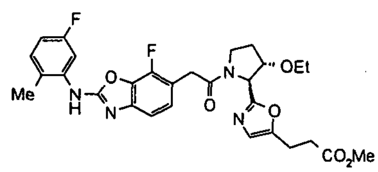

$^1$H-NMR ($CDCl_3$) δ: 1.18-1.28 (3H, m), 2.04-2.39 (5H, m, including 3H, s, at δ 2.30), 2.58-2.66 (2H, m), 2.90-2.99 (2H, m), 3.49-3.88 (total 9H, series of m), 4.04-4.05 and 4.19-4.20 (total 1H, each m), 5. 06 and 5.26 (total 1H, each s), 6.65-6.77 (2H, m), 7.03-7.36 (4H, m), 8.06-8.11 (1H, m). MS (LC-ESI) m/z: 569 ($M^{+}$+1).
3-[2-[1-[[7-Fluoro-2-(5-fluoro-2-methylphenyl)amino-6-benzoxazolyl]acetyl]-(3S)-ethoxy-(2S)-pyrrolidinyl]-5-oxazolyl]propionic acid
[Chem. 288]

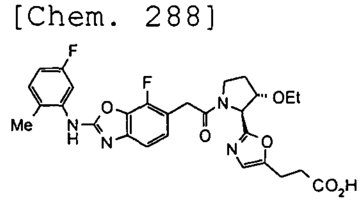

$^1$H-NMR (DMSO-$d_6$) δ: 1.12-1.18 (3H, m), 1.99-2.34 (5H, m, including 3H, s, at δ 2.29), 2. 52-2. 57 (2H, m), 2.81-2.89 (2H, m), 3.45-3.65 (3H, m), 3.72-3.92 (3H, m), 4.00-4.01 and 4.22-4.23 (total 1H, each m), 4.95 and 5.28 (total 1H, each s), 6.77-7.27 (total 5H, series of m), 7.92 (1H, dd, J = 11.4, 2.6 Hz).
IR (ATR) $cm^{-1}$: 1637, 1576, 1281, 1103, 1068, 802.
MS (LC-ESI) m/z: 555 ($M^{+}$+1)

Example 104

3-[2-[1-[[2-(2-Methylphenyl)amino-6-benzoxazolyl]acetyl]-(3S)-ethoxyl-(2S)-pyrrolidinyl]-5-oxazolyl]propionic acid methyl ester

[Chem. 289]

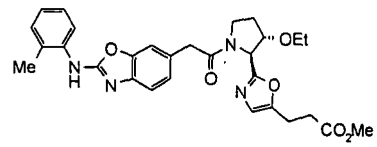

$^1$H-NMR ($CDCl_3$) δ: 1.08 and 1.21 (total 3H, each t, J = 7.1 Hz), 2.04-2.25 (2H, m), 2.35 and 2.37 (total 3H, each s), 2.58-2.67 (2H, m), 2.91-3.00 (2H, m), 3.28-3.81 (total 9H, series of m), 4.05-4.06 and 4.09-4.10 (total 1H, each m), 5.01 and 5.29 (total 1H, each s), 6.70 and 6.71 (total 1H, each s), 6.99-7.41 (total 7H, series of m), 8.07-8.14 (1H, m).
MS (LC-ESI) m/z: 533 ($M^+$+1).

3-[2-[1-[[2-(2-Methylphenyl)amino-6-benzoxazolyl]acetyl]-(3S)-ethoxy-(2S)-pyrrolidinyl]-5-oxazolyl]propion ic acid

[Chem. 290]

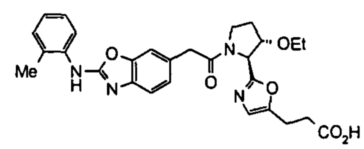

$^1$H-NMR (DMSO-$d_6$) δ: 1.05-1.19 (3H, m), 1.99-2.20 (2H, m), 2.30 (3H, s), 2.51-2.56 (2H, m), 2.82-2.86 (2H, m), 3.44-3.58 (3H, m), 3.66-3.82 (3H, m), 3.97-3.98 and 4.16-4.17 (total 1H, each m), 4.96 and 5.22 (total 1H, each s), 6.76 and 6.84 (total 1H, each s), 6.90-7.09 (2H, m), 7.20-7.31 (4H, m), 7.80-7.83 (1H, m), 9.61 (1H, broad s), 12.33 (1H, broad s).
IR (ATR) $cm^{-1}$: 1635, 1572, 1439, 1244, 1103.
MS (LC-ESI) m/z: 519 ($M^+$+1).
Anal. Calcd for $C_{28}H_{30}N_4O_6 \cdot 1/4H_2O$: C, 64.29; H, 5. 88; N, 10.71. Found: C, 64.46; H, 6.20; N, 10.24.

Example 105

3-[2-[1-[[7-Fluoro-2-(5-fluoro-2-methoxyphenyl)amino-6-benzoxazolyl]acetyl]-(3S)-ethoxy-(2S)-pyrrolidinyl]-5-oxazolyl]propionic acid methyl ester

[Chem. 291]

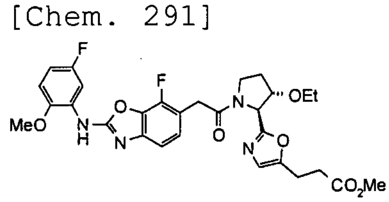

$^1$H-NMR (CDCl$_3$) δ: 1.16-1.28 (3H, m), 2.04-2.38 (2H, m), 2.59-2.66 (2H, m), 2.91-2.99 (2H, m), 3.49-3.69 (5H, m), 3.75-3.91 (7H, m), 4.03-4.04 and 4.18-4.19 (total 1H, each m), 5.06 and 5.26 (total 1H, each s), 6.66-6.73 (2H, m), 6.80-6.84 (1H, m), 7.04-7.27 (2H, m), 7.76 (1H, broad s), 8.26-8.30 (1H, m).
MS (LC-ESI) m/z: 585 ($M^+$+1).

3-[2-[1-[[7-Fluoro-2-(5-fluoro-2-methoxyphenyl)amino-6-benzoxazolyl]acetyl]-(3S)-ethoxy-(2S)-pyrrolidinyl]-5-oxazolyl]propionic acid

[Chem. 292]

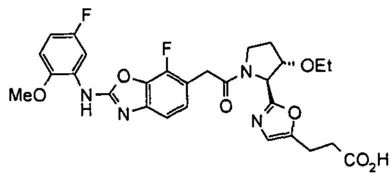

$^1$H-NMR (DMSO-d$_6$) δ: 1.12-1.18 (3H, m), 1.99-2.22 (2H, m), 2.52-2.56 (2H, m), 2.81-2.89 (2H, m), 3.45-3.66 (3H, m), 3.72-3.93 (6H, m, including 3H, s, at δ 3.85), 4.00-4.01 and 4.22-4.23 (total 1H, each m), 4.95 and 5.29 (total 1H, each s), 6.76-6.91 (2H, m), 6.98-7.13 (2H, m), 7.24 (1H, t, J = 8.3 Hz), 8.16-8.20 (1H, m), 10.17 (1H, broad s).
IR (ATR) cm$^{-1}$: 1641, 1577, 1444, 1217, 1068.
MS (LC-ESI) m/z: 571 ($M^+$+1).
Anal. Calcd for $C_{28}H_{28}F_2N_4O_7$: C, 58.94; H, 4.95; N, 9.82; F, 6.66.
Found: C, 58.99; H, 5.33; N, 9.34; F, 6.59.

Example 106

3-[2-[1-[[2-(2-Methoxyphenyl)amino-6-benzoxazolyl]acetyl]-(3S)-ethoxy-(2S)-pyrrolidinyl]-5-oxazolyl]propionic acid methyl ester

[Chem. 293]

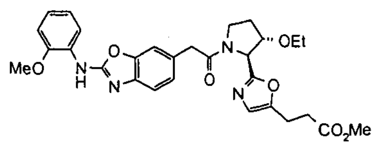

$^1$H-NMR (CDCl$_3$) δ: 1.06-1.09 and 1.19-1.23 (total 3H, each m), 2.04-2.30 (2H, m), 2.57-2.65 (2H, m), 2.88-2.98 (2H, m), 3.26-3.81 (total 9H, series of m), 3.94 (3H, s), 4.03-4.04 and 4.09-4.10 (total 1H, each m), 5.02 and 5.27 (total 1H, each s), 6.69 and 6.71 (total 1H, each s), 6.91-6.93 (1H, m), 7.00-7.13 (3H, m), 7.25-7.26 and 7.32-7.33 (total 1H, each m), 7.37-7.44 (1H, m), 7.62 and 7.66 (total 1H, each s), 8.38-8.42 (1H, m).
MS (LC-ESI) m/z: 549 ($M^+$+1).

3-[2-[1-[[2-(2-Methoxyphenyl)amino-6-benzoxazolyl]acetyl]-(3S)-ethoxy-(2S)-pyrrolidinyl]-5-oxazolyl]propionic acid

[Chem. 294]

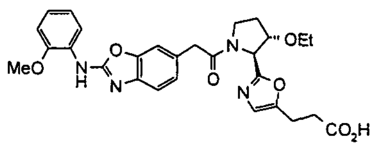

$^1$H-NMR (DMSO-$d_6$) δ: 1.05-1.08 and 1.10-1.14 (total 3H, eachm), 1.99-2.20 (2H, m), 2.52-2.56 (2H, m), 2.82-2.86 (2H, m), 3.44-3.58 (3H, m), 3.66-3.79 (3H, m), 3.85 (3H, s), 3.97-3.98 and 4.16-4.17 (total 1H, each m), 4.96 and 5.23 (total 1H, each s), 6. 76 and 6. 84 (total 1H, each s), 6.92-7.08 (4H, m), 7.21-7.33 (2H, m), 8.16-8.18 (1H, m), 9.53 (1H, broad s), 12.36 (1H, broad s).
IR (ATR) $cm^{-1}$: 1637, 1574, 1437, 1252, 1103, 746.
MS (LC-ESI) m/z: 535 ($M^+$+1).
Anal. Calcd for $C_{28}H_{30}N_4O_7 \cdot 1/4H_2O$: C, 62.39; H, 5.70; N, 10.39. Found: C, 62.26; H, 5.68; N, 10.13.

Example 107

3-[2-[1-[4-(2-Benzoxazolyl)amino-3-chlorophenyl]acetyl-(4S)-methoxy-(2S)-pyrrolidinyl]-5-thyiazolyl]propionic acid methyl ester
[Chem. 295]

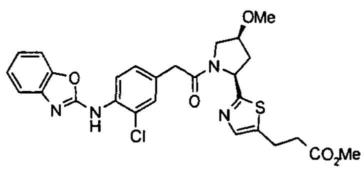

[4-(2-Benzoxazolyl)amino-3-chlorphenyl]acetic acid (Reference Example 1 or 2) (206mg, 0.68mmol), 3-[2-[(4S)-methoxy-(2S)-pyrrolidinyl]-5-thiazolyl]propio nic acid methyl ester (Example 1) (184mg, 0.68mmol), EDC · HCl (196mg, 1.02mmol), HOBt (92mg, 0.68mmol) and triethylamine (142μl, 1.02mmol) were dissolved in N,N-dimethylformamide (7ml), and the solution was stirred at room temperature for 18 hours. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by preparative thin-layer chromatography (thickness 2mmx2 sheets, developing solvent: chloroform: methanol= 20:1) to obtain the title compound (307mg, 81%) as a pale yellow oily substance.
$^1$H-NMR ($CDCl_3$) δ: 2.31-2.72 (4H, m), 3.07-3.22 (5H, m), 3.55-3.85 (7H, m), 4.02-4.08 (1H, m), 5.30-5.32 and 5.46-5.49 (total 1H, each m), 7.13-7.54 (8H, m), 8.44 and 8.54 (total 1H, each d, J = 8.3 Hz).
MS (LC-ESI) m/z : 555 ($M^+$+1).
3-[2-[1-[4-(2-Benzoxazolyl)amino-3-chlorophenyl]acetyl-(4S)-methoxy-(2S)-pyrrolidinyl]-5-thiazolyl]propionic acid
[Chem. 296]

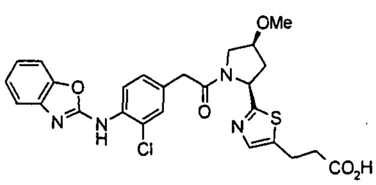

3-[2-[1-[4-(2-Benzoxazolyl)amino-3-chlorophenyl]acetyl-(4S)-methoxy-(2S)-pyrrolidinyl]-5-thiazolyl]propionic

acid methyl ester (307mg, 0.55mmol) was dissolved in tetrahydrofuran (3ml), a 0.5N aqueous sodium hydroxide solution (2.2ml, 1.10mmol) was added, and the mixture was stirred at room temperature for 18 hours. 1N hydrochloric acid was added to the reaction solution to weakly acidic, followed by extraction with ethyl acetate. The extract was washed with an aqueous saturated sodium chloride solution, and dried with anhydrous sodium sulfate, and a solvent was removed under reduced pressure. The resulting residue was purified by column chromatography (developing solvent: chloroform: methanol = 30:1-10:1) using silica gel to obtain the title compound (121mg, 40%) as a colorless amorphous solid.

$^1$H-NMR (DMSO-$d_6$) δ: 2.38-2.59 (4H, m), 2.94-3.09 (total 5H, m, including 3H, s, at δ 3.08), 3.42-3.93 (4H, m), 4.03-4.09 (1H, m), 5.24-5.26 and 5.51-5.53 (total 1H, each m), 7.09-7.48 (8H, m), 7.88 and 7.95 (total 1H, each d, J = 8.3 Hz), 9.99 (1H, broad s), 12.23 (1H, broad s).
IR (ATR) $cm^{-1}$: 1635, 1585, 1566, 1236, 742.
MS (LC-ESI) m/z : 541 ($M^+$+1).
Anal. Calcd for $C_{26}H_{25}ClN_4O_5S \cdot 1/2H_2O$: C, 56.78; H, 4.76; N, 10.19; Cl, 6.45; S, 5.83.
Found: C, 56.87; H, 4.55; N, 10.09; Cl, 6.87; S, 5.61.

Compounds of Example 108 to Example 114 shown below were produced by the same method as that of Example 107.

Example 108

3-[2-[1-[4-(2-Benzoxazolyl)amino-5-chloro-2-fluorophenyl]acetyl-(4S)-methoxy-(2S)-pyrrolidinyl]-5-thiazolyl] propionic acid methyl ester

[Chem. 297]

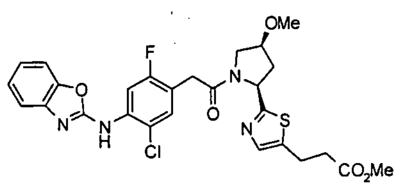

$^1$H-NMR ($CDCl_3$) δ: 2.36-2.70 (4H, m), 3.07-3.21 (5H, m), 3.66-3.89 (total 7H, m, including 3H, s, at δ 3.68), 4.06-4.11 (1H, m), 5.38-5.40 and 5.47-5.49 (total 1H, each m), 7.18-7.57 (7H, m), 8.42 and 8.51 (total 1H, each d, J = 11.8 Hz).
MS (LC-ESI) m/z : 573 ($M^+$+1).

3-[2-1-[4-(2-Benzoxazolyl)amino-5-chloro-2-fluorophenyl]acetyl-(4S)-methoxy-(2S)-pyrrolidinyl]-5-thiazolyl] propionic acid

[Chem. 298]

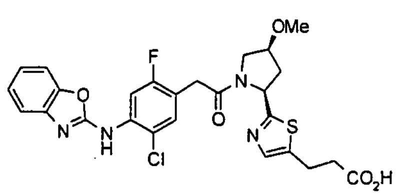

$^1$H-NMR (DMSO-$d_6$) δ: 2.70-2.90 (4H, m), 3.25-3.34 (2H, m), 3.40 (3H, s), 3.71-4.27 (4H, m), 4.35-4.42 (1H, m), 5.53-5.56 and 5.82-5.84 (total 1H, each m), 7.44-7.65 (3H, m), 7.75-7.82 (3H, m), 8.36-8.39 (1H, m), 10.25 (1H, broad s), 12.29 (1H, broad s).
IR (ATR) $cm^{-1}$: 1624, 1570, 1406, 1244, 756, 742.
MS (LC-ESI) m/z : 559 ($M^+$+1).
Anal. Calcd for $C_{26}H_{24}ClFN_4O_5S \cdot 1/2H_2O$: C, 54.98; H, 4.44; N, 9.86; Cl, 6.24; F, 3.34; S, 5.65.

Found: C, 55.05; H, 4.33; N, 9.72; Cl, 6.25; F, 3.38; S, 5.63.

Example 109

3-[2-[1-[4-(2-Benzoxazolyl)amino-2,5-dichlorophenyl]acetyl-(4S)-methoxy-(2S)-pyrrolidinyl]-5-thiazolyl]propionic acid methyl ester

[Chem. 299]

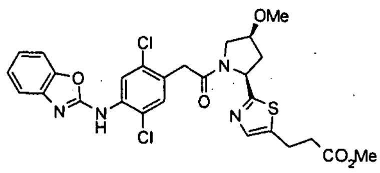

$^1$H-NMR ($CDCl_3$) δ: 2.33-2.74 (4H, m), 3.05-3.22 (5H, m), 3.49-3.91 (total 7H, m, including 3H, s, at δ 3.68), 4.06-4.13 (1H, m), 5.41-5.44 and 5.48-5.51 (total 1H, each m), 7.16-7.21 (1H, m), 7.24-7.57 (6H, m), 8.67 and 8.75 (total 1H, each s).

MS (LC-ESI) m/z : 589 ($M^+$+1).

3-[2-[1-[4-(2-Benzoxazolyl)amino-2,5-dichlorophenyl)acetyl-(4S)-methoxy-(2S)-pyrrolidinyl]-5-thiazolyl]propionic acid

[Chem. 300]

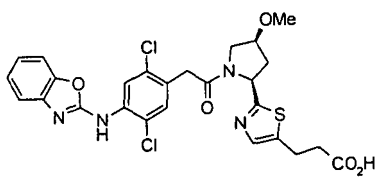

$^1$H-NMR (DMSO-$d_6$) δ: 2.36-2.59 (4H, m), 2.95-3.04 (2H, m), 3.10 and 3.11 (total 3H, each s), 3.49-3.97 (4H, m), 4.05-4.12 (1H, m), 5.23-5.26 and 5.51-5.53 (total 1H, each m), 7.13-7.25 (2H, m), 7.35-7.55 (4H, m), 8.22-8.27 (1H, m). IR (ATR) $cm^{-1}$: 1633, 1583, 1240, 1082, 742.

MS (LC-ESI) m/z : 575 ($M^+$+1).

Anal. Calcd for $C_{26}H_{24}Cl_2N_4O_5S \cdot 1/2H_2O$: C, 53.43; H, 4.31; N, 9.59; Cl, 12.13; S, 5.49.

Found: C, 53.23; H, 4.19; N, 9.46; Cl, 12.13; S, 5.53.

Example 110

3-[2-[1-[4-(2-Benzothiazolyl)amino-3-chlorophenyl]acetyl-(4S)-methoxy-(2S)-pyrrolidinyl]-5-thiazolyl]propionic acid methyl ester

[Chem. 301]

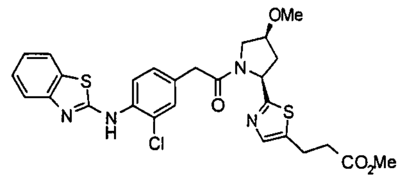

$^1$H-NMR ($CDCl_3$) δ: 2.30-2.72 (4H, m), 3.07-3.24 (5H, m), 3.55-3.89 (7H, m), 4.02-4.08 (1H, m), 5.30-5.33 and 5.47-5.49 (total 1H, each m), 7.06-7.82 (8H, m), 8.27-8.41 (1H, m). MS (LC-ESI) m/z : 571 ($M^+$+1).

3-[2-[1-[4-(2-Benzothiazolyl)amino-3-chlorophenyl]acetyl-(4S)-methoxy-(2S)-pyrrolidinyl]-5-thiazolyl]propionic acid

[Chem. 302]

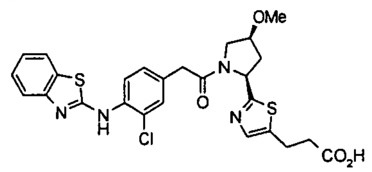

$^1$H-NMR (DMSO-$d_6$) δ: 2.36-2.59 (4H, m), 2.93-3.03 (2H, m), 3.06 and 3.08 (total 3H, each s), 3.40-3.92 (4H, m), 4.02-4.07 (1H, m), 5.22-5.25 and 5.50-5.52 (total 1H, each m), 7.06-7.53 (6H, m), 7.77 (1H, d, J = 8.1 Hz), 8.05 and 8.13 (total 1H, each d, J = 8.1 Hz).
IR (ATR) $cm^{-1}$: 1525, 1441, 1425, 1404, 754.
MS (LC-ESI) m/z : 557 ($M^+$+1).
Anal. Calcd for $C_{26}H_{25}ClN_4O_4S_2 \cdot 1/2H_2O$: C, 55.16; H, 4.63; N, 9.90; Cl, 6.26; S, 11.33.
Found: C, 55.19; H, 4.50; N, 9.81; Cl, 6.46; S, 11.26.

Example 111

3-[2-[1-[4-(2-Benzothiazolyl)amino-5-chloro-2-fluorophenyl]acetyl-(4S)-methoxy-(2S)-pyrrolidinyl]-5-thiazolyl] propionic acid methyl ester

[Chem. 303]

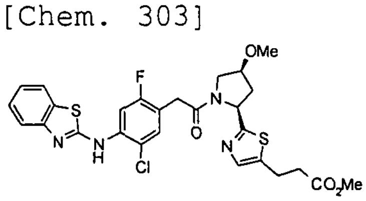

$^1$H-NMR ($CDCl_3$) δ: 2.33-2.78 (4H, m), 3.05-3.26 (5H, m), 3.40-3.97 (total 7H, m, including 3H, s, at δ 3.68), 4.04-4.19 (1H, m), 5.37-5.56 (1H, m), 7.20-7.82 (7H, m), 8.40 and 8.52 (total 1H, each d, J = 11.8 Hz).
MS (LC-ESI) m/z : 589 ($M^+$+1).

3-[2-[1-[4-(2-Benzothiazolyl)amino-5-chloro-2-fluorophenyl]acetyl-(4S)-methoxy-(2S)-pyrrolidinyl]-5-thiazolyl] propionic acid

[Chem. 304]

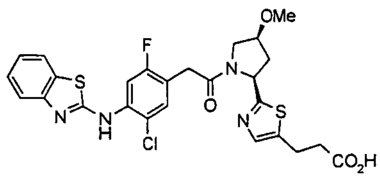

$^1$H-NMR (DMSO-$d_6$) δ: 2.35-2.58 (4H, m), 2.93-3.03 (2H, m), 3.08 (3H, s), 3.38-3.95 (4H, m), 4.03-4.10 (1H, m), 5.22-5.25 and 5.50-5.52 (total 1H, each m), 7.18 (1H, t, J = 7.6 Hz), 7.29-7.33 (2H, m), 7.46 (1H, d, J = 7.6 Hz), 7.61 (1H, d, J = 7.6 Hz), 7.82 (1H, d, J = 7.8 Hz), 8.32-8.34 (1H, m) .
IR (ATR) $cm^{-1}$: 1527, 1404, 1190, 754.
MS (LC-ESI) m/z : 575 ($M^+$+1) .
Anal. Calcd for $C_{26}H_{24}ClFN_4O_4S_2 \cdot 3/4H_2O$: C, 53.06; H, 4.37; N, 9.52; Cl, 6.02; F, 3.23; S, 10.90.
Found: C, 53.15; H, 4.15; N, 9.40; Cl, 6.31; F, 3.33; S, 10.89.

Example 112

3-[2-[1-[4-(2-Benzothiazolyl)amino-2,5-dichlorophenyl]acetyl-(4S)-methoxy-(2S)-pyrrolidinyl]-5-thiazolyl]propionic acid methyl ester
[Chem. 305]

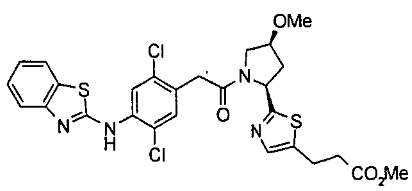

$^1$H-NMR ($CDCl_3$) δ: 2.33-2.78 (4H, m), 3.05-3.29 (5H, m), 3.48-3.94 (7H, m), 4.07-4.13 (1H, m), 5.41-5.44 and 5.49-5.51 (total 1H, each m), 7.20-7.46 (4H, m), 7.51-7.82 (3H, m), 8.59 and 8.70 (total 1H, each s).
MS (LC-ESI) m/z : 605 ($M^+$+1).
3-[2-[1-[4-(2-Benzothiazolyl)amino-2,5-dichlorophenyl]acetyl-(4S)-methoxy-(2S)-pyrrolidinyl]-5-thiazolyl]propionic acid
[Chem. 306]

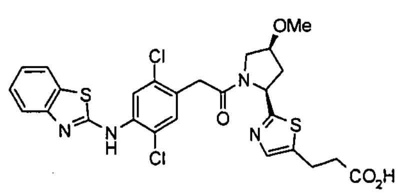

$^1$H-NMR (DMSO-$d_6$) δ: 2.35-2.58 (4H, m), 2.94-3.03 (2H, m), 3.08 and 3.10 (total 3H, each s), 3.47-3.96 (4H, m), 4.04-4.11 (1H, m), 5.22-5.25 and 5.49-5.51 (total 1H, each m), 7.17 (1H, t, J = 7.6 Hz), 7.30-7.60 (4H, m), 7.81 (1H, d, J = 7.8 Hz), 8.44 and 8.50 (total 1H, each broad s).
IR (ATR) $cm^{-1}$: 1520, 1080, 754. MS (LC-ESI) m/z : 591 ($M^+$+1).
Anal. Calcd for $C_{26}H_{24}Cl_2N_4O_4S_2 \cdot 3/4H_2O$: C, 51.61; H, 4.25; N, 9.26; Cl, 11.72; S, 10.60.
Found: C, 51.69; H, 4.03; N, 9.24; Cl, 11.99; S, 10.69.

Example 113

3-[2-[1-[4-(5-Fluoro-2-benzothiazolyl)amino-5-chloro-2-fluorophenyl]acetyl-(4S)-methoxy-(2S)-pyrrolidinyl]-5-thiazolyl]pripionic acid methyl ester

[Chem. 307]

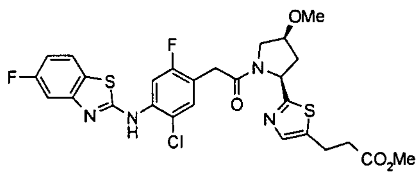

$^1$H-NMR ($CDCl_3$) δ: 2.33-2.78 (4H, m), 3.06-3.28 (5H, m), 3.41-3.90 (7H, m), 4.05-4.14 (1H, m), 5.38-5.40 and 5.47-5.50 (total 1H, each m), 6.94-7.05 (1H, m), 7.25-7.73 (5H, m), 8.34 and 8.45 (total 1H, each d, J = 11.8 Hz).
MS (LC-ESI) m/z : 607 ($M^+$+1).

3-[2-[1-[4-(5-Fluoro-2-benzothiazolyl)amino-5-chloro-2-fluorophenyl]acetyl-(4S)-methoxy-(2S)-pyrrolidinyl]-5-thiazolyl]propionic acid

[Chem. 308]

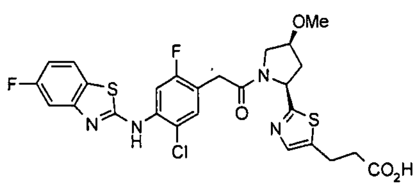

$^1$H-NMR (DMSO-$d_6$) δ: 2.38-2.58 (4H, m), 2.94-3.03 (2H, m), 3.08 (3H, s), 3.38-3.95 (4H, m), 4.03-4.10 (1H, m), 5.21-5.24 and 5.50-5.52 (total 1H, each m), 7.04 (1H, t, J = 8.3 Hz), 7.30-7.34 (1H, m), 7.45-7.48 (2H, m), 7.82-7.85 (1H, m), 8.21-8.29 (1H, m), 10.24 (1H, broad s), 12.22 (1H, broad s).
IR (ATR) $cm^{-1}$: 1720, 1523, 1423, 1404, 1119, 850, 627
MS (LC-ESI) m/z : 593 ($M^+$+1).
Anal. Calcd for $C_{26}H_{23}ClF_2N_4O_4S_2 \cdot 3/4H_2O$: C, 51.48; H, 4.07; N, 9.24; Cl, 5.84; F, 6.26; S, 10.57.
Found: C, 51.55; H, 3.89; N, 9.23; Cl, 6.12; F, 6.27; S, 10.64.

Example 114

3-[2-[1-[4-(4-Choro-2-benzothiazolyl)amino-3-chlorophenyl]acetyl-(4S)-methoxy-(2S)-pyrrolidinyl]-5-thiazolyl] propionic acid methyl ester

[Chem. 309]

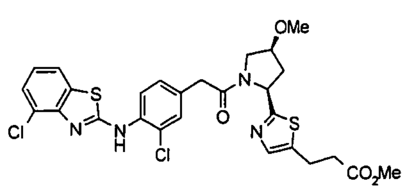

$^1$H-NMR ($CDCl_3$) δ: 2.31-2.72 (4H, m), 3.04-3.26 (5H, m), 3.55-3.89 (7H, m), 4.02-4.10 (1H, m), 5.31-5.34 and 5.47-5.50 (total 1H, each m), 7.10-7.44 (6H, m), 7.54-7.57 (1H, m), 8.17 and 8.29 (total 1H, each d, J = 8.6 Hz).
MS (LC-ESI) m/z : 605 ($M^+$+1).

3-[2-[1-[4-(4- Chloro- 2- benzothiazolyl) amino- 3- chlorophenyl] acetyl-(4S)-methoxy-(2S)-pyrrolidinyl]- 5- thiazolyl]propionic acid

[Chem. 310]

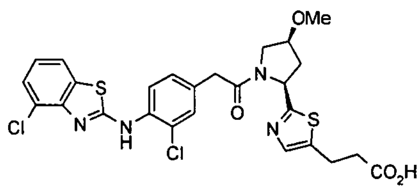

$^1$H-NMR (DMSO-$d_6$) δ: 2.38-2.59 (4H, m), 2.90-3.03 (2H, m), 3.08 and 3.09 (total 3H, each s), 3.42-3.93 (4H, m), 4.03-4.08 (1H, m), 5.24-5.27 and 5.52-5.54 (total 1H, each m), 7.09-7.47 (5H, m), 7.76-7.78 (1H, m), 8.12 and 8.20 (total 1H, each d, J = 8.3 Hz), 10.25 (1H, broad s), 12.25 (1H, broad s).
IR (ATR) $cm^{-1}$: 1525, 1412, 1302, 1093, 766.
MS (LC-ESI) m/z : 591 ($M^+$+1).
Anal. Calcd for $C_{26}H_{24}Cl_2N_4O_4S_2 \cdot 1/4H_2O$: C, 52.39; H, 4.14; N, 9.40; Cl, 11.90; S, 10.76.
Found: C, 52.19; H, 4.10; N, 9.18; Cl, 12.16; S, 10.62.

Example 115

3-[2-[1-[4-(2-Benzothiazolyl)amino-2,5-dichlorophenyl]acetyl-(3S)-ethoxy-(2S)-pyrrolidinyl]-5-thiazolyl]propionic acid

[Chem. 311]

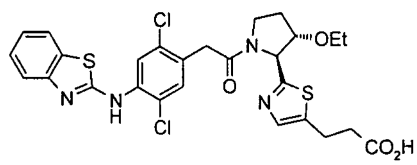

3-[2-((3S)-ethoxy-(2S)-pyrrolidinyl)-5-thiazolyl]propionic acid methyl ester (Example 91) (130mg, 0.46mmol), [4-(2-benzothiazolyl)amino-2,5-dichlorophenyl]acetic acid (Reference Example 2) (161mg, 0.46mnmol), EDC · HCl (131mg, 0.69mmol), HOBt (62mg, 0.46mmol) and triethylamine (96μl, 0.69mmol) were dissolved in N,N-dimethylformamide (7ml), and the solution was stirred at room temperature for 16 hours. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by preparative thin-layer chromatography (thickness 2mmx2 sheets, developing solvent: chloroform: methanol = 20:1) to obtain 3-[2-[1-[4-(2-benzothiazolyl)amino-2,5-dichlorophenyl]acetyl-(3S)-ethoxy-(2S)-pyrrolidinyl]-5-thiazolyl]propionic acid methyl ester (224mg) as a pale yellow oily substance. The resulting ester compound (224mg) was dissolved in tetrahydrofuran (3ml), a 0.5N aqueous sodium hydroxide solution (1.5ml, 0.75mmol) was added, and the mixture was stirred at room temperature for 18 hours. 1N hydrochloric acid was added to the reaction solution to weakly acidic, and a precipitate solid was collected by filtration. The collected solid was dissolved again in ethyl acetate, washed with an aqueous saturated sodium chloride solution, and dried with anhydrous sodium sulfate, and a solvent was removed under reduced pressure. The resulting residue was purified by preparative thin-layer chromatography (thickness 0.5mn×4 sheets, developing solvent: chloroform: methanol = 15:1) to obtain the title compound (57mg, 21%, 2 steps) as a colorless amorphous solid.

$^1$H-NMR (DMSO-$d_6$) δ: 1.13-1.18 (3H, m), 1.98-2.13 (2H, m), 2.53-2.59 (2H, m), 2.96-3.05 (2H, m), 3.41-3.92 (6H, m), 4.13-4.16 (1H, m), 5.18 and 5.43 (total 1H, each s), 7.17 (1H, t, J = 7.7 Hz), 7.32 (1H, t, J = 7.3 Hz), 7.42-7.59

(3H, m), 7.81 (1H, d, J = 7.8 Hz), 8.48-8.51 (1H, m), 10.10 (1H, broad s), 12.27 (1H, broad s).
IR (ATR) $cm^{-1}$: 1520, 1379, 1169, 1078, 754.
MS (LC-ESI) m/z : 605 ($M^{+}$+1). Anal. Calcd for $C_{27}H_{26}Cl_2N_4O_4S_2 \cdot 1/2H_2O$: C, 52.77; H, 4.43; N, 9.12; Cl, 11.54; S, 10.44. Found: C, 52.36; H, 4.37; N, 9.03; Cl, 11.97; S, 10.47.

Example 116

(1-tert-butoxycarbonyl-(3S)-hydroxy-(2S)-pyrrolidinyl)carboxylic acid benzyl ester
[Chem. 312]

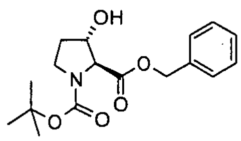

(3S)-hydroxy-L-proline (commercially available, Across) (4.98g, 37.98mmol) was suspended in tetrahydrofuran (70ml), a 1N aqueous sodium hydroxide solution (57ml, 57.00mmol) was added, and the mixture was stirred. To the reaction solution was added a solution (20ml) of di-tert-butyl bicarbonate (9.95g, 45.57mmol) in tetrahydrofuran, and the mixture was stirred at room temperature for 19 hours. 1N hydrochloric acid (60ml, 60.00mmol) was added to the reaction solution to weakly acidic, followed by extraction with ethyl acetate. The extract was washed with an aqueous saturated sodium chloride solution, and dried with anhydrous sodium sulfate, and a solvent was removed under reduced pressure. The resulting residue was dissolved in N,N-dimethylformamide (80ml), potassium bicarbonate (7. 61g, 75.96mmol) and benzyl bromide (4.52ml, 37.98mmol) were added, and the mixture was stirred at room temperature for 15 hours. 1N hydrochloric acid was added to the reaction solution to weakly acidic, followed by extraction with ethyl acetate. The extract was washed with an aqueous saturated sodium chloride solution, and dried with anhydrous sodium sulfate, and a solvent was removed under reduced pressure. The resulting residue was purified by flash column chromatography (Biotage flash chromatography system, column size: 40M, eluting solvent: n-hexane: ethyl acetate= 3:1-1: 1) to obtain the title compound (11.06g, 91%, 2 steps) as a colorless solid.
$^{1}$H-NMR ($CDCl_3$) δ: 1.33 and 1.46 (total 9H, each s), 1.88-1.92 (1H, m), 2.04-2.21 (2H, m), 3.57-3.66 (2H, m), 4.21 and 4.35 (total 1H, each s), 4.43 (1H, s), 5.08-5.27 (2H, m), 7.34-7.36 (5H, m).
[(3S)-acetoxy-1-tert-butoxycarbonyl-(2S)-pyrrolidinyl]carboxylic acid benzyl ester
[Chem. 313]

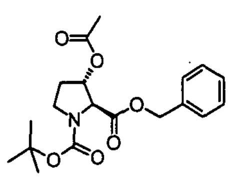

(1-tert-butoxycarbonyl-(3S)-hydroxy-(2S)-pyrrolidinyl)carboxylic acid benzyl ester (11.06g, 34.42mmol) was dissolved in methylene chloride (100ml), pyridine (4.18ml, 51.62mmol) and acetic anhydride (4.22ml, 44.74mmol) were added, and the mixture was stirred at room temperature for 2 days. To the reaction solution was added 1N hydrochloric acid, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent: n-hexane: ethyl acetate= 4:1) using silica gel to obtain the title compound (12.98g, 104%) as a colorless oily substance.
$^{1}$H-NMR ($CDCl_3$) δ: 1.33 and 1.48 (total 9H, each s), 1.96-2.22 (5H, m), 3.47-3.74 (2H, m), 4.29 and 4.44 (total 1H, each s), 5.12-5.30 (3H, m), 7.34-7.36 (5H, m).
[(3S)-acetoxy-1-tert-butoxycarbonyl-(2S)-pyrrolidinyl]carboxylic acid
[Chem. 314]

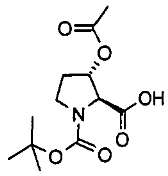

[(3S)-acetoxy-1-tert-butoxycarbonyl-(2S)-pyrrolidinyl]carboxylic acid benzyl ester (12.98g, 35.72mmol) was dissolved in methanol (120ml), and hydrogenation was performed at room temperature for 18 hours in the presence of 10% palladium hydroxide/carbon (2.6g, 20wt%). A catalyst was removed by suction filtration, and the filtrate was concentrated to obtain the title compound (9.70g, 99%) as a colorless oily substance.

$^1$H-NMR ($CDCl_3$) δ: 1.44 and 1.51 (total 9H, each s), 2.03-2.24 (5H, m), 3.31-3.76 (2H, m), 4.28 and 4.40 (total 1H, each s), 5.35 and 5.58 (total 1H, each s).
MS (LC-ESI) m/z : 174 ($M^+$+1-Boc).

5-[[(3S)-acetoxy-1-tert-butoxycarbonyl-(2S)-pyrrolidinyl]carbonylamino]levulinic acid methyl ester

[Chem. 315]

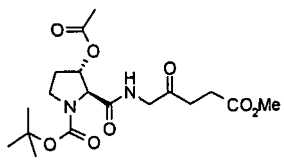

[(3S)-acetoxy-1-tert-butoxycarbonyl-(2S)-pyrrolidinyl]carboxylic acid (9.70g, 35.49mmol), 5-amino-levulinic acid methyl ester hydrochloride (6.45g, 35.49mmol), EDC · HCl (10.21g, 53.24mmol), HOBt (4.80g, 35.49mmol) and triethylamine (24.74ml, 177.47mmol) were dissolved in N,N-dimethylformamide (200ml), and the solution was stirred at room temperature for 4 days. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by column chromatography (eluting solvent: chloroform: methanol= 20:0-20:1) using silica gel to obtain the title compound (10.36g, 73%) as a pale yellow oily substance.

$^1$H-NMR ($CDCl_3$) δ: 1.44 and 1.49 (total 9H, each s), 2.01-2.21 (total 5H, m, including 3H, s, at δ 2.09), 2.66-2.75 (4H, m), 3.48-3.68 (total 5H, m, including 3H, s, at δ 3.68), 4.18-4.38 (3H, m), 5.29 and 5.40 (total 1H, each broad s).
MS (LC-ESI) m/z : 423 ($M^+$+Na).

3-[2-[(3S)-acetoxy-1-tert-butoxycarbonyl-(2S)-pyrrolidinyl]-5-thiazolyl]propionic acid methyl ester

[Chem. 316]

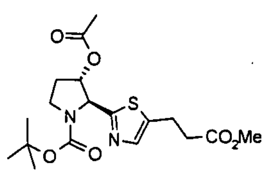

5-[[(3S)-acetoxy-1-tert-butoxycarbonyl-(2S)-pyrrol idinyl]carbonylamino]levulinic acid methyl ester (10.36g, 25.87mmol) was dissolved in toluene (130ml), a Lawesson's reagent (11.51g, 28.46mmol) was added, and the mixture was stirred to 90°C for 3 hours. After allowing to cool to room temperature, water was added to the reaction solution, followed by extraction with ethyl acetate. The extract was washed with an aqueous saturated sodium chloride solution, and dried with anhydrous sodium sulfate, and a solvent was removed under reduced pressure. The resulting residue

was purified by column chromatography (eluting solvent: chloroform: methanol = 100:1-30:1) using silica gel to obtain the title compound (2.66g, 26%) as a brown oily substance.

$^1$H-NMR ($CDCl_3$) δ: 1.36 and 1.49 (total 9H, each s), 2.02-2.13 (total 4H, m, including 3 H, s, at δ 2.10), 2.31-2.33 (1H, m), 2.66 (2H, t, J = 7.4Hz), 3.10-3.15 (2H, m), 3.51-3.69 (total 5H, m, including 3H, s, at δ 3.69), 5. 02 and 5.15 (total 1H, each s), 5.40 and 5.45 (total 1H, each s), 7.42 (1H, s). MS (LC-ESI) m/z : 399 ($M^+$+1).

3-[2-[(3S)-acetoxy-(2S)-pyrrolidinyl]-5-thiazolyl]propionic acid methyl ester

[Chem. 317]

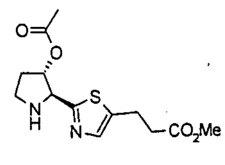

3-[2-[(3S)-acetoxy-1-tert-butoxycarbonyl-(2S)-pyrr olidinyl]-5-thiazolyl]propionic acid methyl ester (2.66g, 6.68mmol) was dissolved in methylene chloride (10ml), trifluoroacetic acid (10ml) was added, and the mixture was stirred at room temperature for 2 hours. An aqueous saturated sodium bicarbonate solution was added to the reaction solution to weakly alkaline, followed by extraction with chloroform. The extract was washed with an aqueous saturated sodium chloride solution, and dried with anhydrous sodium sulfate, and a solvent was removed under reduced pressure to obtain the title compound (1.60g, 80%) as a brown oily substance.

$^1$H-NMR ($CDCl_3$) δ: 1.86-1.95 (1H, m), 2.04-2.16 (total 4H, m, including 3H, s, at δ 2.08), 2.66 (2H, t, J = 7.5 Hz), 3.08-3.14 (2H, m), 3.17-3.27 (2H, m), 3.65-3.74 (total 4H, m, including 3H, s, at δ 3.70), 4.53 (1H, s), 5.39-5.41 (1H, m), 7.42 (1H, s).

MS (LC-ESI) m/z : 299 ($M^+$+1).

3-[2-[(3S)-acetoxy-1-[4-(2-benzoxazolyl)amino-3-chlorophenyl]acetyl-(2S)-pyrrolidinyl]-5-thiazolyl]propionic acid methyl ester

[Chem. 318]

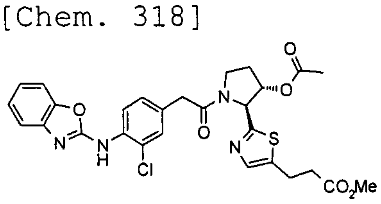

[4-(2-Benzoxazolyl)amino-3-chlorophenyl]acetic acid (see Reference Example 1 or 2) (240mg, 0.79mmol), 3-[2-[(3S)-acetoxy-(2S)-pyrrolidinyl]-5-thiazolyl]propio nic acid methyl ester (237mg, 0.79mmol), EDC · HCl (228mg, 1.19mmol), HOBt (107mg, 0.79mmol) and triethylamine (166μl, 1.19mmol) were dissolved in N, N-dimethylformamide (7ml), and the solution was stirred at room temperature for 17 hours. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by preparative thin-layer chromatography (thickness 2mmx2 sheets, developing solvent: chloroform: methanol= 20:1) to obtain the title compound (364mg, 79%) as a pale brown amorphous solid.

$^1$H-NMR ($CDCl_3$) δ: 2.09-2.67 (total 7H, series of m, including 3H, s, at δ 2.09), 3.09-3.14 (2H, m), 3. 55-3. 90 (total 7H, series of m, including 3H, s, at δ 3.68), 5.23 and 5.43 (total 1 H, each s), 5.33 and 5.50 (total 1H, each d, J = 4.2 Hz), 7.12-7.53 (8H, m), 8.43-8.52 (1H, m).

MS (LC-ESI) m/z : 583 ($M^+$+1).

3-[2-[1-[4-(2-Benzoxazolyl)amino-3-chlorophenyl]ac etyl-(3S)-hydroxy-(2S)-pyrrolidinyl]-5-thiazolyl]propion ic acid

[Chem. 319]

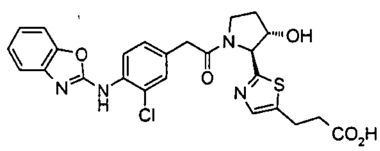

3-[2-[(3S)-acetoxy-1-[4-(2-benzoxzolyl)amino-3-chl orophenyl]acetyl-(2S)-pyrrolidinyl]-5-thiazolyl]propioni c acid methyl ester (364mg, 0.62mmol) was dissolved in tetrahydrofuran (3ml), a 0.5N aqueous sodium hydroxide solution (2.25ml, 1.25mmol) was added, and the mixture was stirred at room temperature for 5 hours. 1N hydrochloric acid was added to the reaction solution to weakly acidic, followed by extraction with ethyl acetate. The extract was washed with an aqueous saturated sodium chloride solution, and dried with anhydrous sodium sulfate, and a solvent was removed under reduced pressure. The resulting residue was purified by preparative thin-layer chromatography (thickness 2mmx2 sheets, developing solvent: chloroform: methanol = 10:1) to obtain the title compound (188mg, 57%) as a colorless amorphous solid.

$^1$H-NMR (DMSO-$d_6$) δ: 1.85-2.15 (2H, m), 2.52-2.60 (2H, m), 2.96-3.04 (2H, m), 3.59-3.80 (4H, m), 4.33-4.34 (1H, m), 5.06 and 5.21 (total 1H, each s), 5.50 (1H, broad s), 7.07-7.56 (8H, m), 7.87 and 7.94 (total 1H, each d, J = 8.3 Hz).
IR (ATR) $cm^{-1}$: 1633, 1585, 1566, 1236, 741.
MS (LC-ESI) m/z : 527 ($M^+$+1).
Anal. Calcd for $C_{25}H_{23}ClN_4O_5S \cdot 3/4H_2O$: C, 55.55; H, 4.57; N, 10.37; Cl, 6.56; S, 5.93.
Found: C, 55.68; H, 4.40; N, 10.24; Cl, 6.64; S, 6.13.

Compounds of Example 117 to Example 123 shown below were produced by the same method as that of Example 116.

Example 117

3-[2-[(3S)-acetoxy-1-[4-(2-benzoxazolyl)amino-5-chloro-2-fluorophenyl]acetyl-(2S)-pyrrolidinyl]-5-thiazolyl] propionic acid methyl ester

[Chem. 320]

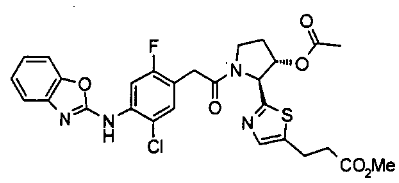

$^1$H-NMR ($CDCl_3$) δ: 2.08-2.72 (total 7H, series of m, including3H, s, at δ 2.10), 3.09-3.20 (2H, m), 3.62-3.91 (total 7H, series of m, including 3H, s, at δ 3.69), 5.28 and 5.42 (total 1 H, each s), 5.38 and 5.51 (total H, each d, J = 4.2 Hz), 7.16-7.62 (7H, m), 8.40-8.50 (1H, m).
MS (LC-ESI) m/z : 601 ($M^+$+1).

3-[2-[1-[4-(2-Benzoxazolyl)amino-5-chloro-2-fluorophenyl]acetyl-(3S)-hydroxy-(2-S)-pyrrolidinyl]-5-thiazolyl] propionic acid

[Chem. 321]

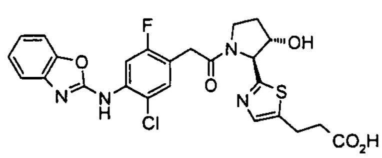

$^1$H-NMR (DMSO-$d_6$) δ: 1.85-2.16 (2H, m), 2.54-2.60 (2H, m), 2.97-3.05 (2H, m), 3.59-3.85 (4H, m), 4.34-4.35 (1H, m), 5.05 and 5.21 (total 1H, each s), 5.52 and 5.65 (total 1H, each broad s), 7.13-7.56 (7H, m), 8.06-8.08 (1H, m).
IR (ATR) $cm^{-1}$: 1729, 1612, 1571, 1539, 1457, 1440, 1425, 1404, 1242, 1184, 735.
MS (LC-ESI) m/z : 545 ($M^+$+1).
Anal. Calcd for $C_{25}H_{22}ClFN_4O_5S \cdot 1/4H_2O$: C, 54.65; H, 4.13; N, 10.20; Cl, 6.45; F, 3.46; S, 5.84.
Found: C, 54.67; H, 4.15; N, 9.94; Cl, 6.64; F, 3.56; S, 5.84.

Example 118

3-[2-[(3S)-acetoxy-1-[4-(2-benzoxazolyl)amino-2,5-dichlorophenyl]acetyl-(2S)-pyrrolidinyl]-5-thiazolyl]propionic acid methyl ester
[Chem. 322]

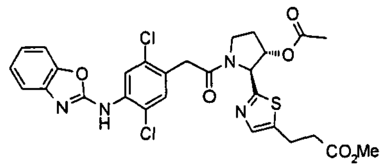

$^1$H-NMR ($CDCl_3$) δ: 2.10-2.70 (total 7H, series of m, including 3H, s, at δ 2.10), 3.09-3.16 (2H, m), 3.67-3.92 (total 7H, series of m, including 3H, s, at δ 3.69), 5.32 and 5.45 (total 1 H, each s), 5.39 and 5.52 (total 1H, each d, J = 4.2 Hz), 7.17-7.62 (7H, m), 8.68 and 8.74 (total 1H, each s).
MS (LC-ESI) m/z : 617 ($M^+$+1).
3-[2-[1-[4-(2-Benzoxazolyl)amino-2,5-dichlorophenyl]acetyl-(3S)-hydroxy-(2S)-pyrrolidinyl]-5-thiazolyl]propionic acid
[Chem. 323]

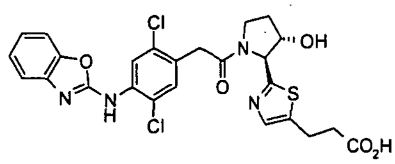

$^1$H-NMR (DMSO-$d_6$) δ: 1.91-2.15 (2H, m), 2.52-2.59 (2H, m), 2.97-3.05 (2H, m), 3.60-3.92 (4H, m), 4.35-4.36 (1H, m), 5.06 and 5.20 (total 1H, each s), 5. 52 (1H, broad s), 7.13-7.25 (2H, m), 7.44-7.56 (4H, m), 8.22-8.26 (1H, m).
IR (ATR) $cm^{-1}$: 1631, 1583, 1525, 1234, 1082, 742.
MS (LC-ESI) m/z : 561 ($M^+$+1).
Anal. Calcd for $C_{25}H_{22}Cl_2N_4O_5S \cdot 1/4H_2O$: C, 53.06; H, 4.01; N, 9.90; S, 5.67.
Found: C, 53.00; H, 4.22; N, 9.28; S, 5.46.

Example 119

3-[2-[(3S)-acetoxy-1-[4-(2-benzothiazolyl)amino-3-chlorophenyl]acetyl-(2S)-pyrrolidinyl]-5-thiazolyl]propionic acid methyl ester
[Chem. 324]

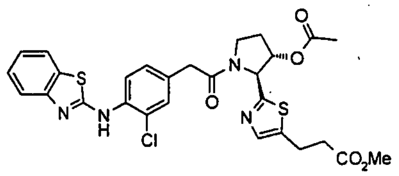

$^1$H-NMR ($CDCl_3$) δ: 2.05-2.71 (total 7H, series of m, including 3H, s, at δ 2.09), 3.05-3.14 (2H, m), 3.55-3.94 (total 7H, series of m, including 3H, s, at δ 3.68), 5.23 and 5.43 (total 1 H, each s), 5.33 and 5.51 (total 1H, each d, J = 4.2 Hz), 7.06-7.82 (8H, m), 8.29-8.40 (1H, m). MS (LC-ESI) m/z : 599 ($M^+$+1).

3-[2-[1-[4-(2-Benzothiazolyl)amino-3-chlorophenyl]acetyl-(3S)-hydroxy-(2S)-pyrrolidinyl]-5-thiazolyl]propionic acid

[Chem. 325]

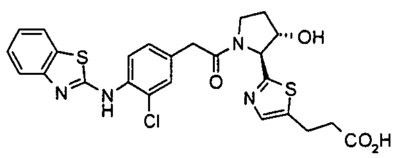

$^1$H-NMR (DMSO-$d_6$) δ: 1.84-2.13 (2H, m), 2.52-2.60 (2H, m), 2.96-3.04 (2H, m), 3.59-3.80 (4H, m), 4.32-4.33 (1H, m), 5.06 and 5.21 (total 1H, each s), 5.50 (1H, broad s), 7.06-7.56 (6H, m), 7.78 (1H, d, J = 7.8 Hz), 8.05-8.14 (1H, m).
IR (ATR) $cm^{-1}$: 1527, 1441, 1404, 1171, 754.
MS (LC-ESI) m/z : 543 ($M^+$+1).
Anal. Calcd for $C_{25}H_{23}ClN_4O_4S_2 \cdot 1/2H_2O$: C, 54.39; H, 4.38; N, 10.15; Cl, 6.42; S, 11.62.
Found: C, 54.23; H, 4.32; N, 9.89; Cl, 6.26; S, 11.47.

Example 120

3-[2-[(3S)-acetoxy-1-[4-(2-benzothiazolyl)amino-5-chloro-2-fluorophenyl]acetyl-(2S)-pyrrolidinyl]-5-thiazolyl] propionic acid methyl ester

[Chem. 326]

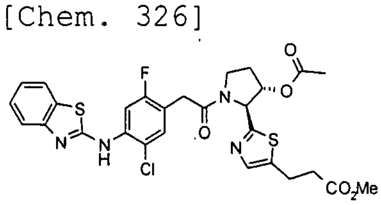

$^1$H-NMR ($CDC1_3$) δ: 2.08-2.31 (4H, m), 2.55-2.71 (3H, m), 3.09-3.20 (2H, m), 3.62-3.96 (total 7H, series of m, including 3H, s, at δ 3.69), 5.28-5.52 (2H, m), 7.22-7.82 (7H, m), 8.40-8.52 (1H, m).
MS (LC-ESI) m/z : 617 ($M^+$+1).

3-[2-[1-[4-(2-Benzothizolyl)amino-5-chloro-2-fluorophenyl]acetyl-(3S)-hydroxy-(2S)-pyrrolidinyl]-5-thiazolyl] propionic acid

[Chem. 327]

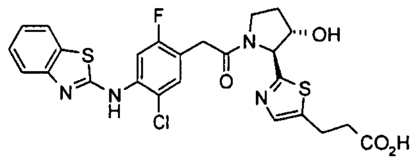

$^1$H-NMR (DMSO-$d_6$) δ: 1.92-2.15 (2H, m), 2.52-2.61 (2H, m), 2.97-3.05 (2H, m), 3.59-3.85 (4H, m), 4.34 (1H, s), 5.05 and 5.20 (total 1H, each s), 5.51-5.52 and 5.65-5.66 (total 1H, eachm), 7.17-7.21 (1H, m), 7.32-7.56 (3H, m), 7.62 (1H, d, J = 7.6 Hz), 7.83 (1H, d, J = 7.8 Hz), 8.34 (1H, broad s).
IR (ATR) $cm^{-1}$: 1712, 1621, 1525, 1404, 1188, 752. MS (LC-ESI) m/z : 561 ($M^+$+1).
Anal. Calcd for $C_{25}H_{22}ClFN_4O_4S_2 \cdot 3/4H_2O$: C, 52.26; H, 4.12; N, 9.75; Cl, 6.17; F, 3.31; S, 11.16.
Found: C, 52.26; H, 4.05; N, 9.57; Cl, 6.30; F, 3.43; S, 11.13.

Example 121

3-[2-[(3S)-acetoxy-1-[4-(2-benzothiazolyl)amino-2, 5-dichlorophenyl]acetyl-(2S)-pyrrolidinyl]-5-thiazolyl]propionic acid methyl ester
[Chem. 328]

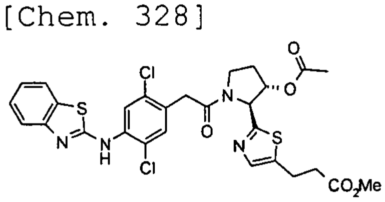

$^1$H-NMR ($CDCl_3$) δ: 2.06-2.71 (total 7H, series of m), 3.05-3.20 (2H, m), 3.65-3.95 (total 7H, series of m, including 3H, s, at δ 3.69), 5.32-5.53 (2H, m), 7.10-7.82 (total 8H, series of m).
MS (LC-ESI) m/z : 633 ($M^+$+1).
3-[2-[1-[4-(2-Benzothiazolyl)amino-2,5-dichlorophenyl]acetyl-(3S)-hydroxy-(2S)-pyrrolidinyl]-5-thiazolyl]propionic acid
[Chem. 329]

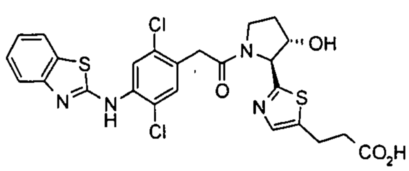

$^1$H-NMR (DMSO-$d_6$) δ: 1.87-2.17 (2H, m), 2.52-2.60 (2H, m), 2.97-3.05 (2H, m), 3.61-3.91 (4H, m), 4.35 (1H, s), 5.06 and 5.20 (total 1H, each s), 5.52 and 5.65 (total 1H, each broad s), 7.18 (1H, t, J = 7.6 Hz), 7.31-7.35 (1H, m), 7.42 and 7.45 (total 1H, each s), 7.55-7.61 (2H, m), 7.82 (1H, d, J=7.6Hz), 8.50 (1H, broad s).
IR (ATR) $cm^{-1}$: 1712, 1635, 1518, 1379, 1169, 1078, 752.
MS (LC-ESI) m/z : 577 ($M^+$+1).
Anal. Calcd for $C_{25}H_{22}Cl_2N_4O_4S_2 \cdot 1/2H_2O$: C, 51.20; H, 3. 95; N, 9.55; Cl, 12.09; S, 10.93.
Found: C, 51.14; H, 3.95; N, 9.38; Cl, 11.92; S, 10.89.

Example 122

3-[2-[(3S)-acetoxy-1-[4-(3-benzo[b]thiophenylcarbonyl)amino-5-chloro-2-fluorophenyl]acetyl]-(2S)-pyrrolidinyl]-5-thiazolyl]propionic acid methyl ester

[Chem. 330]

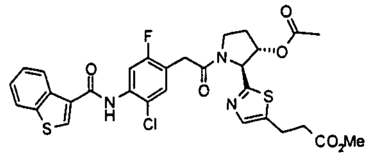

$^1$H-NMR ($CDCl_3$) δ: 1.76-2.41 (total 7H, series of m, including 3H, s, at δ 1.80), 2.78-2.89 (2H, m), 3.32-3.61 (total 7H, series of m, including 3H, s, at δ 3.38), 4.99 and 5.12 (total 1H, each s), 5.09 and 5.20 (total 1H, each d, J = 4.2 Hz), 7.02-7.24 (4H, m), 7.60-8.18 (total 5H, series of m). MS (LC-ESI) m/z: 644 ($M^+$+1).

3-[2-[1-[4-(3-Benzo[b]thiophenylcarbonyl)amino-5-chloro-2-fluorophenyl]acetyl]-(3S)-hydroxy-(2S)-pyrrolidinyl]-5-thiazolyl]propionic acid

[Chem. 331]

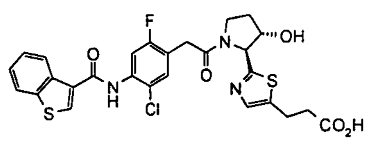

$^1$H-NMR (DMSO-$d_6$) δ: 1.87-2.18 (2H, m), 2.52-2.61 (2H, m), 2.97-3.06 (2H, m), 3.60-3.89 (4H, m), 4.34-4.35 (1H, m), 5.06 and 5.22 (total 1H, each s), 5.53 and 5.67 (total 1H, each broad s), 7.39-7.59 (5H, m), 8.09-8.11 (1H, m), 8.43-8.46 (1H, m), 8.65 and 8.67 (total 1H, each m), 10.10 and 10.12 (total 1H, each s), 12.33 (1H, broad s).
IR (ATR) $cm^{-1}$: 1631, 1525, 1398, 1315, 1228, 762.
MS (LC-ESI) m/z: 588 ($M^+$+1).
Anal. Calcd for $C_{27}H_{23}ClFN_3O_5S_2 \cdot 1/2H_2O$: C, 54.31; H, 4.05; N, 7.04; Cl, 5.94; F, 3.18; S, 10.74. Found: C, 54.46; H, 3.92; N, 7.13; Cl, 5.97; F, 3.26; S, 10.93.

Example 123

3-[2-[(3S)-acetoxy-1-[4-(3-benzo[b]thiophenylcarbonyl)amino-2,5-dichlorophenyl]acetyl]-(2S)-pyrrolidinyl]-5-thiazolyl]propionic acid methyl ester

[Chem. 332]

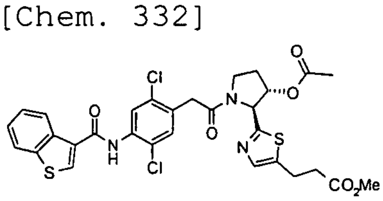

$^1$H-NMR ($CDCl_3$) δ: 2.06-2.70 (total 7H, series of m, including 3H, s, at δ 2.10), 3.08-3.20 (2H, m), 3.65-3.92 (total 7H, series of m, including 3H, s, at δ 3.69), 5.33 and 5.45 (total 1H, each s), 5.39 and 5.53 (total 1H, each d, J = 4.2 Hz), 7.36-7.54 (4H, m), 7.91-7.93 (1H, m), 8.08 and 8.09 (total 1H, each s), 8.28 and 8.30 (total 1H, each s), 8.47-8.49 (1H, m), 8.68 and 8.74 (total 1H, each s).
MS (LC-ESI) m/z: 660 ($M^+$+1).

3-[2-[1-[4-(3-Benzo[b]thiophenylcarbonyl)amino-2,5-dichlorophenyl]acetyl]-(3S)-hydroxy-(2S)-pyrrolidnyl]-5-thiazolyl]propionic acid

[Chem. 333]

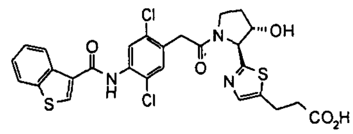

$^1$H-NMR (DMSO-$d_6$) δ: 1.88-2.18 (2H, m), 2.52-2.60 (2H, m), 2.97-3.06 (2H, m), 3.45-3.96 (4H, m), 4.35-4.36 (1H, m), 5.07 and 5.22 (total 1H, each s), 5.53 and 5.67 (total 1H, each broad s), 7.44-7.52 (3H, m), 7.57 and 7.62 (total 1H, each s), 7.73 and 7.78 (total 1H, each s), 8.08-8.11 (1H, m), 8.43-8.46 (1H, m), 8.64 and 8.65 (total 1H, each s), 10.14 and 10.16 (total 1H, each s), 12.30 (1H, broad s).

IR (ATR) $cm^{-1}$: 1631, 1500, 1375, 1080, 764.

MS (LC-ESI) m/z: 604 ($M^+$+1).

Anal. Calcd for $C_{27}H_{23}Cl_2N_3O_5S_2 \cdot 1/2H_2O$: C, 52.86; H, 3.94; N, 6.85; Cl, 11.56; S, 10.45.

Found: C, 52.73; H, 3.92; N, 6.84; Cl, 11.39; S, 10.40.

Compounds of Example 124 to Example 125 shown below were produced by the same method as that of Example 115 using 3-[2-(1-tert-butoxycarbonyl-(4R)-methoxy-(2S)-pyrrolidin yl)-5-thiazolyl]propionic acid methyl ester using in Example 24, and a carboxylic acid derivative shown in Reference Example 1 or 2.

Example 124

3-[2-[1-[4-(2-Benzoxazolyl)amino-2,5-dichloropheny 1]acetyl-(4R)-methoxy-(2S)-pyrrolidinyl]-5-thiazolyl]propionic acid methyl ester

[Chem. 334]

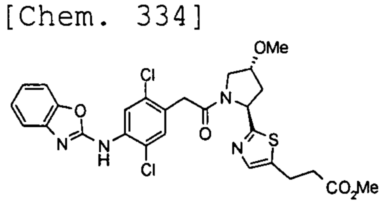

$^1$H-NMR ($CDCl_3$) δ: 2.31-2.39 (1H, m), 2.57-2.67 (3H, m), 3.09-3.15 (2H, m), 3. 34 and 3.35 (total 3H, each s), 3.44-3.86 (7H, m), 4.08-4.27 (1H, m), 5.37-5.40 and 5.48-5.51 (total 1H, each m), 7.16-7.20 (1H, m), 7.25-7.57 (6H, m), 8.65 and 8.73 (total 1H, each s).

MS (LC-ESI) m/z : 589 ($M^+$+1).

3-[2-[1-[4-(2-Benzoxazolyl)amino-2,5-dichloropheny 1]acetyl-(4R)-methoxy-(2S)-pyrrolidinyl]-5-thiazolyl]pro pionic acid

[Chem. 335]

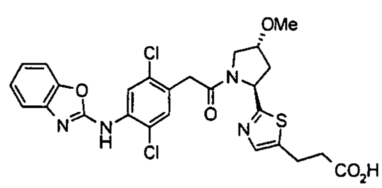

$^1$H-NMR (DMSO-$d_6$) δ: 2.26-2.60 (4H, m), 2.98 and 3.04 (total 2H, t, J=7.1 Hz), 3.26 and 3.30 (total 3H, each s), 3.56-3.88 (4H, m), 4.08-4.18 (1H, m), 5.23-5.27 and 5.48-5.52 (total 1H, each m), 7.13-7.25 (2H, m), 7.40-7.53 (4H, m), 8.21-8.26 (1H, m).
IR (ATR) $cm^{-1}$: 1633, 1583, 1240, 1082, 741.
MS (LC-ESI) m/z : 575 ($M^+$+1).
Anal. Calcd for $C_{26}H_{24}Cl_2N_4O_5S \cdot 1/4H_2O$: C, 53.84; H, 4.26; N, 9.66; Cl, 12.23; S, 5.53.
Found: C, 53.81; H, 4.42; N, 9.51; Cl, 12.15; S, 5.55.

Example 125

3-[2-[1-[4-(2-Benzothiazolyl)amino-2,5-dichlorophenyl]acetyl-(4R)-methoxy-(2S)-pyrrolidinyl]-5-thiazolyl]propionic acid methyl ester
[Chem. 336]

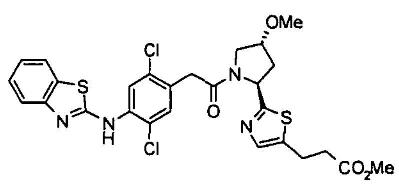

$^1$H-NMR ($CDCl_3$) δ: 2.30-2.42 (1H, m), 2.58-2.70 (3H, m), 3.09-3.17 (2H, m), 3.35 and 3.36 (total 3H, each s), 3.57-3.97 (total 7H, m, including 3H, s, at δ 3.69), 4.09-4.32 (1H, m), 5.37-5.40 and 5.49-5.57 (total 1H, each m), 7.21-7.54 (5H, m),7.62-7.82 (2H, m) 8.59 and 8.68 (total 1H, each s).
MS (LC-ESI) m/z : 605 ($M^+$+1).
3-[2-[1-[4-(2-Benzothiazolyl)amino-2,5-dichlorophenyl]acetyl-(4R)-methoxy-(2S)-pyrrolidinyl]-5-thiazolyl[propionic acid
[Chem. 337]

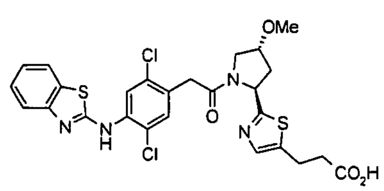

$^1$H-NMR (DMSO-$d_6$) δ: 2.26-2.60 (4H, m), 2.97-3.06 (2H, m), 3.26 and 3.30 (total 3H, each s), 3.57-4.18 (5H, m), 5.23-5.27 and 5.48-5.50 (total 1H, each m), 7.18 (1H, t, J = 7.5Hz), 7.31-7.61 (4H, m), 7.82 (1H, d, J = 7.6 Hz), 8.49-8.51 (1H, m).
IR (ATR) $cm^{-1}$: 1520, 1441, 1379, 1078, 754.
MS (LC-ESI) m/z : 591 ($M^+$+1).
Anal. Calcd for $C_{26}H_{24}Cl_2N_4O_4S_2$: C, 52.79; H, 4.09; N, 9.47; Cl, 11.99; S, 10.84.
Found: C, 52.71; H, 4.14; N, 9.18; Cl, 11.73; S, 10.95.

Example 126

1-Benzyloxycarbonyl-(2S)-carbamoylpyrrolidine
[Chem. 338]

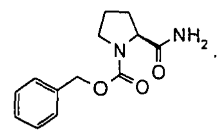

1-Benzyloxycarbonyl-L-proline (5.21g, 20.9mmol) and N-methylmorpholine (2.30ml, 20.9mmol) were dissolved in tetrahydrofuran (50ml), ethyl chloroformate (2.00ml, 20.9mmol) was added at 0˚C, and the mixture was stirred for 15 minutes. To the reaction solution was added 27% aqueous ammonia (2.71ml), and the mixture was stirred for 15 hours while a temperature was gradually raised to room temperature. The reaction solution was diluted with water, and extracted with ethyl acetate. The extract was washed with an aqueous saturated sodium chloride solution, and dried with anhydrous sodium sulfate, and a solvent was removed under reduced pressure to obtain the title compound (5.20g, 100%) as a colorless amorphous solid.

$^1$H-NMR ($CDCl_3$) δ: 1.89-2.41 (4H, m), 3.41-3.57 (2H, m), 4.23-4.38 (1H, m), 5.12-5.20 (2H, m), 5.58 (1H, broad s), 5.98 and 6.71 (total 1H, each broad s), 7.36 (5H, broad s). MS (LC-ESI) m/z: 249 ($M^+$+1).

1-Benzyloxycarbonyl-(2S)-cyanopyrrolidine

[Chem. 339]

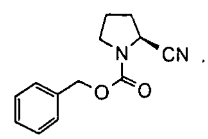

1-Benzyloxycarbonyl-(2S)-carbamoylpyrrolidine (5.20g, 20.9mmol) was dissolved in pyridine (60ml), p-toluenesulfonyl chloride (4.79g, 25.1mmol) was added, and the mixture was stirred at 100˚C for 3 hours. After allowing to cool to room temperature, 1N hydrochloric acid was added to the reaction solution to acidic, followed by extraction with ethyl acetate. The extract was washed with 1N hydrochloric acid and an aqueous saturated sodium chloride solution, and dried with anhydrous sodium sulfate, and a solvent was removed under reduced pressure. The resulting residue was purified by column chromatography (eluting solvent: n-hexane:ethyl acetate= 2:1) using silica gel to obtain the title compound (5.02g, 104%) as a colorless oily substance.

$^1$H-NMR ($CDCl_3$) δ: 2.05-2.29 (4H, m), 3.38-3.49 (1H, m), 3.56-3.64 (1H, m), 4.55-4.64 (1H, m), 5.17-5.22 (2H, m), 7.28-7.43 (5H, m).
MS (LC-ESI) m/z: 231 ($M^+$+1).

1-(1-Benzyloxycarbonyl-(2S)-pyrrolidinyl)-1 or 2H-tetrazole

[Chem. 340]

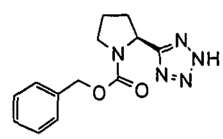

1-Benzyloxycarbonyl-(2S)-cyanopyrrolidine (5.02g, 21.8mmol), sodium azide (2.83g, 43.5mmol) and zinc bromide (2.45g, 10.9mmol) were dissolved in 2-propanol (30ml) and water (60ml), and the solution was heated to reflux for 15 hours. After allowing to cool to room temperature, 3N hydrochloric acid was added to the reaction solution to acidic, followed by extraction with ethyl acetate. The extract was washed with an aqueous saturated sodium chloride solution, and dried with anhydrous sodium sulfate, and a solvent was removed under reduced pressure to obtain the title compound (5.72g, 96%) as a colorless amorphous solid.

$^1$H-NMR ($CDCl_3$) δ: 2.06-2.39 (4H, m), 2.94-2.99 (1H, m), 3.51-3.54 (2H, m), 5.10-5.24 (3H, m), 7.30-7.37 (5H, m).
MS (LC-ESI) m/z: 274 ($M^+$+1).

3-[5-(1-Benzyloxycarbonyl-(2S)-pyrrolidinyl)-2H-tetrazol-2-yl]propionic acid ethyl ester

[Chem. 341]

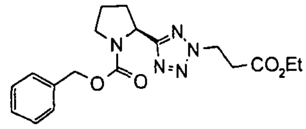

5-(1-Benzyloxycarbonyl-(2S)-pyrrolidinyl)-1 or 2H-tetrazole (3.41g, 12.5mmol) and potassium carbonate (2.59g, 18.7mmol) were dissolved in N,N-dimethylformamide (30ml), ethyl 3-bromopropionate (3.20ml, 25.0mmol) was added, and the mixture was stirred at 60˚C for 6 hours. After allowing to cool to room temperature, 1N hydrochloric acid was added to acidic, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent: n-hexane: ethyl acetate = 4:1-1:1) using silica gel to obtain the title compound (2H-tetrazole derivative, low polar isomer: 1.26g, 27%) as a colorless oily substance.

2H- isomer $^1$H-NMR ($CDCl_3$) δ: 1.24-1.28 (3H, m), 1.97-2.10 (3H, m), 2.29-2.34 (1H, m), 2.92 (1H, t, J = 7.1 Hz), 3.06 (1H, t, J = 7.1 Hz), 3.52-3.63 (1H, m), 3.71-3.75 (1H, m), 4.14-4.20 (2H, m), 4.75 and 4.86 (total 2H, each t, J= 7.1 Hz), 4.96-5.31 (3H, m), 7.12-7.36 (5H, m).
MS (LC-ESI) m/z: 374 ($M^+$+1).

3-[5-((2S)-pyrrolidinyl)-2H-tetrazol-2-yl]propioni c acid ethyl ester

[Chem. 342]

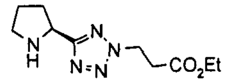

3-[5-(1-Benzyloxycarbonyl-(2S)-pyrrolidinyl)-2H-te trazol-2-yl]propionic acid ethyl ester (1.26g, 3.37mmol) was dissolved in methanol (30ml), 10% palladium hydroxide/ carbon (0.25g) was added, and hydrogenation was performed at room temperature for 16 hours. A catalyst was removed by filtration, and a solvent was removed under reduced pressure to obtain the title compound (810mg, 100%) as a colorless oily substance.
$^1$H-NMR ($CDCl_3$) δ: 1.26 (3H, t, J = 7.1 Hz), 2.10-2.25 (2H, m), 2.30-2.39 (1H, m), 2.46-2.55 (1H, m), 3.10 (2H, t, J = 7.1 Hz), 3.49-3.52 (2H, m), 4.18 (2H, q, J = 7.1 Hz), 4.92 (2H, t, J = 7.1 Hz), 5.04-5.07 (1H, m).
MS (LC-ESI) m/z: 240 ($M^+$+1).

3-[5-[1-[[4-[(3-Benzo[b]thiophenylcarbonyl)amino]-5-chloro-2-fluorophenyl]acetyl]-(2)-pyrrolidinyl]-(2H)-t etrazol-2-yl]propionic acid ethyl ester

[Chem. 343]

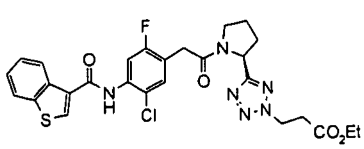

[4-[(3-Benzo[b]thiophenylcarbonyl)amino]-5-chloro-2-fluorophenyl]acetic acid (355mg, 0.98mmol), 3-[5-((2S)-pyrrolidinyl)-2H-tetrazol-2-yl]propionic acid ethyl ester (220mg, 0.92mmol), EDC · HCl (281mg, 1.47mmol), HOBt (198mg, 1.47mmol) and triethylamine (204μl, 1.46mmol) were dissolved in N,N-dimethylformamide (5ml), and the solution was stirred at room temperature for 4 days. The reaction solution was diluted with water, and extracted with ethyl acetate. The extract was washed with an aqueous saturated sodium chloride solution, and dried with anhydrous sodium sulfate, and a solvent was removed under reduced pressure. The resulting residue was purified by flash column chromatography (Biotage flash chromatography system, column size: 40M, eluting solvent: n-hexane: ethyl acetate= 1: 1-1:2) to obtain the title compound (511mg, 95%) as a pale yellow amorphous solvent.

$^1$H-NMR ($CDCl_3$) δ: 1.25 (3H, t, J = 7.1 Hz), 2.02-2.45 (4H, m), 3.04-3.09 (2H, m), 3.62-3.86 (4H, m), 4.17 (2H, q, J=7.1 Hz), 4.83-4.91 (2H, m), 5.38 and 5.50 (total 1H, each dd, J = 7.8, 2.1 Hz), 7.36-7.54 (3H, m), 7.90-7.93 (1H, m), 8.09 (1H, s), 8.33-8.49 (3H, m). MS (LC-ESI) m/z: 585 ($M^+$+1).

3-[5-[1-[[4-[(3-Benzo[b]thiophenylcarbonyl)amino]-5-chloro-2-fluorophenyl]acetyl]-(2S)-pyrrolidinyl]-2H-te trazol-2-yl]propionic acid

[Chem. 344]

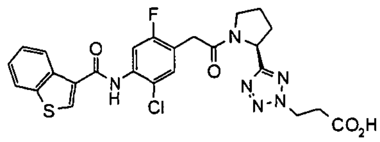

3-[5-[1-[[4-[(3-Benzo[b]thiophenylcarbonyl)amino]-5-chloro-2-fluorophenyl]acetyl]-(2S)-pyrrolidinyl]-2H-te trazol-2-yl]propionic acid ethyl ester (298mg, 0.51mmmol) was dissolved in tetrahydrofuran (3ml), a 0.25N aqueous sodium hydroxide solution (3.0ml, 0.75mmol) was added, and the mixture was stirred at room temperature for 30 minutes. 1N hydrochloric acid was added to the reaction solution to acidic, followed by extraction with ethyl acetate. The extract was washed with an aqueous saturated sodium chloride solution, and dried with anhydrous sodium sulfate, and a solvent was removed under reduced pressure. The resulting residue was purified by column chromatography (eluting solvent: chloroform methanol: 50:1-5:1) using silica gel to obtain the title compound (82mg, 29%), as a colorless amorphous solid.

$^1$H-NMR (DMSO-$d_6$) δ: 1.82-2.40 (4H, m), 2.97-3.04 (2H, m), 3.38-3.52 (1H, m), 3.70-3.88 (3H, m), 4.79 and 4. 85 (total 2H, each t, J = 6.6 Hz), 5.29-5.32 and 5. 60-5. 62 (total 1H, each m), 7.39-7.57 (4H, m), 8.10 (1H, d, J = 7.3 Hz), 8.44 (1H, d, J = 7.3 Hz), 8.65 (1H, s), 10.07-10.10 (1H, m), 12.56 (1H, broad s).
IR (ATR) $cm^{-1}$: 1620, 1518, 1402, 1217, 766.
MS (LC-ESI) m/z: 557 ($M^+$+1).
Anal. Calcd for $C_{25}H_{22}ClFN_6O_4S$ · 1/2$H_2O$: C, 53.05; H, 4.10; N, 14.85; Cl, 6.26, F; 3.36; S, 5.67.
Found: C, 53.01; H, 4.20; N, 14.70; Cl, 6.21; F, 3.31; S, 5.69.

Example 127

3-[2-[1-tert-butoxycarbonyl-(4S)-N,O-dimethylhydroxylamino-(2S)-pyrrolidinyl]-5-thiazolyl]propionic acid methyl ester

[Chem. 345]

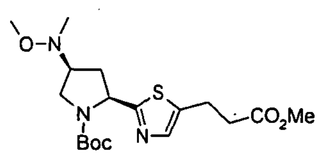

Sodium triacetoxyborohydride (1.75mg, 8.24mmol) was added to a solution (100ml) of 3-[2-[1-tert-butoxycarbonyl-4-oxo-(2S)-pyrrolidinyl]-5-t hiazolyl]propionic acid methyl ester (1.46g, 4.12mmol) and N,O-dimethylhydroxylamine hydrochloride (1.61g, 16.48mmol) in 1,2-dichloroethane, and the mixture was stirred for 14 hours. To the reaction solution

was added an aqueous saturated sodium bicarbonate solution, and the mixture was stirred for 0.5 hour, followed by extraction with methylene chloride. The combined extracts were washed with an aqueous saturated sodium chloride solution, and dried with anhydrous sodium sulfate, and a solvent was removed under reduced pressure. The resulting residue was purified by flash chromatography (Biotage flash chromatography system, column size: 40M, eluting solvent: n-hexane/ethyl acetate = 40% - 80%) to obtain the title compound (1.28g, 78%) as a yellow oily substance.

$^1$H-NMR ($CDCl_3$) δ: 1.23-1.52 (4H, m), 1.57 (9H, s), 2.52 and 2.56 (total 3H, each s, amide isomers), 2.65 (2H, t, J = 7.6 Hz), 3.05-3.28 (3H, m), 3.42 and 3.51 (total 3H, each s, amide isomers), 3.689-3.694 (total 3H, each s, amide isomers), 4.97-5.31 (1H, m), 7.34 and 7.38 (total 1H, each s, amide isomers).
MS (ESI) m/z : 400 ($M^+$+1).

3-[2-[(4S)-N,O-dimethylhydroxylamino-(2S)-pyrrolidinyl]-5-thiazolyl]propionic acid methyl ester dihydrochloride

[Chem. 346]

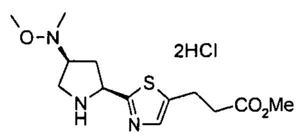

A 4N hydrochloric acid/dioxane solution (100ml) was added to 3-[2-[1-tert-butoxycarbonyl-(4S)-N,O-dimethylhydroxylami no-(2S)-pyrrolidinyl]-5-thiazolyl]propionic acid methyl ester (1.28g, 3.20mmol), and the mixture was stirred for 15 hours. The reaction solution was concentrated under reduced pressure to obtain the title compound as a brown oily substance. The present compound was used in the next reaction without further purification.
MS (ESI) m/z : 300 ($M^+$+1).

3-[2-[1-[5-Chloro-2-fluoro-4-(1-methyl-3-indolylcarbonylamino)phenylacetyl]-(4S)-N,O-dimethylhydroxylamino-(2S)-pyrrolidinyl]-5-thiazolyl]propionicacidmethyl ester

[Chem. 347]

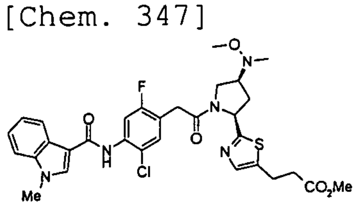

Triethylamine (0.37ml, 2.65mmol) was added to a solution (10ml) of 5-chloro-2-fluoro-4-(1-methyl-3-indolylcarbonylamino)phenylacetic acid (191mg, 0.53mmol), 3-[2-[(4S)-N,O-dimethylhydroxylamino-(2S)-pyrrolidinyl]-5-thiazolyl]propionic acid methyl ester dihydrochloride (188mg, 0.53mmol), EDC · HCl (152mg, 0.80mmol) andHOBt (107mg, 0.80mmol) in methylene chloride at room temperature, and the mixture was stirred for 2 days. The reaction solution was concentrated under reduced pressure, and the resulting residue was purified by flash chromatography (Biotage flash chromatography system, column size: 25S, eluting solvent: methanol/ethyl acetate = 0%-10%) to obtain the title compound (193mg, 57%) as a yellow oily substance.

$^1$H-NMR ($CDCl_3$) δ: 2.21-2.81 (7H, m), 3.03-3.35 (3H, m), 3.34-3.79 (8H, m), 3.84-4.01 (4H, m), 4.09-4.19 (1H, m), 5.13-5.64 (1H, m), 7.28-7.52 (5H, m), 7.80 (1H, s), 7.81 (0H, s), 8.11-8.17 (1H, m), 8.28 and 8.30 (total 1H, each s, amide isomers), 8.45 and 8.54 (total 1H, each d, J = 12.3 and 12.0 Hz respectively, amide isomers).
MS (ESI) m/z : 642 ($M^+$+1), 644 ($M^+$+3).

3-[2-[1-[5-Chloro-2-fluoro-4-(1-methyl-3-indolylcarbonylamino)phenylacetyl]-(4S)-N,O-dimethylhydroxylamino-(2S)-pyrrolidinyl]-5-thiazolyl]propionic acid

[Chem. 348]

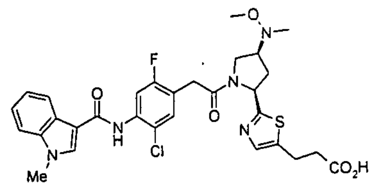

A 1N aqueous sodium hydroxide solution (2ml, 2mmol) was added to a solution of 3-[2-[1-[5-chloro-2-fluoro-4-(1-methyl-3-indolylcarbonyl amino)phenylacetyl]-(4S)-N,O-dimethylhydroxylamino-(2S)-pyrrolidinyl]-5-thiazolyl]propionic acid methyl ester (193mg, 0.30mmol) in a mixture of tetrahydrofuran/methanol (4ml/2ml) at room temperature, and the mixture was stirred for 17 hours. The reaction solution was concentrated under reduced pressure, and the resulting residue was adjusted to a pH 5 with 1N hydrochloric acid, followed by extraction with chloroform/methanol [10/1 (v/v)]. The combined extracts were washed with an aqueous saturated sodium chloride solution. The resulting organic layer was dried with anhydrous sodium sulfate, and a solvent was removed under reduced pressure. The resulting residue was subjected to thin-layer chromatography [eluting solvent: chloroform: methanol = 10/1 (v/v)] to obtain the title compound (152mg, 81%) as a colorless glassy solid, which was lyophilized from dioxane to obtain 152mg of a colorless glassy solid.

$^1$H-NMR ($CDCl_3$) δ: 2.19-2.39 (1H, m), 2.44-2.80 (6H, m), 3.03-3.16 (2H, m), 3.18-3.46 (3H, m), 3.47-3.82 (4H, m), 3.83-4.21 (4H, m), 5.33-5.58 (1H, m), 7.30-7.47 (5H, m), 7.80 and 7.82 (total 1H, each s, amide isomers), 8.14-8.07 (1H, m), 8.34-8.26 (1H, m), 8.47 (1H, dd, J = 11. 9, 2.3 Hz).
MS (ESI) m/z : 628 ($M^+$+1), 630 ($M^+$+3).
IR (ATR) $cm^{-1}$ : 3421, 3107, 3049, 2951, 2854, 1724, 1655, 1624, 1585.

Compounds of Example 128 to Example 132 shown below were produced by the same method as that of Example 127.

Example 128

3-[2 [-[1-[2,5- Dichloro- 4-(1- methyl- 3- indolylcarbonylamino) phenylacetyl]-(4S)-N, O- dimethylhydroxylamino-(2S)-pyrrolidinyl]-5-thiazolyl]propionic acid methyl ester

[Chem. 349]

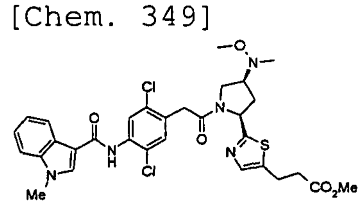

$^1$H-NMR ($CDCl_3$) δ: 2.13-2.41 (1H, m), 2.45-2.77 (6H, m), 3.04-3.33 (3H, m), 3.37-3.54 (4H, m), 3.58-4.04 (8H, m), 4.07-4.25 (1H, m), 5.30-5.61 (1H, m), 7.27-7.52 (5H, m), 7.75-7.83 (1H, m), 8.05-8.31 (2H, m), 8.83-8.71 (1H, m).
MS (ESI) m/z : 658 ($M^+$+1), 660 ($M^+$+3).

3-[3-[1-[2,5-Dichloro-4-(1-methyl-3-indolylcarbonylamino)phenylacetyl]-(4S)-N,O-dimethylhydroxylamino-(2S) -pyrrolidinyl]-5-thiazolyl]propionic acid

[Chem. 350]

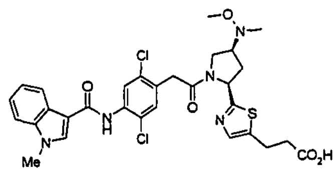

$^1$H-NMR ($CDCl_3$) δ: 2.20-2.39 (1H, m), 2.48-2.75 (6H, m), 3.03-3.35 (3H, m), 3.33-3.78 (6H, m), 3.79-4.25 (5H, m),
5.34-5.61 (1H, m), 7.29-7.51 (5H, m), 7.79 and 7.80 (1H, s), 8.07-8.14 (1H, m), 8.21 and 8.24 (total 1H, each s), 8.59 (1H, s), 8.73 and 8.74 (total 1H, each s, amide isomers).
MS (ESI) m/z : 644 ($M^+$+1), 646 ($M^+$+3)
IR (ATR) $cm^{-1}$ : 3423, 3111, 3047, 2952, 2856, 1724, 1653, 1568.

Example 129

3-[2-[1-[4-(3-Benzo[b]thiophenylcarbonylamino)-5-chloro-2-fluorophenylacetyl]-(4S)-N,O-dimethylhydroxylamino-(2S)-pyrrolidinyl]-5-thiazolyl]propionic acid methyl ester
[Chem. 351]

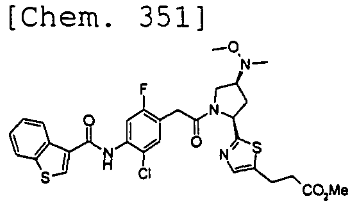

$^1$H-NMR ($CDCl_3$) δ: 2.27-2.81 (7H, m), 3.04-3.35 (3H, m), 3.37-3.54 (4H, m), 3.61-3.90 (5H, m), 3.94-4.24 (1H, m), 5.32-5.59 (1H, m), 7.28-7.57 (4H, m), 7.89-7.94 (1H, m), 8.08 and 8.09 (total 1H, each s, amide isomers), 8.26 and 8.29 (total 1H, each s, amide isomers), 8.45-8.50 (1H, m), 8.66 and 8.74 (total 1H, each s, amide isomers).
MS (ESI) m/z : 645 ($M^+$+1), 647 ($M^+$+3).
3-[2-[1-[4-(3-Benzo[b]thiophenylcarbonylamino)-5-chloro-2-fluorophenylacetyl]-(4S)-N,O-dimethylhydroxylamino-(2S)-pyrrolidinyl]-5-thiazolyl]propionic acid
[Chem. 352]

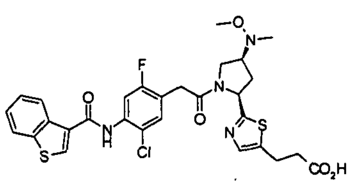

$^1$H-NMR ($CDCl_3$) δ: 2.17-2.41 (1H, m), 2.46-2.79 (6H, m), 3.04-3.34 (3H, m), 3.36-3.67 (4H, m), 3.70-3.78 (1H, m), 3.91-4.24 (1H, m), 5.34-5.43 (1H, m), 7.27-7.58 (4H, m), 7.91 (1H, d, J = 7.8 Hz), 8.09 and 8.10 (total 1H, each s, amide isomers), 8.34 (1H, d, J = 15.9 Hz), 8.41-8.50 (2H, m).
MS (ESI) m/z : 631 ($M^+$+1), 633 ($M^+$+3).
IR (ATR) $cm^{-1}$: 2956, 2889, 2856, 1720, 1645, 1585, 1516.
Anal. Calcd for $C_{29}H_{28}ClFN_4O_5S \cdot H_2O$: C, 53. 66; H, 4. 66; N, 8.63; S, 9.88.
Found: C, 53.88; H, 4.49; N, 8.34; S, 9.58.

Example 130

3-[2-[1-[4-(3- Benzo [b] thiophenylcarbonylamino)- 2,5- dichlorophenylacetyl]-(4S)-N, O- dimethylhydroxylamino-(2S)-pyrrolidinyl]-5-thiazolyl]propionic acid methyl ester

[Chem. 353]

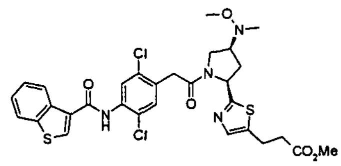

$^1$H-NMR ($CDCl_3$) δ: 2.49-2.69 (5H, m), 3.07-3.78 (16H, m), 4.05 (1H, tt, J = 35.8, 12.3 Hz), 5.34-5.39 (1H, m), 7.34-7.53 (6H, m), 7.87 (2H, dd, J = 26. 8, 8.2 Hz), 8.09 (1H, q, J = 2.0 Hz), 8.34 (2H, t, J = 11.6 Hz), 8.43-8.48 (2H, m).
MS (ESI) m/z: 661 ($M^+$+1), 663 ($M^+$+3).

3-[2-[1-[4-(3- Benzo [b] thiophenylcarbonylamino)- 2,5- dichlorophenylacetyl]-(4S)-N, O- dimethylhydroxylamino-(2S)-pyrrolidinyl]-5-thiazolyl]propionic acid

[Chem. 354]

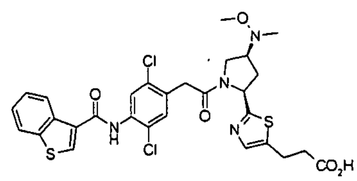

$^1$H-NMR ($CDCl_3$) δ: 1.04-1.72 (10H, m), 1.87-2.73 (7H, m), 2.96-3.34 (7H, m), 3.50-3.78 (5H, m), 3.89-4.31 (2H, m), 7.41 (1H, s), 7.42-7.55 (2H, m), 7.88-7.93 (1H, m), 8.09 (1H, d, J = 2.7 Hz), 8.29 (1H, d, J = 7.1 Hz), 8.47 (1H, d, J = 8.3 Hz), 8.70 (1H, d, J = 6.6 Hz).
MS (ESI) m/z : 647 ($M^+$+1), 649 ($M^+$+3).
IR (ATR) $cm^{-1}$: 2954, 2885, 2852, 1720, 1645, 1570, 1502.
Anal. Calcd for $C_{29}H_{28}Cl_2N_4O_5S_2 \cdot H_2O$: C, 52. 33; H, 4.52; N, 8.42; S, 9.63.
Found: C, 52.71; H, 4.41; N, 8.22; S, 9.51.

Example 131

3-[2-[1-[4-(3-Benzo[b]furanylcarbonylamino)-5-chloro-2-fluorophenylacetyl]-(4S)-N,O-dimethylhydroxylamino-(2S)-pyrrolidinyl]-5-thiazolyl]propionic acid methyl ester

[Chem. 355]

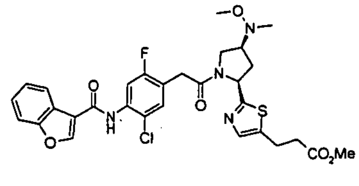

$^1$H-NMR ($CDCl_3$) δ: 2.24-2.79 (7H, m), 3.03-3.35 (3H, m), 3.36-3.82 (9H, m), 3.81-4.23 (1H, m), 5.29-5.55

(1H, m), 7.27-7.49 (4H, m), 7.57-7.64 (1H, m), 8.02-8.08 (1H, m), 8.25 and 8.28 (total 1H, each s, amide isomers), 8.28 and 8.30 (total 1H, each s, amide isomers), 8.38 and 8.47 (total 1H, each d, each J = 11.8 Hz, amide isomers).
MS (ESI) m/z : 629 ($M^+$+1), 631 ($M^+$+3).

3-[2-[1-[4-(3-Benzo[b]furanylcarbonylamino)-5-chloro-2-fluorophenylacetyl]-(4S)-N,O-dimethylhydroxylamino-(2S)-pyrrolidinyl]-5-thiazolyl]propionic acid

[Chem. 356]

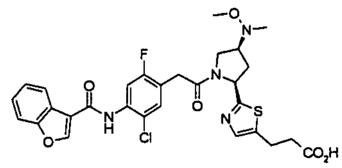

$^1$H-NMR ($CDCl_3$) δ: 2.21-2.38 (1H, m), 2.46-2.75 (6H, m), 3.08 (1H, t, J= 7.4 Hz), 3.13 (1H, t, J = 7.6 Hz), 3.19-3.33 (1H, m), 3.40 and 3.43 (total 3H, each s, amide isomers), 3.47-3.79 (4H, m), 3.81-4.30 (1H, m), 5.32-5.55 (1H, m), 7.30-7.49 (4H, m), 7.56-7.62 (1H, m), 8.00-8.07 (1H, m), 8.25 and 8.29 (total 1H, each s, amide isomers), 8.29 and 8.31 (total 1H, each d, each J = 6.1 Hz, amide isomers), 8.42 (1H, d, J = 11.8 Hz).
MS (ESI) m/z : 615 ($M^+$+1), 617 ($M^+$+3).
IR (ATR) $cm^{-1}$ : 2952, 2887, 2854, 1720, 1643, 1587, 1518.
Anal. Calcd for $C_{29}H_{28}ClFN_4O_6S \cdot 1.25H_2O$: C, 54.63; H, 4.82; N, 8.79; S, 5.03.
Found: C, 55.12; H, 4.67; N, 8.36; S, 4.92.

Example 132

3-[2-[1-[4-(3- Benzo [b] furanylcarbonylamino)- 2,5- dichlorophenylacetyl]-(4S)-N, O- dimethylhydroxylamino-(2S)-pyrrolidinyl]-5-thiazolyl]propionic acid methyl ester

[Chem. 357]

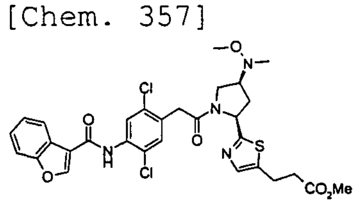

$^1$H-NMR ($CDCl_3$) δ: 2.21-2.42 (1H, m), 2.43-2.81 (6H, m), 3.04-3.36 (3H, m), 3.38-3.56 (3H, m), 3.63-3.94 (5H, m), 3.94-4.25 (1H, m), 5.33-5.60 (1H, m), 7.28-7.52 (5H, m), 7.57-7.63 (1H, m), 8.02-8.08 (1H, m), 8.21 and 8.24 (total 1H, each s, amide isomers), 8.29 and 8.30 (total 1H, each s, amide isomers), 8.63 and 8.70 (total 1H, each s, amide isomers).
MS (ESI) m/z: 645 ($M^+$+1), 647 ($M^+$+3).

3-[2-[1-[-(3- Benzo [b] furanylcarbonylamino)- 2,5- dichlorophenylacetyl]-(4S)-N, O- dimethylhydroxylamino-(2S)-p yrrolidinyl]-5-thiazolyl]propionic acid

[Chem. 358]

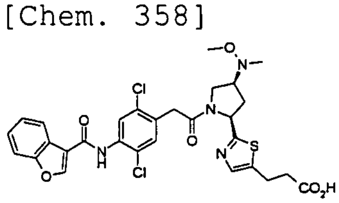

$^1H$-NMR ($CDCl_3$) δ: 1.04-1.72 (10H, m), 1.87-2.73 (7H, m), 2.96-3.34 (7H, m), 3.50-3.78 (5H, m), 3.89-4.31 (2H, m), 7.41 (1H, s), 7.42-7.55 (2H, m), 7.88-7.93 (1H, m), 8.09 (1H, d, J = 2.7 Hz), 8.29 (1H, d, J = 7.1 Hz), 8.47 (1H, d, J = 8.3 Hz), 8.70 (1H, d, J = 6.6 Hz).
MS (ESI) m/z : 631 ($M^+$+1), 633 ($M^+$+3).
IR (ATR) $cm^{-1}$ : 3273, 3136, 2956, 2852, 1720, 1670, 1643, 1560. Anal. Calcd for $C_{29}H_{28}Cl_2N_4O_6S \cdot 0.75H_2O$: C, 54.00; H, 4. 61; N, 8.69; S, 4.91.
Found: C, 54.04; H, 4.52; N, 8.36; S, 4.92.

Example 133

1-Benzyl 2-methyl (2S,4S)-4-methoxy-1,2-pyrrolidinedicarboxylate
[Chem. 359]

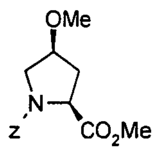

(2S,4S)-1-[(benzyloxy)carbonyl]-4-hydroxy-2-pyrrolidinecarboxylic acid (30.0g, 113mmol) was dissolved in DMF (280ml), and iodomethane (15.5ml, 248mmol) was added. To the reaction mixture was added sodium hydride (9.95g,249mmol) in portions at -0˚C under stirring and, after completion of addition, a temperature of the reaction mixture was gradually raised, followed by stirring at room temperature for 24 hours. The reaction solution was diluted with ethyl acetate (500ml), this was poured into ice-cooled, 1N hydrochloric acid (150ml), and layers were separated, followed by extraction with ethyl acetate. The resulting extract was washed with an aqueous saturated sodium chloride solution, dried with anhydrous sodium sulfate, and concentrated. The resulting residue was purified by silica gel column chromatography (Yamazen, Kilopack Si40-E, diameter 80mm × full length 500mm, flow rate 60ml/min, eluting solvent: hexane/ethyl acetate, 7:3) to obtain a colorless oily substancey title compound (32g, 97%).
(3R,6S,7aS)-6-methoxy-3-(trichloromethyl)tetrahydro-1H-pyrrolo[1,2-c][1,3]oxazole-1-one
[Chem. 360]

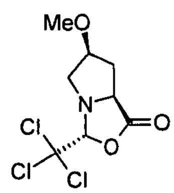

1-Benzyl 2-methyl (2S,4S)-4-methoxy-1,2-pyrrolidinedicarboxylate (32.5g, 111mmol) was dissolved in a mixed solution of tetrahydrofuran and methanol (400ml, 1:1), a 1N aqueous sodium hydroxide solution (222ml, 222mmol) was added, and the mixture was stirred at room temperature for 4 hours. The reaction mixture was concentrated under reduced pressure, and the residue was washed with diethyl ether. Citric acid was added to the aqueous layer to acidic under ice-cooling, followed by extraction with methylene chloride. The extract was dried with anhydrous sodium sulfate, and concentrated. The resulting residue was dissolved in methanol, 5g of 5% palladium hydroxide (WET) was added, and the mixture was stirred at room temperature for 24 hours under a hydrogen gas (3 MPa). A catalyst was filtered, and a solvent of the filtrate was removed under reduced pressure. To the residue were added chloral (21.6ml, 222mmol) and acetonitrile (100ml), and the mixture was heated to reflux for 30 minutes. The reaction solution was concentrated under reduced pressure, to the residue was added acetonitrile (20ml), this was subjected to azeotropy (three times), and the resulting residue was purified by silica gel column chromatography (Yamazen, High Flash-L, diameter 26mmx full length 100mm, flow rate 20ml/min, eluting solvent: methylene chloride) to obtain the title compound (21.1g, 69%) as a colorless amorphous solid matter solid.

$^1$H-NMR (400MHz, $CDCl_3$) δ: 2.04 (1H, ddd, J = 14.0, 9.5, 3.2 Hz), 2.53 (1H, d, J = 14.2 Hz), 3.24 (3H, s), 3.32 (2H, s), 3.86 (1H, s), 4.07 (1H, d, J = 9.6 Hz), 5.24 (1H, s). MS (ESI) m/z: 274 ($M^+$+H).

(3R,6S,7aR)-6-methoxy-7a-(methoxymethyl)-3-(trichloromethyl)tetrahydro-1H-pyrrolo[1,2-c][1,3]oxazole-1-one

[Chem. 361]

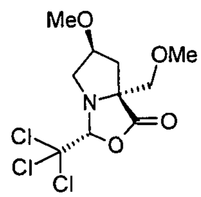

To dry tetrahydrofuran (50ml) were added diisopropylamine (5.11ml, 36.1mmol) and n-butyllithium (22.9ml, 1.58Minhexane, 36.1mmol) at -78˚C under a nitrogen stream while stirring, and the reaction mixture was further stirred at 0˚C for 15 minutes. This was added dropwise to a solution (25ml) of (3R,6S,7aS)-6-methoxy-3-(trichloromethyl) tetrahydro-1H-p yrrolo[1,2-c] [1,3]oxazole-1-one (9.45g, 34.4mmol) in tetrahydrofuran, and the mixture was stirred at the same temperature for 30 minutes. To the reaction mixture was added chloromethyl methyl ether (commercially available, 2.87ml, 37.8mmol) at -78˚C, a temperature was raised to -40˚C for 30 minutes while the reaction solution was stirred, and this was further stirred at -40˚C for 2 hours. The reaction solution was poured into ice water/ethyl acetate (1:1, 600ml), followed by extraction with ethyl acetate three times. The extract was washed with an aqueous saturated sodium chloride solution, and dried with anhydrous sodium sulfate, and a solvent was removed under reduced pressure. The resulting residue was purified by medium pressure silica gel column chromatography (ϕ 30×100mm, 20ml/min, eluting solvent: chloroform) to obtain the title compound (4.02g, 37%) as a colorless oily substance.

$^1$H-NMR ($CDCl_3$) δ: 2.27 (1H, dd, J = 14.4, 3.7 Hz), 2.38 (1H, dd, J= 14.3, 2.3 Hz), 3.18 (1H, dd, J = 13.7, 2.4 Hz), 3.26 (3H, s), 3.42 (3H, s), 3.42 (1H, dd, J = 2.7, 13.4 Hz), 3.66 (2H, dd, J = 10.2, 12.2 Hz), 3.92 (1H, t, J = 3.0 Hz), 5.30 (1H, s).
MS (ESI) m/z: 320 ($M^+$+H).

(2R,4S)-1-(tert-butoxycarbonyl)-4-methoxy-2-(methoxymethyl)-2-pyrrolidinecarboxylic acid

[Chem. 362]

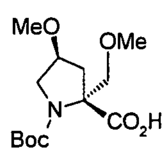

(3R,6S,7aR)-6-methoxy-7a-(methoxymethyl)-3-(trichloromethyl)tetrahydro-1H-pyrrolo[1,2-c][1,3]oxazole-1-one (2.92g, 9.17mmol) was dissolved in tetrahydrofuran (30ml), 6N hydrochloric acid (30ml) was added, and the mixture was heated to reflux at 100˚C for 24 hours under stirring. After completion of the reaction, the reaction solution was cooled to room temperature, and an organic solvent was removed under reduced pressure. The remaining aqueous solution was washed with methylene chloride, and the aqueous layer was concentrated to dryness under reduced pressure. To the resulting residue was added acetonitrile (20ml), di-tert-butyl bicarbonate (2.0g, 9.2mmol) and tetramethylammonium hydroxide · pentahydrate (1.66g, 9.20mol), and the mixture was stirred at room temperature for 3 hours. A solvent of the reaction solution was removed under reduced pressure, to the residue were added methylene chloride and an aqueous saturated citric acid solution, followed by extraction with methylene chloride. The extract was dried with anhydrous sodium sulfate, and a solvent was removed under reduced pressure. The residue was purified by silica gel column chromatography (ϕ 30mm×100mm, 20ml/min, eluting solvent: methylene chloride/methanol, 10:0 to 97: 3) to obtain the title compound (1.16g, 44%) as a colorless oily substance.

$^1$H-NMR ($CD_3OD$) δ: 1.43 and 1.45 (each s, total 9H), 2.11-2.22 (1H, m), 2.46-2.57 (1H, m), 3.30 (3H, s), 3.37 (3H, s), 3.55-3.71 (3H, m), 4.02-4.14 (2H, m).

MS (ESI) m/z: 290 ($M^+$+H).

Tert-butyl (2R,4S)-4-methoxy-2-{[(5-methoxy-2,5-dioxopentyl)amino]carbonyl}-2-(methoxymethyl)-1-pyrrolidinecarboxylate

[Chem. 363]

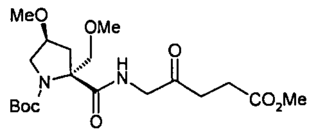

(2R,4S)-1-(tert-butoxycarbonylamino)-4-methoxy-2-(methoxymethyl)-2-pyrrolidinecarboxylic acid (870mg, 3.01mmol) and 5-aminolevulinic acid methyl ester (546mg, 3.01mmol) were dissolved in dimethylformamide (30ml), EDC hydrochloride (865mg, 4.Slmmol), 1-hydroxybenzotriazole (406mg, 3.01mmol), and triethylamine (2.52ml, 18.0mmol) were added, and the mixture was stirred at room temperature for 24 hours and, further, at 50°C for 24 hours. The reaction solution was poured into a mixed solution of ethyl acetate (10ml) and water (20ml), followed by extraction with ethyl acetate. The extract was washed with an aqueous saturated sodium chloride solution, and dried with anhydrous sodium sulfate, and a solvent was removed under reduced pressure. The resulting residue was purified by medium pressure silica gel column chromatography (Biotage flash chromatography system, column size, 40Meluting solvent: hexane/ethyl acetate, 1:1 to 0:10) to obtain the title compound (273mg, 22%) as a colorless oily substance.

$^1$H-NMR ($CDCl_3$) δ: 1.57 (9H, s), 1.97-2.06 (1H, m), 2.32-2.53 (2H, m), 2.57-2.81 (5H, m), 3.35 (6H, s), 3.36-3.47 (1H, m), 3.64-3.72 (1H, m), 3.67 (3H, s), 3.84-4.03 (2H, m), 4.08-4.30 (2H, m).

MS (ESI) m/z: 417 ($M^+$+H).

2-[(2R,4S)-1-tert-butoxycarbonyl-4-methoxy-2-(methoxymethyl)pyrrolidin-2-yl]5-thiazolepropionic acid methyl ester

[Chem. 364]

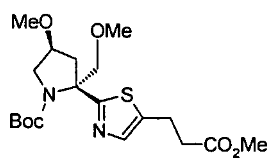

Tert-butyl (2R,4S)-4-methoxy-2-{[(5-methoxy-2,5-dioxopentyl)amino]carbonyl}-2-(methoxymethyl)-1-pyrrolidinecarboxylate (273mg, 0.66mmol) was dissolved in toluene (10ml), a Lawesson's reagent (801mg, 1.98mmol) was added, and the mixture was stirred at 90°C for 24 hours. The reaction solution was allowed to cool, and an aqueous saturated sodium chloride solution was added, followed by extraction with ethyl acetate. The extract was washed with an aqueous saturated sodium chloride solution, and dried with anhydrous sodium sulfate, and a solvent was removed under reduced pressure. The resulting residue was purified by silica gel column chromatography (Yamazen High Flash L, eluting solvent: hexane/ethyl acetate, 2:1), and the resulting crude purified substance was purified by TLC (Whatmann PLK-5F, thickness 1mm, four sheets, developing solvent: methylene chloride/methanol, 96:4) to obtain the title compound (150mg, 69%) as a colorless oily substance.

$^1$H-NMR ($CDCl_3$) δ: 1.23-1.52 (9H, m), 2.35-2.61 (1H, m), 2.62-2.84 (3H, m), 3.07-3.26 (5H, m), 3.35-3.42 (3H, m), 3.49-3.66 (1H, m), 3.64-3.73 (5H, m), 3.74-4.34 (3H, m), 7.22-7.41 (1H, m).

MS (ESI) m/z: 415 ($M^+$+H).

3-{2-[(2R, 4S)-1-[2-(2,5-dichloro-4-{[(1-methyl-1H-indol-3-yl) carbonyl] amino} phenyl) acetyl]-4-methoxy-2-(methoxymethyl)pyrrolidinyl]thiazol-5-yl}propionic acid

[Chem. 365]

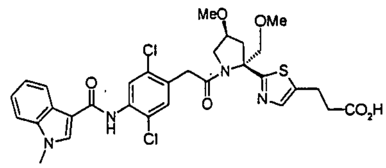

Tert-buxyl (2R,4S)-4-methoxy-2-(methoxymethyl)-2-[5-(3-methoxy-3-oxopropyl)-1,3-thiazol-2-yl]-1-pyrrolidinecarboxylic acid (150mg, 0.36mmol) was dissolved in dioxane (10ml), a 4N hydrochloric acid dioxane solution (10ml) was added, and the mixture was stirred at room temperature for 24 hours. A solvent was removed under reduced pressure, and a procedure of adding toluene to the residue and subjecting this to azeotropy was repeated three times. The resulting residue was dissolving in DMF (5ml), 2-(2,5-dichloro-4-{[(1-methyl-1H-indol-3-yl)carbonyl]ami no}phenyl) acetic acid (137mg, 0.36mmol), 1-hydroxybenzotriazole (49.0mg, 0.36mmol), EDC hydrochloride (104mg, 0.54mmo1), and triethylamine (0.303ml, 2.17mmol) were added, and the mixture was stirred at room temperature for 48 hours. To the reaction solution was added water, followed by extraction with ethyl acetate. The extract was washed with an aqueous saturated sodium chloride solution, and dried with anhydrous sodium sulfate, and a solvent was removed under reduced pressure. The resulting residue was purified by TLC (manufactured by Merck, thickness 2mm, 3 sheets, 20x20cm, eluting solvent: methylene chloride/methanol, 93:7) to obtain the title ester compound. The resulting ester compound was dissolved in a mixed solvent of tetrahydrofuran and methanol (21ml, 2:1, v/v), a 1N aqueous sodium hydroxide solution (7ml, 7.0mmol) was added, and the mixture was stirred at room temperature for 2 hours. 1N hydrochloric acid was added to the reaction solution to acidic, followed by extraction with methylene chloride. The extract was dried with anhydrous sodium sulfate, and a solvent was removed under reduced pressure. The resulting residue was purified by TLC (manufactured by Merck, thickness 2mm, 2 sheets, 20×20cm, developingsolvent:methylene chloride/methanol, 88:12, v/v) to obtain the title compound (194mg, 82%) as a colorless oily substance.

$^1$H-NMR (DMSO-$d_6$) δ: 0.86 (2H, t, J = 6.7 Hz), 2.34 (1H, dd, J = 13.5, 3.1 Hz), 2.59 (1H, dd, J = 13.7, 5.4 Hz), 2.96 (3H, t, J = 7.2 Hz), 3.13 (3H, s), 3.32 (3H, s), 3.80 (1H, d, J = 9.3 Hz), 3.85 (3H, s), 3.89 (3H, s), 4.07-4.09 (1H, m), 4.29 (1H, d, J= 9.3 Hz), 7.22 (1H, t, J = 7.2 Hz), 7.28 (1H, t, J = 7.2 Hz), 7.32 (1H, s), 7.54-7.57 (2H, m), 7.90 (1H, s), 8.15 (1H, d, J = 7.6 Hz), 8.30 (1H, s), 9.40 (1H, s).
IR (cm$^{-1}$) : 2923, 2825, 1654, 1502, 1373, 1099.
MS (ESI) m/z: 659 ($M^+$+H).
Anal. Calcd. for $C_{31}H_{32}Cl_2N_4O_6S \cdot 2.0H_2O$: C, 53.53; H, 5.22; Cl, 10.19; N, 8.05; S, 4.61.
Found: C, 53.62; H, 5.24; Cl, 11.07; N, 7.84; S, 4.52.

[Reference Example 1]

[4-(2-Benzoxazolyl)amino-3-chlorophenyl]acetic acid ethyl ester
[Chem. 366]

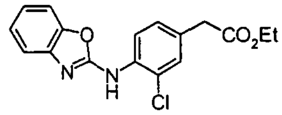

2-Chlorobenzoxazole (743μl, 6.51mmol) and 4-amino-3-chlorophenylacetic acid ethyl ester (see Patent Document 1) (1.30g, 6.51mmol) were heated to reflux for 2 hours in xylene (10ml). The reaction solution was cooled to room temperature, and water (30ml) was added, followed by extraction with chloroform. The extract was dried with anhydrous sodium sulfate, and a solvent was removed under reduced pressure. The resulting residue was purified by column chromatography using silica gel to obtain the title compound (1.70g, 79%) as a pale yellow solid from an-hexane/ethyl acetate (9:1, v/v)-eluted fraction.

$^1$H-NMR ($CDCl_3$) δ 1.25-1.28 (m, 3 H), 3.58 (s, 2 H), 4.14-4.19 (m, 2 H), 7.15-7.19 (m, 1 H), 7.24-7.30 (m, 3 H), 7.36-7.38 (m, 2 H), 7.52-7.54 (m, 1 H), 8.51-8.53 (m, 1 H). MS (ESI), m/z : 331 ($M^+$+1).

[4-(2-Benzoxazolyl)amino-3-chlorophenyl]acetic acid
[Chem. 367]

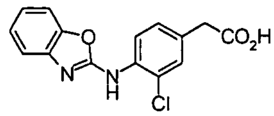

To [4-(2-benzoxazolyl)amino-3-chlorophenyl]acetic acid ethyl ester (1.70g, 5.14mmol) were added tetrahydrofuran (30ml) and 0.5N NaOH (30ml, 15.0mmol), and the mixture was stirred at room temperature for 20 hours. The reaction solution was concentrated under reduced pressured, and 1N HCl was added to acidic under cooling. A precipitated crystal was collected by filtration under reduced pressure, washed with water, and dried to obtain the title compound (1.24g, 80%) as a pale yellow solid.

$^1$H-NMR (DMSO-$d_6$) δ 3.62 (s, 2 H), 7.10-7.19 (m, 1 H), 7.21-7.28 (m, 1 H), 7.30-7.31 (m, 1 H), 7.38-7.40 (m, 1 H), 7.45-7.49 (m, 3 H), 7.94-7.96 (m, 1 H).

Compounds of Reference Example Nos. 1a to 1d shown below were produced by the same method as that of Reference Example 1.

[Table 1]

| Reference Example No. | Compound name | Structural formula | Molecular weight | Instrument data |
|---|---|---|---|---|
| 1a | [4-(2-Benz oxazolyl) a mino-5-chloro-2-fluo rophenyl]acetic acid ethyl ester | 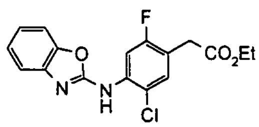 | 348 | $^1$H-NMR ($CDCl_3$) δ 1.27 (t, J=7.1 Hz, 3 H), 3.62 (s, 2 H), 4.19 (q, J = 7.1 Hz, 2 H), 7.16-7.38 (m, 3 H), 7.53-7.56 (m, 2 H), 8.47-8.50 (m, 1 H), MS (ESI), m/z : 349 ($M^+$+1) . |
| 1b | [4-(2-Benzoxazolyl) a mino-5-chl oro-2-fluo rophenyl] a cetic acid | 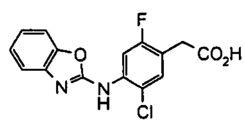 | 320 | $^1$H-NMR (DMSO-$d_6$) δ 3.64 (s, 2 H), 7.14-7.17 (m, 1 H), 7.21-7.26 (m, 1 H), 7.45-7.55 (m, 3 H), 8.07-8.10 (m, 1 H), MS (ESI), m/z : 321 ($M^+$+1). |
| 1c | [4-(2-Benzoxazolyl) a mino-2,5-dichlorophe nyl] acetic acid ethyl ester | 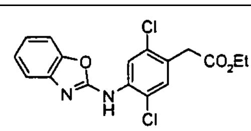 | 364 | $^1$H-NMR ($CDCl_3$) δ: 1.28 (3H, t, J = 6.8 Hz), 3.72 (2H, s), 4.20 (2H, q, J = 6.8 Hz), 7.17-7.21 (1H, m), 7.25-7.30 (1H, m), 7.36-7.39 (2H, m), 7.49 (1H, broad s), 7.56-7.58 (1H, m), 8.75 (1H, s), MS (LC-ESI), m/z : 365 ($M^+$+1). |

(continued)

| Reference Example No. | Compound name | Structural formula | Molecular weight | Instrument data |
|---|---|---|---|---|
| 1d | [4-(2-benzoxazolyl) a mino-2,5-dichlorophe nyl] acetic acid | 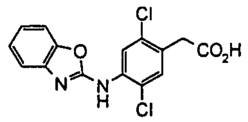 | 336 | $^1$H-NMR (DMSO-$d_6$) δ: 3.73 (2H, s), 7.14-7.26 (2H, m), 7.46-7.52 (2H, m), 7.63 (1H, s), 8.28 (1H, broad s), MS (LC-ESI), m/z : 337 ($M^+$+1). |

Reference Example 2

2-Bromo-5-fluorobenzothiazole

[Chem. 368]

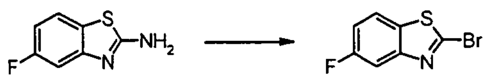

Cuprous bromide (1.26g, 7.31mmol) was suspended in acetonitrile (25ml), isoamyl nitrite (1.47ml, 10.9mmol) was added, and the mixture was stirred at 60˚C for 10 minutes. To the reaction solution was added 2-amino-5-fluorobenzothiazole (1.23 g, 7.31 mmol), and the reaction mixture was further stirred at 60˚C for 1 hour. The reaction solution was allowed to cool to room temperature, and poured into 1N HCl (50 ml). A precipitated crystal was collected by filtration under reduced pressure, washed with water, and dissolved in ethyl acetate (50 ml). The organic layer was washed successively with 1N HCl, and an aqueous saturated sodium chloride solution, and dried with anhydrous sodium sulfate, and a solvent was removed under reduced pressure. The resulting residue was purified by column chromatography using silica gel to obtain the title substance (1.07 g, 63%) as a yellow solid from a N-hexane/ethyl acetate (7:1, v/v)-eluted fraction.

$^1$H-NMR ($CDCl_3$) δ 7.18-7.23 (m, 1 H), 7.64-7.77 (m, 2 H).

[5-Chloro-4-(5-fluoro-2-benzothiazolyl)amino-2-fluorophenyl]acetic acid methyl ester

[Chem. 369]

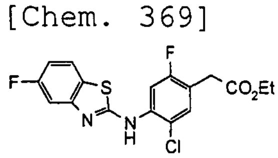

2-Bromo-5-fluorobenzothiazole (548 mg, 2.36 mmol), (4-amino-5-chloro-2-fluorophenyl)acetic acid ethyl ester) acetate (see Patent Document 1) (547 mg, 2.36 mmol), and pyridinium p-toluene sulfonate (PPPS) (178 mg, 0.71 mmol) were heated to reflux in xylene (5 ml) for 3 hours under stirring. The reaction solution was allowed to cool to room temperature, and a solvent was removed under reduced pressure. The resulting residue was purified by column chromatography using silica gel to obtain the title substance (298 mg, 33%) as a pale yellow solid from a n-hexane/ethyl acetate (7:1, v/v)-eluted fraction.

$^1$H-NMR ($CDCl_3$) δ 1.28 (t, J = 7.1 Hz, 3 H), 3.61 (s, 2H), 4.17 (q, J = 7.1 Hz, 2H), 6.95-6.98 (m, 1H), 7.28-7.30 (m, 1H), 7.39-7.42 (m, 1 H), 7.54-7.57 (m, 1 H), 7.69 (broad s, 1 H), 8.40-8.43 (m, 1 H).

[5-Chloro-4-(5-fluoro-2-benzothiazolyl)amino-2-fluorophenyl]acetic acid

[Chem. 370]

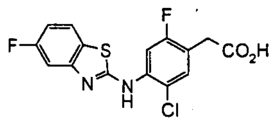

[5-Chloro-4-(5-fluoro-2-benzothiazolyl)amino-2-flu orophenyl]acetic acid methyl ester (298 mg, 0.78 mmol) was dissolved in tetrahydrofuran (5 ml), 0.5N NaOH (5.0ml, 2.50 mmol) was added, and the mixture was stirred at room temperature for 3 hours. The reaction solution was poured into ice (20 ml) and 1N HCl (10 ml). A precipitated crystal was collected by filtration under reduced pressure, washed with water, and dried under reduced pressure to obtain the title substance (237 mg, 86%) as a pale yellow solid.

$^1$H-NMR (DMSO-$d_6$) δ 3.64 (s, 2 H), 7.03-7.08 (m, 1 H), 7.45-7.55 (m, 2 H), 7.83-7.86 (m, 1 H), 8.28-8.31 (m, 1 H), 10.26 (broad s, 1 H), 12.56 (broad s, 1 H).

Compounds of Reference Examples 2a to 2h shown below were produced by the same method as that of Reference Example 2.

[Table 2]

| Reference Example No. | Compound name | Structural formula | Molecular weight | Instrument data |
|---|---|---|---|---|
| 2a | [4-(2-Benzothiazolyl ) amino-3-chloropheny 1] acetic acid methyl ester | 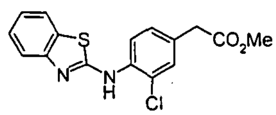 | 332 | $^1$H-NMR ($CDCl_3$) δ 3.58 (s, 2 H), 3.71 (s, 3 H), 7.16-7.24 (m, 2 H), 7.33-7.38 (m, 2 H), 7.63-7.69 (m, 2 H), 8.33-8.35 (m, 1 H), MS (ESI), m/z : 333 ($M^+$+1). |
| 2b | [4-(2-Benzothiazolyl ) amino-3-chloropheny 1] acetic acid | 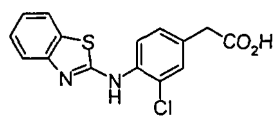 | 318 | $^1$H-NMR (DMSO-$d_6$) δ 3.59 (s, 2 H), 7.11-7.15 (m, 1 H), 7.25-7.31 (m, 2 H), 7.42 (d, J = 2.0 Hz, 1H), 7.52 (d, J = 8.1 Hz, 1 H), 7.77 (d, J = 8.1 Hz, 1 H), 8.11-8.13 (m, 1H), MS (ESI), m/z : 319 ($M^+$+1). |
| 2c | [4-(2-Benzothiazolyl ) amino-5-chloro-2-fl uorophenyl ] acetic acid ethyl ester | 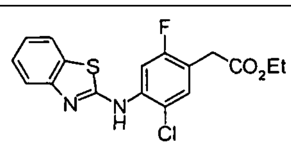 | 364 | $^1$H-NMR ($CDCl_3$) δ 1.26-1.30 (m, 3 H), 3.62 (s, 2 H), 4.10 (broad s, 1 H), 4.15-4.21 (m, 2 H), 7.21-7.42 (m, 3 H), 7.67-7.76 (m, 2 H), 8.47-8.50 (m, 1H), MS (ESI) m/z : 365 ($M^+$+1). |

(continued)

| Reference Example No. | Compound name | Structural formula | Molecular weight | Instrument data |
|---|---|---|---|---|
| 2d | [4-(2-Benzothiazolyl ) amino-5-chloro-2-fl uorophenyl ] acetic acid | 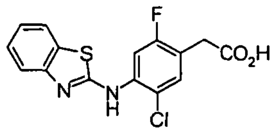 | 336 | $^1$H-NMR (DMSO-$d_6$) δ 3.63 (s, 2 H), 7.17-7.21 (m, 1 H), 7.32-7.39 (m, 1 H), 7.52-7.54 (m, 1 H), 7.61-7.63 (m, 1 H), 7.82-7.84 (m, 1 H), 8.38 (m, 1 H), 10.17 (broad s, 1H), 12.52 (broad s, 1 H). |
| 2e | [4-(4-Chloro-2-benzothiazolyl) amino-3-ch lorophenyl]acetic acid methyl ester | 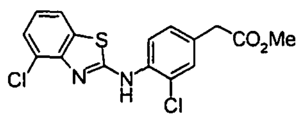 | 366 | $^1$H-NMR ($CDCl_3$) δ 3.61 (s, 2 H), 3.72 (s, 3 H), 7.10-7.14 (m, 1 H), 7.27-7.29 (m, 1 H), 7.39-7.41 (m, 2 H), 7.54-7.56 (m, 1 H), 7.75 (broad s, 1H), 8.25-8.27 (m, 1 H). |

[Table 3]

| Reference Example No. | Compound name | Structural formula | Molecular weight | Instrument data |
|---|---|---|---|---|
| 2f | [4-(4-Chloro-2-benZothiazolyl) amino-3-ch lorophenyl]acetic acid | 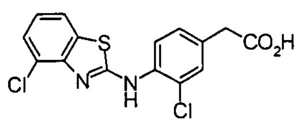 | 352 | $^1$H-NMR (DMSO-$d_6$) δ 3.63 (s, 2 H), 7.12-7.16 (m, 1 H), 7.29-7.31 (m, 1 H), 7.38-7.41 (m, 1 H), 7.46-7.47 (m, 1 H), 7.76-7.78 (m, 1 H), 8.18-8.21 (m, 1 H), 10.26 (broad s, 1H), 12.45 (broad s, 1H). |
| 2g | [4-(2-Benz othiazolyl )amino-2,5-dichlorop henyl]acetic acid ethyl ester | 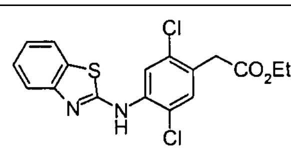 | 380 | $^1$H-NMR ($CDCl_3$) δ: 1.28 (3H, t, J = 7.1 Hz), 3.71 (2H, s), 4.20 (2H, q, J = 7.1 Hz), 7.22-7.28 (1H, m), 7.38-7.42 (3H, m), 7.69-7.78 (2H, m), 8.70 (1H, s), MS (LC-ESI) m/z: 381 ($M^+$+1) . |

(continued)

| Reference Example No. | Compound name | Structural formula | Molecular weight | Instrument data |
|---|---|---|---|---|
| 2h | [4-(2-Benz othiazolyl )amino-2,5-dichlorop henyl]acet ic acid | 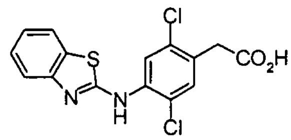 | 352 | $^1$H-NMR (DMSO-$d_6$) δ : 3.72 (2H, s), 7.17-7.21 (1H, m), 7.32-7.36 (1H, m), 7.59-7.61 (2H, m), 7.83 (1H, d, J = 7.6 Hz), 8.53 (1H, broad s), MS (LC-ESI) m/z: 353 ($M^+$+1). |

Test Example 1 In vitro evaluation of test substance

CHO cells which were forced to be expressed by transduction of h$\alpha_4$ and $\beta_1$ integrins are seeded ($3\times10^4$ cells/100μl/well) on a Costar 3599 plate, and cultured for 2 days. A medium is washed with a buffer A* two times, and $Eu^{3+}$-hVCAM-1 DID7-IgG which was diluted with an assay buffer A** to 2 nM is added at 50μl/well. A test substance which was diluted with 2% DMSO-assay buffer (in the presence or the absence of 6% human serum albumin) is added at 50μl/well (A solution which was separately diluted is added to a well

in which Scatchard analysis is performed), this is stirred with a plate mixer for 5 minutes, and allowed to stand at room temperature for 1 hour. Thereafter, this is washed with a buffer A four times, a potentiating reagent (manufactured by DELFIA) is added at 100μl/well, this is shaken for 5 minutes using a plate mixer, and a fluorescence intensity is measured with a time-resolved fluorometer (DELFIA Wallac). IC50 (concentration at which binding of CHO cell and $Eu^{3+}$-hVCAM-1 D1D7-IgG is inhibited by 50%) of a test substance is obtained from an integration rate obtained from a calculation equation: $[(F_T-F_{NS})-(F_I-F_{NS})]/[(F_T-F_{NS})]\times100$, wherein $F_T$ indicates a fluorescence intensity of a well not containing a test substance, $F_{NS}$ indicates a fluorescence intensity of a well not containing a test substance and hVCAM-1 D1D7-IgG, and $F_I$ indicates a fluorescence intensity containing a test substance. A Kd value showing strength of binding and Bmax (maximum binding amount) were presumed in a range of 0.06 to 20 nM according to the Scachard analysis method. Results are shown in Tables 4 to 7.

[Equation 1]

$$Ki = \frac{IC_{50}}{1+\frac{[L]}{Kd}}$$

A Ki value was calculated according to the above equation ([L] is ligand concentration)

[* buffer A : 25 mM HEPES (pH 7.5), 150 mM NaCl, 1 mM $Ca^{2+}$, 1 mM $Mg^{2+}$, 4 mM $Mn^{2+}$ ; ** assay buffer : 25 mM HEPES (pH 7.5), 150 mM NaCl, 1 mM $Ca^{2+}$, 1 mM $Mg^{2+}$, 4 mM $Mn^{2+}$, 0.1% BSA , 20 μM DTPA, with/without 6% ALBUMIN, HUMAN SERUM (C/N A-1653 , SIGMA)]

Results of in vitro evaluation of test substance

[Table 4]

| Example No. | MW | Ki (nM) |
|---|---|---|
| 1 | 615.5 | 1.3 |
| 2 | 599.1 | 0.85 |

(continued)

| Example No. | MW | Ki (nM) |
|---|---|---|
| 3 | 618.6 | 0.58 |
| 4 | 602.1 | 0.72 |
| 5 | 602.5 | 0.6 |
| 6 | 586.0 | 0.4 |
| 7 | 600.1 | 0.97 |
| 8 | 556.6 | 0.34 |
| 9 | 572.6 | 0.96 |
| 10 | 554.6 | 0.56 |
| 11 | 562.6 | 0.5 |
| 12 | 599.5 | 18 |
| 13 | 583.0 | 17 |
| 14 | 602.5 | 4 |
| 15 | 586.0 | 4.5 |
| 16 | 556.5 | 22 |
| 17 | 538.5 | 15 |
| 18 | 603.5 | 1.1 |
| 19 | 587.0 | 0.94 |
| 20 | 606.5 | 0.69 |
| 21 | 590.1 | 0.61 |
| 22 | 574.0 | 0.23 |
| 23 | 542.6 | 0.35 |
| 24 | 615.5 | 6.5 |
| 25 | 602.1 | 0.84 |
| 26 | 585.5 | 2.5 |
| 27 | 569.0 | 1.2 |
| 28 | 588.5 | 0.86 |
| 29 | 572.0 | 0.26 |
| 30 | 599.5 | 5.8 |
| 31 | 583.0 | 5.8 |
| 32 | 602.4 | 2.1 |
| 33 | 586.0 | 2 |
| 34 | 643.6 | 5.1 |
| 35 | 627.1 | 3 |

[Table 5]

| Example No. | MW | Ki (nM) |
|---|---|---|
| 36 | 630.1 | 1.7 |

(continued)

| Example No. | MW | Ki (nM) |
|---|---|---|
| 37 | 630.5 | 5.9 |
| 38 | 614.1 | 3.3 |
| 39 | 655.6 | 6.3 |
| 40 | 639.1 | 4 |
| 41 | 642.2 | 2.2 |
| 42 | 642.5 | 7 |
| 43 | 626.1 | 2.9 |
| 44 | 678.6 | 3.9 |
| 45 | 665.2 | 1.6 |
| 46 | 668.6 | 1.6 |
| 47 | 655.2 | 0.93 |
| 48 | 643.6 | 36 |
| 49 | 630.2 | 22 |
| 50 | 614.1 | 51 |
| 51 | 584.6 | 4.6 |
| 52 | 600.6 | 34 |
| 53 | 613.1 | 16 |
| 54 | 629.6 | 24 |
| 55 | 616.1 | 17 |
| 56 | 600.1 | 66 |
| 57 | 570.6 | 4.4 |
| 58 | 552. 6 | 6.7 |
| 59 | 623.1 | 17 |
| 60 | 639.6 | 8.6 |
| 61 | 626.2 | 6 |
| 62 | 580.6 | 2.3 |
| 63 | 654.2 | 0.89 |
| 64 | 670.6 | 3.8 |
| 65 | 657.2 | 0.93 |
| 66 | 673.6 | 0.84 |
| 67 | 641.1 | 1.2 |
| 68 | 657.6 | 0.73 |
| 69 | 668.2 | 0.71 |
| 70 | 684.6 | 2.3 |

[Table 6]

| Example No. | MW | Ki (nM) |
|---|---|---|
| 71 | 671.2 | 0.5 |
| 72 | 687.7 | 0.68 |
| 73 | 688.2 | 1.1 |
| 74 | 704.6 | 1.2 |
| 75 | 691.2 | 0.47 |
| 76 | 707.6 | 0.79 |
| 77 | 675.1 | 0.72 |
| 78 | 691.6 | 1.2 |
| 79 | 735.2 | 1.5 |
| 80 | 751.6 | 2.0 |
| 81 | 738.2 | 1.3 |
| 82 | 754.7 | 1.3 |
| 83 | 722.1 | 1.6 |
| 84 | 738.6 | 1.5 |
| 85 | 682.2 | 1.0 |
| 86 | 698.7 | 1.0 |
| 87 | 685.2 | 1.0 |
| 88 | 701.7 | 1.2 |
| 89 | 669.2 | 1.3 |
| 90 | 685.6 | 1.3 |
| 91 | 613.1 | 4.1 |
| 92 | 629.6 | 10 |
| 93 | 616.1 | 2.9 |
| 94 | 632.6 | 6.2 |
| 95 | 600.1 | 4.2 |
| 96 | 616.5 | 6.4 |
| 97 | 597.0 | 9.2 |
| 98 | 613.5 | 11 |
| 99 | 600.1 | 5.3 |
| 100 | 616.5 | 7.7 |
| 101 | 584.0 | 9.8 |
| 102 | 600.4 | 17 |

[Table 7]

| Example No. | MW | Ki (nM) |
|---|---|---|
| 103 | 554.5 | 2.6 |
| 104 | 518.6 | 4.5 |

(continued)

| Example No. | MW | Ki (nM) |
|---|---|---|
| 105 | 570.5 | 11 |
| 106 | 534.6 | 21 |
| 107 | 541.0 | 9.2 |
| 108 | 559.0 | 19 |
| 109 | 575.5 | 6.3 |
| 110 | 557.1 | 2.5 |
| 111 | 575.1 | 3.3 |
| 112 | 591.5 | 1.9 |
| 113 | 593.1 | 5.0 |
| 114 | 591.5 | 6.9 |
| 115 | 605.6 | 8.4 |
| 116 | 527.0 | 40 |
| 117 | 545.0 | 35 |
| 118 | 561.4 | 36 |
| 119 | 543.1 | 6.2 |
| 120 | 561.0 | 9.8 |
| 121 | 577.5 | 8.5 |
| 122 | 588.1 | 1.6 |
| 123 | 604.5 | 3.5 |
| 124 | 575.5 | 76 |
| 125 | 591.5 | 11 |
| 126 | 557.0 | 19 |
| 127 | 628.1 | 4.5 |
| 128 | 644.6 | 6.7 |
| 129 | 631.1 | 2.4 |
| 130 | 647.6 | 3.5 |
| 131 | 615.1 | 5.9 |
| 132 | 631.5 | 13 |
| 133 | 659.6 | 4.5 |
| CP664511 [1] | 522.6 | 1.5 |
| [1]A representative compound of WO 01/051487, Bioorganic and Medicinal Chemistry Letters 2001, 11: 19 (2593-2596), Journal of Pharmacology and Experimental Therapeutics 2002, 301: 2 (747-752) | | |

Evaluation Example 2 In vivo evaluation of test substance

Ascaris (Ascaris suum Antigen in pigs) active sensitization-induced mouse eosinophil infiltration test:

It has been reported that cell infiltration of eosinophil is induced by inducing Ascaris active sensitization {Int. Arch. Immunol., 108, 11-18, (1995)}. In this evaluation method, a test compound was evaluated. A test substance was orally administered two times per day, the total count of cells and the count of eosinophils in BALF 48 hours after inducement were calculated, and the effect was determined by comparing with a non-test substance administered group.

[Table 8]

| Results of mouse eosinophil infiltration test | | | |
|---|---|---|---|
| | Saline-induced group | Ascaris-induced group | Ascaris inducement+R1-2 group [1] |
| Average total eosinophil count ($\times 10^5$) | 0.02 | 2.55 | 1.13 |
| Inhibition rate (%) | - | - | 56** |

[Table 9]

| Results of mouse eosinophil infiltration test | | | |
|---|---|---|---|
| | Saline-induced group | Ascaris-induced group | Ascaris inducement+Example 1 |
| Average total eosinophil count ($\times 10^5$) | 0.01 | 3.51 | 1.52 |
| Inhibition rate | - | - | 57** |

[Table 10]

| Results of mouse eosinophil infiltration test | | | |
|---|---|---|---|
| | Saline-induced group | Ascaris-induced group | Ascaris inducement+Example 46 |
| Average total eosinophil count ($\times 10^5$) | 0.01 | 3.07 | 1.44 |
| Inhibition rate (%) | - | - | 53** |

[Table 11]

| | Saline-induced group | Ascaris-induced group | Ascaris inducement+CP6 64511 |
|---|---|---|---|
| Average total eosinophil count ($\times 10^5$) | 0.01 | 3.18 | 2.96 |
| Inhibition rate (%) | - | - | 16 |
| Results of mouse eosinophil infiltration test<br>[1] anti-VLA-4 antibody, two times/1 day s.c. administration;<br>**p<0.01: vs D, cont by student's t-test | | | |

Evaluation Example 3

Intracorporeal kinetics and oral absorbing property test:

A pharmacokinetic parameter when a test substance was orally administered to a dog by 0.5 mg/kg cassette was as follows. Results are shown in the following Table.
Results of dog intracorporeal kinetics and oral absorbing property test
[Table 12]

| | AUC[1] (ng · h/ml) | Cltot[2] (mL/min/kg) |
|---|---|---|
| Example 1 | 7040 | 1.23 |
| Example 2 | 10690 | 0.81 |
| Example 4 | 10240 | 0.82 |
| Example 36 | 14935 | 0.57 |
| Example 51 | 32310 | 0.26 |
| Example 57 | 27620 | 0.31 |
| Example 58 | 14860 | 0.59 |
| Example 60 | 19770 | 0.42 |
| CP-664511 | N.D. [3] | N.D. [3] |
| [1] AUC (ng h/ml) : total area under the plasma concentration (measured by LC/MS/MS method) versus time curve; [2] CLtot (mL/min/kg) apparent plasma clearance;[3] not determined. | | |

Evaluation Example 4

Dispersibility test

Dispersibility of a test substance in Japanese Pharmacopoeia First Solution (JP1, pH 1.2) and Japanese Pharmacopoeia Second Solution (JP2, pH 6.8) was evaluated. Results are shown in Tables 13 and 14.
[Table 13]

| Results of dispersibility test | | |
|---|---|---|
| | Japanese Pharmacopoeia First Solution (μg/ml) | Japanese Pharmacopoeia Second Solution (μg/ml) |
| Example 1 | 33 | >2000 |
| Example 2 | 97 | >2000 |
| Example 10 | 1700 | 2000 |
| Example 25 | 7 | 330 |
| Example 26 | 4 | 660 |
| Example 45 | 2000 | 200 |
| Example 46 | >2000 | 570 |
| Example 47 | >2000 | 510 |
| Example 51 | 88 | >800 |
| Compound 1[1] | 0.1 | 20 |

(continued)

| Results of dispersibility test | | |
|---|---|---|
| | Japanese Pharmacopoeia First Solution (μg/ml) | Japanese Pharmacopoeia Second Solution (μg/ml) |
| Example 63 | 1000 | 1000 |
| Example 64 | 1000 | 1000 |
| Example 65 | 1000 | 1000 |
| Example 66 | 1000 | 1000 |
| Example 67 | 1000 | 1000 |
| Example 68 | 1000 | 1000 |
| Example 69 | 940 | 940 |
| Example 70 | 960 | 960 |
| Example 71 | 940 | 940 |
| Example 72 | 960 | 960 |
| Example 73 | 230 | 290 |
| Example 74 | 130 | 150 |
| Example 75 | 220 | 160 |

[Table 14]

| | Japanese Pharmacopoeia First Solution (μg/ml) | Japanese Pharmacopoeia Second Solution (μg/ml) |
|---|---|---|
| Example 76 | 110 | 83 |
| Example 77 | 430 | 380 |
| Example 78 | 260 | 230 |
| Example 79 | 250 | 670 |
| Example 80 | 110 | 730 |
| Example 81 | 210 | 680 |
| Example 82 | 96 | 320 |
| Example 83 | 540 | 1000 |
| Example 84 | 280 | 760 |
| Example 85 | 1200 | 1300 |
| Example 86 | 1200 | 1400 |
| Example 87 | 1200 | 1300 |
| Example 88 | 1200 | 1400 |
| Example 89 | 1100 | 1300 |
| Example 90 | 1100 | 1300 |
| Example 107 | 700 | 700 |
| Example 108 | 730 | 730 |
| Example 109 | 480 | 750 |

(continued)

| | Japanese Pharmacopoeia First Solution (μg/ml) | Japanese Pharmacopoeia Second Solution (μg/ml) |
|---|---|---|
| Example 110 | 730 | 730 |
| Example 111 | 570 | 750 |
| Example 112 | 420 | 770 |
| Example 113 | 180 | 770 |
| Example 119 | 730 | 730 |
| Example 120 | 320 | 760 |
| Example 121 | 160 | 780 |
| Compound 1[1] | 0.1 | 20 |
| [1] Compound of WO 02/053534 | | |

Industrial applicability

The present compound or a salt thereof selectively inhibits of binding of a cell adhesion molecule to VLA-4 and, at the same time, has high oral adsorbing property, can be used as a preventive and/or a therapeutic for various diseases mediated with leukocyte chemotaxis and adhesion, for example, inflammatory disease, autoimmune disease, cancer metastasis, bronchial asthma (congested nose), diabetes, arthritis, psoriasis, multiple sclerosis, inflammatory bowl disease and rejection reaction at transplantation and, moreover, since exhibits the high effect by oral administration, and can be administered for a long term, it has extremely high usefulness in clinic.

## Claims

1. A compound represented by the following general formula (I) :

[Chem. 1]

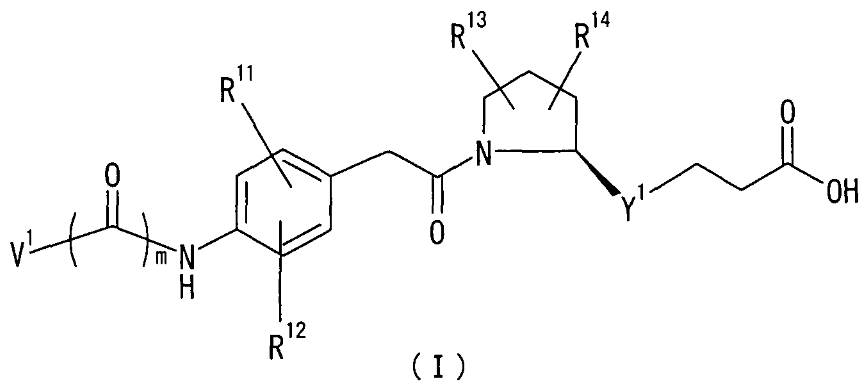

(I)

[wherein $Y^1$ represents a divalent aryl group optionally having a substituent, or a divalent heteroaryl group optionally having a substituent, $V^1$ represents an aryl group optionally having a substituent, or a heteroaryl group optionally having a substituent, $R^{11}$ and $R^{12}$ each independently represent a hydrogen atom, a hydroxy group, a halogen atom, a lower alkyl group, a lower alkoxy group, or an amino group optionally having a substituent, $R^{13}$ and $R^{14}$ each independently represent a hydrogen atom, a hydroxy group, a halogen atom, an amino group, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkylamino group optionally having a substituent, a dialkylamino group optionally having a substituent, a cyclic amino group optionally having a substituent, an alkylsulfonylamino group optionally having a substituent, an arylsulfonylamino group optionally having a substituent, a heteroaryloxy group optionally having a substituent, or an aryloxy group optionally having a substituent, $R^{13}$ and $R^{14}$ may be taken together with a carbon atom constituting a pyrrolidine ring to which $R^{13}$ and $R^{14}$ are bound, to form a 3- to 7-membered cyclic hydrocarbon or a heterocycle (optionally having 1 to 3 substituents independently selected from a hydroxy group, a halogen atom, an amino group, an alkyl group, an alkoxy group, an aryl group, an aryloxy group, an alkylamino group, a cyclic amino group, a benzyloxy group and a heteroaryl group on the ring), and m represents a number of 0 or 1]
or a salt thereof.

**2.** A compound represented by the following general formula (II) :

[Chem. 2]

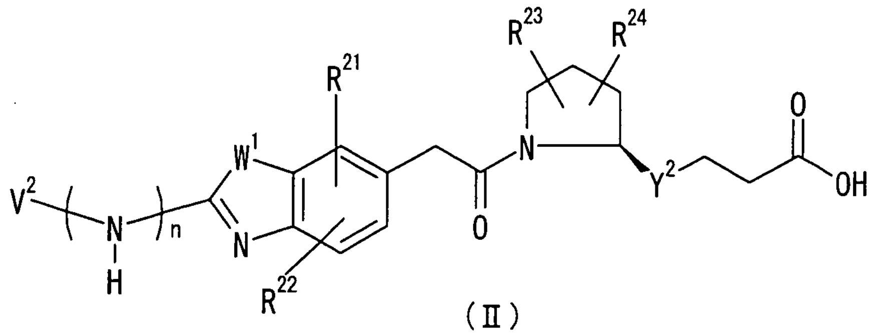

(II)

[wherein $R^2$ represents a divalent aryl group optionally having a substituent, or a divalent heteroaryl group optionally having a substituent, $V^2$ represents an aryl group optionally having a substituent, or a heteroaryl group optionally having a substituent, $W^1$ represents an oxygen atom or a sulfur atom, $R^{21}$ and $R^{22}$ each independently represent a hydrogen atom, a hydroxy group, a halogen atom, a lower alkyl group, a lower alkoxy group, or an amino group optionally having a substituent, $R^{23}$ and $R^{24}$ each independently represent a hydrogen atom, a hydroxy group, an amino group, a halogen atom, an alkyl group optionally having a substituent, an aryl group optionally having a substituent, a heterocyclic group optionally having a substituent, an alkoxy group optionally having a substituent, an alkoxyalkyl group optionally having a substituent, a cycloalkoxy group optionally having a substituent, a monoalkylamino group optionally having a substituent, a dialkylamino group optionally having a substituent, a cyclic amino group optionally having a substituent, an alkylsulfonylamino group optionally having a substituent, an arylsulfonylamino group optionally having a substituent, a heteroaryloxy group optionally having a substituent, or an aryloxy group optionally having a substituent, $R^{23}$ and $R^{24}$ may be taken together with a carbon atom constituting a pyrrolidine ring to which $R^{23}$ and $R^{24}$ are bound, to form a 3-to 7-membered cyclic hydrocarbon, or a heterocycle (optionally having 1 to 3 substituents independently selected from a hydroxy group, a halogen atom, an amino group, an alkyl group, an alkoxy group, an aryl group, an aryloxy group, an alkylamino group, a cyclic amino group, a benzyloxy group and a heteroaryl group on the ring), and n represents a number of 0 or 1]
or a salt thereof.

**3.** The compound according to claim 1, wherein $V^1$ in the formula (I) is any one of the following formulas (i-a) to (i-e):

[Chem. 3]

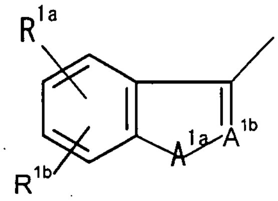

(i-a)

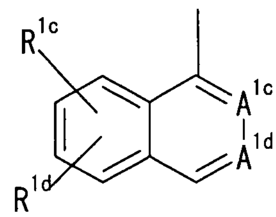

(i-b)

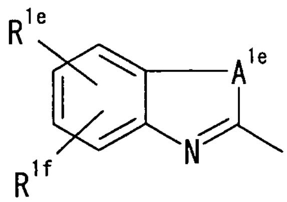

(i-c)

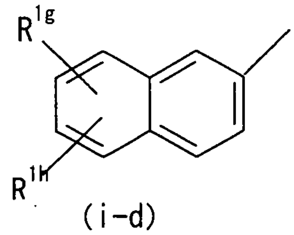

(i-d)

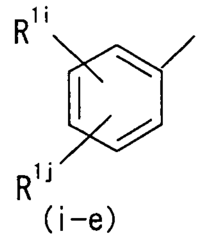

(i-e)

[wherein $A^{1a}$ represents an oxygen atom, a sulfur atom or N-$R^{1k}$ (wherein $R^{1k}$ represents a hydrogen atom or a lower alkyl group), $A^{1b}$ represents a nitrogen atom or C-$R^{1m}$ (wherein $R^{1m}$ represents a hydrogen atom or a lower alkyl group), $A^{1c}$ represents a nitrogen atom or C-$R^{1n}$ (wherein $R^{1n}$ represents a hydrogen atom or a lower alkyl group), $A^{1d}$ represents a nitrogen atom or C-$R^{10}$ (wherein $R^{10}$ represents a hydrogen atom or a lower alkyl group), $A^{1e}$ represents an oxygen atom, a sulfur atom or N-$R^{1p}$ (wherein $R^{1p}$ represents a hydrogen atom or a lower alkyl group), $R^{1a}$ represents a hydrogen atom, a hydroxy group, an amino group, a halogen atom, an alkyl group optionally having a substituent, an alkoxy group optionally having a substituent, a monoalkylamio group optionally having a substituent, a dialkylamino group optionally having a substituent, an alkylsulfonylamino group optionally having a substituent, an alkylthio group optionally having a substituent, or an alkylsulfonyl group optionally having a substituent, $R^{1b}$ is as defined for $R^{1a}$, $R^{1c}$ is as defined for $R^{1a}$, $R^{1d}$ is as defined for $R^{1a}$, $R^{1e}$ is as defined for $R^{1a}$, $R^{1f}$ is as defined for $R^{1a}$, $R^{1g}$ is as defined for $R^{1a}$, $R^{1h}$ is as defined for $R^{1a}$, $R^{1i}$ is as defined for $R^{1a}$, and $R^{1j}$ is as defined for $R^{1a}$],
or a salt thereof.

**4.** The compound according to claim 2, wherein $V^2$ in the formula (II) is any one of the following formulas (ii-a) to (ii-e) :

[Chem. 4]

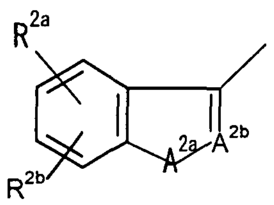

(ii-a)

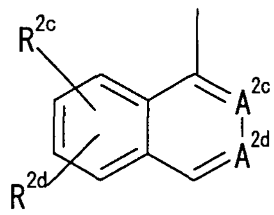

(ii-b)

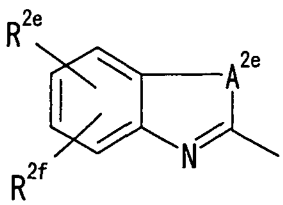

(ii-c)

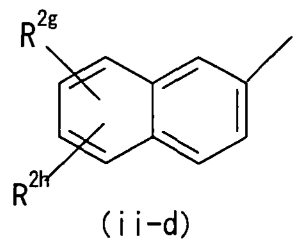

(ii-d)

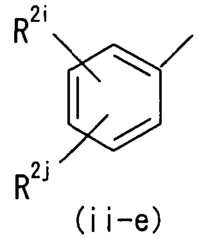

(ii-e)

[wherein $A^{2a}$ represents an oxygen atom, a sulfur atom or N-$R^{2k}$ (wherein $R^{2k}$ represents a hydrogen atom or a lower alkyl group), $A^{2b}$ represents a nitrogen atom or C-$R^{2m}$ (wherein $R^{2m}$ represents a hydrogen atom or a lower alkyl group), $A^{2c}$ represents a nitrogen atom or C-$R^{2n}$ (wherein $R^{2n}$ represents a hydrogen atom or a lower alkyl group), $A^{2d}$ represents a nitrogen atom or C-$R^{2o}$ (wherein $R^{2o}$ represents a hydrogen atom or a lower alkyl group), $A^{2e}$ represents an oxygen atom, a sulfur atom or N-$R^{2p}$ (wherein $R^{2p}$ represents a hydrogen atom or a lower alkyl group), $R^{2a}$ represents a hydrogen atom, a hydroxy group, an amino group, a halogen atom, an alkyl group optionally having a substituent, an alkoxy group optionally having a substituent, a monoalkylamino group optionally having a substituent, a dialkylamino group optionally having a substituent, an alkylsulfonylamino group optionally having a substituent, an alkylthio group optionally having a substituent, or an alkylsulfonyl group optionally having a substituent, $R^{2b}$ is as defined for $R^{2a}$, $R^{2c}$ is as defined for $R^{2a}$, $R^{2d}$ is as defined for $R^{2a}$, $R^{2e}$ is as defined for $R^{2a}$, $R^{2f}$ is as defined for $R^{2a}$, $R^{2g}$ is as defined for $R^{2a}$, $R^{2h}$ is as defined for $R^{2a}$, $R^{2i}$ is as defined for $R^{2a}$, and $R^{2j}$ is as defined for $R^{2a}$],
or a salt thereof.

**5.** The compound according to claim 3, wherein $V^1$ in the formula (I) is represented by the formula (I-a) or (I-c), or a salt thereof.

**6.** The compound according to claim 4, wherein when n = 0 in the formula (II), $V^2$ is represented by the formula (ii-a), or a salt thereof.

**7.** The compound according to claim 4, wherein when n = 1 in the formula (II), $V^2$ is represented by the formula (ii-e), or a salt thereof.

**8.** The compound according to claim 1, 3 or 5, wherein $Y^1$ in the formula (I) is any one of the following formulas (vii-a) to (vii-f):

[Chem. 5]

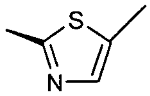
(vii-a)

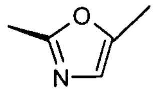
(vii-b)

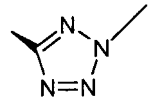
(vii-c)

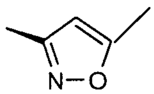
(vii-d)

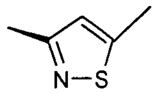
(vii-e)

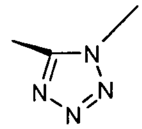
(vii-f)

(wherein a left bond indicates that $Y^1$ is bound to a carbon atom on a pyrrolidine ring, and a right bond indicates that $Y^1$ is bound to a 3-position carbon atom of propionic acid), or a salt thereof.

**9.** The compound according to claim 2, 4 or 6, wherein $Y^2$ in the formula (II) is any one of the following formulas (viii-a) to (viii-f):

[Chem. 6]

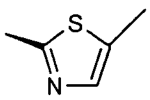
(viii-a)

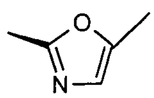
(viii-b)

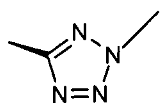
(viii-c)

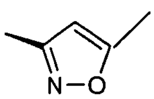
(viii-d)

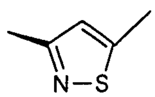
(viii-e)

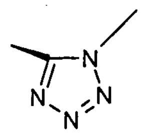
(viii-f)

(wherein a left bond indicates that $Y^2$ is bound to a carbon atom of a pyrrolidine ring, and a right bond indicates that $Y^2$ is bound to a 3-position carbon atom of propionic acid),
or a salt thereof.

**10.** The compound according to claim 8, wherein $Y^1$ in the formula (I) is represented by the formula (vii-a), (vii-b), (vii-c) or (vii-d), or a salt thereof.

**11.** The compound according to claim 9, wherein when n = 0 in the formula (II), $Y^2$ is represented by the formula (viii-a), or a salt thereof.

**12.** The compound according to claim 9, wherein when n = 1 in the formula (II), $Y^2$ is represented by the formula (viii-a) or (viii-b), or a salt thereof.

**13.** The compound according to claim 5, wherein $Y^1$ in the formula (I) is any one of the following formulas (vii-a), (vii-b), (vii-c) and (vii-d):

[Chem. 7]

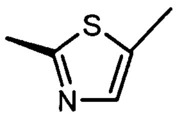

(vii-a)

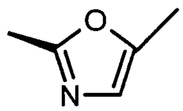

(vii-b)

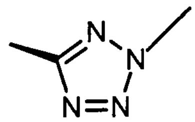

(vii-c)

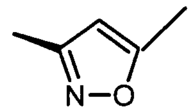

(vii-d)

(wherein a left bond indicates that $Y^1$ is bound to a carbon atom of a pyrrolidine, and a right bond indicates that $Y^1$ is bound to a 3-position carbon atom of propionic acid),
or a salt thereof.

**14.** The compound according to claim 6, wherein when n = 0 in the formula (II), $Y^2$ is the following formula (viii-a):

[Chem. 8]

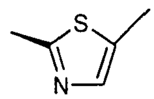

(viii-a)

(wherein a left bond indicates that $Y^2$ is bound to a carbon atom of a pyrrolidine ring, and a right bond indicates that $Y^2$ is bound to a 3-position carbon atom of propionic acid), or a salt thereof.

**15.** The compound according to claim 7, wherein when n = 1 in the formula (II), $Y^2$ is the following formula (viii-a) or (viii-b):

[Chem. 9]

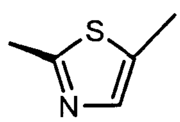

(viii-a)

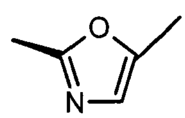

(viii-b)

(wherein a left bond indicates that $Y^2$ is bound to a carbon atom of a pyrrolidine ring, and a right bond indicates that $Y^2$ is bound to a 3-position carbon atom of propionic acid), or a salt thereof.

**16.** The compound according to claim 13, wherein $Y^1$ in the formula (I) is represented by the formula (vii-a), or a salt thereof.

**17.** The compound according to claim 7, wherein when n = 1 in the formula (II), $Y^2$ is the following formula (viii-a):

[Chem. 10]

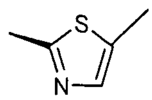

(viii-a)

(wherein a left bond indicates that $Y^2$ is bound to a carbon atom of a pyrrolidine ring, and a right bond indicates that $Y^2$ is bound to a 3-position carbon atom of propionic acid), or a salt thereof.

**18.** A compound represented by any one of the following formulas (18-1) to (18-133):

[Chem. 11]

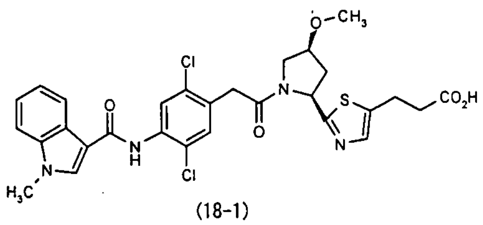

(18-1)

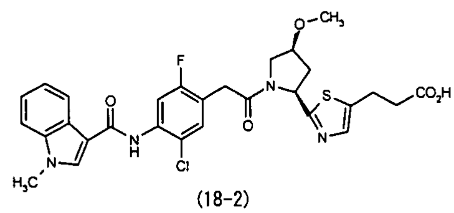

(18-2)

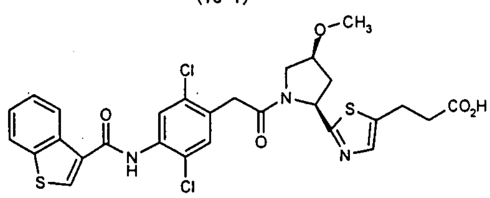

(18-3)

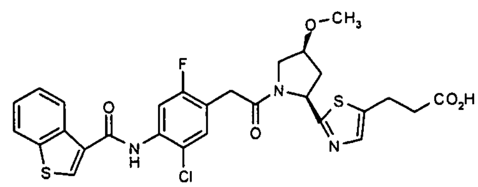

(18-4)

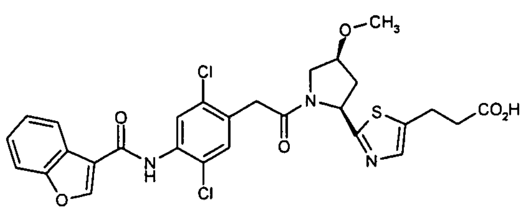

(18-5)

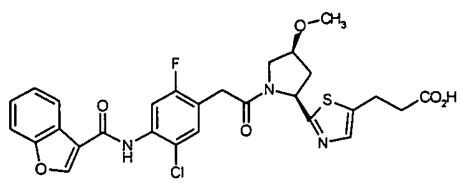

(18-6)

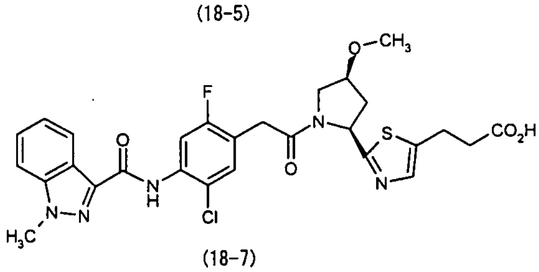

(18-7)

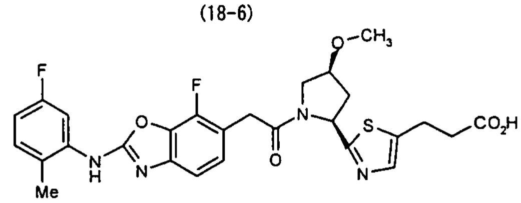

(18-8)

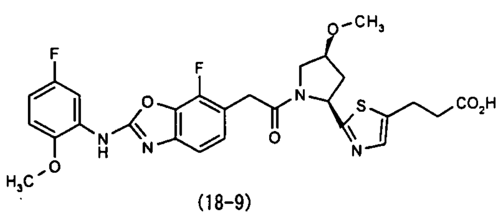

(18-9)

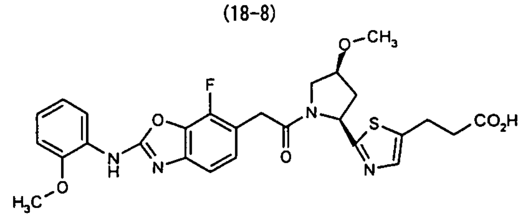

(18-10)

[Chem. 12]

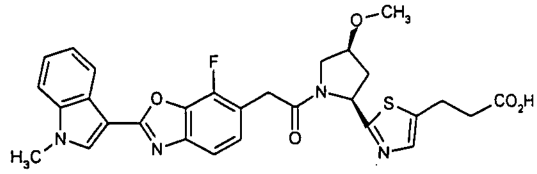

(18-11)

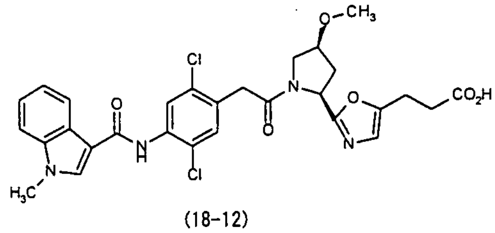

(18-12)

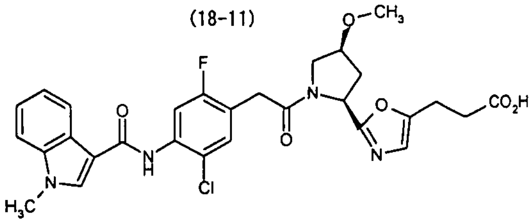

(18-13)

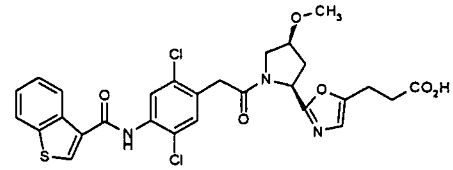

(18-14)

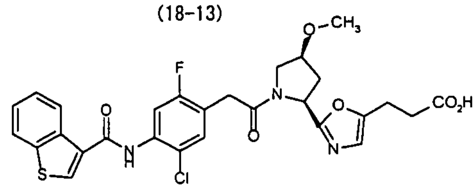

(18-15)

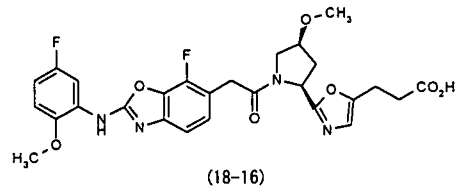

(18-16)

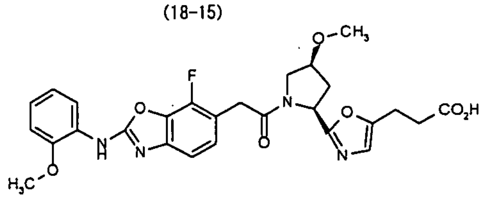

(18-17)

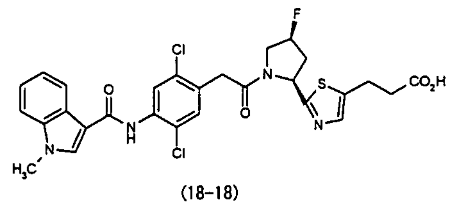

(18-18)

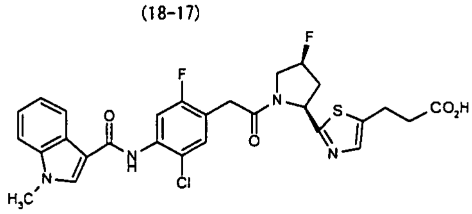

(18-19)

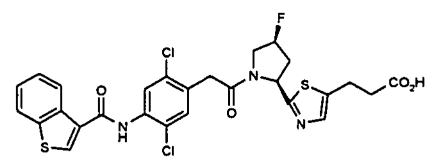

(18-20)

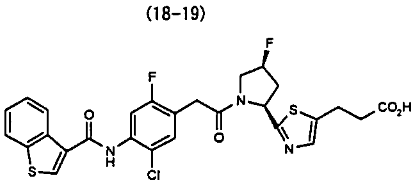

(18-21)

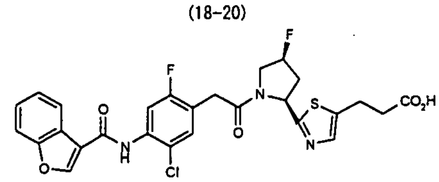

(18-22)

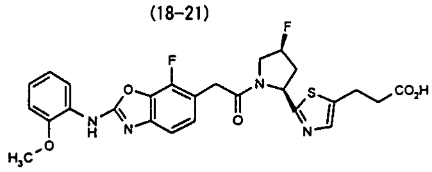

(18-23)

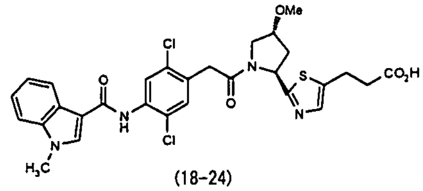

(18-24)

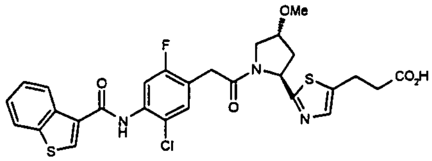

(18-25)

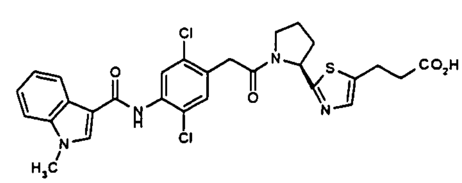

(18-26)

[Chem. 13]

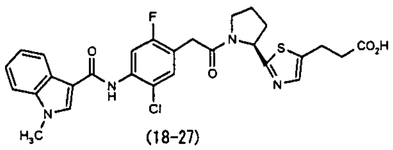

(18-27)

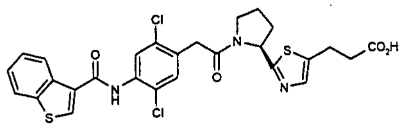

(18-28)

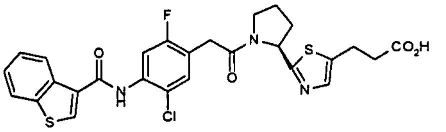

(18-29)

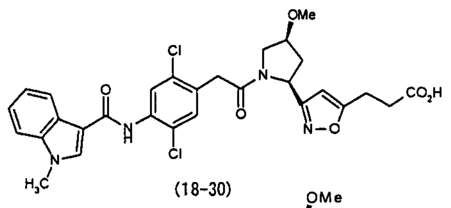

(18-30)

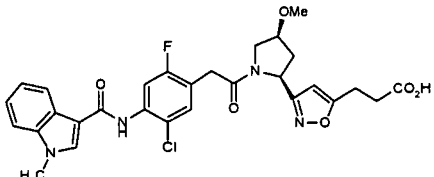

(18-31)

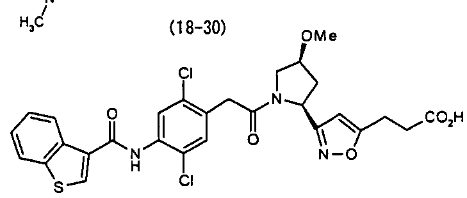

(18-32)

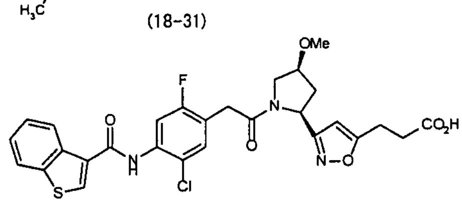

(18-33)

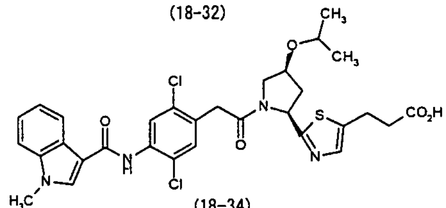

(18-34)

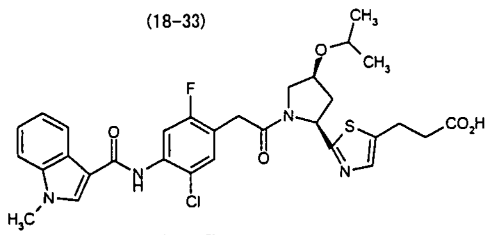

(18-35)

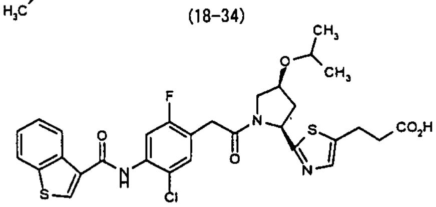

(18-36)

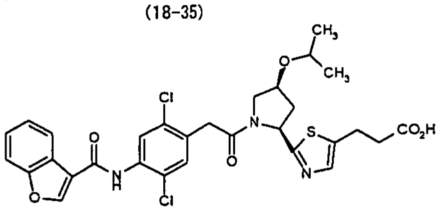

(18-37)

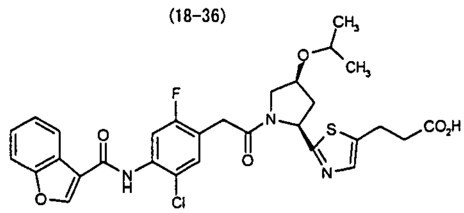

(18-38)

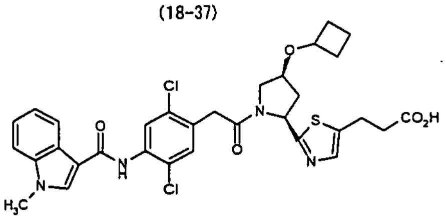

(18-39)

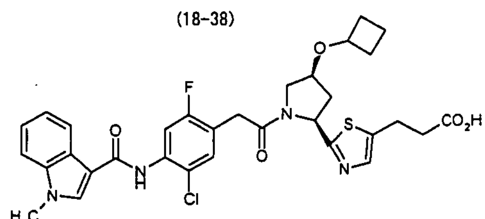

(18-40)

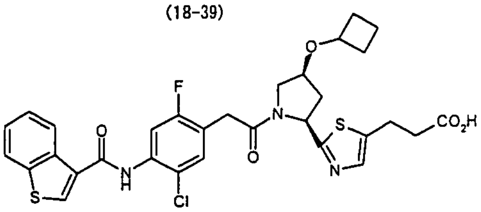

(18-41)

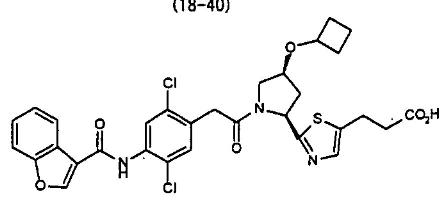

(18-42)

[Chem. 14]

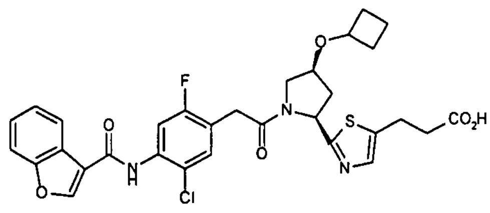

(18-43)

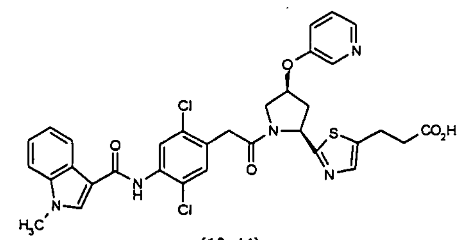

(18-44)

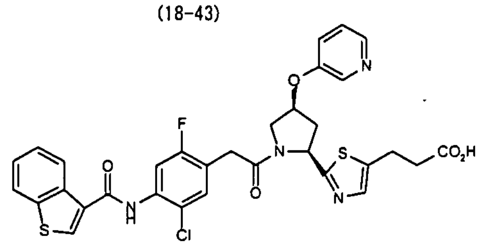

(18-45)

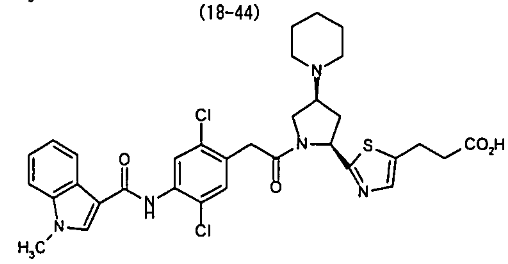

(18-46)

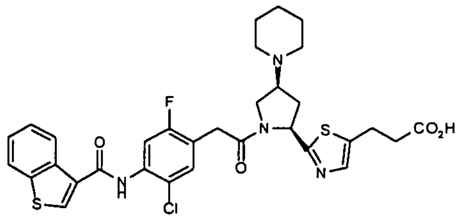

(18-47)

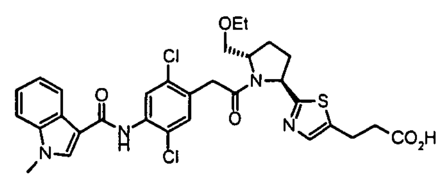

(18-48)

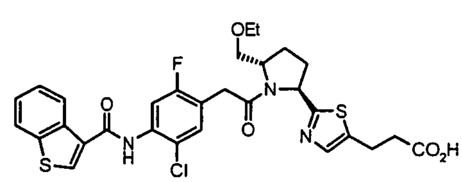

(18-49)

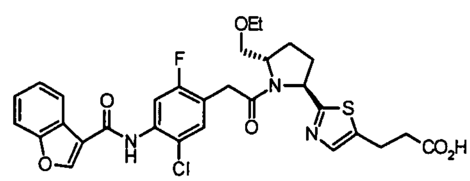

(18-50)

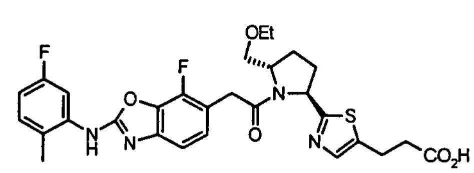

(18-51)

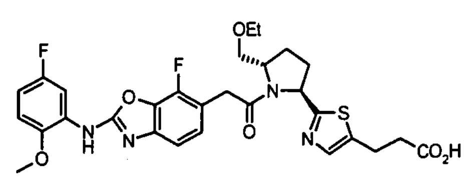

(18-52)

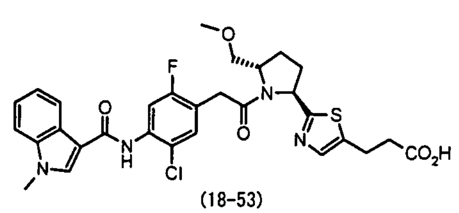

(18-53)

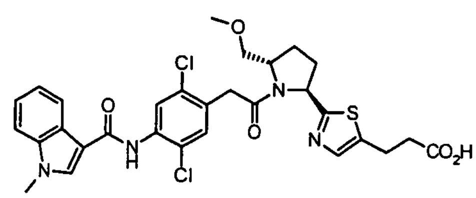

(18-54)

[Chem. 15]

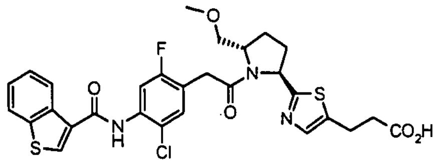

(18-55)

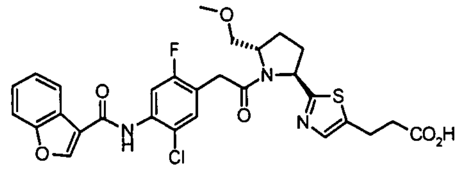

(18-56)

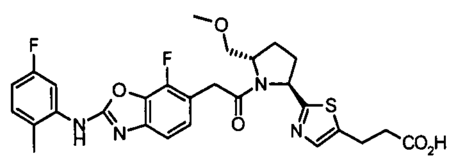

(18-57)

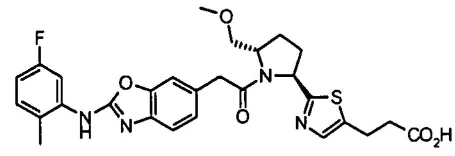

(18-58)

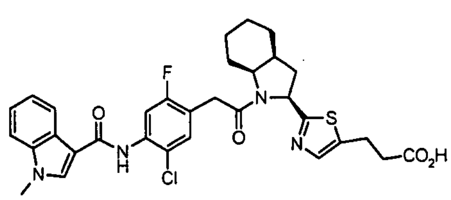

(18-59)

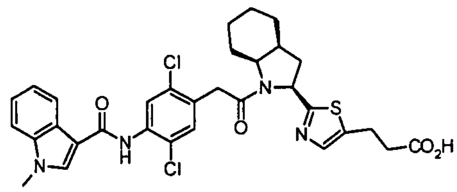

(18-60)

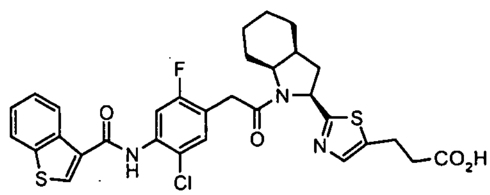

(18-61)

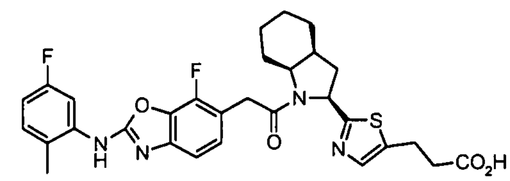

(18-62)

[Chem. 16]

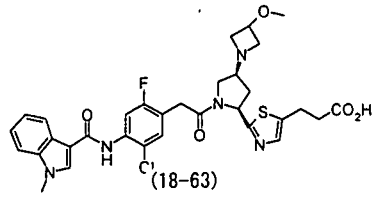
(18-63)

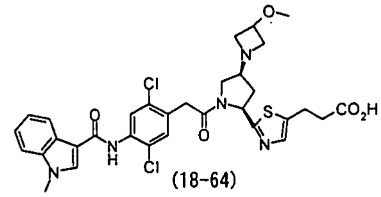
(18-64)

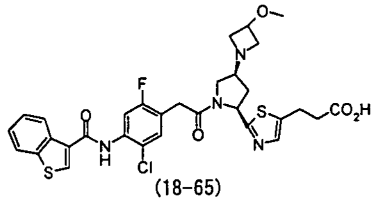
(18-65)

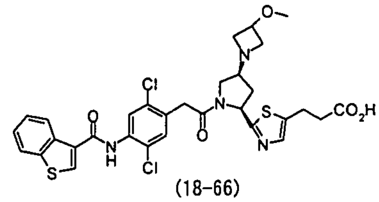
(18-66)

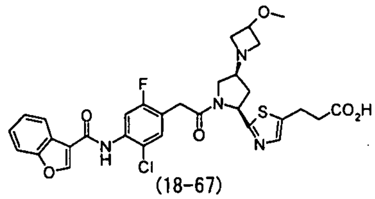
(18-67)

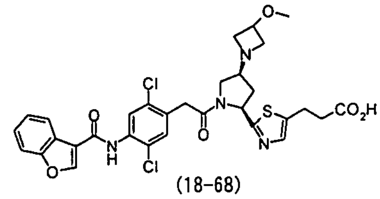
(18-68)

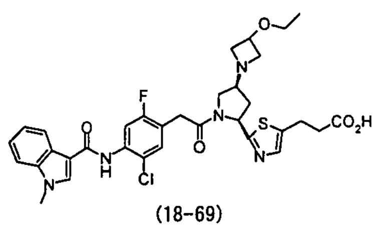
(18-69)

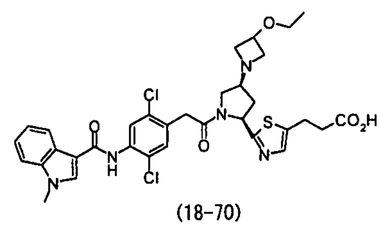
(18-70)

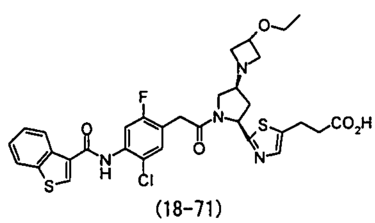
(18-71)

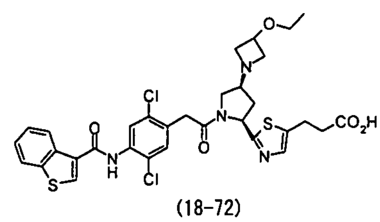
(18-72)

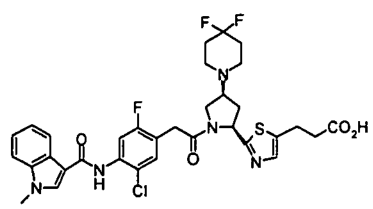
(18-73)

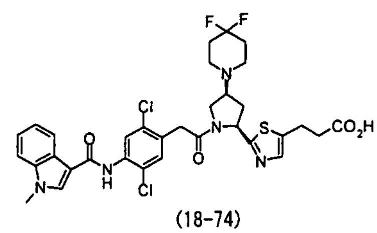
(18-74)

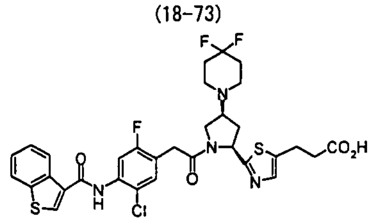
(18-75)

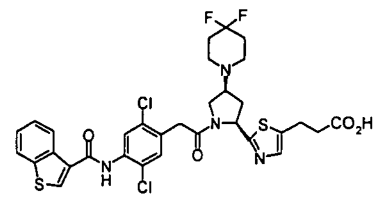
(18-76)

[Chem. 17]

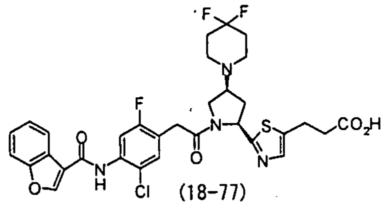

(18-77)

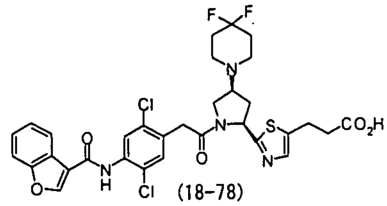

(18-78)

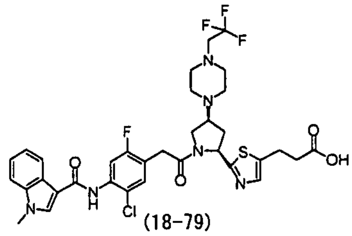

(18-79)

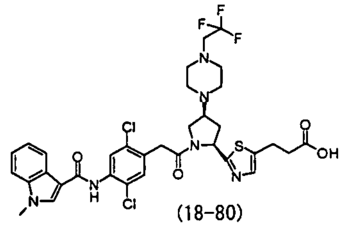

(18-80)

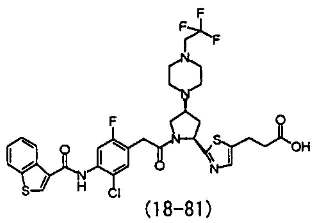

(18-81)

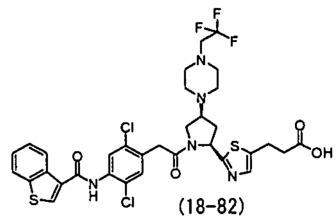

(18-82)

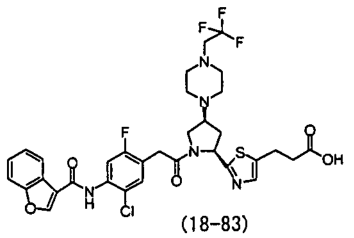

(18-83)

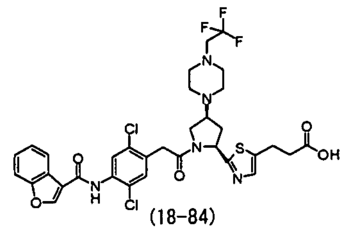

(18-84)

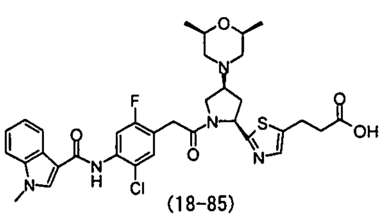

(18-85)

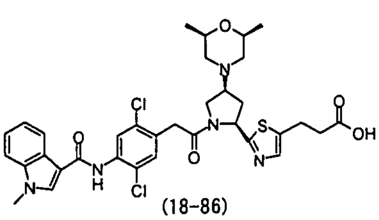

(18-86)

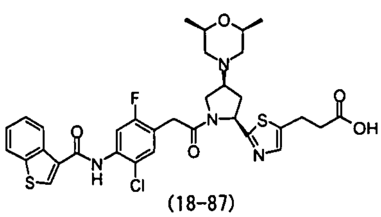

(18-87)

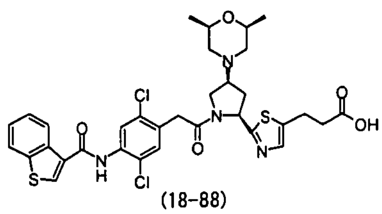

(18-88)

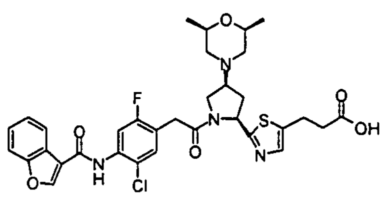

(18-89)

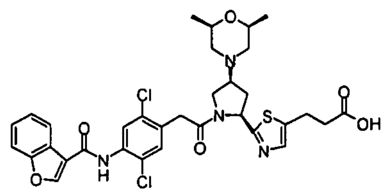

(18-90)

[Chem. 18]

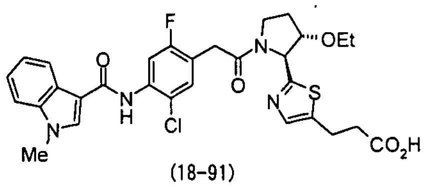

(18-91)

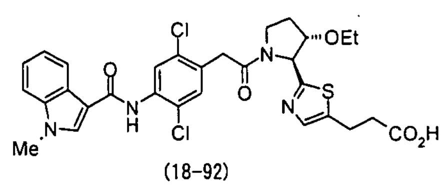

(18-92)

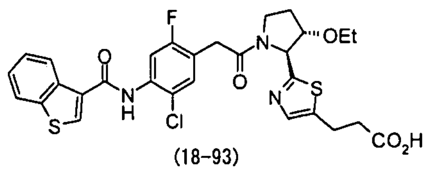

(18-93)

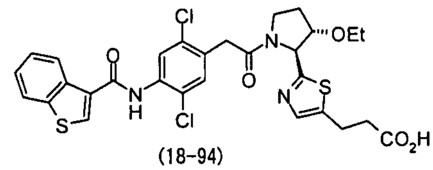

(18-94)

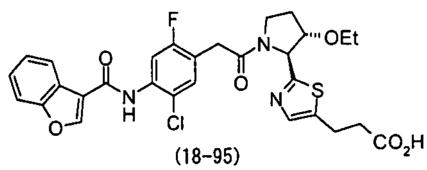

(18-95)

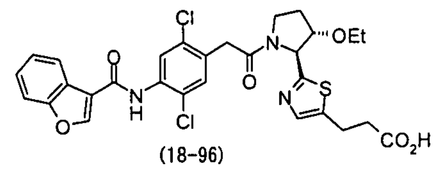

(18-96)

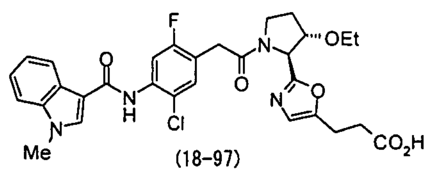

(18-97)

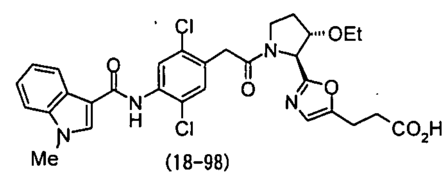

(18-98)

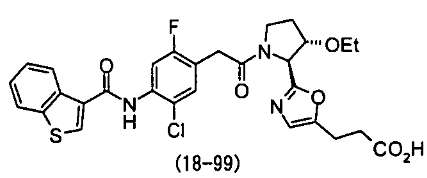

(18-99)

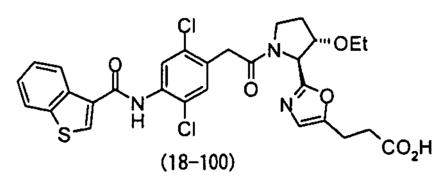

(18-100)

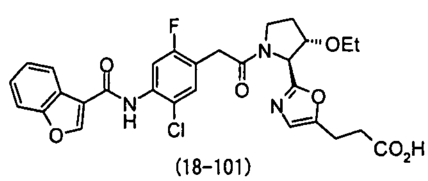

(18-101)

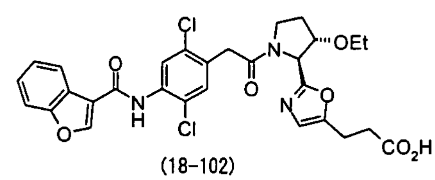

(18-102)

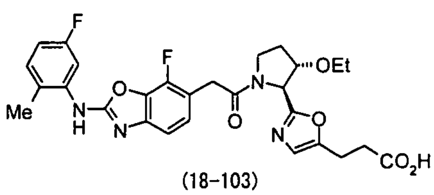

(18-103)

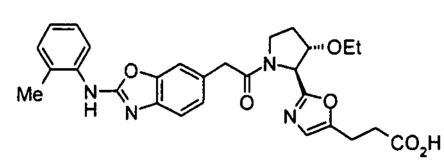

(18-104)

[Chem. 19]

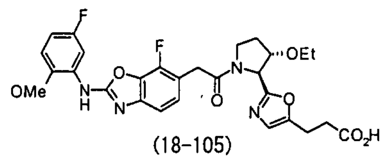
(18-105)

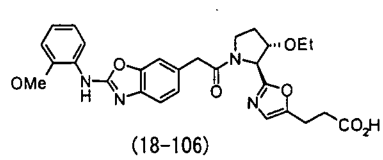
(18-106)

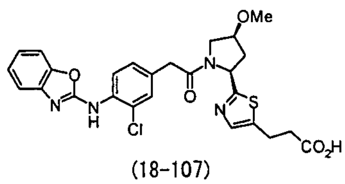
(18-107)

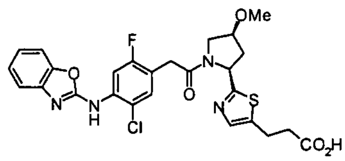
(18-108)

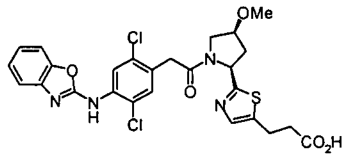
(18-109)

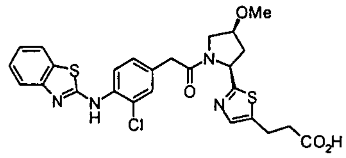
(18-110)

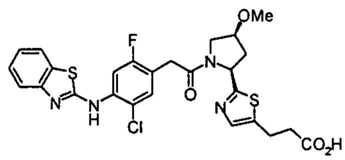
(18-111)

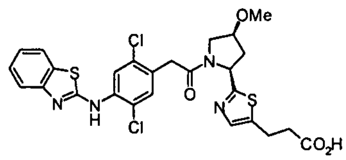
(18-112)

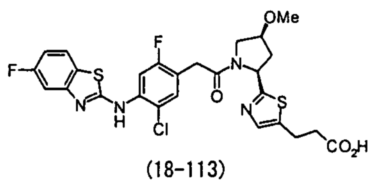
(18-113)

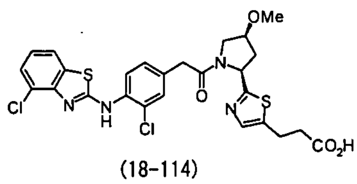
(18-114)

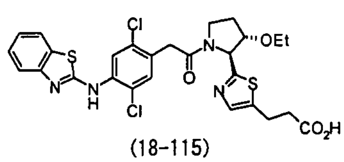
(18-115)

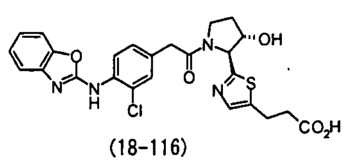
(18-116)

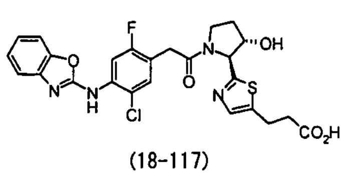
(18-117)

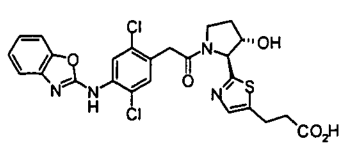
(18-118)

[Chem. 20]

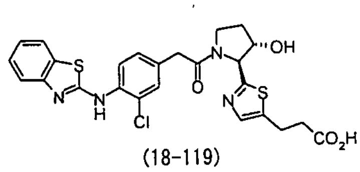

(18-119)

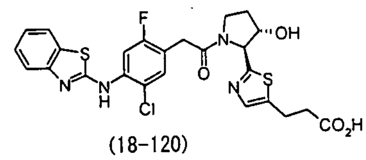

(18-120)

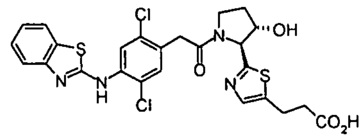

(18-121)

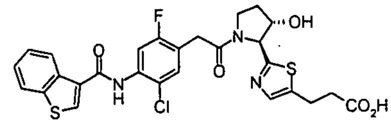

(18-122)

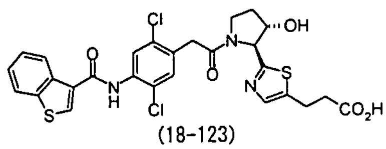

(18-123)

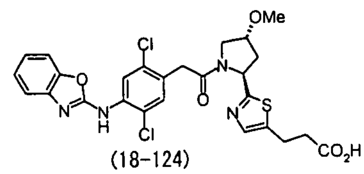

(18-124)

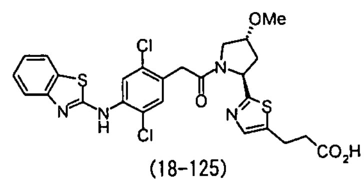

(18-125)

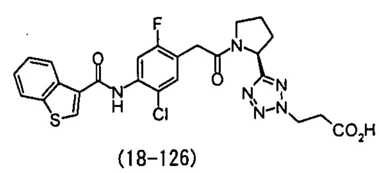

(18-126)

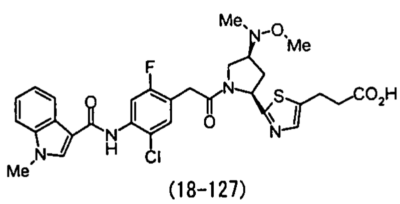

(18-127)

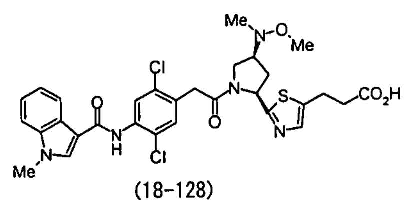

(18-128)

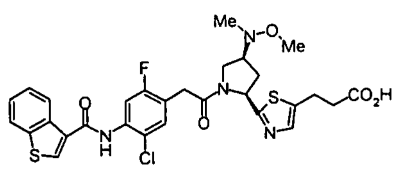

(18-129)

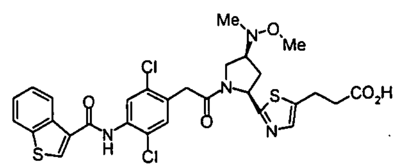

(18-130)

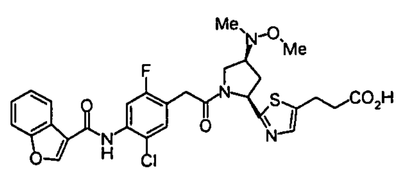

(18-131)

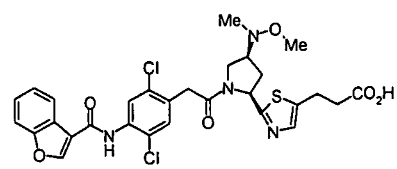

(18-132)

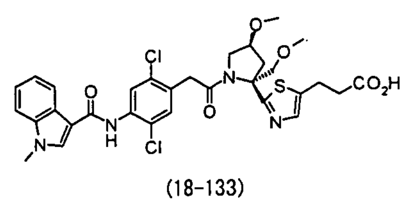

(18-133)

or a salt thereof.

**19.** The compound represented by the following formula (18-1), (18-36), (18-46) or (18-47):

[Chem. 21]

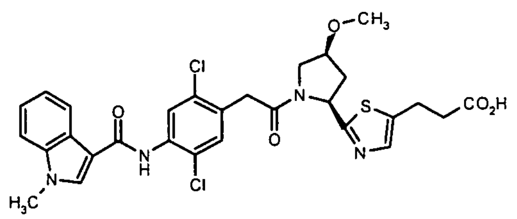

(18-1)

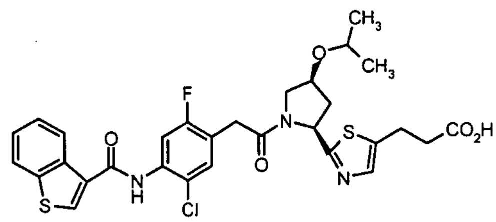

(18-36)

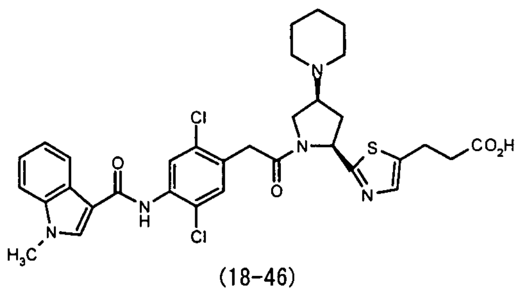

(18-46)

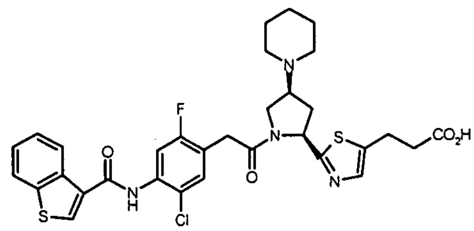

(18-47)

or a salt thereof.

**20.** A medicament containing a compound as defined in any one of claims 1 to 19, or a salt thereof.

**21.** A preventive and/or a therapeutic agent for a disease caused by cell adhesion, containing a compound as defined in any one of claims 1 to 19 or a salt thereof as a primary component.

**22.** The preventive and/or the therapeutic agent for a disease caused by cell adhesion according to claim 21, wherein the disease caused by cell adhesion is selected from the following group;
inflammatory reaction,
autoimmune disease,
cancer metastasis,
bronchial asthma,
nasal obstruction
diabetes,
arthritis,
psoriasis,
multiple sclerosis,
inflammatory bowel disease,
and
rejection reaction at transplantation.

**23.** Use of a compound as defined in any one of claims 1 to 19 or a salt thereof for producing a medicament.

**24.** A method of preventing and/or treating a disease cause by cell adhesion, comprising administering a compound as defined in any one claims 1 to 19 or a salt thereof.

**25.** The preventing method and/or the treating method according to claim 24, wherein the disease caused by cell adhesion is selected from the following group;
inflammatory reaction,
autoimmune disease,

cancer metastasis,
bronchial asthma,
nasal obstruction
diabetes,
arthritis,
psoriasis,
multiple sclerosis,
inflammatory bowel disease,
and
rejection reaction at transplantation.

**INTERNATIONAL SEARCH REPORT**

International application No.
PCT/JP2005/010894

A. CLASSIFICATION OF SUBJECT MATTER
Int.Cl$^7$ C07D413/14, A61K31/41, 31/422, 31/423, 31/427, 31/4439, 31/454, 31/496, 31/5377, A61P1/00, 3/10, 11/02, 11/06, 17/06, 19/02, 25/00, 29/00, 35/00, 37/02, 37/06, 43/00, C07D417/14
According to International Patent Classification (IPC) or to both national classification and IPC

B. FIELDS SEARCHED
Minimum documentation searched (classification system followed by classification symbols)
Int.Cl$^7$ C07D413/14, A61K31/41, 31/422, 31/423, 31/427, 31/4439, 31/454, 31/496, 31/5377, A61P1/00, 3/10, 11/02, 11/06, 17/06, 19/02, 25/00, 29/00, 35/00, 37/02, 37/06, 43/00, C07D417/14

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br><br>Y | JP 2003-519697 A (Pfizer Products Inc.),<br>24 June, 2003 (24.06.03),<br>Claims 1 to 53<br>& WO 01/51487 A1 & EP 1244656 A1 | 1,3,5,8,10,<br>13,16,18-23<br>2,4,6-7,9,<br>11-12,14-15,<br>17 |
| Y | JP 2002-521376 A (Astra Zeneca AB.),<br>16 July, 2002 (16.07.02),<br>Full text<br>& WO 00/05224 A1 & EP 1144393 A1 | 2,4,6-7,9,<br>11-12,14-15,<br>17 |
| A | JP 2002-501039 A (Novartis Pharma AG.),<br>15 January, 2002 (15.01.02),<br>Full text<br>& WO 99/37605 A1 & EP 1049665 A1 | 1-23 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| | |
|---|---|
| Date of the actual completion of the international search<br>02 August, 2005 (02.08.05) | Date of mailing of the international search report<br>16 August, 2005 (16.08.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/010894

C (Continuation). DOCUMENTS CONSIDERED TO BE RELEVANT

| Category* | Citation of document, with indication, where appropriate, of the relevant passages | Relevant to claim No. |
|---|---|---|
| A | JP 2002-519344 A (Pfizer Products Inc.),<br>02 July, 2002 (02.07.02),<br>Full text<br>& WO 00/00477 A1 & EP 1091943 A1 | 1-23 |
| A | WO 99/25685 A1 (DELASZLO),<br>27 May, 1999 (27.05.99),<br>Full text<br>& US 6020347 A | 1-23 |

Form PCT/ISA/210 (continuation of second sheet) (January 2004)

INTERNATIONAL SEARCH REPORT

International application No.
PCT/JP2005/010894

**Box No. II Observations where certain claims were found unsearchable (Continuation of item 2 of first sheet)**

This international search report has not been established in respect of certain claims under Article 17(2)(a) for the following reasons:

1. [x] Claims Nos.: 24-25
because they relate to subject matter not required to be searched by this Authority, namely:
Claims 24 to 25 are relevant to methods for treatment of the human body by surgery or therapy and diagnostic methods.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest**

[ ] The additional search fees were accompanied by the applicant's protest.

[ ] No protest accompanied the payment of additional search fees.

Form PCT/ISA/210 (continuation of first sheet (2)) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6355662 B
- WO 01051487 A
- WO 02053534 A
- WO 2002053534 A, Nakayama

### Non-patent literature cited in the description


- **SPRINGER, T.** *Ann. Rev. Physiol.,* 1995, vol. 57, 827
- **CARLOS ; HARLAN.** *Blood,* 1994, vol. 82, 2068
- **HEYNES, R.** *Cell,* 1992, vol. 69, 11
- **HELMER, M.** *Ann. Rev. Immunol.,* 1990, vol. 8, 365
- **ELICES et al.** *Cell,* 1990, vol. 60, 577
- **LOBB et al.** *J. Clin., Invest.,* 1994, vol. 94, 1722-28
- **PRETOLANI et al.** *J. Exp. Med.,* 1994, vol. 180, 795
- **NAKAJIMA et al.** *J. Exp. Med.,* 1994, vol. 179, 1145
- **CHISHOLM et al.** *Eur. J. Immunol.,* 1994, vol. 179, 1145
- **SILBER et al.** *J. Clin., Invest.,* 1993, vol. 93, 1554
- **ISOBE et al.** *J. Immunol.,* 1994, vol. 153, 5810
- **YANG et al.** *Proc. Natl. Acad. Sci. USA,* 1993, vol. 90, 10494
- **YEDNOCK et al.** *Nature,* 1992, vol. 356, 63
- **BARON et al.** *J. Exp. Med.,* 1993, vol. 177, 57
- **JAKUBOWSKI et al.** *J. Immunol.,* 1995, vol. 155, 938
- **WANG et al.** *Proc. Natl. Acad. Sci. USA,* 1995, vol. 92, 5714
- **MOLOSSI et al.** *J. Clin. Invest.,* 1995, vol. 95, 2601
- **JEFFERSON W. TILLEY ; ACHYUTHARAO SIDDURI.** *Drugs of the Future,* 2001, vol. 26 (1), 985-998
- **E. KUDLUCZ et al.** *J. Pharmacol. Exp. Ther.,* 2002, vol. 301, 747-752
- **M. H. JUNG et al.** *J.Med.Chem.,* 1999, vol. 9, 56
- **DOYLE, M.P. et al.** *J.Org.Chem.,* 1977, vol. 42, 2426
- **LAURENCE E. BURGESS.** *Synth. Communication,* 1997, vol. 27, 2181-2191
- Protective Groups in Organic Synthesis. John Wiley & Sons, Inc, 1991
- **H. WANG et al.** *Synlett,* 1999, 33-36
- **M. AMEDJKOUH et al.** *Tetrahedron: Asymmetry,* 2002, vol. 13 (20), 2229-2234
- **GIORDANO, C. ; CAVICCHIOLI, S. ; LEVI, S. ; VILLA, M.** *J Org. Chem.,* 1991, vol. 56 (21), 6114-6118
- **KONRADI, A. W. ; PEDERSEN, S. F.** *J Org. Chem.,* 1992, vol. 57 (1), 28-32
- **JURCZAK, J. ; PROKOPOWICZ, P. ; GOLEBIOWSKI, A.** *Tetrahedron Letters,* 1993, vol. 34 (44), 7107-7110
- **DE LUCA, L. ; GIACOMELLI, G. ; PORCHEDDU, A.** *J.Org. Chem.,* 2001, vol. 66 (23), 7907-7909
- **DE LUCA, L. ; GIACOMELLI, G. ; PORCHEDDU, A.** *Org Letters,* 2001, vol. 3 (19), 3041-3043
- **KONRADI, A. W. ; PEDERSEN, S. F.** *J.Org. Chem.,* 1990, vol. 55 (15), 4506-4508
- **TAKEMOTO, Y. ; MATSUMOTO, T. ; ITO, Y. ; TERASHIMA, S.** *Chem.Pharm. Bull.,* 1991, vol. 39 (9), 2425-2428
- **GORDON, D. W. ; STEELE, J.** *Bioorg. Med. Chem. Letters,* 1995, vol. 5 (1), 47-50
- **KELLEY, J. L. ; MCLEAN, E. W. ; FERRIS, R. M. ; HOWARD, J. L.** *Jmed. Chem.,* 1990, vol. 33 (7), 1910-1914
- **K. B. SHARPLESS.** *Organic Letters,* 2002, vol. 4 (15), 2525-2527
- *Bioorganic and Medicinal Chemistry Letters,* 2001, vol. 11 (19), 2593-2596
- *Journal of Pharmacology and Experimental Therapeutics,* 2002, vol. 301 (2), 747-752
- *Int. Arch. Immunol.,* 1995, vol. 108, 11-18